(19) Europäisches Patentamt
European Patent Office
Office européen des brevets

(11) Publication number: **0 520 336 A2**

(12) **EUROPEAN PATENT APPLICATION**

(21) Application number: **92110388.3**

(22) Date of filing: **19.06.92**

(51) Int. Cl.[5]: **C07D 205/04**, C07D 207/16, C07D 211/36, A61K 31/395, A61K 31/40, A61K 31/445, A61K 31/16, C07D 401/06, C07D 401/12, C07D 401/14, C07D 333/16

(30) Priority: **19.06.91 JP 173377/91**
**17.12.91 JP 352877/91**
**26.12.91 JP 357647/91**

(43) Date of publication of application:
**30.12.92 Bulletin 92/53**

(84) Designated Contracting States:
**CH DE FR GB IT LI NL**

(71) Applicant: **FUJIREBIO Inc.**
**7-1, Nishi-shinjuku 2-chome**
**Shinjuku-ku, Tokyo 163(JP)**

(72) Inventor: **Hosoda, Akihiko**
**1-3-25, Shimomuneoka**
**Shiki-shi, Saitame-ken(JP)**
Inventor: **Nakayama, Yukihide**
**239-5, Terayama-cho, Midori-ku**
**Yokohama-shi, Kanagawa-ken(JP)**
Inventor: **Shibata, Masahiro**
**No. 208, Asahisou, 4-13-3, Sakae-cho**
**Tachikawa-shi, Tokyo(JP)**
Inventor: **Sekine, Yasuo**
**2-23-7, Fujizuka, Kohoku-ku**
**Yokohama-shi, Kanagawa-ken(JP)**
Inventor: **Inaba, Niro**
**2-1-5-403, Toyogaoka**
**Tama-shi, Tokyo(JP)**
Inventor: **Ikawa, Hiroshi**
**1-26-10, Miyasaka, Setagaya-ku**
**Tokyo(JP)**
Inventor: **Yamaura, Tetsuaki**
**2-17-8, Niza**
**Niiza-shi, Saitama-ken(JP)**
Inventor: **Tanabe, Naoko**
**1-11-15, Ogawahigashi-cho**
**Kodaira-shi, Tokyo(JP)**

(74) Representative: **Sandmair, Kurt, Dr. Dr.**
**Patentanwälte Schwabe, Sandmair, Marx**
**Stuntzstrasse 16**
**W-8000 München 80(DE)**

(54) **Aldehyde derivatives and their use as calpain inhibitors.**

(57) Aldehyde derivatives with a specific calpain inhibiting activity and a platelet-aggregation inhibiting effect with formula (I) or formula (II):

$$\text{Z-CONH-}\underset{}{\overset{\displaystyle CHO \atop |}{\text{CH}}}\text{-}(CH_2)_n\text{-}R^1 \qquad (I)$$

wherein $R^1$ represents an aromatic hydrocarbon group, a heterocyclic group, or a group of $-X-R^3$ in which X represents O, $-S(O)_m-$ (m = 0, 1, or 2), and $R^3$ represents an aromatic hydrocarbon group, a heterocyclic group, or an alkyl group; Z represents $R^4$-Y- or $R^6$O-CH($R^5$)- in which Y represents a 3- to 7-membered nitrogen-containing saturated heterocyclic group, or a single cyclic saturated hydrocarbon group, $R^4$ represents an alkyl group, an alkenyl group, an alkynyl group, an acyl group, a sulfonyl group, an alkoxycarbonyl group, a

EP 0 520 336 A2

carbamoyl group, or a thiocarbamoyl group, $R^5$ represents hydrogen, an alkyl group, or an aromatic hydrocarbon group, and $R^6$ represents an acyl group, a carbamoyl group, a thiocarbamoyl group, or an alkyl group; and n is an integer of 1 to 5.

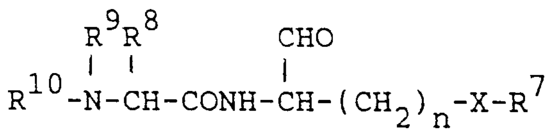

(II)

wherein $R^7$, $R^8$, $R^9$, and $R^{10}$ are defined in the specification.

## BACKGROUND OF THE INVENTION

### Field of the Invention

The present invention relates to aldehyde derivatives which serve as calpain inhibitors including a non-natural amino acid structure, and more particularly to aldehyde derivatives with a specific calpain inhibiting effect (i.e., inhibitory effect on the activity of calpain) and a platelet-aggregation inhibiting effect for increasing the curative effect of ischemic diseases.

### Discussion of Background

Calpain is a proteolytic enzyme (cysteine protease) which can be activated by the calcium ion, and is widely present in the cells of vital tissues. Calpain is known to have physiological roles such as the histodialysis of tissues whose substrates are, for instance, myoprotein, zymoprotein, receptor protein and cytoskeleton protein, the activation of an inactivated enzyme precursor, and intracellular processing as described in Trends in Pharmacological Science Vol. 11, 139(1990).

It is reported that the abnormal acceleration of the enzyme activity of calpain in vivo may cause inveterate diseases such as ischemic diseases, inflammation, progressive muscular dystrophy, cataract, hypoimmunity, and essential hypertension.

It is considered that in ischemic diseases, calpain is sufficiently activated by the increase in the amount of the calcium ion in the cells during ischemia to cause cellular impairment and necrosis.

Conventional remedies for such ischemic diseases are calcium antagoists and $\beta$-blockers which have a vasodilation effect. However these remedies are not effective for curing and/or prophyaxis of the cellular impairment and necrosis in ischemic diseases.

Calpain inhibitors are therefore considered to be useful as remedies for such inveterate diseases. Conventionally known calpain inhibitors are compounds with a peptide structure, such as compounds with a dipeptide structure as disclosed in Japanese Laid-Open Patent Applications 1-121257, 2-268145, and Journal of Medicinal Chemistry 33, 11 (1990), compounds with a tripeptide structure as disclosed in Japanese Laid-Open Patent Applications 58-116616, 60-28990, and 61-103897, compounds with a tetrapeptide structure as disclosed in Japanese Laid-Open Patent Application 58-198453, and compounds with a pentapeptide structure as disclosed in Japanese Laid-Open Patent Application 61-10600.

However, the above-mentioned compounds which have capability of inhibiting the calpain activity do not have satisfactory effects in the treatment of the inveterate diseases. This is because each of the above compounds has a natural amino acid structure therein and can be cleaved by the proteolytic enzyme in vivo.

Furthermore, the following compounds are conventionally known as calpain inhibitors: epoxy succinic acid derivatives (J. Biochem., 87,339(1980)), piperazine derivatives (Japanese Laid-Open Patent Applications 57-169478, 58-126879, and 63-25575), aminoaldehyde derivatives (Japanese Laid-Open Patent Applications 61-103997, 1-12157, and 2-268145), and aminoaldehyde derivatives and amino ketone derivatives (Japanese Laid-Open Patent Application 2-256654). Of these compounds, the piperazine derivatives are reported to have a myocardium protecting effect. It is therefore considered that such piperazine derivatives may probably be used as remedies for ischemic diseases. However, those piperazine derivatives do not have a sufficient myocardium protection effect and a calpain inhibiting effect for use as remedies for ischemic diseases. The compounds other than the piperazine derivatives cannot be used satisfactorily as remedies for ischemic diseases although they have a calpain inhibiting effect.

## SUMMARY OF THE INVENTION

It is therefore an object of the present invention to provide compounds which do not have a natural amino acid structure, but have a specific calpain inhibiting activity and a platelet-aggregation inhibiting effect for increasing the curative effect of ischemic diseases.

This object of the present invention can be achieved by aldehyde derivatives represented by the following formula (I):

$$Z\text{-}CONH\text{-}\underset{}{\overset{\displaystyle CHO}{\overset{|}{C}H}}\text{-}(CH_2)_n\text{-}R^1 \qquad (I)$$

wherein $R^1$ represents an aromatic hydrocarbon group, a heterocyclic group, an alkyl group having 1 to 10 carbon atoms, an alkenyl group having 2 to 10 carbon atoms, or a group of $-X-R^3$ in which X represents O, $-S(O)_m-$ (m = 0, 1, or 2), and $R^3$ represents an aromatic hydrocarbon group, a heterocyclic group, or an alkyl group having 1 to 10 carbon atoms; Z represents $R^4$-Y- or $R^6$O-CH($R^5$)- in which Y represents a 3- to 7-membered nitrogen-containing saturated heterocyclic group, or a monocyclic saturated hydrocarbon group having 3 to 7 carbon atoms, $R^4$ represents an alkyl group having 1 to 10 carbon atoms, an alkenyl group having 2 to 10 carbon atoms, an alkynyl group having 2 to 10 carbon atoms, an acyl group, a sulfonyl group, an alkoxycarbonyl group, a carbamoyl group, or a thiocarbamoyl group, $R^5$ represents hydrogen, an alkyl group having 1 to 10 carbon atoms, or an aromatic hydrocarbon group, and $R^6$ represents an acyl group, a carbamoyl group, a thiocarbamoyl group, or an alkyl group having 1 to 10 carbon atoms; and n is an integer of 1 to 5.

The above object of the present invention can also be achieved by aldehyde derivatives represented by the following formula (II):

$$R^{10}\text{-}\overset{\displaystyle R^9}{\overset{|}{N}}\text{-}\overset{\displaystyle R^8}{\overset{|}{C}}H\text{-}CONH\text{-}\overset{\displaystyle CHO}{\overset{|}{C}}H\text{-}(CH_2)_n\text{-}X\text{-}R^7$$

wherein $R^7$ represents an aromatic hydrocarbon group, a heterocyclic group, an alkyl group having 1 to 10 carbon atoms with a substituent, or a cyclic alkyl group having 3 to 6 carbon atoms; $R^8$ represents hydrogen, an alkyl group having 1 to 10 carbon atoms, or an aromatic hydrocarbon group; $R^9$ represents hydrogen, or an alkyl group having 1 to 10 carbon atoms; $R^{10}$ represents an alkoxycarbonyl group, an acyl group, a carbamoyl group, or a sulfonyl group; X represents oxygen, or a group represented by $-S(O)_m-$ in which m is 0, 1 or 2; and n is an integer of 1 to 5.

## DETAILED DESCRIPTION OF THE PREFERRED EMBODIMENTS

In the aldehyde derivatives of the present invention having the previously mentioned formula (I), $R^1$ represents an aromatic hydrocarbon group, a heterocyclic group, an alkyl group having 1 to 10 carbon atoms, an alkenyl group having 2 to 10 carbon atoms, or a group represented by $-X-R^3$ in which X represents O, $-S(O)_m-$ (m = 0, 1, or 2), and $R^3$ represents an aromatic hydrocarbon group, a heterocyclic group, or an alkyl group having 1 to 10 carbon atoms.

Examples of the aromatic hydrocarbon group represented by $R^1$ are phenyl group, naphthyl group and anthranyl group.

Examples of the heterocyclic group represented by $R^1$ are furyl group, thienyl group, pyrrolyl group, pyridyl group, quinolyl group, isoquinolyl group, and indolyl group.

The alkyl group represented by $R^1$ may be a substituted or unsubstituted straight-chain, branched or cyclic alkyl group having 1 to 10 carbon atoms. Specific examples of the alkyl group represented by $R^1$ are methyl group, ethyl group, propyl group, butyl group, pentyl group, hexyl group, heptyl group, octyl group, nonyl group, decyl group, iso-propyl group, s-butyl group, t-butyl group, iso-pentyl group, neopentyl group, t-pentyl group, iso-hexyl group, cyclopropyl group, cyclobutyl group, cyclopentyl group, and cyclohexyl group. Examples of the substituent of the substituted alkyl group include the previously mentioned aromatic hydrocarbon group and heterocyclic group.

The alkenyl group represented by $R^1$ may be a substituted or unsubstituted straight-chain, branched or cyclic alkenyl group having 2 to 10 carbon atoms. Specific examples of the alkenyl group represented by $R^1$ are ethenyl or vinyl group, 1-propenyl group, 2-propenyl group, iso-propenyl group, 1-butenyl group, 2-butenyl group, 3-butenyl group, 2-methyl-2-propenyl group, 1-pentenyl group, 1-hexenyl group, 1-heptenyl group, 1-cyclohexenyl group and 2-cylcohexenyl group. Examples of the substituent of the substituted alkenyl group are the previously mentioned aromatic hydrocarbon group and heterocyclic group.

Moreover, the aromatic hydrocarbon group and the heterocyclic group represented by $R^1$ in the

previously mentioned formula (I), and the aromatic hydrocarbon group and the heterocyclic group which are the substituents of the alkyl group or the alkenyl group represented by $R^1$ may have a substituent. Specific examples of the substituent are an alkyl group having 1 to 10 carbon atoms such as methyl group, ethyl group, propyl group, butyl group, pentyl group, hexyl group, and heptyl group; an alkoxyl group having 1 to 10 carbon atoms such as methoxy group, ethoxy group, propoxy group, butoxy group, and benzyloxy group; a halogen atom such as fluorine, chlorine, bromine and iodine; an amino group such as amino group, dimethylamino group, and diethylamino group; hydroxyl group; and a nitro group.

$R^1$ in formula (I) may also be a group represented by $-X-R^3$ in which X represents O, $-S(O)_m-$ (m = 0, 1, or 2), and $R^3$ represents an aromatic hydrocarbon group, a heterocyclic group, or an alkyl group having 1 to 10 carbon atoms. Examples of the aromatic hydrocarbon group, the heterocyclic group and the alkyl group represented by $R^3$ are respectively the same as those represented by $R^1$ in formula (I). The alkyl group represented by $R^3$ may have as a substituent the same aromatic hydrocarbon group or heterocyclic group represented by $R^1$.

Z in formula (I) represents $R^4$-Y- in which Y represents a 3 to 7-membered nitrogen-containing saturated heterocyclic group, or a monocyclic saturated hydrocarbon having 3 to 7 carbon atoms, and $R^4$ represents an alkyl group having 1 to 10 carbon atoms, an alkenyl group having 2 to 10 carbon atoms, an alkynyl group having 2 to 10 carbon atoms, which may be a straight-chain alkynyl group or a branched alkynyl group, an acyl group, a sulfonyl group, an alkoxycarbonyl group, a carbamoyl group, or a thiocarbamoyl group.

Examples of the 3- to 7-membered nitrogen-containing saturated heterocyclic ring represented by Y are aziridine, azetidine, pyrrolidine, piperidine, and perhydroazepine.

Examples of the 3- to 7-membered nitrogen-containing saturated heterocyclic group represented by Y are azetidine-1,2-diyl group, pyrrolidine-1,2-diyl group, pyrrolidine-1,3-diyl group, piperidine-1,2-diyl group, piperidine-1,3-diyl group, piperidine-1,4-diyl group, perhydroazepine-1,2-diyl group, perhydroazepine-1,3-diyl group, and perhydroazepine-1,4-diyl group.

Examples of the monocyclic saturated hydrocarbon having 3 to 7 carbon atoms represented by Y are cyclopropane, cyclobutane, cyclopentane, and cycloheptane.

Examples of the corresponding monocyclic saturated hydrocarbon group having 3 to 7 carbon atoms are

1,2-cyclobutylene group, 1,3-cyclobutylene group,
1,2-cyclopentylene group, 1,3-cyclopentylene group,
1,2-cyclohexylene group, 1,3-cyclohexylene group,
1,4-cyclohexylene group, 1,2-cycloheptylene group,
1,3-cycloheptylene group, and 1,4-cycloheptylene group.

The alkyl group represented by $R^4$ is a straight-chain, branched, or cyclic alkyl group having 1 to 10 carbon atoms, which may be the same represented by $R^1$. More specifically, examples of the alkyl group represented by $R^4$ are methyl group, ethyl group, propyl group, butyl group, pentyl group, hexyl group, heptyl group, octyly group, nonyl group, decyl group, iso-propyl group, s-butyl group, t-butyl group, iso-pentyl group, neopentyl group, t-pentyl group, iso-hexyl group, cyclopropyl group, cyclobutyl group, cyclopentyl group, and cyclohexyl group.

The alkyl group represented by $R^4$ may have a substituent such as an aromatic hydrocarbon group or a heterocyclic group, which may be the same substituent as for the alkyl group represented by $R^1$.

The aromatic hydrocarbon group or the heterocyclic group which is the substituent for the above alkyl group may further have a substituent. Specific examples of the substituent are an alkyl group having 1 to 10 carbon atoms such as methyl group, ethyl group, propyl group, butyl group, pentyl group, hexyl group, and heptyl group; an alkoxyl group having 1 to 10 carbon atoms such as methoxy group, ethoxy group, propoxy group, butoxy group, and benzyloxy group; a halogen atom such as fluorine, chlorine, bromine and iodine; an amino group such as amino group, dimethylamino group, and diethylamino group; a hydroxyl group; and a nitro group.

The alkenyl group represented by $R^4$ is a straight-chain, branched or cyclic alkenyl group having 2 to 10 carbon atoms, which may have a substituent and is the same alkenyl group as represented by $R^1$. More specifically, examples of the alkenyl group represented by $R^4$ are vinyl group, 1-propenyl group, 2-propenyl group, iso-propenyl group, 1-butenyl group, 2-butenyl group, 3-butenyl group, 2-methyl-2-propenyl group, 1-pentenyl group, 1-hexenyl group, 1-heptenyl group, 1-cyclohexenyl group and 2-cyclohexenyl group. Examples of the substituent of the alkenyl group represented by $R^4$ are the same aromatic hydrocarbon group or heterocyclic group as represented by $R^1$. The aromatic hydrocarbon group or the heterocyclic group may further have any of the same substituents as in the alkyl group represented by $R^4$.

Examples of the alkynyl group having 2 to 10 carbon atoms represented by $R^4$ are 2-propynyl group, 1-propynyl group, 1-butynyl group, 2-butynyl group, 1-pentynyl group, 1-hexynyl group, 1-heptynyl group, 1-optynyl group, 1-nonyl group, 1-denyl group and 1-methyl-2-propynyl group.

Examples of the acyl group represented by $R^4$ are acetyl group, propionyl group, butyryl group, valeryl group, hexanoyl group, heptanoyl group, iso-valeryl group, cyclohexane carbonyl group, benzoyl group, 1-naphthoyl group, 2-naphthoyl group, toluoyl group, 1-(benzyloxycarbonyl)piperidine-4-carbonyl group, cinnamoyl group, phenylacetyl group, 2-thienylcarbonyl group, trimethyl acetyl group, cyclopentane carbonyl group, 2,6-dichlorobenzoyl group, 3,4-dichlorobenzoyl group, 4-phenylbenzoyl group, 2-chlorocinnamoyl group, 3-chlorocinnamoyl group, 4-chlorocinnamoyl group, 2-nitrocinnamoyl group, indolyl-2-carbonyl group, indolyl-3-carbonyl group, quinolyl-2-carbonyl group, quinolyl-3-carbonyl group, isoquinolyl-3-carbonyl group, diphenyl acetyl group, fluorenyl-9-carbonyl group, 3-phenylpropionyl group, 4-phenyl-butyryl group, 3-(3-pyridyl)acryloyl group, 3-(3-thienyl)acryloyl group, 3-phenyl-2-methylacryloyl group, 3-(2-naphthyl)acryloyl group, (2S)-3-phenyl-2-(benzyloxycarbonylamino)propionyl group, and (2R)-3-phenyl-2-(benzyloxycarbonylamino)propionyl group.

The sulfonyl group represented by $R^4$ may have a substituent such as an alkyl group or an aromatic hydrocarbon group. Specific examples of the sulfonyl group represented by $R^4$ are methane sulfonyl group, ethane sulfonyl group, propane sulfonyl group, butane sulfonyl group, pentane sulfonyl group, hexane sulfonyl group, trifluoromethane sulfonyl group, benzene sulfonyl group, naphthalene sulfonyl group, 4-methyl benzene sulfonyl group, iso-quinoline-5-sulfonyl group, and quinoline-8-sulfonyl group.

The alkoxycarbonyl group represented by $R^4$ is a substituted or unsubstituted, saturated or unsaturated alkoxycarbonyl group. Specific examples of the alkoxycarbonyl group represented by $R^4$ are methoxy carbonyl group, ethoxy carbonyl group, propoxy carbonyl group, butoxy carbonyl group, pentyloxy carbonyl group, hexyloxy carbonyl group, heptyloxy carbonyl group, octyloxy carbonyl group, nonyloxy carbonyl group, decyloxy carbonyl group, iso-propoxy carbonyl group, iso-butoxy carbonyl group, s-butoxy carbonyl group, t-butoxy carbonyl group, iso-pentyloxy carbonyl group, neopentyloxy carbonyl group, t-pentyloxy carbonyl group, iso-hexyloxy carbonyl group, cinnamyloxy carbonyl group, and benzyloxy carbonyl group.

The carbamoyl group represented by $R^4$ may have a substituent, such as the so far mentioned substituted or unsubstituted alkyl group or substituted or unsubstituted aromatic hydrocarbon group. Examples of the carbamoyl group represented by $R^4$ are N-methylcarbamoyl group, N-ethylcarbamoyl group, N-phenylcarbamoyl group, N-(2-chlorophenyl)carbamoyl group, N-(3-chlorophenyl)carbamoyl, N-(4-chlorophenyl)carbamoyl, N-(1-naphthyl)carbamoyl group, and N-benzylcarbamoyl group.

The thiocarbamoyl group represented by $R^4$ may have a substituent. Examples of the substituent are a substituted or unsubstituted alkyl group and a substituted or unsubstituted aromatic hydrocarbon group. Examples of the thiocarbamoyl group represented by $R^4$ are N-methylthiocarbamoyl group, N-ethylthiocarbamoyl group, N-phenylthiocarbamoyl group, N-(2-chlorophenyl)thiocarbamoyl group, N-(1-naphthyl)-thiocarbamoyl group, N-(1-naphthyl)thiocarbamoyl group, and N-benzylthiocarbamoyl group.

Z also represents $R^6O\text{-}CH(R^5)\text{-}$ in which $R^5$ represents hydrogen, an alkyl group having 1 to 10 carbon atoms, or an aromatic hydrocarbon group, and $R^6$ represents an acyl group, a carbamoyl group, a thiocarbamoyl group, or an alkyl group having 1 to 10 carbon atoms.

The alkyl group represented by $R^5$ may be a straight-chain, branched or cyclic alkyl group having 1 to 10 carbon atoms. Specific examples of the alkyl group are methyl group, ethyl group, propyl group, butyl group, pentyl group, hexyl group, heptyl group, octyl group, nonyl group, decyl group, iso-propyl group, iso-butyl group, s-butyl group, t-butyl group, iso-pentyl group, neopentyl group, t-pentyl group, iso-hexyl group, cyclopropyl group, cyclobutyl group, cyclopentyl group, and cyclohexyl group.

Examples of the aromatic hydrocarbon group represented by $R^5$ are phenyl group, naphthyl group and anthranyl group, which are the same as those represented by $R^1$ in formula (I).

The alkyl group represented by $R^5$ may have a substituent. Examples of the substituent are the aromatic-hydrocarbon group and heterocyclic group represented by $R^1$ in formula (I).

Examples of the acyl group represented by $R^6$ are acetyl group, propionyl group, butyryl group, valeryl group, hexanoyl group, heptanoyl group, iso-valeryl group, cylcohexane carbonyl group, benzoyl group, 1-naphthoyl group, 2-naphthoyl group, toluoyl group, and 1-(benzyloxycarbonyl)piperidine-4-carbonyl group.

The carbamoyl group represented by $R^6$ may have a substituent. Examples of the carbamoyl group are N-methylcarbamoyl group, N-ethylcarbamoyl group, N-phenylcarbamoyl group, N-(2-chlorophenyl)-carbamoyl group, N-(1-naphthyl)carbamoyl group and N-benzylcarbamoyl group.

The thiocarbamoyl group represented by $R^6$ may have a substituent. Examples of the thiocarbamoyl group are N-methylthiocarbamoyl group, N-ethylthiocarbamoyl group, N-phenylthiocarbamoyl group, N-(2-chlorophenyl)thiocarbamoyl group, N-(2-naphthyl)thiocarbamoyl group and N-benzylthiocarbamoyl group.

The alkyl group represented by $R^6$ is the same as the alkyl group represented by $R^5$ and may have a substituent. Specifically, examples of the alkyl group represented by $R^6$ are methyl group, ethyl group,

propyl group, butyl group, pentyl group, hexyl group, heptyl group, octyl group, nonyl group, decyl group, iso-propyl group, iso-butyl group, s-butyl group, t-butyl group, iso-pentyl group, neopentyl group, t-pentyl group, iso-hexyl group, cyclopropyl group, cyclobutyl group, cyclopentyl group, and cyclohexyl group. Examples of the substituent of the alkyl group are the aromatic hydrocarbon group and heterocyclic group represented by $R^1$ in formula (I).

The object of the present invention can also be achieved by aldehyde derivatives represented by the following formula (II):

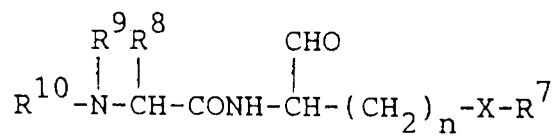

(II)

wherein $R^7$ represents an aromatic hydrocarbon group, a heterocyclic group, an alkyl group having 1 to 10 carbon atoms, which has a substituent such as an aromatic hydrocarbon group, and a heterocyclic group, or a cyclic alkyl group having 3 to 6 carbon atoms; $R^8$ represents hydrogen, an alkyl group having 1 to 10 carbon atoms, or an aromatic hydrocarbon group; $R^9$ represents hydrogen, or an alkyl group having 1 to 10 carbon atoms; $R^{10}$ represents an alkoxycarbonyl group, an acyl group, a carbamoyl group, or a sulfonyl group; X represents oxygen, or a group represented by $-S(O)_m-$ in which m is 0, 1 or 2; and n is an integer of 1 to 5.

Examples of the aromatic hydrocarbon group represented by $R^7$ are phenyl group, naphthyl group and anthranyl group.

Examples of the heterocyclic group represented by $R^7$ are furyl group, thienyl group, pyrrolyl group, pyridyl group, quinolyl group, isoquinolyl group, and indolyl group.

The substituted alkyl group represented by $R^7$ is a substituted straight-chain or branched alkyl group having 1 to 10 carbon atoms. Specific examples of the alkyl group represented by $R^7$ are methyl group, ethyl group, propyl group, butyl group, pentyl group, hexyl group, heptyl group, octyl group, nonyl group, decyl group, iso-propyl group, iso-butyl group, s-butyl group, t-butyl group, iso-pentyl group, neopentyl group, t-pentyl group, and iso-hexyl group.

Examples of the cyclic alkyl group having 3 to 6 carbon atoms represented by $R^7$ are cyclopropyl group, cyclobutyl group, cyclopentyl group, and cyclohexyl group.

Moreover, the aromatic hydrocarbon group and the heterocyclic group represented by R in formula (II), and the aromatic hydrocarbon group and the heterocyclic group which are the substituents of the alkyl group represented by $R^7$ may have a substituent. Specific examples of the substituent are an alkyl group having 1 to 10 carbon atoms such as methyl group, ethyl group, propyl group, butyl group, pentyl group, hexyl group, heptyl group, octyl group, nonyl group, decyl group, iso-propyl group, iso-butyl group, s-butyl group, t-butyl group, iso-pentyl group, neopentyl group, t-pentyl group, and iso-hexyl group; an alkoxyl group having 1 to 10 carbon atoms such as methoxy group, ethoxy group, propoxy group, butoxy group, pentyloxy group, hexyloxy group, butyloxy group, octyloxy group, nonyloxy group, decyloxy group, iso-propoxy group, iso-butoxy group, s-butoxy group, t-butoxy group, iso-pentyloxy group, neopentyloxy group, t-pentyloxy group and iso-hexyloxy group, and benzyloxy group; a halogen atom such as fluorine, chlorine, bromine and iodine; an amino group such as amino group, dimethylamino group, and diethylamino group; hydroxyl group; and a nitro group.

$R^8$ represents hydrogen, an alkyl group having 1 to 10 carbon atoms, or an aromatic hydrocarbon group.

The alkyl group represented by $R^8$ may be a straight-chain or branched alkyl group having 1 to 10 carbon atoms. Specific examples of the alkyl group are methyl group, ethyl group, propyl group, butyl group, pentyl group, hexyl group, heptyl group, octyl group, nonyl group, decyl group, iso-propyl group, iso-butyl group, s-butyl group, t-butyl group, iso-pentyl group, neopentyl group, t-pentyl group, and iso-hexyl group. The alkyl group represented by $R^8$ may have a substituent such as the aromatic hydrocarbon group and heterocyclic group represented by $R^7$.

Examples of the aromatic hydrocarbon group represented by $R^8$ are the same as those represented by $R^7$ in formula (II). Specifically, examples of the aromatic hydrocarbon group represented by $R^8$ are phenyl group, naphthyl group and anthranyl group.

$R^9$ represents hydrogen or an alkyl group having 1 to 10 carbon atoms. The alkyl group represented by $R^9$ may be the same as represented by $R^8$. More specifically, the alkyl group represented by $R^9$ may be a straight-chain or branched alkyl group having 1 to 10 carbon atoms. Specific examples of the alkyl group

are methyl group, ethyl group, propyl group, butyl group, pentyl group, hexyl group, heptyl group, octyl group, nonyl group, decyl group, iso-propyl group, iso-butyl group, s-butyl group, t-butyl group, iso-pentyl group, neopentyl group, t-pentyl group, and iso-hexyl group. The alkyl group represented by $R^9$ may have a substituent such as the aromatic hydrocarbon group and heterocyclic group represented by $R^7$.

As mentioned above, $R^{10}$ represents an alkoxy carbonyl group, an acyl group, a carbamoyl group or a sulfonyl group.

Specific examples of the alkoxy carbonyl group represented by $R^{10}$ are methoxy carbonyl group, ethoxy carbonyl group, propoxy carbonyl group, butoxy carbonyl group, pentyloxy carbonyl group, hexyloxy carbonyl group, heptyloxy carbonyl group, octyloxy carbonyl group, nonyloxy carbonyl group, decyloxy carbonyl group, iso-propoxy carbonyl group, iso-butoxy carbonyl group, s-butoxy carbonyl group, t-butoxy carbonyl group, iso-pentyloxy carbonyl group, neopentyloxy carbonyl group, t-pentyloxy carbonyl group, iso-hexyloxy carbonyl group, cinnamyloxy carbonyl group, and benzyloxy carbonyl group.

Examples of the acyl group represented by $R^{10}$ are acetyl group, propionyl group, butyryl group, valeryl group, hexanoyl group, heptanoyl group, iso-valeryl group, cyclohexane carbonyl group, benzoyl group, 1-naphtholyl group, 2-naphthoyl group, toluoyl group, and 1-(benzyloxycarbonyl)piperidine-4-carbonyl group.

The carbamoyl group represented by $R^{10}$ may have a substituent. Examples of the carbamoyl group are N-methylcarbamoyl group, N-ethylcarbamoyl group, N-phenylcarbamoyl group, N-(2-chlorophenyl)-carbamoyl group, N-(1-naphthyl)carbamoyl group and N-benzylcarbamoyl group.

Examples of the sulfonyl group represented by $R^{10}$ are methane sulfonyl group, ethane sulfonyl group, propane sulfonyl group, butane sulfonyl group, pentane sulfonyl group, hexane sulfonyl group, trifluoromethane sulfonyl group, benznenesulfonyl group, 4-methylbenzene sulfonyl group, isoquinoline-5-sulfonyl group, and quinoline-8-sulfonyl group.

Aldehyde derivatives (I) of the present invention, which are represented by the previously mentioned formula (I) in which Z is $R^4$-Y-, more specifically represented by the following formula (IA), can be prepared in accordance with the following reaction scheme:

$$R^4\text{-Y-CONH-}\underset{}{\overset{\text{CHO}}{\overset{|}{\text{CH}}}}\text{-(CH}_2)_n\text{-}R^1 \qquad \text{(IA)}$$

wherein $R^1$ represents an aromatic hydrocarbon group, a heterocyclic group, an alkyl group having 1 to 10 carbon atoms, an alkenyl group having 2 to 10 carbon atoms, or a group of -X-$R^3$ in which X represents O, -$S(O)_m$- (m = 0, 1, or 2), and $R^3$ represents an aromatic hydrocarbon group, a heterocyclic group, or an alkyl group having 1 to 10 carbon atoms; $R^4$ represents an alkyl group having 1 to 10 carbon atoms, an alkenyl group having 2 to 10 carbon atoms, an alkynyl group having 2 to 10 carbon atoms, an acyl group, a sulfonyl group, an alkoxycarbonyl group, a carbamoyl group, or a thiocarbamoyl group; and n is an integer of 1 to 5.

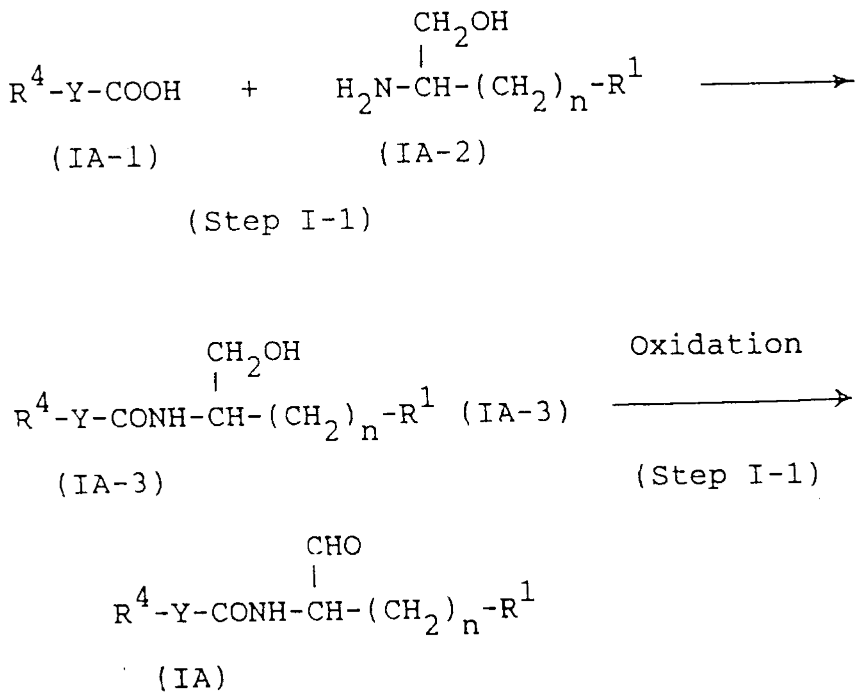


(IA-1) (IA-2) (IA-3) (IA-3) (IA)

wherein $R^1$, $R^4$, Y, and n are respectively the same as in formula (I).

**[Step I-1]**

In this step, a carboxylic acid derivative of formula (IA-1) is allowed to react with an amine derivative of formula (IA-2), so that an alcohol derivative of formula (IA-3) is produced.

Specific examples of the carboxylic acid derivative of formula (IA-1) are as follows:
1-benzyl-piperidine-4-carboxylic acid,
1-(2-naphthylmethyl)piperidine-4-carboxylic acid,
1-(3,4-dichlorobenzyl)piperidine-4-carboxylic acid,
1-cinnamylpiperidine-4-carboxylic acid,
1-benzoylpiperidine-4-carboxylic acid,
1-phenylacetylpiperidine-4-carboxylic acid,
1-(3-phenylpropionyl)piperidine-3-carboxylic acid,
1-(3-phenylpropionyl)piperidine-4-carboxylic acid,
1-(4-phenylbutyryl)piperidine-4-carboxylic acid,
1-cinnamoylpyrrolidine-2-carboxylic acid,
1-cinnamoylpyrrolidine-3-carboxylic acid,
1-cinnamoylpyrrolidine-4-carboxylic acid,
1-(1-naphthoyl)piperidine-3-carboxylic acid,
1-(2-naphthoyl)piperidine-3-carboxylic acid,
1-(1-naphthoyl)piperidine-4-carboxylic acid,
1-(2-naphthoyl)piperidine-4-carboxylic acid,
1-(2-naphthoyl)pyrrolidine-2-carboxylic acid,
1-(2-naphthoyl)azetidine-2-carboxylic acid,
1-(2-naphthoyl)perhydroazepine-3-carboxylic acid,
1-(2-naphthoyl)perhydroazepine-4-carboxylic acid,
1-(2-thienylcarbonyl)piperidine-4-carboxylic acid,
1-(3-pyridylcarbonyl)piperidine-3-carboxylic acid,
1-acetylpiperidine-4-carboxylic acid,
1-trimethylacetylpiperidine-4-carboxylic acid,
1-diphenylacetylpiperidine-4-carboxylic acid,
1-(9-fluorenylcarbonyl)piperidine-4-carboxylic acid,
1-(2,6-dichlorobenzoyl)piperidine-4-carboxylic acid,

1-(3,4-dichlorobenzoyl)piperidine-3-carboxylic acid,
1-(3,4-dichlorobenzoyl)piperidine-4-carboxylic acid,
1-(2-chlorocinnamoyl)piperidine-2-carboxylic acid,
1-(2-chlorocinnamoyl)piperidine-3-carboxylic acid,
1-(2-chlorocinnamoyl)piperidine-4-carboxylic acid,
1-(2-chlorocinnamoyl)pyrrolidine-3-carboxylic acid,
1-(3-chlorocinnamoyl)piperidine-4-carboxylic acid,
1-(4-chlorocinnamoyl)piperidine-3-carboxylic acid,
1-(4-chlorocinnamoyl)piperidine-4-carboxylic acid,
1-(2-chlorocinnamoyl)perhydroazepine-3-carboxylic acid,
1-(2-chlorocinnamoyl)perhydroazepine-4-carboxylic acid,
1-(cyclopentylcarbonyl)azetidine-3-carboxylic acid,
1-(cyclopentylcarbonyl)piperidine-4-carboxylic acid,
1-(cyclohexylcarbonyl)piperidine-4-carboxylic acid,
1-(methylsulfonyl)azetidine-3-carboxylic acid,
1-(ethylsulfonyl)pyrrolidine-2-carboxylic acid,
1-(trifluoromethylsulfonyl)piperidine-4-carboxylic acid,
1-(4-methylphenylsulfonyl)piperidine-3-carboxylic acid,
1-(4-methylphenylsulfonyl)piperidine-4-carboxylic acid,
1-(1-naphthylsulfonyl)pyrrolidine-3-carboxylic acid,
1-(2-naphthylsulfonyl)pyrrolidine-3-carboxylic acid,
1-(2-naphthylsulfonyl)piperidine-3-carboxylic acid,
1-(2-naphthylsulfonyl)piperidine-4-carboxylic acid,
1-(2-naphthylsulfonyl)perhydroazepine-3-carboxylic acid,
1-(2-naphthylsulfonyl)perhydroazepine-4-carboxylic acid,
1-benzyloxycarbonylazetidine-2-carboxylic acid,
1-benzyloxycarbonylpyrrolidine-2-carboxylic acid,
1-benzyloxycarbonylpiperidine-2-carboxylic acid,
1-benzyloxycarbonylpiperidine-3-carboxylic acid,
1-benzyloxycarbonylpiperidine-4-carboxylic acid,
1-ethoxycarbonylpiperidine-4-carboxylic acid,
1-(t-butoxycarbonyl)piperidine-3-carboxylic acid,
1-cinnamyloxycarbonylpiperidine-3-carboxylic acid,
1-cinnamyloxycarbonylpiperidine-4-carboxylic acid,
1-cinnamyloxycarbonylpyrrolidine-3-carboxylic acid,
1-(N-benzylcarbamoyl)piperidine-3-carboxylic acid,
1-(N-benzylcarbamoyl)piperidine-4-carboxylic acid,
1-(N-phenylcarbamoyl)pyrrolidine-3-carboxylic acid,
1-(N-phenylcarbamoyl)piperidine-2-carboxylic acid,
1-(N-phenylcarbamoyl)piperidine-3-carboxylic acid,
1-(N-phenylcarbamoyl)piperidine-4-carboxylic acid,
1-[N-(2-chlorophenyl)carbamoyl]piperidine-4-carboxylic acid,
1-[N-(3-chlorophenyl)carbamoyl]piperidine-4-carboxylic acid,
1-[N-(4-chlorophenyl)carbamoyl]piperidine-4-carboxylic acid,
1-[N-(2-naphthyl)carbamoyl]piperidine-3-carboxylic acid,
1-[N-(2-naphthyl)carbamoyl]piperidine-4-carboxylic acid,
1-[N-(2-naphthyl)carbamoyl]perhydroazepine-3-carboxylic acid,
1-[N-(2-naphthyl)carbamoyl]perhydroazepine-4-carboxylic acid,
1-(N-phenylthiocarbamoyl)piperidine-3-carboxylic acid,
1-(N-phenylthiocarbamoyl)piperidine-4-carboxylic acid,
1-[N-(2-naphthyl)thiocarbamoyl]piperidine-3-carboxylic acid, and
1-[N-(2-naphthyl)thiocarbamoyl]piperidine-4-carboxylic acid.

The amine derivatives of formula (IA-2) are commercially available. However, they can be easily produced from the corresponding amino acids.

Specific examples of the amine derivative of formula (IA-2) are as follows:

(2S)-2-aminobutanol,
(2S)-2-amino-3-methylbutanol,
(2S)-2-aminopentanol,

(2S)-2-amino-4-methylpentanol,
(2S)-2-aminohexanol,
(2S)-2-aminoheptanol,
(2S)-2-amino-3-phenylpropanol,
(2S)-2-amino-4-phenylbutanol,
(2S)-2-amino-5-phenylpentanol,
(2S)-2-amino-6-phenylhexanol,
(2S)-2-amino-7-phenylheptanol,
(2S)-2-amino-3-(2-fluorophenyl)propanol,
(2S)-2-amino-3-(4-hydroxyphenyl)propanol,
(2S)-2-amino-3-(4-benzyloxyphenyl)propanol,
(2S)-2-amino-3-(3-indolyl)propanol,
(2R)-2-amino-3-benzyloxypropanol,
(2R)-2-amino-3-benzylthiopropanol,
(2R)-2-amino-3-(2-fluorobenzylthio)propanol,
(2R)-2-amino-3-(4-chlorobenzylthio)propanol,
(2R)-2-amino-3-methylthiopropanol,
(2R)-2-amino-3-ethylthiopropanol,
(2S)-2-amino-4-phenyloxybutanol,
(2S)-2-amino-4-(3-chlorophenyloxy)butanol,
(2S)-2-amino-4-(4-chlorophenyloxy)butanol,
(2S)-2-amino-4-benzyloxybutanol,
(2S)-2-amino-4-ethoxybutanol,
(2S)-2-amino-4-methylthiobutanol,
(2S)-2-amino-4-phenylthiobutanol,
(2S)-2-amino-4-(4-chlorophenylthio)butanol,
(2S)-2-amino-4-benzylthiobutanol,
(2S)-2-amino-4-(2-chlorobenzylthio)butanol,
(2S)-2-amino-4-(4-chlorobenzylthio)butanol,
(2S)-2-amino-4-(2-fluorobenzylthio)butanol,
(2S)-2-amino-4-(1-thienylmethylthio)butanol,
(2S)-2-amino-4-(2-thienylmethylthio)butanol,
(2S)-2-amino-5-phenyloxypentanol,
(2S)-2-amino-5-(4-chlorophenyloxy)pentanol,
(2S)-2-amino-5-benzyloxypentanol,
(2S)-2-amino-5-ethoxypentanol,
(2S)-2-amino-5-methylthiopentanol,
(2S)-2-amino-5-phenylthiopentanol,
(2S)-2-amino-5-(4-chlorophenylthio)pentanol,
(2S)-2-amino-5-benzylthiopentanol,
(2S)-2-amino-5-(2-chlorobenzylthio)pentanol,
(2S)-2-amino-5-(4-chlorobenzylthio)pentanol,
(2S)-2-amino-5-(2-fluorobenzylthio)pentanol,
(2S)-2-amino-5-(1-thienylmethylthio)pentanol,
(2S)-2-amino-5-(2-thienylmethylthio)pentanol,
(2S)-2-amino-5-phenyl-4-pentene-1-ol,
(2S)-2-amino-5-(2-chlorophenyl)-4-pentane-1-ol,
(2S)-2-amino-4-phenyl-3-butene-1-ol, and
(2S)-2-amino-6-phenyl-5-hexene-1-ol.

The ester derivative with the following formula (IA-2a) can be used as the material in Step I-1 instead of the amino derivative of formula (IA-2):

$H_2N\text{-}CH(COOR)\text{-}(CH_2)_n\text{-}R^1$ (IA-2a)

In the case where the ester derivative of formula (IA-2a) is used in Step I-1, the product obtained contains an ester group therein. Therefore, it is necessary to reduce the ester group in order to convert the ester derivative of formula (IA-2a) into the alcohol derivative of formula (IA-3).

It is preferable that the reaction in Step I-1 be carried out in the presence of a condensation agent. As

the condensation agent, for instance, carbodiimide reagents such as dicyclohexylcarbodiimide (DCC) and 1-ethyl-3-(3-dimethylaminopropyl)-carbodiimide hydrochloride (WSC•HCl) can be employed. It is also preferable that the above-mentioned condensation agent be used in an amount of 1 to 3 equivalents, more preferably in an amount of 1.5 to 2 equivalents, with respect to the carboxylic acid derivative of formula (IA-1) or the amine derivative of formula (IA-2). in order to carry out the reaction efficiently.

Furthermore, it is preferable that the above reaction in Step A-1 be carried out in an inert solvent. Examples of the inert solvent are halogenated hydrocarbons such as dichloromethane, chloroform, and dichloroethane; aromatic hydrocarbons such as benzene, toluene, and xylene; ethers such as diethyl ether, dimethoxyethane, tetrahydrofuran (THF), and dioxane; amides such as dimethylformamide; dimethyl sulfoxide (DMSO); and acetonitrile. These inert solvents can be used alone or in combination.

The reaction proceeds in the temperature range of -50 °C to the reflux temperature under atmospheric pressure. However, it is preferable that the reaction temperature be set in the range of -30 °C to 30 °C in order to carry out the reaction efficiently.

The carboxyl group in the carboxylic acid derivative of formula (IA-1) can be converted to, for example, an active ester group, a carboxylic acid halide group, or an acid anhydride group for use in the above reaction in Step I-1.

**[Step I-2]**

In this step, the alcohol derivative of formula (IA-3) obtained in the reaction of Step I-1 is oxidized, so that an aldehyde derivative of formula (IA) is produced.

When $R^4$ in the formula (IA-3) is an alkoxycarbonyl group or a substituted carbamoyl group, the alcohol derivative of formula (IA-3) can be produced by the reaction in Step A-1, as mentioned previously. However, it is also possible to produce the alcohol derivative of formula (IA-3) by allowing a compound having formula (IA-1b) to react with the amine derivative of formula (IA-2), and allowing the resultant compound to react with a corresponding alcohol or amine having the desired group:

HOOC-Y-COOH (IA-1b)

wherein Y is the same as in formula (I).

An active dimethyl sulfoxide (DMSO) oxidization method is employed for the oxidization in Step I-2. DMSO is an oxidizing agent and can be used in combination with an activating agent such as dicyclohexylcarbodiimide, phosphorus pentoxide, pyridine•sulfur trioxide complex, oxalyl chloride, acetic anhydride, and trifluoroacetic anhydride. It is preferable that the oxidization agent be employed in an amount of 1 to 4 equivalents to the alcohol derivative of formula (IA-3).

It is preferable that the above reaction in Step B-1 be carried out in a solvent. As the solvent, halogenated hydrocarbons such as dichloromethane, dichloroethane, and chloroform can be employed. Alternatively, DMSO employed as the oxidization agent can be used as the solvent for this reaction by using the same in an excessive amount. The above reaction in Step B-1 can be carried out at -20 °C to 30 °C.

Aldehyde derivatives (II) of the present invention, which are represented by the previously mentioned formula (I) in which Z is $R^6O\text{-}CH(R^5)$, more specifically represented by the following formula (IB), can be prepared in accordance with the following reaction scheme:

$$R^6O\text{-}CH(R^5)\text{-}CONH\text{-}\underset{}{\overset{\displaystyle CHO}{\overset{|}{C}H}}\text{-}(CH_2)_n\text{-}R^1 \qquad (IB)$$

wherein $R^1$ represents an aromatic hydrocarbon group, a heterocyclic group, an alkyl group having 1 to 10 carbon atoms, an alkenyl group having 2 to 10 carbon atoms, or a group of $-X-R^3$ in which X represents O, $-S(O)_m-$ (m = 0, 1, or 2), and $R^3$ represents an aromatic hydrocarbon group, a heterocyclic group, or an alkyl group having 1 to 10 carbon atoms; $R^5$ represents hydrogen, an alkyl group having 1 to 10 carbon atoms, or an aromatic hydrocarbon group, and $R^6$ represents an acyl group, a carbamoyl group, a thiocarbamoyl group, or an alkyl group having 1 to 10 carbon atoms; and n is an integer of 1 to 5.

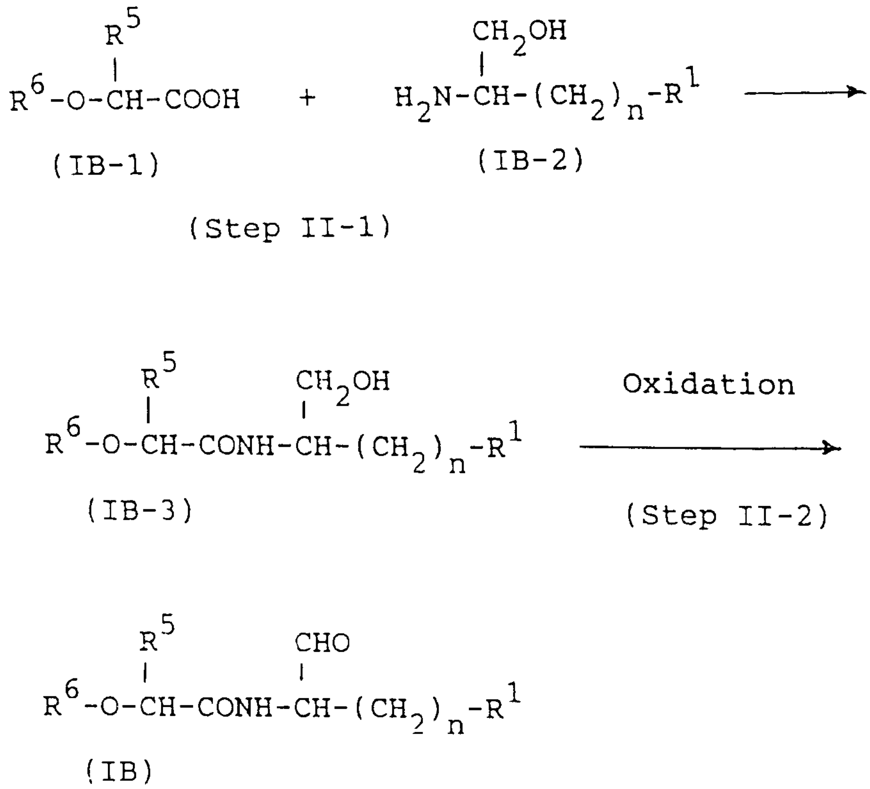


wherein $R^5$, $R^6$, and n are respectively the same as in formula (I).

**[Step II-1]**

In this step, a carboxylic acid derivative of formula (IB-1) is allowed to react with an amine derivative of formula (IB-2), which is the same as the amine derivative of formula (IA-2) employed in the preparation of aldehyde derivatives (I), whereby an alcohol derivative of formula (IB-3) is produced.

The carboxylic acid derivative of formula (IB-1) can be easily synthesized from the following amino compound:

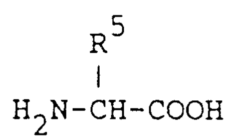

Specific examples of the carboxylic acid derivative of formula (IB-1) are as follows:

(2S)-2-(3-phenylpropyloxy)-4-methylpentanoic acid,
(2S)-2-(N-phenylcarbamoyloxy)-4-methylpentanoic acid,
(2S)-2-(N-phenylthiocarbamoyloxy)-4-methylpentanoic acid,
(2S)-2-[N-(2-chlorophenyl)carbamoyloxy]-4-methylpentanoic acid,
(2S)-2-[N-(1-naphthyl)carbamoyloxy]-4-methylpentanoic acid,
(2S)-2-[N-(2-naphthyl)carbamoyloxy]-4-methylpentanoic acid,
(2S)-2-(N-t-butylcarbamoyloxy)-4-methylpentanoic acid,
(2S)-2-(N-benzylcarbamoyloxy)-4-methylpentanoic acid,
(2S)-2-[(3-phenylpropyl)carbonyloxy]-4-methylpentanoic acid,
(2S)-2-[N-(3,4-dichlorophenyl)carbamoyloxy]-4-methylpentanoic acid,
(2S)-2-[N-(3-chlorophenyl)carbamoyloxy]-4-methylpentanoic acid,
(2S)-2-[N-(4-chlorophenyl)carbamoyloxy]-4-methylpentanoic acid,
(2S)-2-(N-n-propylcarbamoyloxy)-4-methylpentanoic acid,
(2S)-2-(4-phenylbutyloxy)-3-methylbutanoic acid,

(2S)-2-(N-phenylcarbamoyloxy)-3-methyl-butanoic acid,
(2S)-2-[N-(1-naphthyl)carbamoyloxy]-3-methylbutanoic acid,
(2S)-2-[N-(2-naphthyl)carbamoyloxy]-3-methylbutanoic acid,
(2S)-2-[N-(1-naphthyl)carbamoyloxy]-3-phenylpropanoic acid,
(2S)-2-[N-(2-naphthyl)carbamoyloxy]-3-phenylpropanoic acid,
(2S,3S)-2-(N-phenylcarbamoyloxy)-3-methylpentanoic acid,
(2S,3R)-2-(N-phenylcarbamoyloxy)-3-methylpentanoic acid,
(2S,3S)-2-[N-(1-naphthyl)carbamoyloxy)-3-methylpentanoic acid, and
(2S,3R)-2-[N-(1-naphthyl)carbamoyloxy]-3-methylpentanoic acid.

The amine derivatives of formula (IB-2) are commercially available. However, they can be easily produced from the corresponding amino acids.

It is preferable that the reaction in Step II-1 be carried out in the presence of a condensation agent. As the condensation agent, for instance, carbodiimide reagents such as dicyclohexylcarbodiimide (DCC) and 1-ethyl-3-(3-dimethylaminopropyl)-carbodiimide hydrochloride (WSC•HCl) can be employed. It is also preferable that the above-mentioned condensation agent be used in an amount of 1 to 3 equivalents, more preferably in an amount of 1.5 to 2 equivalents, with respect to the carboxylic acid derivative of formula (IB-1) or the amine derivative of formula (IB-2). in order to carry out the reaction efficiently.

Furthermore, it is preferable that the above reaction in Step II-1 be carried out in an inert solvent. Examples of the inert solvent are halogenated hydrocarbons such as dichloromethane, chloroform, and dichloroethane; aromatic hydrocarbons such as benzene, toluene, and xylene; ethers such as diethyl ether, dimethoxyethane, tetrahydrofuran (THF), and dioxane; amides such as dimethylformamide; dimethyl sulfoxide (DMSO); and acetonitrile. These inert solvents can be used alone or in combination.

The reaction proceeds in the temperature range of -50 °C to the reflux temperature under atmospheric pressure. However, it is preferable that the reaction temperature be set in the range of -30 °C to 30 °C in order to carry out the reaction efficiently.

The carboxyl group in the carboxylic acid derivative of formula (IB-1) can be converted to, for example, an active ester group, a carboxylic acid halide group, or an acid anhydride group for use in the above reaction in Step II-1.

**[Step II-2]**

In this step, the alcohol derivative of formula (IB-3) obtained in the reaction of Step II-1 is oxidized, so that an aldehyde derivative of formula (IB) is produced.

An active dimethyl sulfoxide (DMSO) oxidization method is employed for the oxidization in Step ii-2. DMSO is an oxidizing agent and can be used in combination with an activating agent such as dicyclohexylcarbodiimide, phosphorus pentoxide, pyridine•sulfur trioxide complex, oxalyl chloride, acetic anhydride, and trifluoroacetic anhydride. It is preferable that the oxidization agent be employed in an amount of 1 to 4 equivalents to the alcohol derivative of formula (IB-3).

It is preferable that the above reaction in Step II-2 be carried out in a solvent. As the solvent, halogenated hydrocarbons such as dichloromethane, dichloroethane, and chloroform can be employed. Alternatively, DMSO employed as the oxidization agent can be used as the solvent for this reaction by using the same in an excessive amount. The above reaction in Step II-2 can be carried out at -20 °C to 30 °C.

Aldehyde derivatives (III) of the present invention, which are represented by the previously mentioned formula (II) can be prepared in accordance with the following reaction scheme:

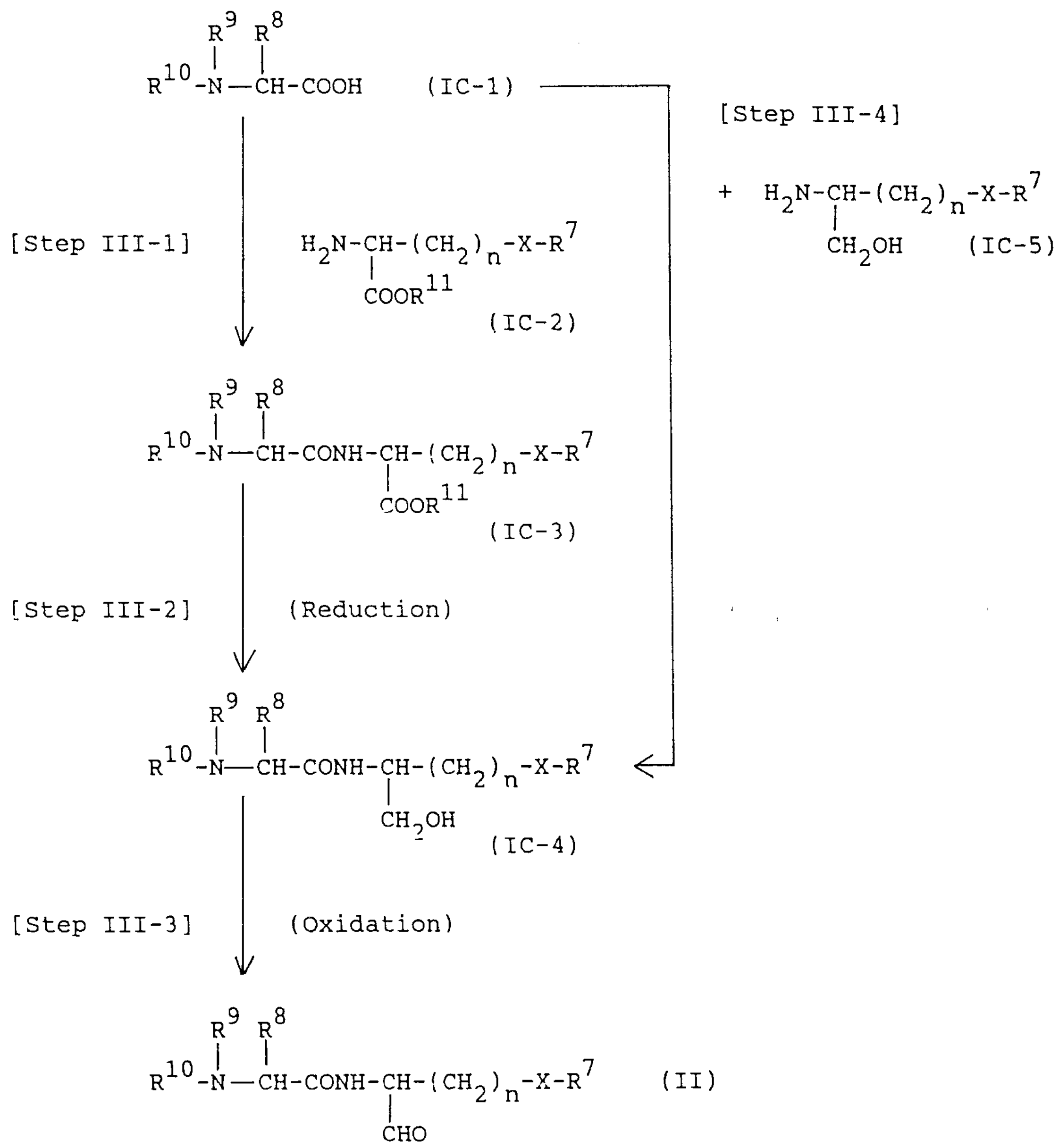


wherein $R^7$ represents an aromatic hydrocarbon group, a heterocyclic group, an alkyl group having 1 to 10 carbon atoms with a substituent, or a cyclic alkyl group having 3 to 6 carbon atoms; $R^8$ represents hydrogen, an alkyl group having 1 to 10 carbon atoms, or an aromatic hydrocarbon group; $R^9$ represents hydrogen, or an alkyl group having 1 to 10 carbon atoms; $R^{10}$ represents an alkoxycarbonyl group, an acyl group, a carbamoyl group, or a sulfonyl group; X represents oxygen, or a group represented by $-S(O)_m-$ in which m is 0, 1 or 2; and n is an integer of 1 to 5.

**[Step III-1]**

In this step, a carboxylic acid derivative of formula (IC-1) is allowed to react with an amine derivative of formula (IC-2), whereby an ester derivative of formula (IC-3) is produced. In formula (IC-3), $R^{11}$ represents an alkyl group having 1 to 15 carbon atoms, such as methyl group, ethyl group, propyl group, butyl group, benzyl group and diphenylmethyl group

It is preferable that the reaction in Step III-1 be carried out in the presence of a condensation agent. As the condensation agent, for instance, carbodiimide reagents such as dicyclohexylcarbodiimide (DCC) and 1-ethyl-3-(3-dimethylaminopropyl)-carbodiimide hydrochloride (WSC•HCl) can be employed. It is also preferable that the above-mentioned condensation agent be used in an amount of 1 to 3 equivalents, more preferably in an amount of 1.5 to 2 equivalents, with respect to the carboxylic acid derivative of formula (IC-1) or the amine derivative of formula (IC-2) in order to carry out the reaction efficiently.

Furthermore, it is preferable that the above reaction in Step III-1 be carried out in an inert solvent. Examples of the inert solvent are halogenated hydrocarbons such as dichloromethane, chloroform, and dichloroethane; aromatic hydrocarbons such as benzene, toluene, and xylene; ethers such as diethyl ether, dimethoxyethane, tetrahydrofuran (THF), and dioxane; amides such as dimethylformamide; dimethyl sulfoxide (DMSO); and acetonitrile. These inert solvents can be used alone or in combination.

The reaction proceeds in the temperature range of -50 °C to the reflux temperature under atmospheric pressure. However, it is preferable that the reaction temperature be set in the range of -30 °C to 30 °C in order to carry out the reaction efficiently.

The carboxylic acid derivatives of formula (IC-1) employed in Step III-1 are commercially available or can by obtained by converting the amino groups of commercially available amino acids to the groups of $R^9$ and $R^{10}$ in formula (IC-2).

Specific examples of the carboxylic acid derivative of formula (IC-1) are as follows:

L-N-(benzyloxycarbonyl)leucine,
L-N-[1-(benzyloxycarbonyl)piperidine-4-carbonyl]leucine,
L-N-(N-phenylaminocarbonyl)leucine,
L-N-(4-methylbenzenesulfonyl)leucine,
L-N-methyl-N-(benzyloxycarbonyl)leucine,
L-N-(cinnamoyl)leucine,
L-N-(2-naphthoyl)leucine,
L-N-(benzyloxycarbonyl)valine, and
L-N-(benzyloxycarbonyl)phenylalanine.

The amine derivative of formula (IC-2) employed in Step III-2 is not only commercially available, but also can be synthesized from an amino acid represented by formula (IC-2a) in accordance with the following procedure:

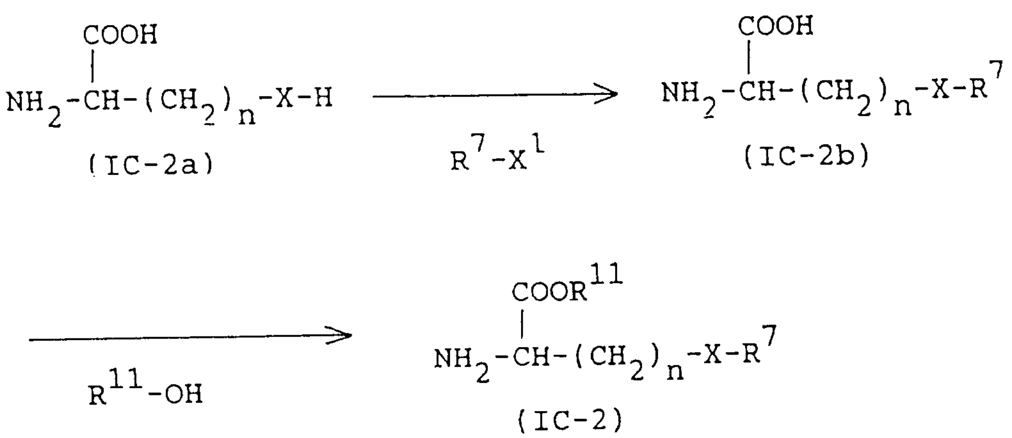

(IC-2a) (IC-2b) (IC-2)

wherein $R^7$, $R^{11}$, X, and n are respectively the same as defined in formula (II), and $X^1$ represents a halogen atom.

The amine derivative of formula (IC-2) can also be synthesized after protecting the amino group of the amino acid of formula (IC-2a) by a protective group for the amino group employed in the peptide synthesis.

Specific examples of the amine derivative of formula (IC-2) are as follows:

L-O-(benzyl)serineethyl ester,
L-S-(2-phenylethyl)cysteinemethyl ester,
L-S-(3-phenylpropyl)cysteinemethyl ester,
L-O-(3-phenylpropyl)serineethyl ester,
L-O-(3-thienylmethyl)serineethyl ester,
L-S-(diphenylmethyl)cysteinemethyl ester,
L-S-(cyclohexylmethyl)cysteineethyl ester,
L-S-(cyclopentyl)cysteineethyl ester,
L-S-(2-thienylmethyl)cysteinemethyl ester,

L-S-(3-thienylmethyl)cysteineethyl ester,
L-S-(1-naphthylmethyl)cysteineethyl ester,
L-S-(2-naphthylmethyl)cysteineethyl ester, and
L-S-(2-chlorobenzyl)cysteineethyl ester.

In Step III-1, the ester derivative of formula (IC-3) can be produced by converting the carboxyl group of the carboxylic acid derivative of formula (IC-1) to, for example, an active ester group, a carboxylic acid halide group, or an acid anhydride group to prepare an active ester compound, a carboxylic acid halide compound or an acid anhydride compound and then by allowing such a compound to react with the amine derivative of formula (IC-2) in an inert solvent in the same manner as in the previously mentioned procedure in Step III-1.

**[Step III-2]**

In this step, the ester derivative of formula (IC-3) is reduced to prepare the alcohol derivative of formula (IC-4).

Boron compounds such as sodium borohydride and lithium borohydride can be used as the reducing agents for the reduction in this step.

It is preferable that the amount of such a reducing agent be in the range of 1 to 4 equivalents per one mole of the ester derivative of formula (IC-3). It is also preferable that the reduction reaction be carried out in an inert solvent, for example, water, alcohols such as methanol and ethanol, ethers such as ether, THF, dimethoxyethane, and dioxane, halogenated hydrocarbons such as dichloromethane, chloroform and dichloroethane, and aromatic hydrocarbons such as benzene, toluene and xylene. These solvents can be used alone or in combination. The reduction reaction can be carried out at temperatures of -20 ° C to 50 ° C.

**[Step III-3]**

In this step, the alcohol derivative of formula (IC-4) obtained in the reaction of Step III-2 is oxidized, so that an aldehyde derivative of formula (II) is produced.

An active dimethyl sulfoxide (DMSO) oxidization method is employed for the oxidization in Step III-3. DMSO is an oxidizing agent and can be used in combination with an activating agent such as dicyclohexylcarbodiimide, phosphorus pentoxide, pyridine•sulfur trioxide complex, oxalyl chloride, acetic anhydride, and trifluoroacetic anhydride. It is preferable that the oxidization agent be employed in an amount of 1 to 4 equivalents to the alcohol derivative of formula (IC-4).

It is preferable that the above reaction in Step III-3 be carried out in a solvent, for example, halogenated hydrocarbons such as dichloromethane, dichloroethane, and chloroform, and DMSO. The above reaction in Step III-3 can be carried out at -20 ° C to 30 ° C.

**[Step III-4]**

In this step, the carboxylic acid of formula (IC-1) and the amino alcohol derivative of formula (IC-5) are allowed to react in the presence of a condensing agent to produce the alcohol derivative of formula (IC-4). This reaction can be carried out by using the same condensing agent and reaction solvent under the same condictions as in Step III-1.

The amino alcohol derivative of formula (IC-5) can be produced from the previously mentioned amino acid of formula (IC-2a) in accordance with the following reaction scheme:

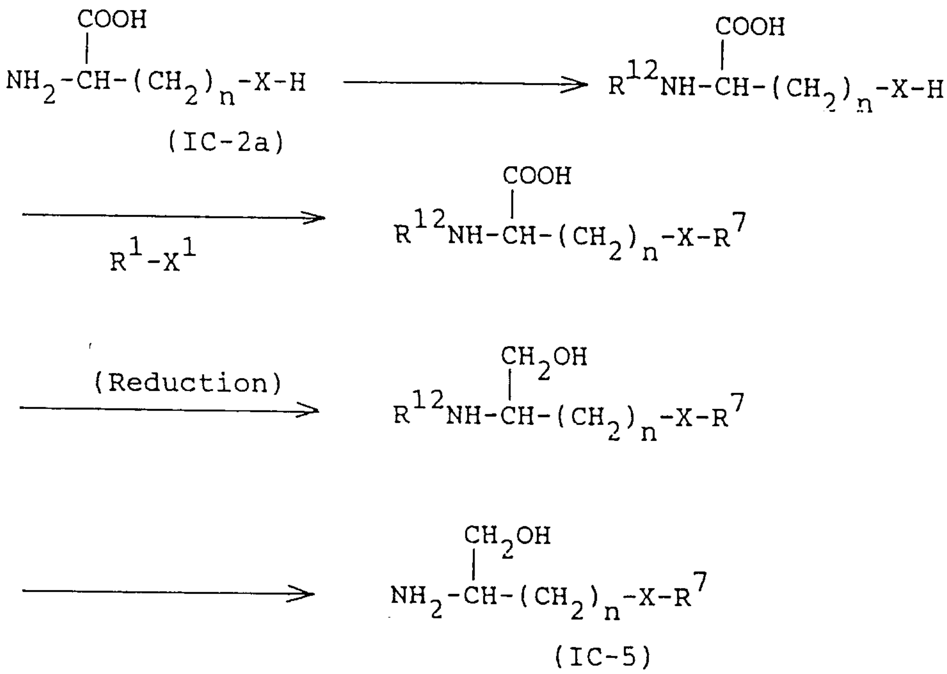


(IC-2a)

(IC-5)

wherein $R^7$, X, and n are respectively the same as in formula (II) and $R^{12}$ represents a protective group for the amino group.

Specific examples of the amino alcohol derivative of formula (IC-5) are as follows:

(2R)-2-amino-3-(2-fluorobenzylthio)propanol,
(2R)-2-amino-3-(3-chlorobenzylthio)propanol,
(2R)-2-amino-3-(4-chlorobenzylthio)propanol,
(2R)-2-amino-3-(3-fluorobenzylthio)propanol,
(2R)-2-amino-3-(2-methoxybenzylthio)propanol,
(2R)-2-amino-3-(3-fluorobenzylthio)propanol,
(2R)-2-amino-3-(3-methoxybenzylthio)propanol,
(2R)-2-amino-3-(4-methoxybenzylthio)propanol,
(2R)-2-amino-3-(3-nitrobenzylthio)propanol,
(2R)-2-amino-3-(4-nitrobenzylthio)propanol,
(2S)-2-amino-4-phenoxybutanol,
(2S)-2-amino-4-(phenylthio)butanol,
(2S)-2-amino-3-(2-chlorobenzyloxy)propanol,
(2S)-2-amino-4-(2-fluorophenoxy)butanol,
(2S)-2-amino-4-(3-fluorophenoxy)butanol,
(2S)-2-amino-4-(2-chlorophenoxy)butanol,
(2S)-2-amino-4-(3-chlorophenoxy)butanol,
(2S)-2-amino-4-benzylthiobutanol,
(2S)-2-amino-4-(2-fluorobenzylthio)butanol,
(2S)-2-amino-4-(2-chlorobenzylthio)butanol,
(2S)-2-amino-4-(2-fluorophenylthio)butanol,
(2S)-2-amino-4-(2-chlorophenylthio)butanol,
(2S)-2-amino-4-(4-chlorophenylthio)butanol,
(2S)-2-amino-4-(2-chlorobenzyloxy)butanol,
(2S)-2-amino-4-(2-fluorobenzyloxy)butanol, and
(2S)-2-amino-4-(3-nitrobenzyloxy)butanol.

In this step, the carboxylic acid derivative of formula (IC-1) can be converted to an active ester compound, a carboxylic acid halide compound or an acid anhydride compound, and such a compound can be allowed to react with the amine alcohol derivative of formula (IC-5) in the same manner as in the previously mentioned procedure in Step III-1 to produce the alcohol derivative of formula (IC-4). The thus produced alcohol derivative of formula (IC-4) is oxidized by the same procedure as in Step III-3 to produce

the aldehyde derivative of formula (II).

Obviously, numerous modifications and variations of the present invention are possible in light of the above teachings. It is therefore to be understood that within the scope of the appended claims, the invention may be practiced otherwise than as specifically described herein.

Other features of this invention will become apparent in the course of the following description of exemplary embodiments, which are given for illustration of the invention and are not intended to be limiting thereof.

**Reference Example 1-1**

Synthesis of (2R,2S)-1-benzyloxycarbonylazetidine-2-carboxylic acid (Reference Compound No. 1-1):

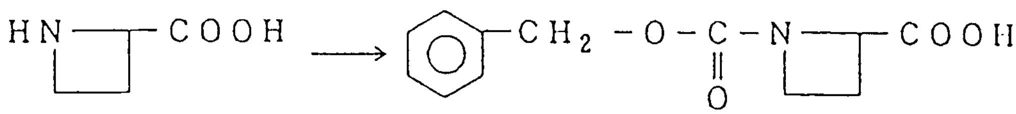

To 60 ml of a 1 N sodium hydroxide solution containing 5.05 g (56 mmol) of (2R,2S)-azetidine-2-carboxylic acid synthesized in accordance with the method described in Agr. Biol. Chem vol 37 (No. 3) 649 (1973), 45 ml of a 32% toluene solution of benzyloxycarbonyl chloride and 60 ml of a 1 N sodium hydroxide solution were added dropwise at the same time under an ice-cooled condition. The reaction mixture was stirred overnight at room temperature and then washed with ether twice. The reaction mixture was made acid (pH = 1 - 2) by the addition of concentrated hydrochloric acid thereto and extracted with ethyl acetate twice. The resultant organic extract layer was washed with a saturated aqueous solution of sodium chloride, dried over anhydrous sodium sulfate, and concentrated under reduced pressure, whereby 9.8 g of the captioned Reference Compound No. 1-1 was obtained in a yield of 74%.

NMR (δ, $CDCl_3$): 7.30 - 7.43 (m, 5H), 5.16 (s, 2H), 4.75 - 4.90 (m, 1H), 3.90 - 4.10 (m, 2H), 2.40 - 2.65 (m, 2H)

**Reference Examples 1-2 to 1-26**

The same reaction procedure as in Reference Example 1-1 was repeated except that the (2R,2S)-azetidine-2-carboxylic acid and the benzyloxycarbonyl chloride used in Reference Example 1-1 were respectively replaced by Material a and Material b shown in Table 1, whereby Reference Compounds No. 1-2 to No. 1-26 were respectively obtained as shown in Table 1.

Table 1

$R^2$-Y-COOH (II-a)

| Ref. Ex. | Material a | Material b | Compound | $R^2$ | Y | NMR(δ, $CDCl_3$) |
|---|---|---|---|---|---|---|
| 1-2 | (2R,2S)-piperidine-2-carboxylic acid | benzyloxycarbonylchloride | (2R,S)-1-benzyloxycarbonylpiperidine-2-carboxylic acid | 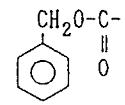 | 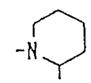 | 7.25 - 7.43 (m, 5H), 5.05 - 5.20 (m, 2H), 3.90 - 4.35 (m, 2H), 2.88 - 3.25 (m, 2H), 2.45 - 2.60 (m, 1H), 2.03 - 2.15 (m, 1H), 1.40 - 1.80 (m, 3H) |
| 1-3 | (3R,3S)-piperidine-3-carboxylic acid | benzyloxycarbonylchloride | (3R,S)-1-benzyloxycarbonylpiperidine-3-carboxylic acid | 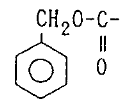 | 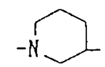 | 7.25 - 7.45 (m, 5H), 5.17 (s, 2H), 4.85 - 5.05 (m, 1H), 4.00 - 4.19 (m, 1H), 2.93 - 3.15 (m, 1H), 2.15 - 2.35 (m, 1H), 1.20 - 1.80 (m, 5H) |
| 1-4 | piperidine-4-carboxylic acid | benzyloxycarbonylchloride | 1-benzyloxycarbonylpiperidine-4-carboxylic acid | 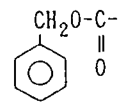 | 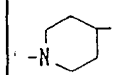 | 7.25 - 7.41 (m, 5H), 5.13 (s, 2H), 4.00 - 4.23 (m, 2H), 2.85 - 3.05 (m, 2H), 2.52 (tt, J=10.8Hz, 3.9Hz, 1H), 1.80 - 2.03 (m, 2H), 1.55 - 1.80 (m, 2H) |
| 1-5 | piperidine-4-carboxylic acid | ethoxycarbonylchloride | 1-ethoxycarbonylpiperidine-4-carboxylic acid | 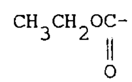 | 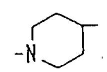 | 4.14 (q, J=7.1Hz, 2H), 3.98 - 4.23 (m, 2H), 2.80 - 3.00 (m, 2H), 2.51 (tt, J=11.5Hz, 3.8Hz, 1H), 1.87 - 2.00 (m, 2H), 1.56 - 1.75 (m, 2H), 1.26 (t, j=7.1Hz, 3H) |
| 1-6 | piperidine-4-carboxylic acid | benzoylchloride | 1-benzoylpiperidine-4-carboxylic acid | 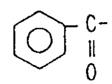 | 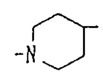 | 7.35 - 7.45 (m, 5H), 4.40 - 4.70 (m, 1H), 3.60 - 3.90 (m, 1H), 2.95 - 3.16 (m, 2H), 2.63 (tt, J=10.6Hz, 4.1Hz, 1H), 1.60 - 2.15 (m, 4H) |

Table 1

| Ref. Ex. | Material a | Material b | Compound | $R^2$ | Y | NMR(δ,$CDCl_3$) |
|---|---|---|---|---|---|---|
| 1-7 | piperidine-4-carboxylic acid | phenylacetyl chloride | 1-phenylacetyl-piperidine-4-carboxylic acid | 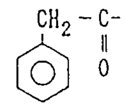 | 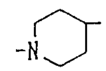 | 7.20 - 7.37 (m, 5H), 4.34 - 4.47 (m, 1H), 3.70 - 3.86 (m, 1H), 3.75 (s, 2H), 3.00 - 3.15 (m, 1H), 2.81 - 2.97 (m, 1H), 2.52 (tt, J=11.5Hz, 3.8Hz, 1H), 1.35 - 2.00 (m, 4H) |
| 1-8 | piperidine-4-carboxylic acid | 3-phenylpropionyl chloride | 1-(3-phenylpropionyl)-piperidine-4-carboxylic acid | 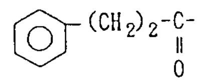 | 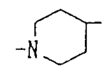 | 7.10 - 7.40 (m, 5H), 4.35 - 4.54 (m, 1H), 3.65 - 3.88 (m, 1H), 2.97 (t, J=8.0Hz, 2H), 2.64 (t, J=8.0Hz, 2H), 2.40 - 3.15 (m, 3H), 1.75 - 2.08 (m, 2H), 1.45 - 1.75 (m, 2H) |
| 1-9 | piperidine-4-carboxylic acid | 4-phenylbutyryl chloride | 1-(4-phenylbutyryl)-piperidine-4-carboxylic acid | 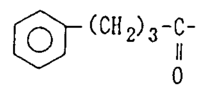 | 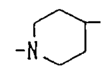 | 7.14 - 7.33 (m, 5H), 4.32 - 4.49 (m, 1H), 3.64 - 3.80 (m, 1H), 2.97 - 3.15 m, 1H), 2.74 - 2.93 (m, 1H), 2.67 (t, J=7.5Hz, 2H), 2.56 (tt, J=11.5Hz, 3.8Hz, 1H), 2.34 (t, J=7.6Hz, 2H), 1.96 (t, J=7.6Hz, 2H), 1.85 - 2.03 (m, 2H), 1.57 - 1.73 (m, 2H) |
| 1-10 | piperidine-4-carboxylic acid | cinnamoyl chloride | 1-cinnamoylpiperidine-4-carboxylic acid | 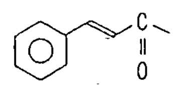 | 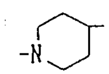 | (Solvent: $CD_3OD$)<br>7.59 - 7.64 (m, 2H), 7.55 (d, J=15.6 Hz, 1H), 7.32 - 7.44 (m, 3H), 7.15 (d, J=15.6Hz, 1H), 4.38 - 4.48 (m, 1H), 4.15 - 4.25 (m, 1H), 3.25 - 3.40 (m, 1H), 2.93 - 3.06 (m, 1H), 2.64 (tt, J=11.5Hz, 3.8Hz, 1H), 1.94 - 2.08 (m, 2H), 1.56 - 1.75 (m, 2H) |

Table 1

| Ref. Ex. | Material a | Material b | Compound | $R^2$ | Y | NMR(δ,$CDCl_3$) |
|---|---|---|---|---|---|---|
| 1-11 | piperidine-4-carboxylic acid | 1-naphthoyl chloride | 1-(1-naphthoyl)-piperidine-4-carboxylic acid | 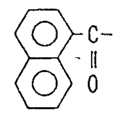 | 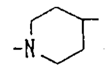 | 7.75 - 7.92 (m, 3H), 7.36 - 7.58 (m, 4H), 4.68 - 4.80 (m, 1H), 3.35 - 3.50 (m, 1H), 2.90 - 3.27 (m, 2H), 2.57 - 2.70 (m, 1H), 1.50 - 2.20 (m, 4H) |
| 1-12 | piperidine-4-carboxylic acid | 2-naphthoyl chloride | 1-(2-naphthoyl)-piperidine-4-carboxylic acid | 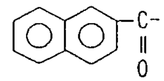 | 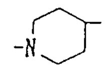 | 7.80 - 8.97 (m, 4H), 7.45 - 7.65 (m, 3H), 4.40 - 4.75 (m, 1H), 3.60 - 4.00 (m, 1H), 3.00 - 3.20 (m, 2H), 2.65 (tt, J=11.5Hz, 3z8Hz, 1H), 1.60 - 2.20 (m, 4H) |
| 1-13 | piperidine-4-carboxylic acid | cyclopentanecarbonyl chloride | 1-cyclopentylcarbonyl-piperidine-4-carboxylic acid | 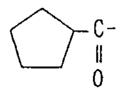 | 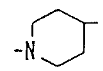 | 4.30 - 4.55 (m, 1H), 3.80 - 4.10 (m, 1H), 2.70 - 3.25 (m, 3H), 2.59 (tt, J=10.6Hz, 4.1Hz, 1H), 1.40 - 2.05 (m, 12H) |
| 1-14 | piperidine-4-carboxylic acid | thiophene-2-carbonyl chloride | 1-(2-thienylcarbonyl)-piperidine-4-carboxylic acid | 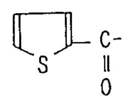 | 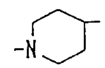 | 7.43 - 7.48 (m, 1H), 7.25 - 7.33 (m, 1H), 7.03 - 7.08 (m, 1H), 4.21 - 4.45 (m, 2H), 3.05 - 3.29 (m, 2H), 2.67 (tt, J=10.5Hz, 4.1Hz, 1H), 1.95 - 2.09 (m, 2H), 1.72 - 1.87 (m, 2H) |
| 1-15 | piperidine-4-carboxylic acid | trimethylacetyl chloride | 1-trimethylacetyl-piperidine-4-carboxylic acid | 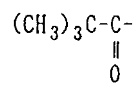 | 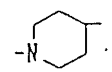 | 4.21 - 4.36 (m, 2H), 2.91 - 3.10 (m, 2H), 2.61 (tt, J=11.2Hz, 4.1Hz, 1H), 1.90 - 2.03 (m, 2H), 1.60 - 1.78 (m, 2H), 1.38 (s, 9H) |

Table 1

| Ref. Ex. | Material a | Material b | Compound | $R^2$ | Y | NMR(δ,$CDCl_3$) |
|---|---|---|---|---|---|---|
| 1-16 | piperidine-4-carboxylic acid | acetic anhydride | 1-acetylpiperidine-4-carboxylic acid | 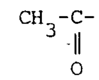 | 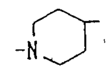 | 4.34 - 4.46 (m, 1H), 3.74 - 3.87 (m, 1H), 3.10 - 3.23 (m, 1H), 2.80 - 2.93 (m, 1H), 2.59 (tt, J=11.5Hz, 3.8Hz, 1H), 2.12 (s, 3H), 1.91 - 2.05 (m, 2H), 1.59 - 1.80 (m, 2H) |
| 1-17 | piperidine-4-carboxylic acid | 3,4-dichlorobenzoyl chloride | 1-(3,4-dichlorobenzoyl)piperidine-4-carboxylic acid | 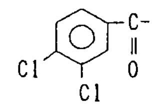 | 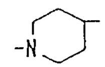 | 7.45 - 7.55 (m, 2H), 7.20 - 7.29 (m, 1H), 3.40 - 4.70 (m, 2H), 2.90 - 3.25 (m, 2H), 2.64 (tt, J=10.9Hz, 4.1Hz, 1H), 1.50 - 2.20 (m, 4H) |
| 1-18 | piperidine-4-carboxylic acid | 2-chlorocinnamoyl chloride | 1-(2-chlorocinnamoyl)-piperidine-4-carboxylic acid | 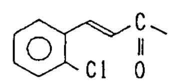 | 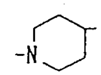 | 7.98 (d, J=15.5Hz, 1H), 7.20 - 7.63 (m, 4H), 6.86 (d, J=15.5Hz, 1H), 4.35 - 4.60 (m, 1H), 3.95 - 4.15 (m, 1H), 2.85 - 3.38 (m, 2H), 2.67 (tt, J=11.5Hz, 3.8Hz, 1H), 1.20 - 2.10 (m, 4H) |
| 1-19 | piperidine-4-carboxylic acid | 3-chlorocinnamoyl-chloride | 1-(3-chlorocinnamoyl)-piperidine-4-carboxylic acid | 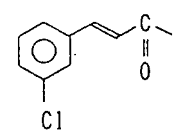 | 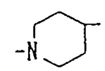 | 7.60 (d, J=15.5Hz, 1H), 7.52 (s, 1H), 7.23 - 7.43 (m, 3H), 6.88 (d, J=15.4Hz, 1H), 4.35 - 4.60 (m, 1H), 3.90 - 4.20 (m, 1H), 2.90 - 3.42 (m, 2H), 2.67 (tt, J=11.5Hz, 3.8Hz, 1H), 1.97 - 2.12 (m, 2H), 1.68 - 1.88 (m, 2H) |
| 1-20 | piperidine-4-carboxylic acid | 4-chlorocinnamoyl chloride | 1-(4-chlorocinnamoyl)-piperidine-4-carboxylic acid | 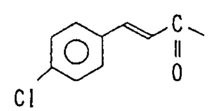 | 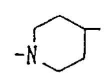 | 7.62 (d, J=15.4Hz, 1H), 7.41 - 7.50 (m, 2H), 7.30 - 7.40 (m, 2H), 6.85 (d, J=15.4Hz, 1H), 4.35 - 4.62 (m, 1H), 3.90 - 4.15 (m, 1H), 2.90 - 3.40 (m, 2H), 2.65 (tt, J=11.5Hz, 3.8Hz, 1H), 1.95 - 2.10 (m, 2H), 1.68 - 1.85 (m, 2H) |

Table 1

| Ref. Ex. | Material a | Material b | Compound | $R^2$ | Y | NMR(δ,$CDCl_3$) |
|---|---|---|---|---|---|---|
| 1-21 | piperidine-4-carboxylic acid | 4-phenylbenzoyl chloride | 1-(4-phenylbenzoyl)-piperidine-4-carboxylic acid | 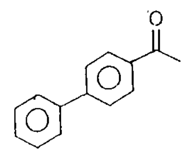 | 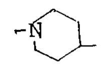 | (Solvent: $CD_3OD$)<br>7.60 - 7.80 (m, 4H), 7.30 - 7.55 (m, 5H), 4.40 - 4.60 (m, 1H), 3.70 - 3.90 (m, 1H), 3.00 - 3.40 (m, 2H), 2.65 (tt, J=10Hz, 4Hz, 1H), 1.60 - 2.15 (m, 4H) |
| 1-22 | piperidine-4-carboxylic acid | 3-(3-thienyl)-acrylic acid chloride | 1-[3-(3-thienyl)-acryloyl]piperidine-4-carboxylic acid | 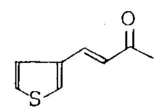 | 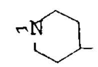 | (Solvent: $CD_3OD$)<br>7.70 (d, J=15Hz, 1H), 7.48 (d, J=5Hz, 1H), 7.34 (d, J=3Hz, 1H), 7.07 (dd, J=5Hz, 3Hz, 1H), 6.90 (d, J=15Hz, 1H), 4.35 - 4.50 (m, 1H), 4.05 - 4.25 (m, 1H), 3.20 - 3.40 (m, 1H), 2.90 - 3.10 (m, 1H), 2.63 (tt, J=10Hz, 4Hz, 1H), 1.90 - 2.10 (m, 2H), 1.50 - 1.80 (m, 2H) |
| 1-23 | piperidine-4-carboxylic acid | 2-nitrocinnamoyl chloride | 1-(2-nitrocinnamoyl)-piperidine-4-carboxylic acid | 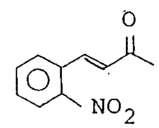 | 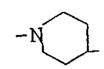 | (Solvent: $CD_3OD$)<br>8.01 (d, J=9Hz, 1H), 7.85 - 7.95 (m, 2H), 7.71 (t, J=7Hz, 1H), 7.59 (t, J=9Hz, 1H), 7.11 (d, J=15Hz, 1H), 4.35 - 4.50 (m, 1H), 4.15 - 4.25 (m, 1H), 4.25 - 4.40 (m, 1H), 2.95 - 3.10 (m, 1H), 2.65 (tt, J=10Hz, 4Hz, 1H), 1.95 - 2.10 (m, 2H), 1.60 - 1.80 (m, 2H) |
| 1-24 | piperidine-4-carboxylic acid | 3-phenyl-2-methyl-acrylic acid chloride | 1-[(3-phenyl-2-methyl)acryloyl]-piperidine-4-carboxylic acid | 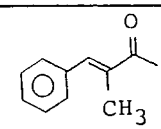 | 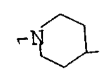 | 7.20 - 7.40 (m, 5H), 6.52 (s, 1H), 3.70 - 4.60 (m, 2H), 2.90 - 3.30 (m, 2H), 2.62 (tt, J=11Hz, 4Hz, 1H), 2.09 (s, 3H), 1.90 - 2.10 (m, 2H), 1.65 - 1.80 (m, 2H) |

Table 1

| | | | | | | |
|---|---|---|---|---|---|---|
| 1-25 | piperidine-4-carboxylic acid | quinoline-8-sulfonyl chloride | 1-(8-quinolylsulfonyl)-piperidine-4-carboxylic acid | 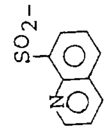 | 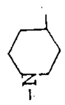 | (Solvent: $CD_3OD$) 9.03 (d, J=4Hz, 1H), 8.40 - 8.50 (m, 2H), 8.20 (d, J=7Hz, 1H), 7.04 (t, J=8Hz, 1H), 7.62 (dd, J=8Hz, 4Hz, 1H), 3.85 - 4.00 (m, 2H), 2.85 - 3.00 (m, 2H), 2.34 (tt, J=10Hz, 4Hz, 1H), 1.85 - 2.00 (m, 2H), 1.55 - 1.70 (m, 2H) |
| 1-26 | piperidine-4-carboxylic acid | 3-(2-naphthyl)-acrylic acid chloride | 1-[3-(2-naphthyl)-acryloyl]piperidine-4-carboxylic acid | 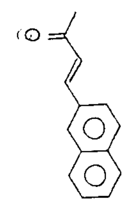 | 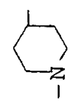 | 8.20 - 9.50 (m, 1H), 7.35 - 8.00 (m, 8H), 6.98 (d, J=15Hz, 1H), 3.80 - 4.75 (m, 2H), 2.80 - 3.50 (m, 2H), 2.65 (tt, J=10Hz, J=4Hz, 1H), 1.92 - 2.14 (m, 2H), 1.65 - 1.90 (m, 2H) |

## Reference Example 1-27

Synthesis of 1-(N-benzylcarbamoyl)piperidine-4-carboxylic acid (Reference Compound No. 1-27):

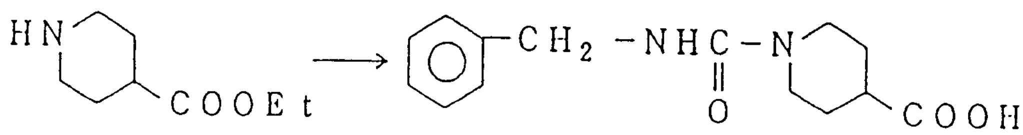

Under an ice-cooled condition, 28.9 ml (0.396 mol) of thionyl chloride was added dropwise to 200 ml of an ethanol suspension containing 25 g (0.193 mol) of piperidine-4-carboxylic acid. The reaction mixture was stirred at room temperature for 18 hours, and then concentrated under reduced pressure. The residue thus obtained was dissolved in ethanol. With the addition of ether to this reaction mixture, crystals separated out. These crystals were separated by filtration and dried, so that 36.3 g of piperidine-4-carboxylic acid ethyl ester hydrochloride was obtained in a yield of 97%. To a methylene chloride suspension containing 7.28 g (37.6 mmol) of the above prepared ester hydrochloride, 10.5 ml (75.2 mmol) of triethylamine and benzyl isocyanate were successively added under an ice-cooled condition. The reaction mixture was then stirred overnight at room temperature. The reaction mixture was washed successively with 1 N hydrochloric acid, a saturated aqueous solution of sodium hydrogencarbonate and a saturated aqueous solution of sodium chloride, and then dried over anhydrous sodium sulfate. The solvent was distilled away from the reaction mixture under reduced pressure, whereby 7.58 g of 1-(N-benzylcarbamoyl)piperidine-4-carboxylic acid ethyl ester was obtained in a yield of 68%.

53 ml of a 1 N sodium hydroxide solution was added to 50 ml of a methanol solution containing 7 g (24.1 mmol) of the above prepared ethyl ester under an ice-cooled condition. The reaction mixture was stirred for 3 hours, and then concentrated under reduced pressure. The residue thus obtained was dissolved in water and washed with ether twice. The resulting water layer was made acid (pH = 1) by the addition of concentrated hydrochloric acid thereto. The water layer was extracted with chloroform twice and dried over anhydrous sulfate. The solvent was distilled away under reduced pressure, whereby 5.94 g of the captioned Reference Compound No. 1-27 was obtained in a yield of 94%.

NMR (δ, $CD_3OD$): 7.17 - 7.38 (m, 5H), 4.35 (s, 2H), 3.87 - 4.05 (m, 2H), 2.84 - 3.04 (m, 2H), 2.52 (tt, J = 11.0Hz, 4.0Hz, 1H), 1.80 - 1.99 (m, 2H), 1.45 - 1.83 (m, 2H)

**Reference Examples 1-28 to 1-32**

The same reaction procedure as in Reference Example 1-27 was repeated except that benzyl isocyanate used in Reference Example 1-27 was replaced by the respective materials shown in Table 2, whereby Reference Compounds No. 1-28 to No. 1-32 were respectively obtained as shown in Table 2.

Table 2

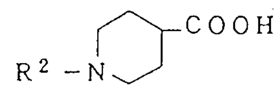
(II-b)

| Ref. Ex. | Material | Compound | $R^2$ | NMR(δ, $CDCl_3$) |
|---|---|---|---|---|
| 1-28 | phenyl isocyanate | 1-(N-phenylcarbamoyl)-piperidine-4-carboxylic acid | 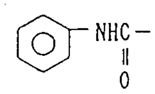 | (Solvent: $CD_3OD$) 7.20 - 7.37 (m, 4H), 6.97 - 7.04 (m, 1H), 4.02 - 4.15 (m, 2H), 2.95 - 3.10 (m, 2H), 2.57 (tt, J=10.9Hz, 4.0Hz, 1H), 1.90 - 2.02 (m, 2H), 1.58 - 1.75 (m, 2H) |
| 1-29 | 4-methylbenzenesulfonyl chloride | 1-(4-methylphenylsulfonyl)piperidine-4-carboxylic acid | 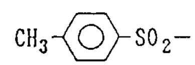 | 7.64 (d, J=8.3Hz, 2H), 7.33 (d, J=7.9Hz, 2H), 3.60 - 3.70 (m, 2H), 2.44 (s, 3H), 2.40 - 2.53 (m, 2H), 2.29 (tt, J=10.7Hz, 4.0Hz, 1H), 1.75 - 2.05 (m, 4H) |
| 1-30 | naphthalene-2-sulfonyl chloride | 1-(2-naphthylsulfonyl)piperidine-4-carboxylic acid | 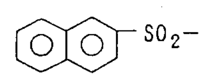 | 8.33 (s, 1H), 7.88 - 8.03 (m, 3H), 7.57 - 7.78 (m, 3H), 3.62 - 3.78 (m, 2H), 2.45 - 2.62 (m, 2H), 2.20 - 2.34 (m, 1H), 1.75 - 2.10 (m, 4H) |
| 1-31 | diphenylacetyl chloride | 1-diphenylacetyl-piperidine-4-carboxylic acid | 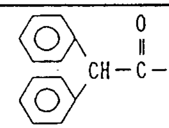 | 7.23 - 7.38 (m, 10H), 5.20 (s, 1H), 4.35 - 4.54 (m, 1H), 3.75 - 3.95 (m, 1H), 2.80 - 3.15 (m, 2H), 2.50 (tt, J=11.2Hz, 3.9Hz, 1H), 1.20 - 2.05 (m, 4H) |
| 1-32 | 2,6-dichlorobenzoyl chloride | 1-(2,6-dichlorobenzoyl)piperidine-4-carboxylic acid | 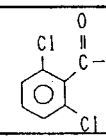 | 7.20 - 7.37 (m, 3H), 4.50 - 4.62 (m, 1H), 3.35 - 3.49 (m, 1H), 3.05 - 3.25 (m, 2H), 2.63 (tt, J=11.0Hz, 4.0Hz, 1H), 1.71 - 2.19 (m, 4H) |

**Reference Example 1-33**

Synthesis of 1-(9-fluorenylcarbonyl)piperidine-4-carboxylic acid (Reference Compound No. 1-33):

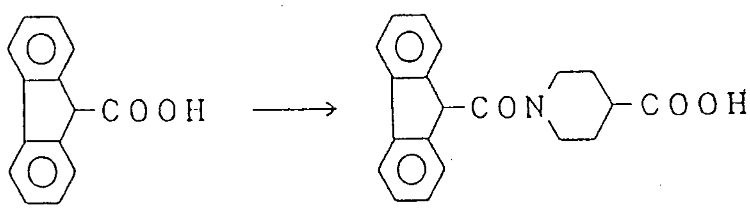

To 100 ml of a tetrahydrofuran solution of 5 g (23.8 mmol) of 9-fluorene carboxylic acid, 2.74 g (23.8 mmol) of N-hydroxysuccinimide and 5.40 g (26.2 mmol) of N,N'-dicyclohexyl carbodiimide were successively added under an ice-cooled condition. After stirring the above mixture for 3 hours, dicyclohexylurea was removed from the reaction mixture by filtration. To the filtrate, 4.61 g (23.8 mmol) of piperidine-4-carboxylic acid ethyl ester hydrochloride and 3.33 ml (23.8 mmol) of triethylamine were added at -0 °C. The reaction mixture was further stirred overnight at room temperature. The solvent was distilled away from the reaction mixture under reduced pressure. Ethyl acetate was added to the residue and the mixture was washed successively with a 1 N hydrochloric acid solution, a saturated aqueous solution of sodium chloride, a saturated aqueous solution of sodium hydrogencarbonate and a saturated aqueous solution of sodium chloride, and then dried over anhydrous sodium sulfate. The solvent was distilled away under reduced pressure, whereby 6.10 g of ethyl 1-(9-fluorenylcarbonyl)piperidine-4-carboxylate was obtained in a yield of 73%.

6.10 g (17.5 mmol) of the above prepared ethyl 9-fluorenylcarbonylpiperidine-4-carboxylate was dissolved in 35 ml of methanol. With the addition of 38.5 ml of a 1 N sodium hydroxide solution under an ice-cooled condition, the reaction mixture was stirred for one hour. Subsequently, a 1 N hydrochloric acid solution was added to the reaction mixture until it became neutral, and then the methanol was distilled away from the reaction mixture under reduced pressure. The residue thus obtained was made basic with the addition of a 1 N sodium hydroxide solution and washed with ether. The resultant water layer was made acid (pH = 1) by the addition of 4 N hydrochloric acid, and extracted with ethyl acetate. The extract layer was washed with a saturated aqueous solution of sodium chloride and then dried over anhydrous sodium sulfate. The solvent was distilled away under reduced pressure, whereby 5.75 g of the captioned Reference compound No. 1-33 was obtained.

NMR ($\delta$, $CDCl_3$): 7.80 (d, 7.3Hz, 2H), 7.28 - 7.70 (m, 6H), 5.06 (s, 1H), 4.25 - 4.45 (m, 1H), 0.60 - 3.15 (m, 8H)

**Reference Examples 1-34 to 1-41**

The same procedure as in Reference Example 1-33 was repeated except that the 9-fluorene carboxylic acid used in Example 1-33 was replaced by the respective carboxylic acids shown in Table 3, whereby Reference Compounds No. 1-34 to No. 1-41 were respectively obtained as shown in Table 3.

Table 3

| Ref. Ex. | Material | Compound | $R^2$ | NMR(δ,$CDCl_3$) |
|---|---|---|---|---|
| 1-34 | quinoline-2-carboxylic acid | 1-(2-quinolyl-carbonyl)piperidine-4-carboxylic acid | 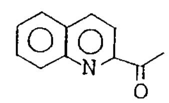 | 8.28 (d, J=8Hz, 1H), 8.15 (d, J=8Hz, 1H), 7.86 (d, J=8Hz, 1H), 7.76 (t, J=7Hz, 1H), 7.68 (d, J=8Hz, 1H), 7.61 (t, J=7Hz, 1H), 4.55 - 4.70 (m, 1H), 3.90 - 4.05 (m, 1H), 3.10 - 3.30 (m, 2H), 2.30 - 2.45 (m, 1H), 1.80 - 2.20 (m, 4H) |
| 1-35 | quinoline-3-carboxylic acid | 1-(3-quinolyl-carbonyl)piperidine-4-carboxylic acid | 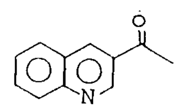 | (Solvent: $CD_3OD$) 8.89 (d, J=2Hz, 1H), 8.44 (d, J=2Hz, 1H), 8.08 (d, J=8Hz, 1H), 8.04 (d, J=8Hz, 1H), 7.85 - 7.95 (m, 1H), 7.65 - 7.75 (m, 1H), 4.45 - 4.60 (m, 1H), 3.70 - 3.90 (m, 1H), 3.10 - 3.40 (m, 2H), 2.67 (tt, J=10Hz, 4Hz, 1H), 1.65 - 2.20 (m, 4H) |
| 1-36 | isoquinoline-3-carboxylic acid | 1-(3-isoquinolyl-carbonyl)piperidine-4-carboxylic acid | 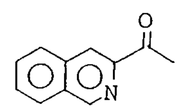 | 9.36 (s, 1H), 8.06 (d, J=8Hz, 1H), 8.02 (s, 1H), 7.92 (d, J=8Hz, 1H), 7.79 (t, J=8Hz, 1H), 7.70 (t, J=8Hz, 1H), 4.50 - 4.70 (m, 1H), 3.70 - 3.90 (m, 1H), 3.00 - 3.30 (m, 4H), 2.55 - 2.70 (m, 1H), 1.80 - 2.20 (m, 4H) |
| 1-37 | 3-(2-pyridyl)acrylic acid | 1-[3-(2-pyridyl)-acryloyl]piperidine-4-carboxylic acid | 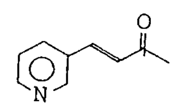 | (Solvent: $CD_3OD$) 8.76 (d, J=2Hz, 1H), 8.50 (dd, J=5Hz, 2Hz, 1H), 8.15 (d, t, J=8Hz, 2Hz, 1H), 7.57 (d, J=15Hz, 1H), 7.47 (dd, J=8Hz, 5Hz, 1H), 7.33 (d, J=15Hz, 1H), 4.40 - 4.50 (m, 1H) 4.15 - 4.30 (m, 1H), 3.25 - 3.40 (m, 1H), 2.90 - 3.10 (m, 1H), 2.65 (tt, J=10Hz, 4Hz, 1H), 1.95 - 2.10 (m, 2H), 1.55 - 1.80 (m, 2H) |

Table 3

| Ref. Ex. | Material | Compound | $R^2$ | NMR(δ, $CDCl_3$) |
|---|---|---|---|---|
| 1-38 | indole-2-carboxylic acid | 1-(2-indolylcarbonyl)-piperidine-4-carboxylic acid | 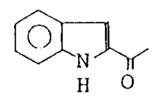 | (Solvent: $CD_3OD$) 7.61 (d, J=8Hz, 1H), 7.42 (d, J=8Hz, 1H), 7.20 (td, J=8Hz, J=1Hz, 1H), 7.06 (td, J=8Hz, J=1Hz, 1H), 6.79 (s, 1H), 4.38 - 4.52 (m, 2H), 3.10 - 3.48 (m, 2H), 2.68 (tt, J=11Hz, J=4Hz, 1H), 1.97 - 2.12 (m, 2H), 1.66 - 1.85 (m, 2H) |
| 1-39 | indole-3-carboxylic acid | 1-(3-indolylcarbonyl)-piperidine-4-carboxylic acid | 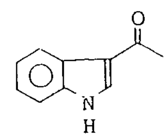 | (Solvent: $CD_3OD$) 7.62 (td, J=7Hz, J=2Hz, 1H), 7.60 (s, 1H), 7.44 (dd, J=7Hz, J=2Hz, 1H), 7.17 (m, 2H), 4.21 - 4.42 (m, 2H), 3.12 - 3.32 (m, 2H), 2.64 (tt, J=10Hz, J=4Hz, 1H), 1.60 - 2.05 (m, 4H) |
| 1-40 | L-N-(benzyloxycarbonyl)-phenylalanine | 1-(N-benzyloxy-carbonyl-L-phenyl-alanyl)piperidine-4-carboxylic acid | 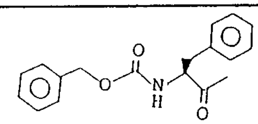 | 7.07 - 7.42 (m, 10H), 5.85 - 5.98 (m, 1H), 4.80 - 5.16 (m, 3H), 4.15 - 4.40 (m, 1H), 3.50 - 3.70 (m, 1H), 2.30 - 3.10 (m, 5H), 0.60 - 1.95 (m, 4H) |
| 1-41 | D-N-(benzyloxycarbonyl)-phenylalanine | 1-(N-benzyloxy-carbonyl-D-phenyl-alanyl)piperidine-4-carboxylic acid | 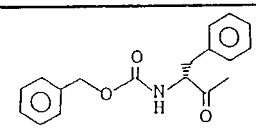 | 7.05 - 7.45 (m, 10H), 5.87 - 6.02 (m, 1H), 4.80 - 5.18 (m, 3H), 4.17 - 4.41 (m, 1H), 3.50 - 3.72 (m, 1H), 2.30 - 3.12 (m, 5H), 0.40 - 1.95 (m, 4H) |

**Reference Example 1-42**

Synthesis of 1-(2-naphthylmethyl)piperidine-4-carboxylic acid (Reference Compound No. 1-42):

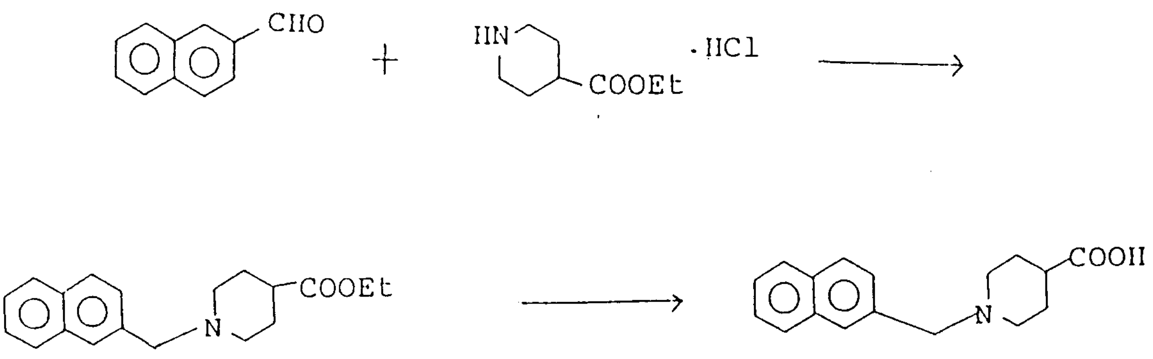

2.17 ml (15.5 mmol) of triethylamine, 4.84 g (31.0 mmol) of 2-naphthoaldehyde and 0.3 g of a 10% palladium carbon were added to an ethanol solution containing 3.0 g (15.5 mmol) of piperidine-4-carboxylic acid ethyl ester hydrochloride. The above prepared reaction mixture was stirred overnight in a stream of hydrogen gas and the palladium carbon was separated therefrom by filtration. The solvent was distilled away under reduced pressure. The residue thus obtained was dissolved in 1 N hydrochloric acid and washed with ether. The resultant water layer was made basic with the addition of sodium hydrogencarbonate and extracted with dichloromethane. The resultant extract organic layer was washed with a saturated aqueous solution of sodium chloride and dried over anhydrous sodium sulfate. The solvent was distilled away under reduced pressure, whereby 4.05 g of N-(2-naphthylmethyl)piperidine-4-carboxylic acid ethyl ester was obtained in a yield of 88%.

4.05 g (13.6 mmol) of the above prepared ester was dissolved in methanol. With the addition of 30 ml (30 mmol) of 1 N sodium hydroxide aqueous solution, the reaction mixture was stirred. The pH of the reaction mixture was adjusted to 7.0 with the addition of 4 N hydrochloric acid. The solvent was distilled away from the reaction mixture under reduced pressure. The residue thus obtained was dissolved in 1 N sodium hydroxide and washed with ether. The pH of the resulting water layer was adjusted to 2 with the addition of 4 N hydrochloric acid and extracted with a mixed solvent of chloroform and 2-butanol. The resultant extract organic layer was dried over anhydrous sodium sulfate and the solvent was distilled away under reduced pressure, whereby 3.70 g of N-(2-naphthyl-methyl)piperidine-4-carboxylic acid was obtained in a yield of 100%.

NMR ($\delta$, $CD_3OD$): 7.90 - 8.08 (m, 4H), 7.54 - 7.63 (m, 3H), 4.46 (s, 2H), 3.40 - 3.55 (m, 2H), 3.05 - 3.27 (m, 2H), 2.55 - 2.71 (m, 1H), 1.82 - 2.25 (m, 4H)

**Reference Example 1-43**

Synthesis of 1-benzyl-piperidine-4-carboxylic acid (Reference Compound No. 1-43):

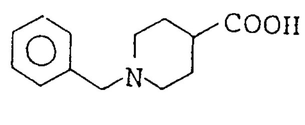

The same procedure as in Reference Example 1-42 was repeated except that the 4.84 g (31.0 mmol) of 2-naphthoaldehyde used in Reference Example 1-42 was replaced by 5.24 ml (31.6 mmol) of benzaldehyde, whereby the captioned Reference Compound No. 1-43 was obtained.

NMR ($\delta$, $CD_3OD$): 7.40 - 7.65 (m, 5H), 4.33 (s, 2H), 2.90 - 3.65 (m, 4H), 2.50 - 2.80 (m, 1H), 1.70 - 2.40 (m, 4H)

**Reference Example 1-44**

Synthesis of 1-(3,4-dichlorobenzyl)-piperidine-4-carboxylic acid (Reference Compound No. 1-44):

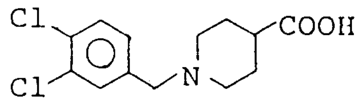

The same procedure as in Reference Example 1-42 was repeated except that the 4.84 g (31.0 mmol) of 2-naphthoaldehyde used in Reference Example 1-42 was replaced by 4.10 g (23.4 mmol) of 3,4-dichlorobenzaldehyde, whereby the captioned Reference Compound No. 1-44 was obtained.

NMR (δ, $CD_3OD$): 7.62 (d, J=2Hz, 1H), 7.55 (d, J=8Hz, 1H), 7.41 (dd, J=8Hz, J=2Hz, 1H), 3.84 (s, 2H), 3.02 - 3.15 (m, 2H), 2.45 - 2.62 (m, 2H), 2.34 (tt, J=10Hz, J=4Hz, 1H), 1.72 - 2.10 (m, 4H)

**Reference Example 1-45**

Synthesis of 1-cinnamylpiperidine-4-carboxylic acid (Reference Compound No. 1-45):

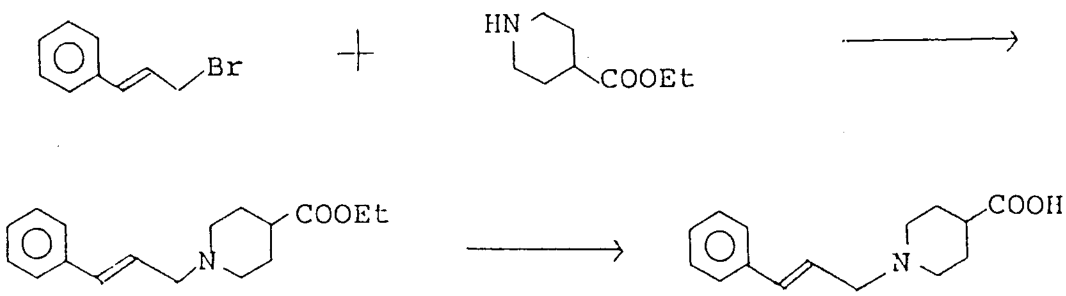

3.53 g (25.7 mmol) of potassium carbonate and 3.80 ml (25.7 mmol) of cinnamyl bromide were added to an acetonitrile solution containing 4.04 g (25.7 mmol) of piperidine-4-carboxylic acid ethyl ester. The above prepared reaction mixture was refluxed for 4 hours and the solvent was distilled away therefrom under reduced pressure. The residue thus obtained was dissolved in chloroform and washed successively with a saturated aqueous solution of sodium chloride containing 5% citric acid, a saturated solution of sodium hydrogencarbonate and a saturated aqueous solution of sodium chloride. The resultant extract organic layer was dried over anhydrous sodium sulfate and the solvent was distilled away under reduced pressure. The residue thus obtained was chromatographed on a silica gel column for purification, whereby 3.97 g of N-cinnamyl-piperidine-4-carboxylic acid ethyl ester was obtained in a yield of 57%.

3.97 g (14.5 mmol) of the above prepared ester was dissolved in methanol and 32 ml (32 mmol) of a 1 N sodium hydroxide aqueous solution was added to the reaction mixture, followed by stirring for 3 hours. The pH of reaction mixture was adjusted to 7 with the addition of 4 N hydrochloric acid and the solvent was distilled away therefrom under reduced pressure. The residue thus obtained was dissolved in 1 N sodium hydroxide and washed with ether. The pH of the resulting water layer was adjusted to 2 with the addition of 4 N hydrochloric acid and extracted with a mixed solvent containing chloroform and 2-butanol. The resultant extract organic layer was dried over anhydrous sodium sulfate and the solvent was distilled away under reduced pressure, whereby 3.25 g of the captioned Reference Compound No. 1-45 was obtained in a yield of 91%.

NMR (δ, $CD_3OD$): 7.25 - 7.60 (m, 5H), 6.91 (d, J=16Hz, 1H), 6.34 (dt, J=16Hz, J=7Hz, 1H), 3.89 (d, J=7Hz, 2H), 3.40 - 3.65 (m, 2H), 2.95 - 3.25 (m, 2H), 2.55 - 2.75 (m, 1H), 1.80 - 2.32 (m, 4H)

**Reference Example 1-46**

Synthesis of (2S)-2-amino-4-methylthiobutanol (Reference Compound No. 1-46):

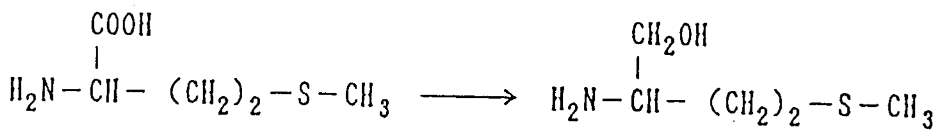

38 ml (300 mmol) of chlorotrimethylsilane was added dropwise to 200 ml of an anhydrous tetrahydrofuran suspension containing 3.3 g (150 mmol) of lithium borohydride under an ice-cooled condition. The reaction mixture was stirred for 30 minutes. 7.5 g (50 mmol) of L-methionine was gradually added to the reaction mixture, followed by stirring overnight at room temperature. Methanol was further added to the reaction mixture under an ice-cooled condition until the evolution of hydrogen gas ceased. The solvent was distilled away from the reaction mixture under reduced pressure. A 10% sodium hydroxide solution was added to the thus obtained residue, followed by the extraction with chloroform twice. After the resultant chloroform extract layer was dried over anhydrous sodium sulfate, the solvent was distilled away under reduced pressure, whereby 5.12 g of the captioned Reference Compound No. 1-46 was obtained in a yield of 75%.

NMR ($\delta$, $CDCl_3$): 3.60 (dd, J=11Hz, J=4Hz, 1H), 3.33 (dd, J=11Hz, J=7Hz, 1H), 2.96 - 3.04 (m, 1H), 2.57 - 2.64 (m, 2H), 2.22 - 2.43 (m, 3H), 2.11 (s, 3H), 1.68 - 1.80 (m, 1H), 1.50 - 1.62 (m, 1H)

**Reference Examples 1-47 to 1-53**

The same reaction procedure in Reference Example 1-46 was repeated except that the L-methionine used in Reference Example 1-46 was replaced by the material shown in Table 4, whereby Reference Compounds No. 1-47 to No. 1-53 were respectively obtained as shown in Table 4.

Table 4

$$H_2N\text{-}CH(CH_2OH)\text{-}(CH_2)n\text{-}R' \quad \text{(III-a)}$$

| Ref. Ex. | Material | Compound | n | $R^1$. | NMR(δ, $CDCl_3$) |
|---|---|---|---|---|---|
| 1-47 | L-leucine | (2S)-2-amino-4-methyl-pentanol | 1 | 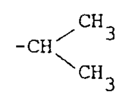 | 3.63 (dd, J=11Hz, 3Hz, 1H), 3.48 (dd, J=11Hz, 1H), 2.60 - 2.80 (m, 1H), 2.05 (brs, 3H), 2.50 - 2.70 (m, 1H), 1.30 - 1.45 (m, 2H), 0.83 - 1.05 (m, 6H) |
| 1-48 | L-phenylalanine | (2S)-2-amino-3-phenyl-propanol | 1 | 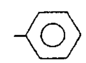 | 7.16 - 7.33 (m, 5H), 3.64 (dd, J=11Hz, J=4Hz, 1H), 3.38 (dd, J=11Hz, J=7Hz, 1H), 3.07 - 3.16 (m, 1H), 2.79 (dd, J=14Hz, J=5Hz, 1H), 2.52 (dd, J=14Hz, J=9Hz, 1H), 2.10 (brs, 3H) |
| 1-49 | L-tyrosine-o-benzylether | (2S)-2-amino-4-(4-benzyl-oxyphenyl)propanol | 1 | 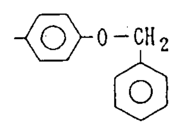 | 7.27 - 7.47 (m, 5H), 7.10 (d, J=8.6Hz, 2H), 6.92 (d, J=8.6Hz, 2H), 5.05 (s, 2H), 3.63 (dd, J=10.6Hz, J=3.9Hz, 1H), 3.37 (dd, J=10.6Hz, J=7.2Hz, 1H), 3.02 - 3.13 (m, 1H) 2.73 (dd, J=13.6Hz, J=5.3Hz, 1H), 2.47 (dd, J=13.7Hz, J=8.6Hz, 1H), 1.70 - 1.95 (m, 3H) |
| 1-50 | L-homophenyl-alanine | (2S)-2-amino-4-phenyl-butanol | 2 | 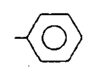 | 7.17 - 7.31 (m, 5H), 3.60 (dd, J=11Hz, J=4Hz, 1H), 3.31 (dd, J=11Hz, J=8Hz, 1H), 2.80 - 2.88 (m, 1H), 2.60 - 2.79 (m, 2H), 1.95 (brs, 3H), 1.70 - 1.80 (m, 1H), 1.52 - 1.64 (m, 1H) |
| 1-51 | L-norleucine | (2S)-2-aminohexanol | 3 | $-CH_3$ | 3.58 (dd, J=10Hz, 3Hz, 1H), 3.27 (dd, J=10Hz, 8Hz, 1H), 2.75 - 2.90 (m, 1H), 2.13 (brs, 3H), 1.20 - 1.50 (m, 6H), 0.80 - 1.00 (m, 3H) |

Table 4

| Ref. Ex. | Material | Compound | n | $R^1$ | NMR(δ,$CDCl_3$) |
|---|---|---|---|---|---|
| 1-52 | L-tryptophan | (2S)-2-amino-3-(3-indolyl)-propanol | 1 | 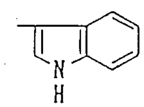 | (Solvent: $CD_3OD$) 7.56 (d, J=7Hz, 1H), 7.34 (d, J=7Hz, 1H), 6.90 - 7.15 (m, 3H), 3.58 (dd, J=10Hz, 4Hz, 1H), 3.39 (dd, J=10Hz, 7Hz, 1H), 3.05 - 3.20 (m, 1H), 2.90 (dd, J=14Hz, 6Hz, 1H), 2.69 (dd, J=14Hz, 7Hz, 1H) |
| 1-53 | L-o-fluoro-phenylalanine | (2S)-2-amino-3-(2-fluoro-phenyl)propanol | 1 | 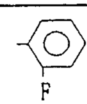 | 6.90 - 7.30 (m, 4H), 3.66 (dd, J=10Hz, 4Hz, 1H), 3.78 (dd, J=10Hz, 7Hz, 1H), 3.05 - 3.20 (m, 1H), 2.81 (dd, J=13Hz, 5Hz, 1H), 2.63 (dd, J=13Hz, 8Hz, 1H), 2.02 (brs, 3H) |

## Reference Example 1-54

Synthesis of (4S)-2,2-dimethyl-3-(t-butoxycarbonyl)-4-(2-hydroxyethyl)-1,3-oxazolidine (Reference Compound No. 1-54):

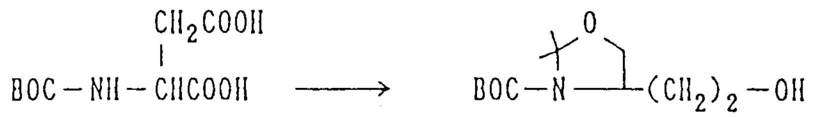

40.1 g (0.4 mol) of potassium hydrogencarbonate was added to an anhydrous N,N'-dimethylformamide solution containing 23.4 g (0.1 mol) of t-butoxycarbonyl-L-aspartic acid. The above mixture was stirred at room temperature for one hour. Furthermore, the reaction mixture was stirred overnight at room temperature after the dropwise addition of 31.1 ml (0.5 mol) of methyl iodide. Water was then added to the reaction mixture, followed by the extraction with ethyl acetate. The resultant organic extract layer was successively washed with 1 N hydrochloric acid and a saturated aqueous solution of sodium chloride, and dried over anhydrous sodium sulfate. The solvent was distilled away under reduced pressure, whereby 25.8 g of a dimethyl ester was obtained in a yield of 99%.

A tetrahydrofuran solution containing 25.7 g (98.4 mmol) of the above prepared dimethyl ester was added to a tetrahydrofuran solution containing 4.3 g (196.8 mmol) of lithium borohydride. To this reaction mixture, 50 ml of methanol was further added dropwise. After stirring for two hours, water was added to the reaction mixture and the solvent was distilled away therefrom under reduced pressure. To the residue thus obtained, 1 N hydrochloric acid was added. The mixture was extracted with chloroform and dried over anhydrous sodium sulfate. The solvent was distilled away under reduced pressure, whereby 18.5 g of a diol was obtained in a yield of 92%.

83 ml (675 mmol) of 2,2-dimethoxypropane and 1.28 g (6.75 mmol) of p-toluene sulfonic acid hydrate were added to a methylene chloride solution containing 27.6 g (135 mmol) of the above prepared diol. The reaction mixture was stirred overnight at room temperature. The reaction mixture was washed with a saturated solution of sodium hydrogencarbonate and dried over anhydrous sodium sulfate. The solvent was distilled away under reduced pressure. The thus obtained residue was chromatographed on a silica gel column for purification, whereby 19.9 g of the captioned Reference Compound No. 1-54 was obtained in a yield of 60%.

NMR (δ, $CDCl_3$): 4.18 - 4.27 (m, 1H), 3.99 - 4.04 (m, 1H), 3.50 - 3.71 (m, 3H), 2.70 - 3.00 (brs, 1H), 1.70 - 1.90 (m, 2H), 1.55 (s, 6H), 1.50 (s, 9H)

**Reference Example 1-55**

Synthesis of (4S)-2,2-dimethyl-3-(t-butoxycarbonyl)-4-(3-hydroxypropyl)-1,3-oxazolidine (Reference Compound No. 1-55):

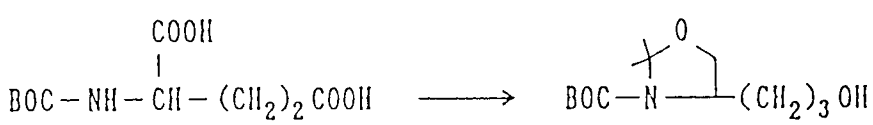

The reaction procedure in Reference Example 1-54 was repeated except that the t-butoxycarbonyl-L-aspartic acid used in Reference Example 1-54 was replaced by the t-butoxycarbonyl-L-glutamic acid, whereby the captioned Reference Compound No. 1-55 was obtained.

NMR (δ, $CDCl_3$): 3.55 - 4.02 (m, 5H), 2.19 (s, 1H), 1.35 - 2.00 (m, 19H)

**Reference Example 1-56**

Synthesis of (2S)-2-amino-5-phenylpentanol (Reference Compound No. 1-56):

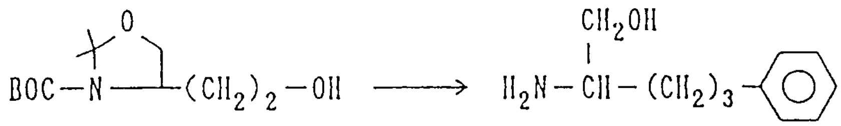

A methylene chloride solution containing 4.1 ml (46.8 mmol) of oxalyl chloride was cooled to -78 ° C. To this solution, 6.84 ml (97.2 mmol) of dimethyl sulfoxide was added dropwise with stirring. After one hour, a methylene chloride solution containing 9.82 g (40 mmol) of the Reference Compound No. 1-54 synthesized in Reference Example 1-54 was added to the above solution. This reaction solution was further stirred for 3 hours, followed by addition of 27.9 ml (200 mmol) of triethylamine. The reaction mixture was further stirred for 30 minutes. After the temperature of the reaction mixture was raised to room temperature, the reaction mixture was poured into water to separate an organic layer therefrom. The remaining water layer was extracted with methylene chloride, and a mixture of the thus obtained extract layer and the organic layer was washed successively with a 1 N hydrochloric acid solution and a saturated aqueous solution of sodium chloride, and dried over anhydrous sodium sulfate. The solvent was distilled away under reduced pressure. The residue thus obtained was chromatographed on a silica gel column for purification, whereby 7.15 g of (4S)-2,2-dimethyl-3-(t-butoxycarbonyl)-4-(formylmethyl)-1,3-oxazolidine was obtained in a yield of 74%.

NMR ($\delta$, $CDCl_3$): 9.79 (s, 1H), 4.25 - 4.40 (m, 1H), 4.04 - 4.09 (m, 1H), 3.73 (dd, J = 9Hz, J = 2Hz, 1H), 2.82 - 3.10 (m, 1H), 2.62 - 2.80 (m, 1H), 1.61 (s, 3H), 1.55 (m, 3H), 1.48 (s, 9H)

An anhydrous tetrahydrofuran solution containing 3.28 g (29.2 mmol) of potassium tert-butoxide was added dropwise to an anhydrous tetrahydrofuran suspension of 18.98 g (43.8 mmol) of triphenylbenzyl phosphonium bromide at -78 ° C with stirring. The temperature of the reaction solution was raised to room temperature over a period of one hour, and then cooled to -78 ° C. To this solution, an anhydrous tetrahydrofuran containing 7.10 g (29.2 mmol) of the above prepared oxazolidine aldehyde was added dropwise.

After the completion of the dropwise addition, the reaction solution was allowed to warm to room temperature, followed by stirring for one hour. With the addition of a saturated ammonium chloride solution to the above reaction solution, the solvent was distilled away therefrom under reduced pressure. A water layer was extracted with ethyl acetate. The resultant organic extract layer was washed with a saturated aqueous solution of sodium chloride and dried over anhydrous sodium sulfate. The solvent was distilled away under reduced pressure. The residue thus obtained was chromatographed on a silica gel column for purification, whereby 7.5 g of an olefin was obtained in a yield of 82%.

To a methanol solution containing 7.5 g (23.9 mmol) of the above obtained olefin, 2.00 g of a 10% palladium carbon was added. This solution was stirred overnight at room temperature under a stream of hydrogen. The palladium carbon was removed from the solution by filtration, and then the solvent was distilled away from the filtrate under reduced pressure. To a methanol solution containing the thus obtained residue, an ethyl acetate solution containing 4 N hydrogen chloride (4 N HCl - AcOEt) was added with being cooled. The reaction mixture was stirred for one hour. The solvent was distilled away under reduced pressure and the residue thus obtained was dissolved in water and then washed with ethyl acetate. Potassium carbonate was added to the resultant water layer to make the reaction product in the water layer basic. The water layer was extracted with chloroform and dried over anhydrous sodium sulfate. The solvent was distilled away under reduced pressure, whereby 1.34 g of the captioned Reference Compound No. 1-56 was obtained in a yield of 31%.

NMR ($\delta$, $CDCl_3$): 7.26 - 7.31 (m, 2H), 7.16 - 7.20 (m, 3H), 3.57(dd, J = 11Hz, J = 4Hz, 1H), 3.25 (dd, J = 11Hz, J = 8Hz, 1H), 2.80 - 2.88 (m, 1H), 2.60 - 2.66 (m, 2H), 1.82 - 1.90 (m, 3H), 1.60 - 1.80 (m, 2H), 1.40 - 1.50 (m, 1H), 1.24 - 1.37 (m, 1H)

**Reference Example 1-57**

Synthesis of (2S)-2-amino-6-phenylhexanol (Reference Compound No. 1-57):

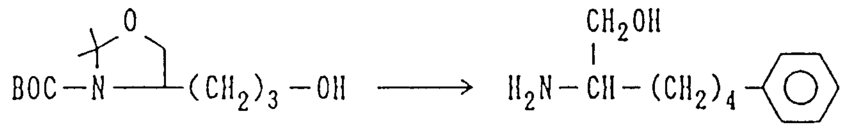

The same reaction procedure as in Reference Example 1-56 was repeated except that the (4S)-2,2-dimethyl-3-(t-butoxycarbonyl)-4-(2-hydroxyethyl)-1,3-oxazolidine used in Reference Example 1-56 was replaced by (4S)-2,2-dimethyl-3-(t-butoxycarbonyl)-4-(3-hydroxypropyl)-1,3-oxazolidine synthesized in Reference Example 1-55, whereby the captioned Reference Compound No. 1-57 was obtained.

NMR (δ, $CDCl_3$): 7.05 - 7.25 (m, 5H), 3.50 (dd, J=10.6Hz, J=3.8Hz, 1H), 3.18 (dd, J=10.6Hz, J=7.8Hz, 1H), 2.65 - 2.80 (m, 1H), 2.55 (t, J=15.2Hz, 2H), 1.75 - 2.00 (m, 3H), 1.10 - 1.65 (m, 6H)

**Reference Example 1-58**

Synthesis of (2S)-2-amino-7-phenylheptanol (Reference Compound No. 1-58):

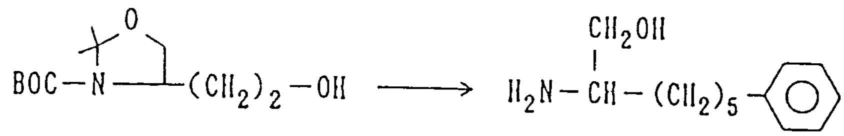

The same reaction procedure as in Reference Example 1-56 was repeated except that, the triphenylbenzyl phosphonium bromide used in Reference Example 1-56 was replaced by triphenyl(3-phenyl)-propylphosphonium bromide, whereby the captioned Reference Compound No. 1-58 was obtained.

NMR (δ, $CDCl_3$): 7.20 - 7.35 (m, 2H), 7.10 - 7.20 (m, 3H), 3.50 - 3.85 (m, 3H), 3.64 (dd, J=10Hz, 3Hz, 1H), 3.35 (dd, J=10Hz, 7Hz, 1H), 2.85 - 3.00 (m, 1H), 2.58 (t, J=7Hz, 2H), 1.50 - 1.80 (m, 2H), 1.20 - 1.55 (m, 6H)

**Reference Example 1-59**

Synthesis of (2R)-2-amino-3(2-fluorobenzylthio)propanol (Reference Compound No. 1-59):

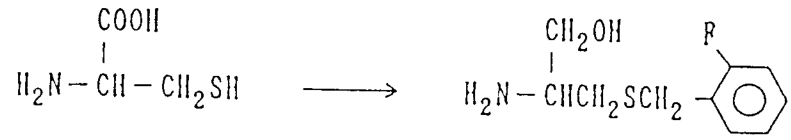

6.9 g (300 mmol) of metallic sodium was dissolved in 500 ml of methanol with stirring. 17.6 g (100 mmol) of a L-cysteine hydrochloride hydrate was added to the above-prepared reaction solution, followed by stirring at room temperature for one hour. 15.0 g (100 mmol) of 2-fluorobenzyl chloride was added dropwise to the reaction mixture, and then the mixture was further stirred overnight. The solvent was distilled away from the reaction mixture under reduced pressure. The residue thus obtained was dissolved in water and washed with diethyl ether. The resulting water layer was made acid (pH=1) by the addition of concentrated hydrochloric acid, so that crystals separated out.

The crystals were separated from the reaction mixture by filtration, washed with water, ethanol and diethyl ether, and dried over under reduced pressure, whereby 16.5 g of L-S-(2-fluorobenzyl)cysteine was obtained in a yield of 72%.

The thus obtained L-S-(2-fluorobenzyl)cysteine was reduced in accordance with the procedure used in

Reference Example 1-46 subsequently, whereby the captioned Reference Compound No. 1-59 was obtained.

NMR (δ, $CDCl_3$): 7.20 - 7.36 (m, 2H), 7.01 - 7.13 (m, 2H), 3.75 (s, 2H), 3.62 (dd, J = 11Hz, J = 5Hz, 1H), 3.38 (dd, J = 11Hz, J = 7Hz, 1H), 2.96 - 3.04 (m, 1H), 2.61 (dd, J = 13Hz, J = 5Hz, 1H), 2.42 (dd, J = 13Hz, J = 8Hz, 1H), 2.00 - 2.10 (m, 3H)

**Reference Example 1-60**

Synthesis of (2R)-2-amino-3-(4-chlorobenzylthio)propanol (Reference Compound No. 1-60):

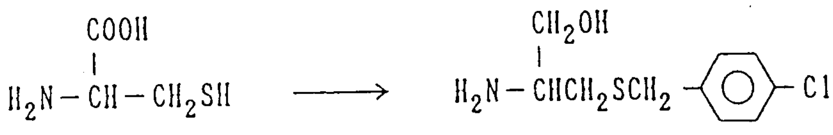

The same reaction procedure as in Reference Example 1-59 was repeated except that the 2-fluorobenzyl chloride used in Reference Example 1-59 was replaced by 4-chlorobenzylchloride, whereby the captioned Reference Compound No. 1-60 was obtained.

NMR (δ, $CDCl_3$): 7.23 - 7.30 (m, 5H), 3.68 (s, 2H), 3.60 (dd, J = 11Hz, J = 4Hz, 1H), 3.38 (dd, J = 11Hz, J = 7Hz, 1H), 2.92 - 3.00 (m, 1H), 2.54 (dd, J = 13Hz, J = 5Hz, 1H), 2.36 (dd, J = 13Hz, J = 8Hz, 1H), 2.16 - 2.28 (m, 3H)

**Reference Example 1-61**

Synthesis of (2S)-2-amino-4-(4-chlorobenzylthio)butanol (Reference Compound No. 1-61):

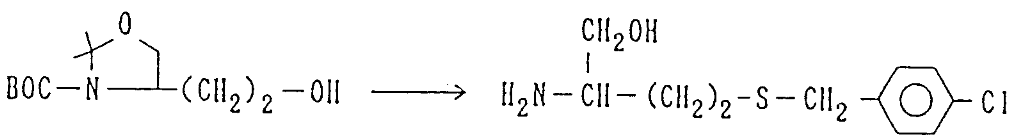

2.77 g (27.4 mmol) of triethylamine was added to an ethyl acetate solution containing 5.6 g (22.8 mmol) of the compound synthesized in Reference Example 1-54. 2.2 ml (27.4 mmol) of methanesulfonyl chloride was further added dropwise to the above-prepared reaction mixture under an ice-cooled condition, followed by stirring for two hours. The reaction mixture was washed with a saturated aqueous solution of sodium chloride and dried over anhydrous sodium sulfate. The solvent was distilled away under reduced pressure, whereby a methanesulfonate was obtained.

1.05 g (26.2 mmol) of sodium hydride was added to an anhydrous dimethylformamide solution containing 3.72 ml (28.6 mmol) of 4-chlorobenzylmercaptan, followed by stirring at room temperature for 30 minutes. With the addition of 7.7 g (23.8 mmol) of the above prepared methanesulfonate, the reaction mixture was further stirred overnight. With the addition of water, the reaction mixture was extracted with ethyl acetate. A resultant organic extract layer was washed with 1 N hydrochloric acid and a saturated aqueous solution of sodium chloride, and dried over anhydrous sodium sulfate. The solvent was distilled away from the reaction mixture under reduced pressure and the residue thus obtained was chromatographed on a silica gel column for purification, whereby 3.95 g of a 4-chlorobenzylthioether was obtained in a yield of 43%.

8 ml of 4 N HCl - AcOEt was added to a methanol solution containing 3.95 g (10.22 mmol) of the above prepared thioether. This reaction mixture was stirred overnight under an ice-cooled condition. The solvent was distilled away from the reaction mixture under reduced pressure, and the residue thus obtained was dissolved in water and then washed with diethyl ether. A resulting aqueous layer was made bacic by the addition of a 10% potassium carbonate solution, and extracted with chloroform. The extract layer was dried over anhydrous sodium sulfate. The solvent was distilled away under reduced pressure, whereby 0.81 g of the captioned Reference Compound No. 1-61 was obtained in a yield of 75%.

NMR (δ, $CDCl_3$): 7.23 - 7.30 (m, 4H), 3.68 (s, 2H), 3.54 (dd, J = 11Hz, J = 4Hz, 1H), 3.27 (dd, J = 11Hz, J = 7Hz, 1H), 2.88 - 2.98 (m, 1H), 2.39 - 2.58 (m, 2H), 1.77 (brs, 3H), 1.64 - 1.74 (m, 1H), 1.45 - 1.55 (m, 1H)

**Reference Examples 1-62 to 1-66**

The same reaction procedure as in Reference Example 1-61 was repeated except that the 4-chlorobenzylmercaptan used in Reference Example 1-61 was replaced by a mercaptan compound or phenol compound with a moiety $R^1$ shown in Table 5, and the compound synthesized in Reference Example 1-54 or Reference Example 1-55 was used, whereby Reference Compounds No. 1-62 to No. 1-66 were respectively obtained as shown in Table 5.

Table 5

$$H_2N-\underset{|}{CH}(CH_2OH)-(CH_2)n-R^1 \qquad \text{(III-b)}$$

| Ref. Ex. | Material | Compound | n | $R^1$ | NMR(δ,$CDCl_3$) |
|---|---|---|---|---|---|
| 1-62 | the same as employed in Reference Example 1-54 | (2S)-2-amino-4-(2-chloro-benzylthio)butanol | 2 | 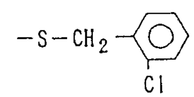 | 7.33 - 7.39 (m, 2H), 7.19 - 7.26 (m, 2H), 3.84 (s, 2H), 3.57 (dd, J=10.69Hz, J=4.01Hz, 1H), 3.29 (dd, J=10.69Hz, J=7.49Hz, 1H), 2.93 - 3.01 (m, 1H), 2.50 - 2.66 (m, 2H), 2.04 (brs, 3H), 1.66 - 1.78 (m, 1H), 1.50 - 1.62 (m, 1H) |
| 1-63 | the same as employed in Reference Example 1-54 | (2S)-2-amino-4-(4-chloro-phenyloxy)butanol | 2 | 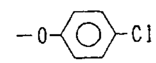 | 7.22 - 7.31 (m, 2H), 6.81 - 6.98 (m, 2H), 4.04 - 4.12 (m, 2H), 3.61 - 3.67 (m, 1H), 3.36 - 3.43 (m, 1H), 3.10 - 3.18 (m, 1H), 1.88 - 2.00 (m, 3H), 1.68 - 1.82 (m, 2H) |
| 1-64 | the same as employed in Reference Exmaple 1-54 | (2S)-2-amino-4-(4-chloro-phenylthio)butanol | 2 | 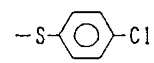 | 7.32 (d, J=9Hz, 2H), 7.27 (d, J=9Hz, 2H), 4.78 - 4.90 (m, 3H), 3.51 (dd, J=11Hz, J=5Hz, 1H), 3.35 (dd, J=11Hz, J=7Hz, 1H), 2.88 (m, 3H), 1.70 - 1.81 (m, 1H), 1.54 - 1.65 (m, 1H) |
| 1-65 | the same as employed in Reference Example 1-55 | (2S)-2-amino-5-(4-chloro-phenyloxy)pentanol•hydro-chloric acid | 3 | 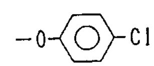 | (Solvent: $CD_3OD$) 7.20 - 7.30 (m, 2H), 6.85 - 6.95 (m, 2H), 4.01 (t, J=6Hz, 2H), 3.80 (dd, J=11Hz, 3Hz, 1H), 3.60 (dd, J=11Hz, 6Hz, 1H), 3.25 - 3.35 (m, 1H), 1.75 - 2.00 (m, 4H) |
| 1-66 | the same as employed in Refernece Example 1-55 | (2S)-2-amino-5-(4-chloro-phenylthio)pentanol | 3 | 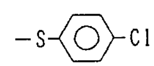 | 7.22 - 7.35 (m, 5H), 3.49 (dd, J=10Hz, 4.5Hz, 1H), 2.94 (t, J=7Hz, 2H), 2.65 - 2.80 (m, 1H), 1.50 - 1.85 (m, 3H), 1.30 - 1.50 (m, 1H) |

**Reference Example 1-67**

Synthesis of (2R,2S)-2-amino-5-phenyl-4-pentenoic acid ethyl ester hydrochloride (Reference Compound No. 1-67):

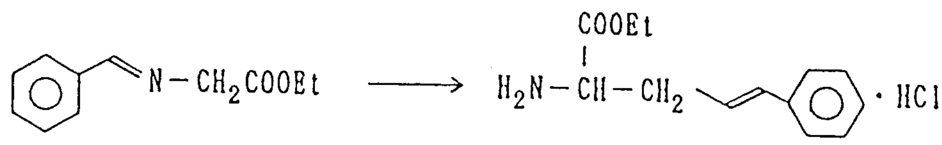

9.32 ml (15.0 mmol) of a hexane solution containing n-butyl lithium was added dropwise to an anhydrous tetrahydrofuran solution of 2.31 ml (16.5 mmol) of diisopropylamine at -78°C in a stream of nitrogen. After the completion of dropping, the reaction mixture was stirred at room temperature for one hour. To this reaction mixture, an anhydrous tetrahydrofuran solution containing 2.868 g (15.0 mmol) of benzylideneglycine ethyl ester was added at -78°C. After stirring for one hour, 1.82 ml (15.0 mmol) of cinnamyl bromide was added to the reaction mixture. The reaction mixture was stirred at -78°C for 4 hours and then at room temperature overnight.

After putting into 150 ml of a cooled saturated ammonium chloride solution, the reaction mixture was extracted with ether and dried over anhydrous sodium sulfate. The solvent was distilled away from the reaction mixture under reduced pressure. To the residue thus obtained, 40 ml of 5% hydrochloric acid was added, followed by stirring for two hours. Furthermore, with the addition of 40 ml of 5% hydrochloric acid, the residue was washed with ether. The resulting water layer was made basic with a sodium hydrogencarbonate solution thereto and extracted with ethyl acetate. The extract layer was washed with a saturated aqueous solution of sodium chloride and dried over anhydrous sodium sulfate. With the addition of 5.5 ml of a mixture of 4 N HCl - AcOEt thereto, the solvent was distilled away under reduced pressure, whereby 2.54 g of the captioned Reference Compound No. 1-67 was obtained in a yield of 66%.

NMR (δ, $CDCl_3$): 8.50 - 8.90 (m, 2H), 7.11 - 7.50 (m, 5H), 6.54 (d, J=15.6Hz, 6.22 (dt, J=15.8Hz, J=8.2Hz, 1H), 4.00 - 4.25 (m, 3H), 2.80 - 3.10 (m, 2H), 1.18 (t, J=7.1Hz, 3H)

**Example 1-1**

Synthesis of (2R,2S)-1-benzyloxycarbonyl-azetidine-2-carboxylic acid-(1S)-(1-formyl-3-methylthio)-propylamide (Compound No. 1-1):

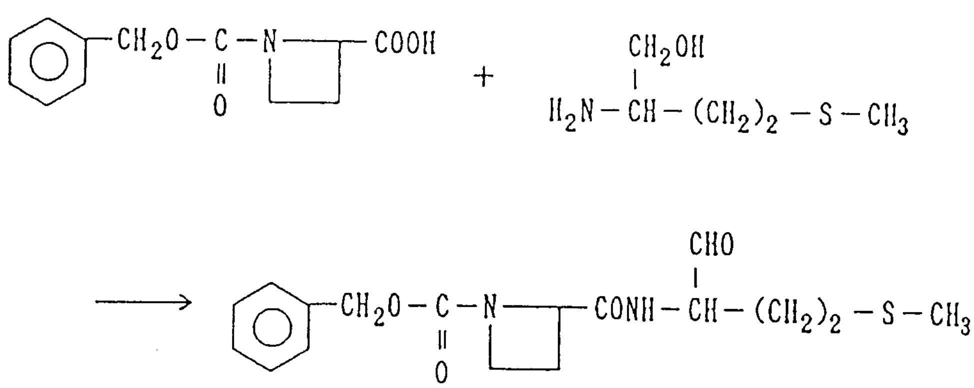

100 ml of a methylene chloride solution containing 1.27 g (5.4 mmol) of Reference Compound No. 1-1 synthesized in Reference Example 1-1, 0.826 g (5.4 mmol) of a N-hydroxybenzotriazole hydrate, 0.546 g (5.4 mmol) of triethylamine and 0.73 g (5.4 mmol) of Reference Compound No. 1-46 synthesized in Reference Example 1-46 was cooled in an ice bath containing sodium chloride. 20 ml of a methylene chloride solution containing 1.22 (5.94 mmol) of N,N'-dicyclohexylcarbodiimide was added dropwise to the above-prepared reaction mixture, followed by stirring for 18 hours.

Insoluble materials were removed from the reaction mixture by filtration. The filtrate was washed successively with 1 N hydrochloric acid, a saturated aqueous solution of sodium hydrogencarbonate, and a saturated aqueous solution of sodium chloride. An organic layer and a water layer in the thus washed filtrate were separated. The resulting water layer was extracted with methylene chloride again to obtain an another organic layer. The thus extracted organic layer and the first obtained organic layer were mixed, dried over

anhydrous sodium sulfate and concentrated under reduced pressure. The residue thus obtained was chromatographed on a silica gel column for purification, whereby 1.28 g of (2R,S)-1-benzyloxycarbonyl-azetidine-2-carboxylic acid-(1S)-(hydroxymethyl-3-methylthio)-propylamide was obtained in a yield of 67%.

10 ml of an anhydrous dimethyl sulfoxide of 1.42 g (8.95 mmol) of pyridine•sulfur trioxide complex was added dropwise to 20 ml of an anhydrous dimethyl sulfoxide solution containing the above prepared (2R,S)-1-benzyloxycarbonyl-azetidine-2-carboxylic acid-(1S)-(hydroxymethyl-3-methylthio)propylamide and 0.905 (8.75 mmol) of triethylamine. The reaction mixture was then stirred for 30 minutes and added to an iced water. The reaction mixture was extracted with ethyl acetate four times. The resultant extract organic layer was washed successively with a 10% citric acid solution, water, a saturated aqueous solution of sodium hydrogencarbonate, and a saturated aqueous solution of sodium chloride, dried over anhydrous sodium sulfate, and then concentrated under reduced pressure. The residue thus obtained was chromatographed on a silica gel column for purification, whereby 1.03 g of the captioned Compound No. 1-1 in the state of a mixture of diastereomers was obtained as an oily material in a yield of 80%.

NMR (δ, $CDCl_3$): 9.59 - 9.62 (m, 1H), 7.50 - 8.10 (bs, 1H), 7.30 - 7.45 (m, 5H), 5.05 - 5.20 (m, 2H), 4.70 - 4.85 (m, 1H), 4.45 - 4.65 (m, 1H), 3.85 - 4.10 (m, 2H), 2.35 - 2.70 (m, 4H), 1.90 - 2.30 (m, 5H)

$R_f$ values: 0.34 (Developing Solvent A: ethyl acetate)
0.16 (Developing Solvent B: mixture of methylene chloride and acetone at a mixing ratio of 10:1)

**Examples 1-2 to 1-21**

The same reaction procedure as in Example 1-1 was repeated except that the carboxylic acid derivative (Reference Compound No. 1-1 synthesized in Reference Example 1-1) and the amine derivative (Reference Compound No. 1-46 synthesized in Reference Example 1-46) used in Example 1-1 were respectively replaced by a carboxylic acid derivative (Material 1) and an amine derivative (Material 2) shown in Table 6, whereby Compounds No. 1-2 to No. 1-21 (cyclic carboxylic acid amide derivatives) shown in Table 6 were obtained.

Furthermore, Table 7 shows the yield, the melting point or state, the NMR analysis data and the $R_f$ values obtained by TLC analysis of each of the thus prepared cyclic carboxylic acid amide derivatives. The developing solvent A used in the TLC analysis is ethyl acetate and the developing solvent B is a mixture of methylene chloride and acetone at a mixing ratio of 10:1.

Table 6

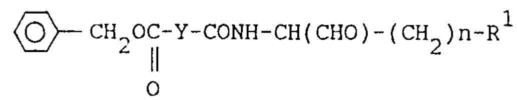 (I-a)

| Ex. | Material 1 (*) | Material 2 (*) | Y | n | $R^1$ | Compound |
|---|---|---|---|---|---|---|
| 1-2 | cbz-L-proline | 1-46 | 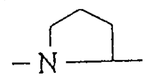 | 2 | $-S-CH_3$ | (2S)-1-benzyloxycarbonylpyrrolidine-2-carboxylic acid-(1S)-(1-formyl-3-methylthio)propylamide |
| 1-3 | 1-2 | 1-46 | 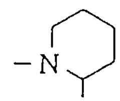 | 2 | $-S-CH_3$ | (2R, 2S)-1-benzyloxycarbonylpiperidine-2-carboxylic acid-(1S)-(1-formyl-3-methylthio)propylamide |
| 1-4-a | 1-3 | 1-46 | 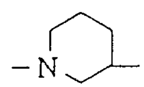 | 2 | $-S-CH_3$ | 1-benzyloxycarbonylpiperidine-3-carboxylic acid-(1S)-(1-formyl-3-methylthio)propylamide |
| 1-4-b | 1-3 | 1-46 | 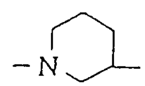 | 2 | $-S-CH_3$ | the same as the diastereomer compound employed in Example 1-4-a |
| 1-5 | 1-4 | 1-46 | 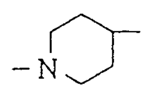 | 2 | $-S-CH_3$ | 1-benzyloxycarbonylpiperidine-4-carboxylic acid-(1S)-(1-formyl-3-methylthio)propylamide |
| 1-6 | 1-4 | 1-47 | 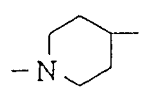 | 1 | 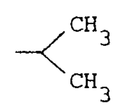 | 1-benzyloxycarbonylpiperidine-4-carboxylic acid-(1S)-(3-methyl)butylamide |

(*) Each material is indicated by the Reference Example Number in which it is synthesized.

Table 6

| Ex. | Material 1 (*) | Material 2 (*) | Y | n | $R^1$ | Compound |
|---|---|---|---|---|---|---|
| 1-7 | 1-4 | 1-48 | 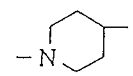 | 1 | 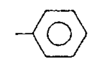 | 1-benzyloxycarbonylpiperidine-4-carboxylic acid-(1S)-(1-formyl-2-phenyl)ethylamide |
| 1-8-a | 1-3 | 1-48 | 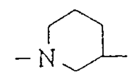 | 1 | 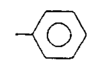 | 1-benzyloxycarbonylpiperidine-3-carboxylic acid-(1S)-(1-formyl-2-phenyl)ethylamide |
| 1-8-b | 1-3 | 1-48 | 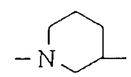 | 1 | 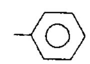 | the same as the diastereomer compound employed in Example 1-8-a |
| 1-9 | 1-2 | 1-48 | 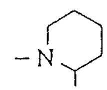 | 1 | 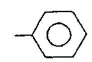 | (2R, 2S)-1-benzyloxycarbonylpiperidine-2-carboxylic acid-(1-formyl-2-phenyl)ethylamide |
| 1-10 | cbz-L-proline | 1-48 | 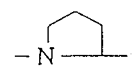 | 1 | 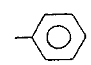 | (2S)-1-benzyloxypyrrolidine-2-carboxylic acid-(1S)-(1-formyl-2-phenyl)ethylamide |
| 1-11 | 1-1 | 1-48 | 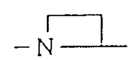 | 1 | 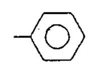 | (2R, 2S)-1-benzyloxycarbonylazetidine-2-carboxylic acid-(1S)-(1-formyl-2-phenyl)ethylamide |
| 1-12 | 1-2 | 1-59 | 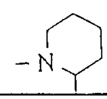 | 1 | 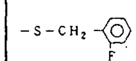 | (2R, 2S)-1-benzyloxycarbonylpiperidine-2-carboxylic acid-(1R)-[1-formyl-2-(2-fluorobenzyl)thio]ethylamide |

(*) Each material is indicated by the Reference Example Number in which it is synthesized.

Table 6

| Ex. | Material 1 (*) | Material 2 (*) | Y | n | $R^1$ | Compound |
|---|---|---|---|---|---|---|
| 1-13 -a | 1-3 | 1-59 | 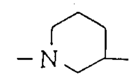 | 1 | $-S-CH_2-$ 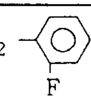 | 1-benzyloxycarbonylpiperidine-3-carboxylic acid (1R)-[1-formyl-2-(2-fluorobenzylthio)]ethylamide |
| 1-13 -b | 1-3 | 1-59 | 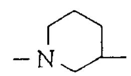 | 1 | $-S-CH_2-$ 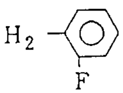 | the same as the diastereomer compound employed in Example 1-13-a |
| 1-14 | 1-4 | 1-59 | 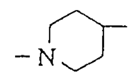 | 1 | $-S-CH_2-$ 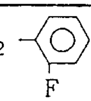 | 1-benzyloxycarbonylpiperidine-4-carboxylic acid-(1R)-[1-formyl-2-(2-fluorobenzylthio)]ethylamide |
| 1-15 | cbz-L-proline | 1-59 | 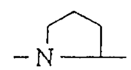 | 1 | $-S-CH_2-$ 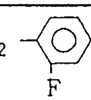 | (2S)-1-benzyloxycarbonylpyrrolidine-2-carboxylic acid-(1R)-[1-formyl-2-(2-fluorobenzylthio)]ethylamide |
| 1-16 | 1-1 | 1-59 | 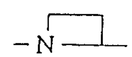 | 1 | $-S-CH_2-$ 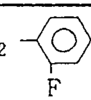 | (2R, 2S)-1-benzyloxycarbonylazetidine-2-carboxylic acid-(1R)-[1-formyl-2-(2-fluorobenzylthio)]-ethylamide |
| 1-17 | 1-2 | 1-50 | 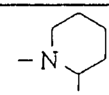 | 2 | 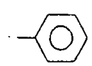 | (2R, 2S)-1-benzyloxycarbonylpiperidine-2-carboxylic acid-(1S)-(1-formyl-3-phenyl)propylamide |

(*) Each material is indicated by the Reference Example Number in which it is synthesized.

Table 6

| Ex. | Material 1 (*) | Material 2 (*) | Y | n | $R^1$ | Compound |
|---|---|---|---|---|---|---|
| 1-18 -a | 1-3 | 1-50 | 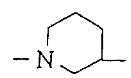 | 2 | 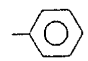 | 1-benzyloxycarbonylpiperidine-3-carboxylic acid-(1S)-(1-formyl-3-phenyl)propylamide |
| 1-18 -b | 1-3 | 1-50 | 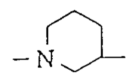 | 2 | 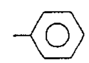 | the same as the diastereomer compound employed in Example 1-18-a |
| 1-19 | 1-4 | 1-50 | 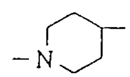 | 2 | 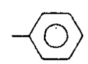 | 1-benzyloxycarbonylpiperidine-4-carboxylic acid-(1S)-(1-formyl-3-phenyl)propylamide |
| 1-20 | cbz-L-proline | 1-50 | 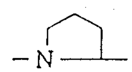 | 2 | 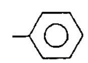 | (2S)-1-benzyloxycarbonylpyrrolidine-2-carboxylic acid-(1S)-(1-formyl-3-phenyl)propylamide |
| 1-21 | 1-1 | 1-50 | 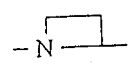 | 2 | 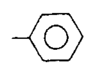 | (2R, 2S)-1-benzyloxycarbonylazetidine-2-carboxylic acid-(1S)-(1-formyl-3-phenyl)propylamide |

(*) Each material is indicated by the Reference Example Number in which it is synthesized.

Table 7

| Ex. | Yield (%) | Melting Point (°C) or State | NMR (δ, $CDCl_3$) | $R_f$ Values |
|---|---|---|---|---|
| 1-2 | 80 | oily | 9.60 - 9.80 (m, 1H), 7.30 - 7.50 (m, 5H) 5.17 (s, 2H), 4.30 - 4.60 (m, 2H), 3.35 - 3.70 (m, 2H), 1.80 - 2.60 (m, 8H), 2.03 (s, 3H) | A: 0.37 B: 0.16 |
| 1-3 | 65 | oily | 9.58 - 9.60 (m, 1H), 7.30 - 7.45 (m, 5H) 6.60 - 6.90 (m, 1H), 5.10 - 5.30 (m, 2H) 4.80 - 4.75 (m, 1H), 4.50 - 4.70 (m, 1H) 4.05 - 4.30 (m, 1H), 2.80 - 3.10 (m, 1H) 2.40 - 2.60 (m, 2H), 2.15 - 2.35 (m, 2H) 2.04 (s, 3H), 1.35 - 1.80 (m, 6H) | A: 0.52 B: 0.17 |
| 1-4 -a | 25 | oily | 9.50 - 9.61 (m, 1H), 7.30 - 7.40 (m, 5H) 6.20 - 7.00 (m, 1H), 5.05 - 5.20 (m, 2H) 4.50 - 4.60 (m, 1H), 2.70 - 4.30 (m, 4H) 2.15 - 2.60 (m, 4H), 2.08 (s, 3H), 1.40 - 2.05 (m, 5H) | A: 0.41 B: 0.20 |
| 1-4 -b | 33 | oily | 9.50 - 9.65 (m, 1H), 7.25 - 7.50 (m, 5H) 6.10 - 6.90 (m, 1H), 5.10 - 5.20 (m, 2H) 4.50 - 4.60 (m, 1H), 2.80 - 4.25 (m, 4H) 1.40 - 2.65 (m, 9H), 2.07 (s, 3H) | A: 0.41 B: 0.17 |
| 1-5 | 46 | amorphous | 9.63 (s, 1H), 7.30 - 7.40 (m, 5H), 6.34 (d, J=6Hz, 1H), 5.12 (s, 2H), 4.61 (q, J=7Hz, 1H), 4.10 - 4.35 (m, 2H), 2.75 - 3.95 (m, 2H), 2.15 - 2.65 (m, 4H), 2.08 (s, 3H), 1.55 - 2.10 (5H) | A: 0.33 B: 0.12 |
| 1-6 | 50 | amorphous | 9.58 (s, 1H), 7.25 - 7.44 (m, 5H), 5.89 - 6.03 (m, 1H), 5.13 (s, 2H), 4.56 - 4.67 (m, 1H), 4.10 - 4.32 (m, 2H), 2.72 - 2.97 (m, 2H), 2.35 (tt, J=11.3Hz, J=3.8Hz, 1H), 1.35 - 1.95 (m, 7H), 0.97 (d, J=6.2Hz, 3H), 0.96 (d, J=6.4Hz, 3H) | A: 0.40 B: 0.18 |
| 1-7 | 46 | 118.1 to 124.5°C | 9.65 (m, 1H), 7.01 - 7.46 (m, 10H), 5.92 - 6.06 (m, 1H), 5.12 (s, 2H), 4.71 - 4.81 (m, 1H), 4.04 - 4.30 (m, 2H), 3.19 (d, J=6.5Hz, 2H), 2.70 - 2.93 (m, 2H), 2.28 (tt, J=11.4Hz, J=3.9Hz, 1H), 1.45 - 1.89 (m, 4H) | A: 0.37 B: 0.13 |

Table 7

| Ex. | Yield | Melting Point (°C) or State | NMR (δ, $CDCl_3$) | $R_f$ Values |
|---|---|---|---|---|
| 1-8 -a | 32 | 124.9 to 130.3°C | 9.59 (s, 1H), 7.24 - 7.36 (m, 8H), 7.14 (m, 2H), 6.50 (brs, 1H), 5.12 (s, 2H), 4.69 (q, J=6Hz, 1H), 3.70 - 4.20 (m, 2H) 3.06 - 3.20 (m, 4H), 2.23 - 2.38 (m, 1H), 1.72 - 1.90 (m, 1H), 1.67 (m, 2H), 1.34 - 1.65 (m, 1H) | A: 0.43 B: 0.13 |
| 1-8 -a | 38 | oily | 9.61 (s, 1H), 7.20 - 7.40 (m, 8H), 7.13 (m, 2H), 6.40 (brs, 1H), 5.11 (s, 2H), 4.65 - 4.75 (m, 1H), 3.80 - 4.10 (m, 2H) 3.06 - 3.20 (m, 4H), 2.22 - 2.39 (m, 1H) 1.70 - 1.95 (m, 1H), 1.62 (m, 2H), 1.37 - 1.55 (m, 1H) | A: 0.42 B: 0.15 |
| 1-9 | 50 | oily | 9.61 (s, 1H), 7.20 - 7.40 (m, 8H), 7.10 (d, J=7Hz, 2H), 6.55 (brs, 1H), 5.14 (s, 2H), 4.82 (m, 1H), 4.64 - 4.72 (m, 1H), 3.75 - 4.20 (m, 1H), 2.93 - 3.25 (m, 1H), 2.75 - 2.93 (m, 1H), 2.20 - 2.50 (m, 1H), 1.23 - 1.75 (m, 6H) | A: 0.54 B: 0.23 |
| 1-10 | 78 | 70.8 to 74.7°C | 9.61 (s, 1H), 7.10 - 7.45 (m, 8H), 7.09 (brs, 2H), 6.39 (brs, 1H), 5.11 (q, J= 12Hz, 1H), 4.64 (m, 1H), 4.36 (m, 1H), 3.27 - 3.55 (m, 2H), 2.90 - 3.25 (m, 2H) 1.50 - 2.40 (m, 4H) | A: 0.41 B: 0.14 |
| 1-11 | 51 | oily | 9.61 (s, 1H), 7.10 - 7.40 (m, 10H), 5.09 (s, 2H), 4.65 - 4.74 (m, 2H), 3.87 - 4.10 (m, 1H), 3.79 (q, J=8Hz, 1H), 3.18 (dd, J=14Hz, J=6Hz, 1H), 3.02 (dd, J= 14Hz, J=7Hz, 1H), 2.45 (m, 2H), 1.70 (m, 1H) | A: 0.36 B: 0.14 |
| 1-12 | 66 | oily | 9.50 - 9.55 (m, 1H), 7.20 - 7.45 (m, 7H) 7.00 - 7.15 (m, 2H), 6.50 - 7.00 (bs, 1H), 5.18 (s, 2H), 4.80 - 5.00 (m, 1H), 4.50 - 4.65 (m, 1H), 4.00 - 4.30 (m, 1H) 3.65 - 3.80 (m, 2H), 2.70 - 3.10 (m, 3H) 2.25 - 2.35 (m, 1H), 1.30 - 1.90 (m, 5H) | A: 0.56 B: 0.36 |

Table 7

| Ex. | Yield | Melting Point (°C) or State | NMR (δ, $CDCl_3$) | $R_f$ Values |
|---|---|---|---|---|
| 1-13 -a | 27 | amorphous | 9.45 - 9.60 (m, 1H), 7.20 - 7.45 (m, 7H) 7.00 - 7.15 (m, 2H), 6.10 - 6.90 (m, 1H) 5.05 - 5.20 (m, 2H), 4.45 - 4.60 (m, 1H) 3.80 - 4.25 (m, 2H), 3.75 (s, 2H), 3.00 - 3.40 (m, 2H), 2.70 - 3.00 (m, 2H), 2.20 - 2.50 (m, 1H), 1.40 - 2.00 (m, 4H) | A: 0.46 B: 0.17 |
| 1-13 -b | 30 | oily | 9.45 - 9.60 (m, 1H), 7.20 - 7.45 (m, 7H) 7.00 - 7.15 (m, 2H), 6.20 - 7.00 (m, 1H) 5.00 - 5.20 (m, 2H), 4.45 - 4.60 (m, 1H) 3.80 - 4.20 (m, 2H), 3.74 (d, J=3Hz, 2H) 2.90 - 3.40 (m, 2H), 2.70 - 3.00 (m, 2H) 2.25 - 3.45 (m, 1H), 1.40 - 2.00 (m, 4H) | A: 0.45 B: 0.17 |
| 1-14 | 46 | amorphous | 9.50 (s, 1H), 7.20 - 7.45 (m, 7H), 7.00 - 7.15 (m, 2H), 6.25 - 6.45 (m, 1H), 5.13 (s, 2H), 4.63 (q, J=6Hz, 1H), 4.10 - 4.30 (m, 2H), 3.76 (s, 2H), 2.75 - 3.05 (m, 4H), 2.34 (tt, J=10Hz, 4Hz, 1H) 1.50 - 1.90 (m, 4H) | A: 0.39 B: 0.11 |
| 1-15 | 44 | oily | 9.30 - 9.60 (m, 1H), 7.20 - 7.55 (m, 8H) 7.00 - 7.17 (m, 2H), 5.05 - 5.57 (m, 2H) 4.30 - 4.60 (m, 2H), 3.35 - 3.85 (m, 4H) 2.70 - 3.00 (m, 2H), 1.85 - 2.30 (m, 4H) | A: 0.42 B: 0.21 |
| 1-16 | 18 | oily | 9.50 - 9.65 (m, 1H), 7.20 - 7.40 (m, 8H) 7.00 - 7.20 (m, 2H), 5.05 - 5.25 (m, 2H) 4.70 - 4.85 (m, 1H), 4.50 - 4.60 (m, 1H) 3.65 - 4.10 (m, 4H), 2.80 - 3.00 (m, 2H) 2.50 - 2.70 (m, 2H) | A: 0.33 B: 0.14 |
| 1-17 | 59 | oily | 9.50 (s, 1H), 7.05 - 7.45 (m, 10H), 6.30 - 6.80 (m, 1H), 5.21 (m, 2H), 4.88 (m, 2H), 4.35 - 4.60 (m, 1H), 4.00 - 4.30 (m, 1H), 2.80 - 3.08 (m, 1H), 2.63 (t, J=7.8Hz, 2H), 2.10 - 2.38 (m, 2H), 1.30 - 2.00 (m, 6H) | A: 0.54 B: 0.40 |
| 1-18 -a | 14 | amorphous | 9.47 (s, 1H), 7.00 - 7.45 (m, 10H), 5.90 - 6.80 (m, 1H), 5.13 (s, 2H), 4.36 - 4.60 (m, 1H), 1.00 - 4.30 (m, 13H) | A: 0.53 B: 0.23 |

Table 7

| Ex. | Yield | Melting Point (°C) or State | NMR (δ, $CDCl_3$) | $R_f$ Values |
|---|---|---|---|---|
| 1-18 -b | 13 | amorphous | 9.50 (s, 1H), 7.12 - 7.44 (m, 10H), 5.80 - 6.70 (m, 1H), 5.06 - 5.21 (m, 2H), 4.43 - 4.59 (m, 1H), 2.75 - 4.24 (m, 4H) 2.66 (t, J=7.7Hz, 2H), 1.40 - 2.55 (m, 7H) | A: 0.48 B: 0.24 |
| 1-19 | 5.8 | amorphous | 9.53 (s, 1H), 7.12 - 7.41 (m, 10H), 6.00 - 6.13 (m, 1H), 5.13 (s, 2H), 4.54 - 4.64 (m, 1H), 4.07 - 4.33 (m, 2H), 2.50 - 2.95 (m, 4H), 2.21 - 2.39 (m, 2H), 1.52 - 2.08 (m, 5H) | A: 0.41 B: 0.14 |
| 1-20 | 89 | amorphous | 9.20 - 9.60 (m, 1H), 7.00 - 7.50 (m, 10H), 6.35 - 6.55 (m, 1H), 5.18 (s, 2H), 4.25 - 4.55 (m, 2H), 3.35 - 3.70 (m, 2H) 1.50 - 2.85 (m, 8H) | A: 0.42 B: 0.26 |
| 1-21 | 49 | oily | 9.52 (s, 0.5H), 9.50 (s, 0.5H), 7.00 - 7.60 (m, 11H), 5.05 - 5.25 (m, 2H), 4.65 - 4.88 (m, 1H), 4.30 - 4.60 (m, 1H), 3.80 - 4.12 (m, 2H), 2.66 (t, J=7.8Hz, 2H), 1.40 - 2.85 (m, 4H) | A: 0.36 B: 0.25 |

**Example 1-22**

Synthesis of 1-ethoxycarbonylpiperidine-4-carboxylic acid-(1S)-(1-formyl-2-phenyl)ethylamide (Compound No. 1-22):

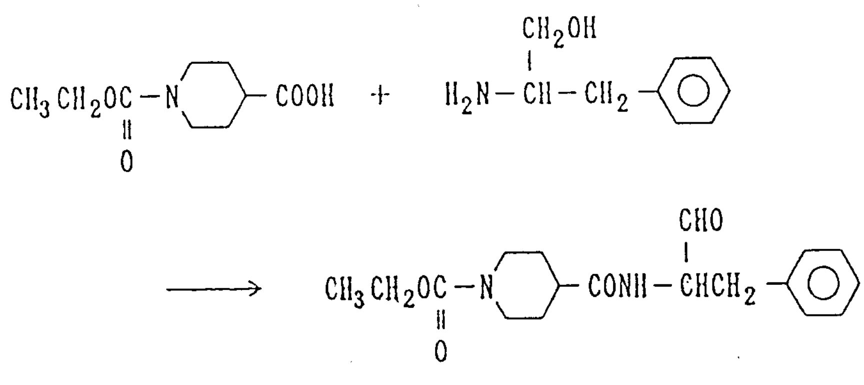

50 ml of a chloroform solution containing 0.84 g (4.17 mmol) of 1-ethoxycarbonylpiperidine-4-carboxylic acid synthesized in Reference Example 1-5 was cooled in an ice bath containing sodium chloride. 0.61 ml (4.36 mmol) of triethylamine and 0.38 ml (3.97 mmol) of ethyl chlorocarbonate were successively added to the above solution. After stirring for 30 minutes, a chloroform solution containing 0.6 g (3.97 mmol) of (2S)-2-amino-3-phenylpropanol synthesized in Reference Example 1-48 was added to the above prepared reaction mixture. The reaction mixture was stirred for one hour at -10 °C and further stirred overnight at

room temperature.

The reaction mixture was washed successively with a 1 N hydrochloric acid solution, a saturated aqueous solution of sodium chloride, a saturated aqueous solution of sodium hydrogencarbonate and then a saturated aqueous solution of sodium chloride. The solvent was distilled away under reduced pressure. The residue thus obtained was crystallized in isopropyl ether and then the crystals were separated by filtration.

0.95 g (2.84 mmol) of the thus obtained crystals was dissolved in 10 ml of dimethyl sulfoxide, 1.60 ml (11.4 mmol) of triethylamine was added thereto. Furthermore, 10 ml of a dimethyl sulfoxide solution in which 1.81 g (11.4 mmol) of pyridine sulfur trioxide was added dropwise to the above reaction mixture. After stirring for one hour, the reaction mixture was poured into 10 ml of iced water and extracted with ethyl acetate. The extract layer was washed successively with a 10% cirtic acid solution, a saturated aqueous solution of sodium chloride, a saturated aqueous solution of sodium hydrogencarbonate and a saturated aqueous solution of sodium chloride. The resultant organic extract layer was dried over anhydrous sodium sulfate and the solvent was distilled away under reduced pressure. The residue thus obtained was chromatographed on a silica gel column for purification, whereby 0.53 g of the captioned Compound No. 1-22 was obtained as crystals in a yield of 41%.

| | |
|---|---|
| Melting Point (°C): | 74.1 to 77.8 |
| NMR (δ, $CDCl_3$): | 9.65 (s, 1H), 7.09 - 7.37 (m, 5H), 5.95 - 6.14 (m, 1H), 4.70 - 4.82 (m, 1H), 4.13 (q, J=7.1Hz, 2H), 4.00 - 4.26 (m, 2H), 3.19 (d, J=6.4Hz, 2H), 2.65 - 2.90 (m, 2H), 2.28 (tt, J=11.4Hz, J=3.9Hz, 1H), 1.43 - 1.86 (m, 4H), 1.26 (t, J=7.1Hz, 3H) |
| $R_f$ values: | 0.13 (B) |
| | 0.31 (A) |

**Examples 1-23 to 1-90**

The same reaction procedure as in Example 1-22 was repeated except that the carboxylic acid derivative (the compound synthesized in Reference Example 1-5) and the amine derivative (the compound synthesized in Reference Example 1-48) used in Example 1-22 were respectively replaced by a carboxylic acid derivative (Material 1) and an amine derivative (Material 2) shown in Table 8, whereby Compound No. 1-23 to No. 1-90 (cyclic carboxylic acid amide derivative) shown in Table 8 were obtained.

Table 9 shows the yield, the melting point or state, the NMR analysis data and the $R_f$ values obtained by TLC analysis of each cyclic carboxylic acid amide derivative. The developing solvent A used in the TLC analysis is ethyl acetate and the developing solvent B is a mixture of methylene chloride and acetone at a mixing ratio of 10:1.

Table 8

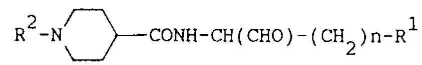

| Ex. | Material 1 (*) | Material 2 (*) | $R^2$ | n | $R^1$ | Compound |
|---|---|---|---|---|---|---|
| 1-23 | 1-6 | 1-48 | 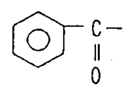 | 1 | 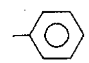 | 1-benzoylpiperidine-4-carboxylic acid-(1S)-(1-formyl-2-phenyl)ethylamide |
| 1-24 | 1-7 | 1-48 | 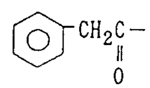 | 1 | 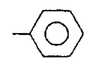 | 1-phenylacetylpiperidine-4-carboxylic acid-(1S)-(1-formyl-2-phenyl)ethylamide |
| 1-25 | 1-8 | 1-48 | 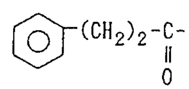 | 1 | 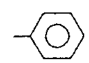 | 1-(3-phenylpropionyl)piperidine-4-carboxylic acid-(1S)-(1-formyl-2-phenyl)ethylamide |
| 1-26 | 1-9 | 1-48 | 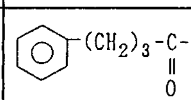 | 1 | 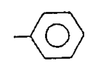 | 1-(4-phenylbutyryl)piperidine-4-carboxylic acid-(1S)-(1-formyl-2-phenyl)ethylamide |
| 1-27 | 1-10 | 1-48 | 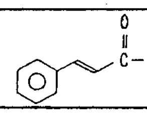 | 1 | 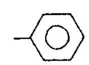 | 1-cinnamoylpiperidine-4-carboxylic acid-(1S)-(1-formyl-2-phenyl)ethylamide |

(*) Each material is indicated by the Reference Example Number in which it is synthesized.

Table 8

| Ex. | Material 1 (*) | Material 2 (*) | $R^2$ | n | $R^1$ | Compound |
|---|---|---|---|---|---|---|
| 1-28 | 1-12 | 1-48 | 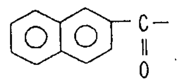 | 1 | 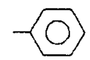 | 1-(2-naphthoyl)piperidine-4-carboxylic acid-(1S)-(1-formyl-2-phenyl)ethylamide |
| 1-29 | 1-27 | 1-48 | 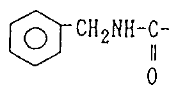 | 1 | 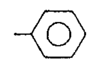 | 1-(N-benzylcarbamoyl)piperidine-4-carboxylic acid (1S)-(1-formyl-2-phenyl)ethylamide |
| 1-30 | 1-29 | 1-48 | 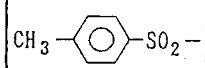 | 1 | 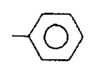 | 1-(4-methylphenylsulfonyl)piperidine-4-carboxylic acid-(1S)-(1-formyl-2-phenyl)ethylamide |
| 1-31 | 1-13 | 1-48 | 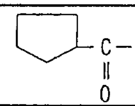 | 1 | 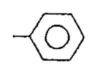 | 1-cyclopentylcarbonylpiperidine-4-carboxylic acid-(1S)-(1-formyl-2-phenyl)ethylamide |
| 1-32 | 1-14 | 1-48 | 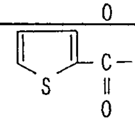 | 1 | 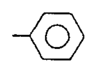 | 1-(2-thienylcarbonyl)piperidine-4-carboxylic acid-(1S)-(1-formyl-2-phenyl)ethylamide |

(*) Each material is indicated by the Reference Example Number in which it is synthesized.

Table 8

| Ex. | Material 1 (*) | Material 2 (*) | $R^2$ | n | $R^1$ | Compound |
|---|---|---|---|---|---|---|
| 1-33 | 1-11 | 1-48 | 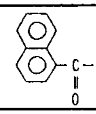 | 1 | 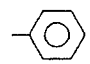 | 1-(1-naphthoyl)piperidine-4-carboxylic acid-(1S)-(1-formyl-2-phenyl)ethylamide |
| 1-34 | 1-30 | 1-48 | 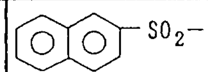 | 1 | 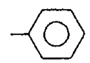 | 1-(2-naphthylsulfonyl)piperidine-4-carboxylic acid-(1S)-(1-formyl-2-phenyl)ethylamide |
| 1-35 | 1-32 | 1-48 | 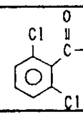 | 1 | 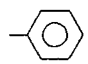 | 1-(2,6-dichlorobenzoyl)piperidine-4-carboxylic acid-(1S)-(1-formyl-2-phenyl)ethylamide |
| 1-36 | 1-17 | 1-48 | 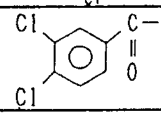 | 1 | 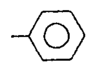 | 1-(3,4-dichlorobenzoyl)piperidine-4-carboxylic acid-(1S)-(1-formyl-2-phenyl)ethylamide |
| 1-37 | 1-15 | 1-48 | 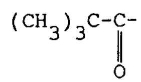 | 1 | 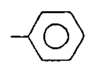 | 1-trimethylacetylpiperidine-4-carboxylic acid-(1S)-(1-formyl-2-phenyl)ethylamide |

(*) Each material is indicated by the Reference Example Number in which it is synthesized.

Table 8

| Ex. | Material 1 (*) | Material 2 (*) | $R^2$ | n | $R^1$ | Compound |
|---|---|---|---|---|---|---|
| 1-38 | 1-31 | 1-48 | 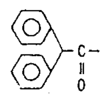 | 1 | 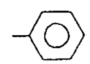 | 1-(diphenylacetyl)piperidine-4-carboxylic acid-(1S)-(1-formyl-2-phenyl)ethylamide |
| 1-39 | 1-33 | 1-48 | 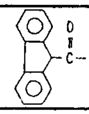 | 1 | 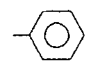 | 1-(9-fluorenylcarbonyl)piperidine-4-carboxylic acid-(1S)-(1-formyl-2-phenyl)ethylamide |
| 1-40 | 1-16 | 1-48 | 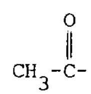 | 1 | 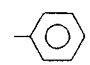 | 1-acetylpiperidine-4-carboxylic acid-(1S)-(1-formyl-2-phenyl)ethylamide |
| 1-41 | 1-28 | 1-48 | 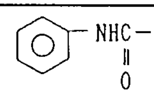 | 1 | 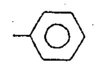 | 1-(N-phenylcarbamoyl)piperidine-4-carboxylic acid-(1S)-(1-formyl-2-phenyl)ethylamide |
| 1-42 | 1-18 | 1-48 | 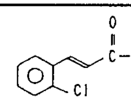 | 1 | 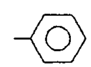 | 1-(2-chlorocinnamoyl)piperidine-4-carboxylic acid-(1S)-(1-formyl-2-phenyl)ethylamide |

(*) Each material is indicated by the Reference Example Number in which it is synthesized.

Table 8

| Ex. | Material 1 (*) | Material 2 (*) | $R^2$ | n | $R^1$ | Compound |
|---|---|---|---|---|---|---|
| 1-43 | 1-19 | 1-48 | 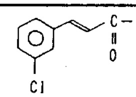 | 1 | 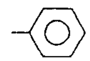 | 1-(3-chlorocinnamoyl)piperidine-4-carboxylic acid-(1S)-(1-formyl-2-phenyl)ethylamide |
| 1-44 | 1-20 | 1-48 | 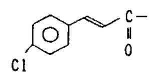 | 1 | 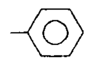 | 1-(4-chlorocinnamoyl)piperidine-4-carboxylic acid-(1S)-(1-formyl-2-phenyl)ethylamide |
| 1-45 | 1-12 | 1-51 | 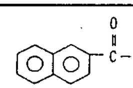 | 3 | $-CH_3$ | 1-(2-naphthoyl)piperidine-4-carboxylic acid-(1S)-1-formyl-pentylamide |
| 1-46 | 1-12 | 1-56 | 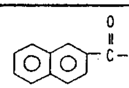 | 3 | 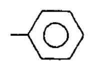 | 1-(2-naphthoyl)piperidine-4-carboxylic acid-(1S)-(1-formyl-4-phenyl)butylamide |
| 1-47 | 1-12 | 1-57 | 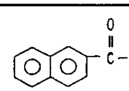 | 4 | 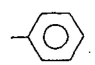 | 1-(2-naphthoyl)piperidine-4-carboxylic acid-(1S)-(1-formyl-5-phenyl)pentylamide |

(*) Each material is indicated by the Reference Example Number in which it is synthesized.

Table 8

| Ex. | Material 1 (*) | Material 2 (*) | $R^2$ | n | $R^1$ | Compound |
|---|---|---|---|---|---|---|
| 1-48 | 1-12 | 1-58 | 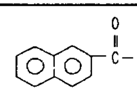 | 5 | 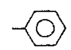 | 1-(2-naphthoyl)piperidine-4-carboxylic acid-(1S)-(1-formyl-6-phenyl)hexylamide |
| 1-49 | 1-12 | 1-52 | 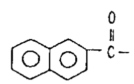 | 1 | 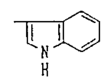 | 1-(2-naphthoyl)piperidine-4-carboxylic acid-(1S)-[1-formyl-2-(3-indolyl)]ethylamide |
| 1-50 | 1-12 | (S)-2-phenyl-glycinol | 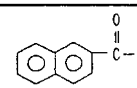 | 0 | 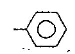 | 1-(2-naphthoyl)piperidine-4-carboxylic acid-(1S)-(1-formyl-1-phenyl)methylamide |
| 1-51 | 1-12 | 1-62 | 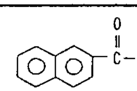 | 2 | 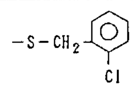 | 1-(2-naphthoyl)piperidine-4-carboxylic acid-(1S)-[1-formyl-3-(2-chlorobenzylthio)]propylamide |
| 1-52 | 1-12 | 1-46 | 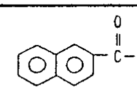 | 2 | $-S-CH_3$ | 1-(2-naphthoyl)piperidine-4-carboxylic acid-(1S)-(1-formyl-3-methylthio)propylamide |

(*) Each material is indicated by the Reference Example Number in which it is synthesized.

Table 8

| Ex. | Material 1 (*) | Material 2 (*) | $R^2$ | n | $R^1$ | Compound |
|---|---|---|---|---|---|---|
| 1-53 | 1-12 | 1-49 | 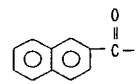 | 1 | 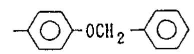 | 1-(2-naphthoyl)piperidine-4-carboxylic acid-(1S)-[1-formyl-2-(4-benzyloxyphenyl)]ethylamide |
| 1-54 | 1-12 | 1-60 | 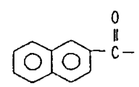 | 1 | 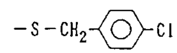 | 1-(2-naphthoyl)piperidine-4-carboxylic acid-(1R)-[1-formyl-2-(4-chlorobenzylthio)]ethylamide |
| 1-55 | 1-12 | 1-53 | 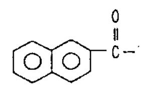 | 1 | 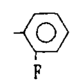 | 1-(2-naphthoyl)piperidine-4-carboxylic acid-(1S)-[1-formyl-2-(2-fluorophenyl)]ethylamide |
| 1-56 | 1-30 | 1-52 | 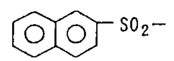 | 1 | 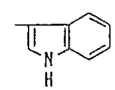 | 1-(2-naphtylsulfonyl)piperidine-4-carboxylic acid-(1S)-[1-formyl-2-(3-indolyl)]ethylamide |
| 1-57 | 1-10 | 1-52 | 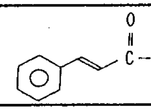 | 1 | 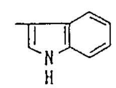 | 1-cinnamoylpiperidine-4-carboxylic acid-(1S)-[1-formyl-2-(3-indolyl)]ethylamide |

(*) Each material is indicated by the Reference Example Number in which it is synthesized.

Table 8

| Ex. | Material 1 (*) | Material 2 (*) | $R^2$ | n | $R^1$ | Compound |
|---|---|---|---|---|---|---|
| 1-58 | 1-18 | 1-52 | 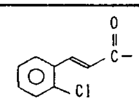 | 1 | 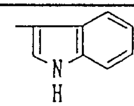 | 1-(2-chlorocinnamoyl)piperidine-4-carboxylic acid-(1S)-[1-formyl-2-(3-indolyl)]ethylamide |
| 1-59 | 1-18 | 1-49 | 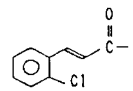 | 1 | 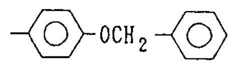 | 1-(2-chlorocinnamoyl)piperidine-4-carboxylic acid-(1S)-[1-formyl-2-(4-benzyloxyphenyl)]ethylamide |
| 1-60 | 1-30 | 1-49 | 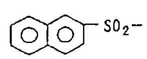 | 1 | 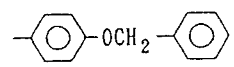 | 1-(2-naphthylsulfonyl)piperidine-4-carboxylic acid-(1S)-[1-formyl-2-(4-benzyloxyphenyl)]ethylamide |
| 1-61 | 1-10 | 1-49 | 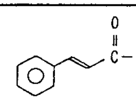 | 1 | 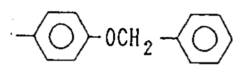 | 1-cinnamoylpiperidine-4-carboxylic acid-(1S)-[1-formyl-2-(4-benzyloxyphenyl)]ethyl |
| 1-62 | 1-18 | 1-61 | 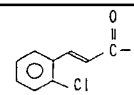 | 2 | 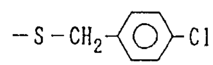 | 1-(2-chlorocinnamoyl)piperidine-4-carboxylic acid-(1S)-[1-formyl-3-(4-chlorobenzylthio)]-propylamide |

(*) Each material is indicated by the Reference Example Number in which it is synthesized.

Table 8

| Ex. | Material 1 (*) | Material 2 (*) | $R^2$ | n | $R^1$ | Compound |
|---|---|---|---|---|---|---|
| 1-63 | 1-30 | 1-61 | 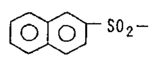 | 2 | 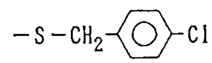 | 1-(2-naphthylsulfonyl)piperidine-4-carboxylic acid-(1S)-[1-formyl-3-(4-chlorobenzylthio)]-propylamide |
| 1-64 | 1-30 | 1-65 | 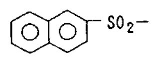 | 3 | 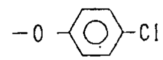 | 1-(2-naphthylsulfonyl)piperidine-4-carboxylic acid-(1S)-[1-formyl-4-(4-chlorophenyloxy)]butyl-amide |
| 1-65 | 1-30 | 1-66 | 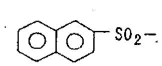 | 3 | 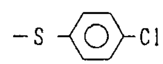 | 1-(2-naphthylsulfonyl)piperidine-4-carboxylic acid-(1S)-[1-formyl-4-(4-chlorophenylthio)]butyl-amide |
| 1-66 | 1-18 | 1-65 | 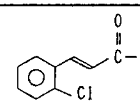 | 3 | 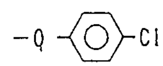 | 1-(2-chlorocinnamoyl)piperidine-4-carboxylic acid-(1S)-[1-formyl-4-(4-chlorophenyloxy)]butyl-amide |
| 1-67 | 1-18 | 1-66 | 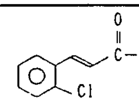 | 3 | 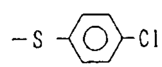 | 1-(2-chlorocinnamoyl)piperidine-4-carboxylic acid-(1S)-[1-formyl-4-(4-chlorophenylthio)]butyl-amide |

(*) Each material is indicated by the Reference Example Number in which it is synthesized.

Table 8

| Ex. | Material 1 (*) | Material 2 (*) | $R^2$ | n | $R^1$, | Compound |
|---|---|---|---|---|---|---|
| 1-68 | 1-30 | 1-63 | 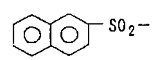 | 2 | 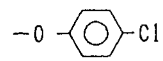 | 1-(2-naphthylsulfonyl)piperidine-4-carboxylic acid-(1S)-[1-formyl-3-(4-chlorophenyloxy)]propylamide |
| 1-69 | 1-30 | 1-64 | 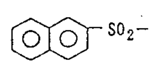 | 2 | 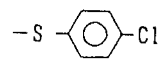 | 1-(2-naphthylsulfonyl)piperidine-4-carboxylic acid-(1S)-[1-formyl-3-(4-chlorophenylthio)]propylamide |
| 1-70 | 1-18 | 1-63 | 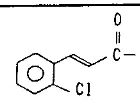 | 2 | 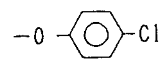 | 1-(2-chlorocinnamoyl)piperidine-4-carboxylic acid-(1S)-[1-formyl-3-(4-chlorophenyloxy)]propylamide |
| 1-71 | 1-18 | 1-64 | 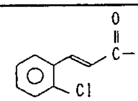 | 2 | 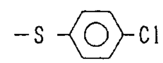 | 1-(2-chlorocinnamoyl)piperidine-4-carboxylic acid-(1S)-[1-formyl-3-(4-chlorophenylthio)]-propylamide |
| 1-72 | 1-34 | 1-48 | 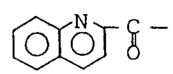 | 1 | 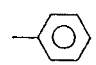 | 1-(2-quinolylcarbonyl)piperidine-4-carboxylic acid-(1S)-(1-formyl-2-phenyl)ethylamide |
| 1-73 | 1-35 | 1-52 | 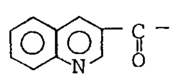 | 1 | 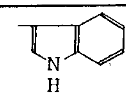 | 1-(3-quinolylcarbonyl)piperidine-4-carboxylic acid-(1S)-[1-formyl-2-(3-indolyl)]ethylamide |
| 1-74 | 1-36 | 1-49 | 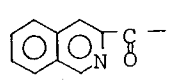 | 1 | 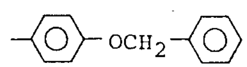 | 1-(3-isoquinolylcarbonyl)piperidine-4-carboxylic acid-(1S)-[1-formyl-2-(4-benzyloxyphenyl)]ethylamide |
| 1-75 | 1-21 | 1-48 | 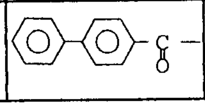 | 1 | 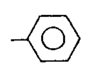 | 1-(4-phenylbenzoyl)piperidine-4-carboxylic acid-(1S)-(1-formyl-2-phenyl)ethylamide |

(*) Each material is indicated by the Reference Example Number in which it is synthesized.

Table 8

| Ex. | Material 1 (*) | Material 2 (*) | $R^2$ | n | $R^1$ | Compound |
|---|---|---|---|---|---|---|
| 1-76 | 1-37 | 1-49 | 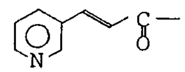 | 1 | 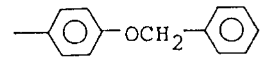 | 1-[3-(3-pyridyl)acryloyl]piperidine-4-carboxylic acid-(1S)-[1-formyl-2-(4-benzyloxyphenyl)]ethylamide |
| 1-77 | 1-22 | 1-49 | 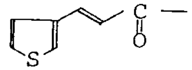 | 1 | 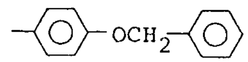 | 1-[3-(3-thienyl)acryloyl]piperidine-4-carboxylic acid-(1S)-[1-formyl-2-(4-benzyloxyphenyl)]ethylamide |
| 1-78 | 1-23 | 1-52 | 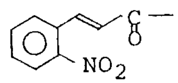 | 1 | 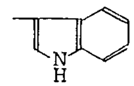 | 1-(2-nitrocinnamoyl)piperidine-4-carboxylic acid-(1S)-[1-formyl-2-(3-indolyl)]ethylamide |
| 1-79 | 1-24 | 1-52 | 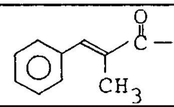 | 1 | 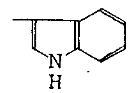 | 1-[(3-phenyl-2-methyl)acryloyl]piperidine-4-carboxylic acid-(1S)-[1-formyl-2-(3-indolyl)]-ethylamide |
| 1-80 | 1-25 | 1-48 | 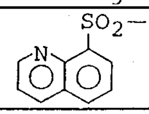 | 1 | 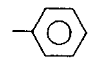 | 1-(8-quinolylsulfonyl)piperidine-4-carboxylic acid-(1S)-(1-formyl-2-phenyl)ethylamide |
| 1-81 | 1-40 | 1-48 | 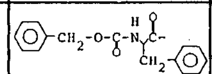 | 1 | 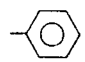 | 1-(benzyloxycarbonyl-L-phenylalanyl)piperidine-4-carboxylic acid-(1S)-(1-formyl-2-phenyl)ethylamide |
| 1-82 | 1-41 | 1-48 | 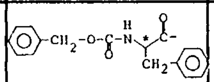 | 1 | 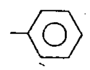 | 1-(benzyloxycarbonyl-D-phenylalanyl)piperidine-4-carboxylic acid-(1S)-(1-formyl-2-phenyl)ethylamide |
| 1-83 | 1-38 | 1-48 | 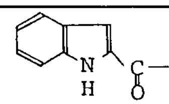 | 1 | 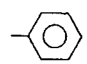 | 1-(2-indolylcarbonyl)piperidine-4-carboxylic acid-(1S)-(1-formyl-2-phenyl)ethylamid |

(*) Each material is indicated by the Reference Example Number in which it is synthesized.

Table 8

| Ex. | Material 1 (*) | Material 2 (*) | $R^2$ | n | $R^1$ | Compound |
|---|---|---|---|---|---|---|
| 1-84 | 1-39 | 1-48 | 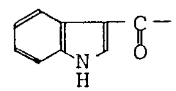 | 1 | 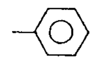 | 1-(3-indolylcarbonyl)piperidine-4-carboxylic acid-(1S)-(1-formyl-2-phenyl)ethylamide |
| 1-85 | 1-26 | 1-49 | 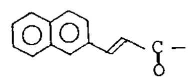 | 1 | 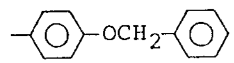 | 1-(2-naphthylacryloyl)piperidine-4-carboxylic acid-(1S)-[1-formyl-2-(4-benzyloxy)phenyl]ethylamide |
| 1-86 | 1-43 | 1-48 | 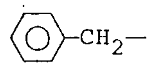 | 1 | 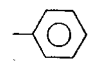 | 1-benzyl-piperidine-4-carboxylic acid-(1S)-(1-formyl-2-phenyl)ethylamide |
| 1-87 | 1-42 | 1-48 | 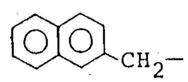 | 1 | 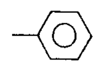 | 1-(2-naphthylmethyl)piperidine-4-carboxylic acid-(1S)-(1-formyl-2-phenyl)ethylamide |
| 1-88 | 1-42 | 1-52 | 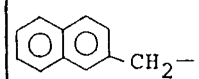 | 1 | 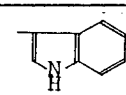 | 1-(2-naphthylmethyl)piperidine-4-carboxylic acid-(1S)-[1-formyl-2-(3-indolyl)]ethylamide |
| 1-89 | 1-44 | 1-48 | 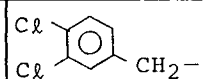 | 1 | 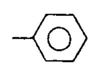 | 1-(3,4-dichlorobenzyl)piperidine-4-carboxylic acid-(1S)-(1-formyl-2-phenyl)ethylamide |
| 1-90 | 1-45 | 1-49 | 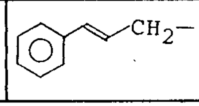 | 1 | 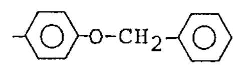 | 1-cinnamyl-piperidine-4-carboxylic acid-(1S)-[1-formyl-2-(4-benzyloxy)phenyl]ethylamide |

(*) Each material is indicated by the Reference Example Number in which it is synthesized.

Table 9

| Ex. | Yield | Melting Point (°C) or State | NMR (δ, $CDCl_3$) | $R_f$ Values |
|---|---|---|---|---|
| 1-23 | 40 | amorphous | 9.65 (s, 1H), 7.09 - 7.49 (m, 10H), 6.02 - 6.21 (m, 1H), 4.35 - 4.90 (m, 2H), 3.60 - 3.95 (m, 1H), 3.19 (d, J=6.3Hz, 2H), 2.60 - 3.20 (m, 2H), 2.28 - 2.46 (m, 1H), 1.40 - 2.00 (m, 4H) | A: 0.16 B: 0.05 |
| 1-24 | 51 | 109.9 to 114.1°C | 9.64 (s, 1H), 7.05 - 7.45 (m, 10H), 5.93 - 6.09 (m, 1H), 4.67 - 4.80 (m, 1H), 4.50 - 4.67 (m, 1H), 3.78 - 3.95 (m, 1H) 3.73 (s, 2H), 3.17 (d, J=6.5Hz, 2H), 2.90 - 3.07 (m, 1H), 2.60 - 2.76 (m, 1H) 2.29 (tt, J=11.5Hz, J=4.0Hz, 1H), 1.30 - 1.90 (m, 4H) | A: 0.14 B: 0.05 |
| 1-25 | 38 | 110.8 to 112.7°C | 9.64 (s, 1H), 7.08 - 7.40 (m, 10H), 5.90 - 6.10 (m, 1H), 4.69 - 4.82 (m, 1H), 4.35 - 4.75 (m, 1H), 3.60 - 3.95 (m, 1H) 3.19 (d, J=6.5Hz, 2H), 2.96 (t, J=8.0Hz, 2H), 2.62 (t, J=7.8Hz, 2H), 2.45 - 3.10 (m, 2H), 2.31 (tt, J=11.3Hz, J=3.8Hz, 1H), 1.35 - 1.90 (m, 4H) | A: 0.16 B: 0.06 |
| 1-26 | 38 | amorphous | 9.62 (s, 1H), 7.07 - 7.38 (m, 10H), 5.95 - 6.18 (m, 1H), 4.68 - 4.81 (m, 1H), 4.20 - 4.80 (m, 1H), 3.50 - 4.10 (m, 1H) 3.19 (d, J=6.5Hz, 2H), 2.68 (t, J=7.4Hz, 2H), 2.45 - 3.15 (m, 2H), 2.32 (t, J= 7.6Hz, 2H), 2.25 - 2.41 (m, 1H), 1.97 (t, J=7.5Hz, 2H), 1.40 - 1.90 (m, 4H) | A: 0.16 B: 0.06 |
| 1-27 | 70 | amorphous | 9.66 (s, 1H), 7.65 (d, J=15.5Hz, 1H), 7.09 - 7.60 (m, 10H), 6.87 (d, J=15.5Hz, 1H), 6.00 - 6.15 (m, 1H), 4.70 - 4.85 (m, 1H), 3.95 - 4.85 (m, 2H), 3.20 (d, J=6.4Hz, 2H), 2.60 - 3.35 (m, 2H), 2.40 (tt, J=11.2Hz, J=4.0Hz, 1H), 1.58 - 2.05 (m, 4H) | A: 0.16 B: 0.05 |
| 1-28 | 59 | amorphous | 9.65 (s, 1H), 7.80 - 7.95 (m, 4H), 7.42 - 7.65 (m, 3H), 7.09 - 7.40 (m, 5H), 6.00 - 6.20 (m, 1H), 4.70 - 4.84 (m, 1H) 3.60 - 4.90 (m, 2H), 3.20 (d, J=6.5Hz, 2H), 2.70 - 3.15 (m, 2H), 2.34 - 2.48 (m, 1H), 1.40 - 4.90 (m, 4H) | A: 0.17 B: 0.05 |

Table 9

| Ex. | Yield | Melting Point (°C) or State | NMR (δ, $CDCl_3$) | $R_f$ Values |
|---|---|---|---|---|
| 1-29 | 56 | 163.8 to 185.1°C | 9.65 (s, 1H), 7.08 - 7.42 (m, 10H), 5.95 - 6.10 (m, 1H), 4.65 - 4.83 (m, 2H), 4.42 (s, 2H), 3.87 - 4.03 (m, 2H), 3.19 (d, J=6.5Hz, 2H), 2.75 - 2.93 (m, 2H), 2.29 (tt, J=11.3Hz, J=4.0Hz, 1H), 1.50 - 1.87 (m, 4H) | A: 0.36<br>B: 0.11 |
| 1-30 | 44 | 101.9 to 116.6°C | 9.61 (s, 1H), 7.56 - 7.70 (m, 2H), 7.05 - 7.45 (m, 7H), 5.90 - 6.08 (m, 1H), 4.67 - 4.80 (m, 1H), 3.65 - 3.81 (m, 2H) 3.16 (d, J=6.5Hz, 2H), 2.43 (s, 3H), 2.28 - 2.42 (m, 2H), 2.00 - 2.13 (m, 1H) 1.60 - 1.90 (m, 4H) | A: 0.45<br>B: 0.22 |
| 1-31 | 46 | oil | 9.63 (s, 1H), 7.08 - 7.43 (m, 5H), 6.03 - 6.30 (m, 1H), 4.65 - 4.84 (m, 1H), 3.75 - 4.85 (m, 2H), 3.19 (d, J=6.3Hz, 2H), 2.45 - 3.29 (m, 3H), 2.28 - 2.45 (m, 1H), 1.47 - 2.05 (m, 12H) | A: 0.22<br>B: 0.09 |
| 1-32 | 47 | amorphous | 9.63 (s, 1H), 7.44 (dd, J=5.1Hz, J= 1.1Hz, 1H), 7.20 - 7.38 (m, 4H), 7.13 (d, J=7.1Hz, 2H), 7.04 (dd, J=5.0Hz, J=3.6Hz, 1H), 6.10 - 6.32 (m, 1H), 4.76 (q, J=6.6Hz, 1H), 4.20 - 4.58 (m, 2H), 3.20 (d, J=6.6Hz, 2H), 2.86 - 3.17 (m, 2H), 2.42 (tt, J=11.0Hz, J=4.2Hz, 1H), 1.55 - 2.00 (m, 4H) | A: 0.23<br>B: 0.12 |
| 1-33 | 56 | amorphous | 9.62 (s, 1H), 7.00 - 7.95 (m, 12H), 6.09 - 6.28 (m, 1H), 4.68 - 4.93 (m, 2H), 3.30 - 3.55 (m, 1H), 3.10 - 3.27 (m, 2H) 2.70 - 3.09 (m, 2H), 2.29 - 2.46 (m, 1H) 1.40 - 2.05 (m, 4H) | A: 0.21<br>B: 0.07 |
| 1-34 | 44 | 137.6 to 143.9°C | 9.59 (s, 1H), 8.33 (s, 1H), 7.89 - 8.04 (m, 3H), 7.58 - 7.78 (m, 3H), 7.03 - 7.35 (m, 5H), 5.90 - 6.10 (m, 1H), 4.71 (q, J=6.6Hz, 1H), 3.77 - 3.92 (m, 2H), 3.15 (d, J=6.5Hz, 2H), 2.35 - 2.53 (m, 2H), 1.99 - 2.12 (m, 1H), 1.50 - 1.95 (m, 4H) | A: 0.48<br>B: 0.25 |

Table 9

| Ex. | Yield | Melting Point (°C) or State | NMR (δ, $CDCl_3$) | $R_f$ Values |
|---|---|---|---|---|
| 1-35 | 52 | amorphous | 9.63 (d, J=2.6Hz, 1H), 7.09 - 7.42 (m, 8H), 6.03 - 6.25 (m, 1H), 4.62 - 4.82 (m, 2H), 3.37 - 3.51 (m, 1H), 3.19 (d, J=6.5Hz, 2H), 2.90 - 3.16 (m, 2H), 2.30 - 2.48 (m, 1H), 1.50 - 2.02 (m, 4H) | A: 0.36 B: 0.18 |
| 1-36 | 68 | amorphous | 9.66 (s, 1H), 7.10 - 7.58 (m, 8H), 5.98 - 6.14 (m, 1H), 4.79 (q, J=6.5Hz, 1H), 4.30 - 4.80 (m, 1H), 3.50 - 4.00 (m, 1H) 3.20 (d, J=6.5Hz, 2H), 2.75 - 3.20 (m, 2H), 2.40 (tt, J=10.9Hz, J=4.1Hz, 1H), 1.50 - 2.00 (m, 4H) | A: 0.23 B: 0.08 |
| 1-37 | 33 | amorphous | 9.63 (s, 1H), 7.20 - 7.36 (m, 3H), 7.09 - 7.17 (m, 2H), 6.08 - 6.26 (m, 1H), 4.74 (q, J=6.5Hz, 1H), 4.39 (d, J= 13.3Hz, 2H), 3.19 (d, J=6.4Hz, 2H), 2.75 - 2.92 (m, 2H), 2.38 (tt, J=11.2Hz, J= 4.1Hz, 1H), 1.50 - 1.90 (m, 4H), 1.27 (s, 9H) | A: 0.25 B: 0.09 |
| 1-38 | 53 | amorphous | 9.60 (s, 1H), 7.06 - 7.45 (m, 15H), 5.90 - 6.14 (m, 1H), 5.19 (s, 1H), 4.52 - 4.80 (m, 2H), 3.80 - 4.05 (m, 1H), 3.17 (d, J=6.4Hz, 2H), 2.55 - 3.10 (m, 2H), 2.28 (tt, J=11.2Hz, J=3.9Hz, 1H), 1.20 - 1.90 (m, 4H) | A: 0.35 B: 0.12 |
| 1-39 | 9.9 | amorphous | 9.56 (s, 1H), 6.95 - 7.87 (m, 13H), 5.85 - 6.15 (m, 1H), 5.06 (s, 1H), 4.48 - 4.72 (m, 2H), 3.13 (d, J=5.8Hz, 2H), 0.70 - 3.05 (m, 8H) | A: 0.26 B: 0.09 |
| 1-40 | 42 | oil | 9.62 (s, 1H), 7.10 - 7.35 (m, 5H), 6.20 - 6.45 (m, 1H), 4.67 - 4.83 (m, 1H), 4.40 - 4.64 (m, 1H), 3.65 - 3.98 (m, 1H) 3.18 (dd, J=6.7Hz, J=2.8Hz, 2H), 2.95 - 3.23 (m, 1H), 2.57 - 2.78 (m, 1H), 2.61 (s, 3H), 2.37 (tt, J=11.1Hz, J=4.0Hz, 1H), 2.30 - 2.53 (m, 1H), 1.50 - 1.95 (m, 3H) | A: 0.06 B: 0.04 |

Table 9

| Ex. | Yield | Melting Point (°C) or State | NMR (δ, $CDCl_3$) | $R_f$ Values |
|---|---|---|---|---|
| 1-41 | 12 | 152.7 to 156.3°C | 9.62 (s, 1H), 6.95 - 7.45 (m, 10H), 6.35 - 6.53 (m, 1H), 6.00 - 6.28 (m, 1H), 4.77 (q, J=6.5Hz, 1H), 3.95 - 4.15 (m, 2H), 3.20 (d, J=6.4Hz, 2H), 2.83 - 3.04 (m, 2H), 2.35 (tt, J=11.1Hz, J=4.0Hz, 1H), 1.55 - 2.00 (m, 4H) | A: 0.22<br>B: 0.03 |
| 1-42 | 75 | 109.9 to 111.6°C | 9.64 (s, 1H), 7.96 (d, J=15.6Hz, 1H), 7.51 - 7.65 (m, 1H), 7.10 - 7.50 (m, 8H) 6.84 (d, J=15.5Hz, 1H), 6.01 - 6.30 (m, 1H), 4.77 (q, J=6.5Hz, 1H), 3.90 - 4.90 (m, 2H), 3.20 (d, J=6.6Hz, 2H), 2.60 - 3.40 (m, 2H), 2.42 (tt, J=11.2Hz, J=4.0Hz, 1H), 1.60 - 2.10 (m, 4H) | A: 0.23<br>B: 0.07 |
| 1-43 | 70 | 115.2 to 120.7°C | 9.65 (s, 1H), 7.58 (d, J=15.5Hz, 1H), 7.51 (s, 1H), 7.08 - 7.47 (m, 8H), 6.87 (d, J=15.5Hz, 1H), 5.97 - 6.23 (m, 1H), 4.78 (q, J=6.5Hz, 1H), 3.85 - 4.90 (m, 2H), 3.20 (d, J=6.5Hz, 2H), 2.65 - 3.40 (m, 2H), 2.41 (tt, J=11.0Hz, J=4.0Hz, 1H), 1.55 - 2.05 (m, 4H) | A: 0.20<br>B: 0.06 |
| 1-44 | 63 | 128.7 to 147.7°C | 9.66 (s, 1H), 7.59 (d, J=15.5Hz, 1H), 7.09 - 7.50 (m, 9H), 6.84 (d, J=15.5Hz, 1H), 6.00 - 6.18 (m, 1H), 4.50 - 4.88 (m, 2H), 4.00 - 4.30 (m, 1H), 3.20 (d, J=6.5Hz, 2H), 3.04 - 3.40 (m, 1H), 2.68 - 3.00 (m, 1H), 2.41 (tt, J=11.2Hz, J=3.9Hz, 1H), 1.59 - 2.10 (m, 4H) | A: 0.16<br>B: 0.06 |
| 1-45 | 43 | amorphous | 9.56 (s, 1H), 7.80 - 7.90 (m, 4H), 7.45 - 7.55 (m, 2H), 7.46 (dd, J=5Hz, 1Hz, 1H), 6.40 - 6.50 (m, 1H), 4.50 - 4.90 (m, 1H), 4.52 (q, J=7Hz, 1H), 3.55 - 4.10 (m, 1H), 2.70 - 3.10 (m, 2H), 2.35 - 2.50 (m, 1H), 1.50 - 2.05 (m, 6H), 1.20 - 1.40 (m, 4H), 0.88 (t, J=6Hz, 3H) | A: 0.20<br>B: 0.07 |
| 1-46 | 38 | amorphous | 9.54 (s, 1H), 7.80 - 7.93 (m, 4H), 7.40 - 7.60 (m, 3H), 7.20 - 7.35 (m, 5H), 6.10 - 6.25 (m, 1H), 4.50 - 4.90 (m, 2H) 3.60 - 4.10 (m, 1H), 2.80 - 3.15 (m, 2H) 2.50 - 2.75 (m, 2H), 2.40 - 2.50 (m, 1H) 1.50 - 2.10 (m, 8H) | A: 0.20<br>B: 0.06 |

Table 9

| Ex. | Yield | Melting Point (°C) or State | NMR (δ, $CDCl_3$) | $R_f$ Values |
|---|---|---|---|---|
| 1-47 | 40 | amorphous | 9.54 (s, 1H), 7.80 - 7.90 (m, 4H), 7.40 - 7.60 (m, 3H), 7.00 - 7.30 (m, 5H), 6.20 - 6.30 (m, 1H), 4.50 - 4.85 (m, 1H) 4.54 (q, J=7Hz, 1H), 3.60 - 4.10 (m, 1H) 2.70 - 3.10 (m, 2H), 2.59 (t, J=7Hz, 2H) 2.35 - 2.50 (m, 1H), 1.50 - 2.05 (m, 8H) 1.20 - 1.45 (m, 2H) | A: 0.20 B: 0.07 |
| 1-48 | 6.4 | amorphous | 9.56 (s, 1H), 7.80 - 7.90 (m, 4H), 7.45 - 7.60 (m, 3H), 7.05 - 7.30 (m, 5H), 6.17 (d, J=7Hz, 1H), 4.55 - 4.90 (m, 1H) 4.57 (q, J=6Hz, 1H), 3.40 - 4.15 (m, 1H) 2.75 - 3.20 (m, 2H), 2.58 (t, J=8Hz, 2H) 2.35 - 2.55 (m, 1H), 1.50 - 2.10 (m, 8H) 1.25 - 1.50 (m, 4H) | A: 0.23 B: 0.09 |
| 1-49 | 19 | amorphous | 9.65 (s, 1H), 8.33 (bs, 1H), 7.80 - 7.95 (m, 4H), 7.50 - 7.65 (m, 3H), 7.44 (dd, J=8Hz, 1Hz, 1H), 7.36 (d, J=7Hz, 1H), 7.20 (t, J=7Hz, 1H), 7.13 (t, J=7Hz, 1H) 6.98 (d, J=0.5Hz, 1H), 6.22 (d, J=6Hz, 1H), 4.84 (q, J=7Hz, 1H), 4.40 - 4.80 (m, 1H), 3.50 - 4.10 (m, 1H), 3.40 (dd, J=15Hz, 5Hz, 1H), 3.30 (dd, J=15Hz, 6Hz, 1H), 2.90 - 3.10 (m, 2H), 2.30 - 2.40 (m, 1H), 1.50 - 2.00 (m, 4H) | A: 0.16 B: 0.02 |
| 1-50 | 8.4 | amorphous | 9.58 (s, 1H), 7.78 - 7.96 (m, 4H), 7.20 - 7.60 (m, 8H), 6.67 - 6.77 (m, 1H), 5.61 (d, J=6.0Hz, 1H), 4.30 - 5.00 (m, 1H), 3.60 - 4.20 (m, 1H), 2.80 - 3.23 (m, 2H), 2.53 (tt, J=11.1Hz, J=4.3Hz, 1H), 1.40 - 2.20 (m, 4H) | A: 0.30 B: 0.09 |
| 1-51 | 72 | amorphous | 9.51 (s, 1H), 7.80 - 7.93 (m, 4H), 7.13 - 7.58 (m, 7H), 6.45 - 6.61 (m, 1H), 4.50 - 4.60 (m, 1H), 4.40 - 4.90 (m, 1H) 3.82 (s, 2H), 3.60 - 4.10 (m, 1H), 2.80 - 3.15 (m, 2H), 2.36 - 2.65 (m, 3H), 2.17 - 2.34 (m, 1H), 1.55 - 2.05 (m, 5H) | A: 0.21 B: 0.07 |

Table 9

| Ex. | Yield | Melting Point (°C) or State | NMR (δ, $CDCl_3$) | $R_f$ Values |
|---|---|---|---|---|
| 1-52 | 30 | amorphous | 9.59 (s, 1H), 7.80 - 7.92 (m, 4H), 7.44 - 7.58 (m, 3H), 6.48 - 6.66 (m, 1H), 4.57 - 4.69 (m, 1H), 4.30 - 5.00 (m, 1H) 3.60 - 4.30 (m, 1H), 2.80 - 3.15 (m, 2H) 2.43 - 2.65 (m, 3H), 2.21 - 2.38 (m, 1H) 2.09 (s, 3H), 1.65 - 2.10 (m, 5H) | A: 0.16 B: 0.06 |
| 1-53 | 74 | amorphous | 9.64 (s, 1H), 7.80 - 7.95 (m, 4H), 7.22 - 7.60 (m, 8H), 7.03 (d, J=8.7Hz, 2H), 6.90 (d, J=8.6Hz, 2H), 6.04 - 6.18 (m, 1H), 5.03 (s, 2H), 4.68 - 4.82 (m, 1H), 4.40 - 4.85 (m, 1H), 3.55 - 4.25 (m, 1H) 3.14 (d, J=6.4Hz, 2H), 2.75 - 3.20 (m, 2H), 2.32 - 2.48 (m, 1H), 1.40 - 2.00 (m, 4H) | A: 0.20 B: 0.06 |
| 1-54 | 52 | amorphous | 9.51 (s, 1H), 7.80 - 7.93 (m, 4H), 7.45 - 7.59 (m, 3H), 7.20 - 7.35 (m, 4H), 6.30 - 6.50 (m, 1H), 4.56 - 4.70 (m, 1H) 4.35 - 4.95 (m, 1H), 3.74 - 4.33 (m, 1H) 3.70 (s, 2H), 2.91 (d, J=5.9Hz, 2H), 2.80 - 3.18 (m, 2H), 2.38 - 2.51 (m, 1H) 1.50 - 2.10 (m, 4H) | A: 0.18 B: 0.05 |
| 1-55 | 68 | amorphous | 9.64 (d, J=1.9Hz, 1H), 7.80 - 7.93 (m, 4H), 7.40 - 7.60 (m, 3H), 6.95 - 7.32 (m, 4H), 6.15 - 6.37 (m, 1H), 4.70 - 4.82 (m, 1H), 4.50 - 4.85 (m, 1H), 3.90 - 4.10 (m, 1H), 3.13 - 3.35 (m, 2H), 2.75 - 3.14 (m, 2H), 2.33 - 2.46 (m, 1H) 1.50 - 2.10 (m, 4H) | A: 0.22 B: 0.07 |
| 1-56 | 18 | 104.5 to 137.7°C | 9.61 (s, 1H), 8.32 (s, 1H), 8.08 (bs, 1H), 7.90 - 8.10 (m, 3H), 7.55 - 7.80 (m, 3H), 7.54 (d, J=8Hz, 1H), 7.35 (d, J=8Hz, 1H), 7.20 (t, J=7Hz, 1H), 7.10 (t, J=8Hz, 1H), 6.96 (d, J=2Hz, 1H), 5.95 - 6.10 (m, 1H), 4.80 (q, J=5Hz, 1H) 3.75 - 4.90 (m, 2H), 3.38 (dd, J=15Hz, 5Hz, 1H), 3.25 (dd, J=15Hz, 7Hz, 1H), 2.30 - 2.50 (m, 2H), 1.90 - 2.05 (m, 1H) 1.60 - 1.90 (m, 4H) | A: 0.45 B: 0.14 |

Table 9

| Ex. | Yield | Melting Point (°C) or State | NMR (δ, $CDCl_3$) | $R_f$ Values |
|---|---|---|---|---|
| 1-57 | 3.5 | amorphous | 9.66 (s, 1H), 8.16 (bs, 1H), 7.64 (d, J=15Hz, 1H), 7.05 - 7.60 (m, 9H), 7.02 (d, J=2Hz, 1H), 6.85 (d, J=15Hz, 1H), 6.10 - 6.20 (m, 1H), 4.86 (q, J=5Hz, 1H), 4.40 - 4.80 (m, 1H), 3.90 - 4.30 (m, 1H), 3.43 (dd, J=14Hz, 5Hz, 1H), 3.30 (dd, J=14Hz, 1H), 2.60 - 3.30 (m, 2H), 2.37 (tt, J=8Hz, 3Hz, 1H), 1.50 - 1.90 (m, 4H) | A: 0.13<br>B: 0.02 |
| 1-58 | 20 | 171.3 to 172.0°C | 9.63 (s, 1H), 8.19 (bs, 1H), 7.95 (d, J=15Hz, 1H), 7.56 - 7.60 (m, 2H), 7.35 - 7.45 (m, 2H), 7.11 - 7.30 (m, 4H), 7.01 (d, J=2Hz, 1H), 6.83 (d, J=15Hz, 1H), 6.15 - 6.30 (m, 1H), 4.85 (q, J=6Hz, 1H) 3.85 - 4.85 (m, 2H), 3.41 (dd, J=15Hz, 6Hz, 1H), 3.30 (dd, J=15Hz, 7Hz, 1H), 2.50 - 3.30 (m, 2H), 2.37 (tt, J=10Hz, 3Hz, 1H), 1.40 - 1.95 (m, 4H) | A: 0.17<br>B: 0.03 |
| 1-59 | 82 | 114.1 to 118.9°C | 9.65 (s, 1H), 7.97 (d, J=15.5Hz, 1H), 7.20 - 7.65 (m, 9H), 7.03 (d, J=8.7Hz, 2H), 6.91 (d, J=8.7Hz, 2H), 6.85 (d, J=15.5Hz, 1H), 5.95 - 6.11 (m, 1H), 5.04 (s, 2H), 4.50 - 4.81 (m, 2H), 3.95 - 4.24 (m, 1H), 3.14 (d, J=6.8Hz, 2H), 2.65 - 3.30 (m, 2H), 2.41 (tt, J=11.1Hz, J=3.8Hz, 1H), 1.50 - 1.95 (m, 4H) | A: 0.23<br>B: 0.05 |
| 1-60 | 58 | 137.2 to 176.2°C | 9.58 (s, 1H), 8.33 (s, 1H), 7.85 - 8.05 (m, 3H), 7.55 - 7.80 (m, 3H), 7.25 - 7.50 (m, 5H), 6.97 (d, J=8.5Hz, 2H), 6.86 (d, J=8.6Hz, 2H), 5.86 - 6.05 (m, 1H), 5.02 (s, 2H), 4.60 - 4.72 (m, 1H), 3.75 - 3.93 (m, 2H), 3.08 (d, J=6.4Hz, 2H), 2.34 - 2.54 (m, 2H), 1.70 - 2.10 (m, 5H) | A: 0.53<br>B: 0.24 |
| 1-61 | 61 | 133.2 to 135.8°C | 9.64 (s, 1H), 7.65 (d, J=15.5Hz, 1H), 7.30 - 7.58 (m, 10H), 7.03 (d, J=8.6Hz, 2H), 6.91 (d, J=8.7Hz, 2H), 6.87 (d, J=15.4Hz, 1H), 6.00 - 6.18 (m, 1H), 5.04 (s, 2H), 3.90 - 4.90 (m, 3H), 3.14 (d, J=6.4Hz, 2H), 2.60 - 3.40 (m, 2H), 2.41 (tt, J=11.1Hz, J=3.9Hz, 1H), 1.50 - 2.10 (m, 4H) | A: 0.20<br>B: 0.04 |

Table 9

| Ex. | Yield | Melting Point (°C) or State | NMR (δ, $CDCl_3$) | $R_f$ Values |
|---|---|---|---|---|
| 1-62 | 0.58 | amorphous | 9.55 (s, 1H), 7.97 (d, J=15.6Hz, 1H), 7.53 - 7.64 (m, 1H), 7.19 - 7.45 (m, 7H) 6.86 (d, J=15.6Hz, 1H), 6.25 - 6.45 (m, 1H), 3.90 - 4.85 (m, 3H), 3.66 (s, 2H), 2.60 - 3.37 (m, 2H), 2.15 - 2.55 (m, 4H) 1.60 - 2.04 (m, 5H) | A: 0.23 B: 0.06 |
| 1-63 | 64 | amorphous | 9.51 (s, 1H), 8.34 (s, 1H), 7.90 - 8.03 (m, 3H), 7.59 - 7.79 (m, 3H), 7.15 - 7.22 (m, 4H), 6.00 - 6.17 (m, 1H), 4.46 - 4.55 (m, 1H), 3.79 - 3.95 (m, 2H), 3.62 (s, 2H), 1.60 - 2.55 (m, 11H) | A: 0.54 B: 0.25 |
| 1-64 | 19 | amorphous | 9.54 (s, 1H), 8.31 (s, 1H), 7.85 - 8.00 (m, 3H), 7.55 - 7.85 (m, 3H), 7.17 (d, J=9Hz, 2H), 6.74 (d, J=9Hz, 2H), 6.20 - 6.35 (m, 1H), 4.45 - 4.60 (m, 1H), 3.70 3.95 (m, 4H), 2.30 - 2.50 (m, 2H), 2.00 - 2.20 (m, 2H), 1.60 - 2.00 (m, 7H) | A: 0.45 B: 0.23 |
| 1-65 | 64 | 107.6 to 117.6°C | 9.52 (s, 1H), 8.35 (s, 1H), 7.80 - 8.10 (m, 3H), 7.55 - 7.80 (m, 3H), 7.20 - 7.30 (m, 4H), 5.96 (d, J=6Hz, 1H), 4.55 (q, J=5Hz, 1H), 3.80 - 3.90 (m, 2H), 2.75 - 2.90 (m, 2H), 2.40 - 2.50 (m, 1H), 2.00 - 2.10 (m, 1H), 1.40 - 1.90 (m, 7H) | A: 0.47 B: 0.24 |
| 1-66 | 20 | amorphous | 9.60 (s, 1H), 7.95 (d, J=15Hz, 1H), 7.55 - 7.62 (m, 1H), 7.35 - 7.45 (m, 1H), 7.15 - 7.35 (m, 4H), 6.85 (d, J=15Hz, 1H), 6.70 - 6.85 (m, 2H), 6.35 - 6.45 (m, 1H), 4.55 - 4.80 (m, 2H), 4.00 - 4.25 (m, 1H), 3.85 - 4.00 (m, 2H), 3.00 - 3.30 (m, 1H), 2.70 - 3.00 (m, 1H), 2.48 (tt, J=8Hz, 4Hz, 1H), 2.10 - 2.30 (m, 1H), 1.65 - 2.00 (m, 7H) | A: 0.17 B: 0.05 |
| 1-67 | 52 | 147.8 to 151.5°C | 9.56 (s, 1H), 7.97 (d, J=15Hz, 1H), 7.55 - 7.65 (m, 2H), 7.35 - 7.45 (m, 2H), 7.20 - 7.40 (m, 4H), 6.85 (d, J=15Hz, 1H), 6.10 (d, J=7Hz, 1H), 4.55 - 4.80 (m, 1H), 4.59 (q, J=5Hz, 1H), 4.05 - 4.20 (m, 1H), 3.10 - 3.30 (m, 1H), 2.70 - 3.05 (m, 3H), 2.35 - 2.50 (m, 1H), 2.05 - 2.25 (m, 1H), 1.60 - 2.00 (m, 7H) | A: 0.19 B: 0.05 |

Table 9

| Ex. | Yield | Melting Point (°C) or State | NMR (δ, $CDCl_3$) | $R_f$ Values |
|---|---|---|---|---|
| 1-68 | 37 | amorphous | 9.63 (s, 1H), 8.33 (s, 1H), 7.96 (d, J=15Hz, 2H), 7.92 (dd, J=9Hz, J=1Hz, 1H), 7.73 (dd, J=9Hz, J=1Hz, 1H), 7.60 - 7.69 (m, 2H), 7.26 (t, J=7Hz, 1H), 6.96 (t, J=7Hz, 1H), 6.79 (d, J=8Hz, 2H), 6.37 - 6.42 (m, 1H), 4.56 (q, J=5Hz, 1H), 4.00 4.09 (m, 1H), 3.82 - 3.92 (m, 3H), 2.22 2.48 (m, 4H), 2.04 - 2.18 (m, 1H), 1.77 - 1.94 (m, 4H) | A: 0.47<br>B: 0.20 |
| 1-69 | 40 | amorphous | 9.53 (s, 1H), 8.34 (s, 1H), 7.99 (d, J=8Hz, 2H), 7.93 (d, J=7Hz, J=1Hz, 1H), 7.75 (dd, J=10Hz, J=2Hz, 1H), 7.60 - 7.70 (m, 2H), 7.21 - 7.28 (m, 4H), 6.11 - 6.18 (m, 1H), 4.59 (t, J=5Hz, 1H), 3.82 - 3.91 (m, 2H), 2.78 - 2.98 (m, 2H) 2.42 - 2.52 (m, 2H), 2.25 - 2.37 (m, 2H) 2.04 - 2.16 (m, 1H), 1.82 - 1.96 (m, 4H) | A: 0.47<br>B: 0.20 |
| 1-70 | 37 | amorphous | 9.69 (s, 1H), 7.96 (d, J=15Hz, 1H), 7.57 - 7.60 (m, 1H), 7.39 - 7.42 (m, 1H), 7.21 - 7.31 (m, 4H), 6.97 (t, J=7Hz, 1H) 6.73 - 6.87 (m, 2H), 6.48 - 6.52 (m, 1H), 4.57 - 4.72 (m, 2H), 4.02 - 4.14 (m, 2H), 3.88 - 3.98 (m, 1H), 3.10 - 3.24 (m, 1H), 2.75 - 2.90 (m, 1H), 2.40 - 2.57 (m, 3H), 1.89 - 1.97 (m, 2H), 1.62 - 1.82 (m, 2H) | A: 0.19<br>B: 0.06 |
| 1-71 | 50 | amorphous | 9.57 (s, 1H), 7.97 (d, J=16Hz, 1H), 7.56 - 7.59 (m, 1H), 7.40 - 7.42 (m, 1H), 7.23 - 7.29 (m, 6H), 6.85 (t, J=16Hz, 1H), 6.40 - 6.47 (m, 1H), 4.63 (q, J=7Hz, 1H), 4.61 - 4.71 (m, 1H), 4.10 - 4.20 (m, 1H), 3.10 - 3.30 (m, 1H), 2.85 - 3.03 (m, 2H), 2.75 - 2.95 (m, 1H), 2.45 - 2.54 (m, 1H), 2.28 - 2.39 (m, 1H) 1.89 - 2.01 (m, 3H), 1.67 - 1.82 (m, 2H) | A: 0.18<br>B: 0.04 |

Table 9

| Ex. | Yield | Melting Point (°C) or State | NMR (δ, $CDCl_3$) | $R_f$ Values |
|---|---|---|---|---|
| 1-72 | 65 | amorphous | 9.66 (s, 1H), 8.26 (d, J=9Hz, 1H), 8.15 (d, J=9Hz, 1H), 7.86 (d, J=8Hz, 1H), 7.76 (t, J=7Hz, 1H), 6.67 (dd, J=8Hz, 1Hz, 1H), 7.60 (t, J=8Hz, 1H), 7.10 - 7.35 (m, 5H), 6.14 (m, 1H), 4.65 - 4.85 (m, 2H), 4.00 - 4.15 (m, 1H), 3.19 (d, J=6Hz, 2H), 3.10 - 3.25 (m, 1H), 2.85 - 3.00 (m, 1H), 2.40 - 2.50 (m, 1H), 1.70 - 2.00 (m, 4H) | A: 0.10<br>B: 0.02 |
| 1-73 | 69 | amorphous | 9.67 (s, 1H), 8.91 (d, J=2Hz, 1H), 8.29 (brs, 1H), 8.23 (d, J=2Hz, 1H), 8.15 (d, J=8Hz, 1H), 7.87 (d, J=8Hz, 1H), 7.80 (td, J=8Hz, 1Hz, 1H), 7.55 - 7.65 (m, 2H), 7.37 (d, J=8Hz, 1H), 7.05 - 7.30 (m, 2H), 7.00 (d, J=2Hz, 1H), 6.17 (d, J=6Hz, 1H), 4.80 - 4.90 (m, 1H), 4.50 - 4.80 (m, 1H), 3.60 - 3.90 (m, 1H) 3.45 (dd, J=15Hz, 5Hz, 1H), 3.30 (dd, J= 15Hz, 7Hz, 1H), 2.80 - 3.20 (m, 2H), 2.30 - 2.45 (m, 1H), 1.60 - 1.90 (m, 4H) | A: 0.04<br>B: 0.01 |
| 1-74 | 20 | amorphous | 9.65 (s, 1H), 9.22 (s, 1H), 8.05 (s, 1H) 8.02 (d, J=8Hz, 1H), 7.90 (d, J=8Hz, 1H) 7.65 - 7.80 (m, 2H), 7.20 - 7.50 (m, 5H) 7.05 (d, J=8Hz, 2H), 6.90 (d, J=8Hz, 2H) 6.05 (d, J=6Hz, 1H), 5.03 (s, 2H), 4.90 - 5.05 (m, 1H), 4.70 - 4.85 (m, 2H) 3.15 (d, J=6Hz, 2H), 2.85 - 3.30 (m, 2H) 2.35 - 2.50 (m, 1H), 1.60 - 1.90 (m, 4H) | A: 0.05<br>B: 0.01 |
| 1-75 | 34 | amorphous | 9.66 (s, 1H), 7.10 - 7.70 (m, 14H), 6.05 (d, J=6Hz, 1H), 4.50 - 4.85 (m, 2H), 3.75 - 4.05 (m, 1H), 3.20 (d, J=6Hz, 2H) 2.70 - 3.10 (m, 2H), 2.30 - 2.50 (m, 1H) 1.60 - 1.95 (m, 4H) | A: 0.18<br>B: 0.04 |

Table 9

| Ex. | Yield | Melting Point (°C) or State | NMR (δ, $CDCl_3$) | $R_f$ Values |
|---|---|---|---|---|
| 1-76 | 61 | amorphous | 9.65 (s, 1H), 8.76 (s, 1H), 8.57 (d, J=3Hz, 1H), 7.82 (d, J=8Hz, 1H), 7.63 (d, J=15Hz, 1H), 7.30 - 7.45 (m, 6H), 6.80 - 7.10 (m, 5H), 6.04 (d, J=6Hz, 1H), 5.04 (s, 2H), 4.55 - 4.80 (m, 2H), 4.05 - 4.20 (m, 1H), 3.10 - 3.30 (m, 1H), 3.14 (d, J=6Hz, 2H), 2.75 - 2.90 (m, 1H), 2.41 (tt, J=11Hz, 4Hz, 1H), 1.60 - 2.00 (m, 4H) | A: 0.02<br>B: 0.01 |
| 1-77 | 59 | amorphous | 9.65 (s, 1H), 7.78 (d, J=15Hz, 1H), 7.20 - 7.50 (m, 6H), 7.00 - 7.10 (m, 3H), 6.90 (d, J=8Hz, 2H), 6.67 (d, J=15Hz, 1H), 5.99 (d, J=6Hz, 1H), 5.04 (s, 2H), 4.55 - 4.80 (m, 2H), 3.95 - 4.25 (m, 1H) 3.00 - 3.20 (m, 1H), 3.14 (d, J=6Hz, 2H) 2.70 - 2.95 (m, 1H), 2.39 (tt, 11Hz, 4Hz, 1H), 1.50 - 1.95 (m, 4H) | A: 0.18<br>B: 0.04 |
| 1-78 | 10 | amorphous | 9.68 (s, 1H), 8.37 (s, 1H), 8.02 (d, J=8Hz, 1H), 7.84 (d, J=15Hz, 1H), 7.45 - 7.70 (m, 4H), 7.36 (d, J=8Hz, 1H), 7.00 - 7.25 (m, 3H), 6.66 (d, J=15Hz, 1H), 5.97 (d, J=7Hz, 1H), 4.80 - 4.90 (m, 1H) 4.40 - 4.65 (m, 1H), 4.00 - 4.15 (m, 1H) 3.00 - 3.20 (m, 3H), 2.65 - 2.90 (m, 1H) 2.25 - 2.35 (m, 1H), 1.50 - 1.80 (m, 4H) | A: 0.03<br>B: 0.02 |
| 1-79 | 15 | amorphous | 9.64 (s, 1H), 8.24 (s, 1H), 7.60 (d, J=8Hz, 1H), 7.05 - 7.42 (m, 8H), 7.00 (d, J=2Hz, 1H), 6.51 (s, 1H), 6.15 - 6.30 (m, 1H), 4.80 - 4.90 (m, 1H), 3.85 - 4.55 (m, 2H), 3.45 (dd, J=15Hz, 5Hz, 1H) 3.30 (dd, J=15Hz, 8Hz, 1H), 2.70 - 3.10 (m, 2H), 2.35 (tt, J=11Hz, 3Hz, 1H), 2.08 (s, 3H), 1.60 - 1.90 (m, 4H) | A: 0.05<br>B: 0.03 |

Table 9

| Ex. | Yield | Melting Point (°C) or State | NMR (δ, $CDCl_3$) | $R_f$ Values |
|---|---|---|---|---|
| 1-80 | 25 | amorphous | 9.61 (s, 1H), 9.04 (dd, J=4Hz, 1Hz, 1H) 8.47 (dd, J=8Hz, 1Hz, 1H), 8.23 (dd, J=8Hz, 1Hz, 1H), 8.03 (d, J=8Hz, 1H), 7.61 (t, J=7Hz, 1H), 7.50 - 7.60 (m, 1H), 7.05 - 7.40 (m, 5H), 5.95 (d, J=6Hz, 1H) 4.65 - 4.75 (m, 1H), 4.00 - 4.20 (m, 2H) 3.15 (d, J=6Hz, 2H), 2.80 - 3.00 (m, 2H) 2.10 - 2.20 (m, 1H), 1.60 - 1.90 (m, 4H) | A: 0.28<br>B: 0.09 |
| 1-81 | 71 | amorphous | 9.60 - 9.70 (m, 1H), 7.00 - 7.43 (m, 15H), 5.60 - 6.00 (m, 2H), 4.98 - 5.16 (m, 2H), 4.80 - 4.98 (m, 1H), 4.67 - 4.80 (m, 1H), 4.40 - 4.55 (m, 1H), 3.50 - 3.77 (m, 1H), 3.10 - 3.25 (m, 2H), 2.80 - 3.10 (m, 2H), 2.49 - 2.68 (m, 1H) 1.20 - 2.35 (m, 6H) | A: 0.30<br>B: 0.07 |
| 1-82 | 69 | amorphous | 9.60 - 9.70 (m, 1H), 7.02 - 7.43 (m, 15H), 5.60 - 6.00 (m, 2H), 4.99 - 5.15 (m, 2H), 4.80 - 4.96 (m, 1H), 4.65 - 4.80 (m, 1H), 4.35 - 4.45 (m, 1H), 3.53 - 3.75 (m, 1H), 3.10 - 3.27 (m, 2H), 2.84 - 3.10 (m, 2H), 2.48 - 2.68 (m, 1H) 1.20 - 2.40 (m, 6H) | A: 0.30<br>B: 0.07 |
| 1-83 | 88 | 174.0 - 176.0 | 9.67 (s, 1H), 9.41 (s, 1H), 7.64 (d, J=8Hz, 1H), 7.43 (d, J=8Hz, 1H), 7.08 - 7.50 (m, 7H), 6.75 (d, J=2Hz, 1H), 6.05 - 6.30 (m, 1H), 4.75 - 4.87 (m, 1H), 4.53 - 4.74 (m, 2H), 3.20 (d, J=6Hz, 2H) 2.90 - 3.40 (m, 2H), 2.43 (tt, J=11Hz, J=4Hz, 1H), 1.50 - 2.05 (m, 4H) | A: 0.21<br>B: 0.04 |
| 1-84 | 73 | amorphous | 9.74 (s, 1H), 9.62 (s, 1H), 7.55 - 7.75 (m, 1H), 7.00 - 7.45 (m, 9H), 6.41 (d, J=7Hz, 1H), 4.64 - 4.80 (m, 1H), 4.15 - 4.50 (m, 2H), 3.05 - 3.35 (m, 2H), 2.75 - 3.05 (m, 2H), 1.50 - 2.45 (m, 5H) | A: 0.06<br>B: 0.01 |

Table 9

| Ex. | Yield | Melting Point (°C) or State | NMR (δ, $CDCl_3$) | $R_f$ Values |
|---|---|---|---|---|
| 1-85 | 87 | 183.0 - 184.8 | 9.65 (s, 1H), 7.23 - 8.00 (m, 13H), 7.03 (d, J=9Hz, 2H), 6.99 (d, J=16Hz, 1H), 6.91 (d, J=9Hz, 2H), 5.97 - 6.13 (m, 1H) 5.04 (s, 2H), 4.70 - 4.83 (m, 1H), 4.00 - 4.85 (m, 2H), 3.15 (d, J=7Hz, 2H), 2.60 - 3.40 (m, 2H), 2.42 (tt, J=11Hz, J=4Hz, 1H), 1.50 - 2.00 (m, 4H) | A: 0.17 B: 0.04 |
| 1-86 | 72 | 105.5 - 109.3 | 9.64 (s, 1H), 7.08 - 7.45 (m, 10H), 5.92 - 6.08 (m, 1H), 4.73 (q, J=7Hz, 1H), 3.40 - 3.65 (m, 2H), 3.18 (d, J=6Hz, 2H) 2.83 - 3.04 (m, 2H), 1.50 - 2.30 (m, 7H) | A: 0.11 B: 0.02 |
| 1-87 | 20 | amorphous | 9.64 (s, 1H), 7.05 - 7.90 (m, 12H), 5.90 - 6.10 (m, 1H), 4.69 - 4.80 (m, 1H), 3.66 (s, 2H), 3.18 (d, J=6Hz, 2H), 2.85 - 3.05 (m, 2H), 1.50 - 2.25 (m, 7H) | A: 0.10 B: 0.02 |
| 1-88 | 54 | amorphous | 9.62 (s, 1H), 8.24 (s, 1H), 6.90 - 7.90 (m, 12H), 6.14 (d, J=7Hz, 1H), 4.74 - 4.87 (m, 1H), 3.63 (s, 2H), 3.20 - 3.48 (m, 2H), 2.80 - 3.05 (m, 2H), 1.60 - 2.25 (m, 7H) | A: 0.10 B: 0.01 |
| 1-89 | 35 | amorphous | 9.65 (s, 1H), 7.05 - 7.47 (m, 8H), 5.90 - 6.10 (m, 1H), 4.69 - 4.82 (m, 1H), 3.35 - 3.60 (m, 2H), 3.19 (d, J=6Hz, 2H), 2.75 - 3.00 (m, 2H), 1.50 - 2.25 (m, 7H) | A: 0.17 B: 0.05 |
| 1-90 | 89 | amorphous | 9.63 (s, 1H), 7.19 - 7.50 (m, 10H), 7.03 (d, J=9Hz, 2H), 6.90 (d, J=9Hz, 2H), 6.51 (d, J=16Hz, 1H), 6.27 (td, J=7Hz, J=16Hz, 1H), 6.00 (d, J=6Hz, 1H), 5.03 (s, 2H), 4.65 - 4.75 (m, 1H), 2.90 - 3.25 (m, 6H), 2.14 (tt, J=11Hz, J=4Hz, 1H), 1.92 - 2.08 (m, 2H), 1.60 - 2.91 (m, 4H) | A: 0.05 B: 0.01 |

**Example 1-91**

Synthesis of 1-benzyloxycarbonyl-piperidine-4-carboxylic acid-(1R,1S)-(1-formyl-4-phenyl)-3-butenylamide (Compound No. 1-91):

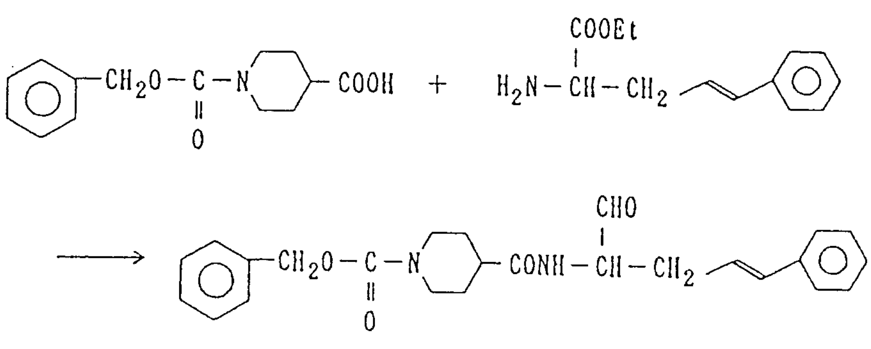

0.70 ml (4.97 mmol) of triethylamine and 1.14 g (5.96 mmol) of 1-ethyl-3-(3-dimethylaminopropyl)-carbodiimide hydrochloride were successively added to 50 ml of a dichloromethane solution containing 1.31 g (4.97 mmol) of 1-benzyloxycarbonylpiperidine-4-carboxylic acid synthesized in Reference Example 1-4 and 1.27 g (4.97 mmol) of (2R,2S)-2-amino-5-phenyl-4-pentanoic acid ethyl ester hydrochloride (Compound No. 1-67) synthesized in Reference Example 1-67 under an ice-cooled condition. After stirring overnight at room temperature, the solvent was distilled away from the reaction mixture under reduced pressure and the residue thus obtained was dissolved in ethyl acetate. This solution was washed successively with a 1 N hydrochloric acid solution, a saturated aqueous solution of sodium chloride, a saturated aqueous solution of sodium hydrogencarbonate and a saturated aqueous solution of sodium chloride. The resultant organic layer was dried over anhydrous sodium sulfate, the solvent was distilled away under reduced pressure, the residue thus obtained was separated with chromatograph, whereby 1.65 g of 1-benzyloxycarbonyl-piperidine-4-carboxylic acid-(2R,2S)-(1-ethoxycarbonyl-1-cinnamyl)methylamide was obtained in a yield of 71%.

1.65 g (3.55 mmol) of the above prepared compound was dissolved in 30 ml of tetrahydrofuran. 0.193 g (8.88 mmol) of lithium boron hydride was added to the tetrahydrofuran solution in an ice bath, followed by dropping of a mixture of 4.5 ml of methanol and 5.5 ml of tetrahydrofuran. After stirring for two hours, 20 ml of water was further added to the reaction mixture and tetrahydrofuran was distilled away therefrom under reduced pressure. With the addition of ice and 1 N hydrochloric acid to the residue, the mixture was extracted with ethyl acetate. The resultant organic extract layer was washed with a saturated aqueous solution of sodium chloride and dried over anhydrous sodium sulfate. The solvent was distilled away under reduced pressure. 1.35 g (3.20 mmol) of the residue thus obtained was made acid with the addition of pyridine•sulfur trioxide complex in dimethyl sulfoxide in accordance with the same procedure as in Example 1-1, whereby 0.9 g of the captioned Compound No. 1-91 was obtained as an oily material in a yield of 43%.

NMR (δ, $CDCl_3$): 9.65 (s, 1H), 7.15 - 7.45 (m, 10H), 6.47 (d, J = 15.7Hz, 1H), 6.06 - 6.18 (m, 1H), 5.95 - 6.10 (m, 1H), 5.16 (s, 2H), 4.65 - 4.75 (m, 1H), 4.02 - 4.35 (m, 2H), 2.67 - 2.95 (m, 4H), 2.33 (tt, J = 11.3Hz, J = 3.9Hz, 1H), 1.50 - 1.98 (m, 4H)

**Examples 1-92 to 1-95**

The same reaction procedure as in Example 1-91 was repeated except that the carboxylic acid derivative (the compound synthesized in Reference Example 1-4) and the amine derivative (Reference Compound No. 1-67 synthesized in Reference Example 1-67) used in Example 1-91 were respectively replaced by a carboxylic acid derivative (Material 1) and an amine derivative (Material 2) shown in Table 10, whereby Compounds No. 1-92 to No. 1-95 (cyclic carboxylic acid amide derivative) shown in Table 10 were respectively obtained.

Table 11 shows the yield, melting point or state, the NMR analysis data and the $R_f$ values obtained by TLC analysis of each cylic carboxylic acid amide derivative. The developing solvent A used in the TLC analysis is ethyl acetate and the developing solvent B is a mixture of methylene chloride and acetone at a mixing ratio of 10:1.

Table 10

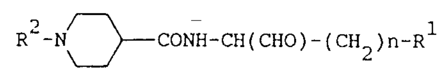

| Ex. | Material 1 (*) | Material 2 | $R^2$ | n | $R^1$ | Compound |
|---|---|---|---|---|---|---|
| 1-92 | 1-12 | L-tyrosine-ethylester | 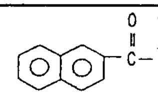 | 1 | 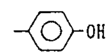 | 1-(2-naphthoyl)piperidine-4-carboxylic acid-(1S)-[1-formyl-2-(4-hydroxyphenyl)]ethylamide |
| 1-93 | 1-18 | L-tyrosine-ethylester | 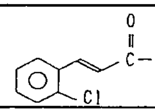 | 1 | 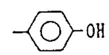 | 1-(2-chlorocinnamoyl)piperidine-4-carboxylic acid-(1S)-[1-formyl-2-(4-hydroxyphenyl)]ethylamide |
| 1-94 | 1-10 | L-tyrosine-ethylester | 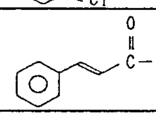 | 1 | 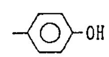 | 1-cinnamoylpiperidine-4-carboxylic acid-(1S)-[1-formyl-2-(4-hydroxyphenyl)]ethylamide |
| 1-95 | 1-30 | L-tyrosine-ethylester | 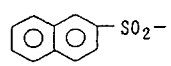 | 1 | 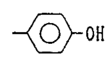 | 1-(2-naphthylsulfonyl)piperidine-4-carboxylic acid-(1S)-[1-formyl-2-(4-hydroxyphenyl)]ethylamide |

(*) Each material is indicated by the Reference Reference Example Number in which it is synthesized.

Table 11

| Ex. | Yield | Melting Point (°C) or State | NMR (δ, $CDCl_3$) | $R_f$ Values |
|---|---|---|---|---|
| 1-92 | 37 | amorphous | 9.58 (s, 1H), 7.77 - 7.93 (m, 4H), 7.32 - 7.60 (m, 3H), 6.93 (d, J=8.4Hz, 2H), 6.76 (d, J=8.4Hz, 2H), 6.30 - 6.58 (m, 1H), 4.30 - 4.83 (m, 2H), 3.55 - 4.10 (m, 1H), 2.60 - 3.22 (m, 4H), 2.30 - 2.48 (m, 1H), 1.50 - 2.00 (m, 4H) | A: 0.16 B: 0.02 |
| 1-93 | 45 | 166.6 to 178.2°C | 9.64 (s, 1H), 7.97 (d, J=15.6Hz, 1H), 7.20 - 7.65 (m, 4H), 6.97 (d, J=8.4Hz, 2H), 6.85 (d, J=15.5Hz, 1H), 6.78 (d, J=8.3Hz, 2H), 6.02 - 6.17 (m, 1H), 3.90 - 4.90 (m, 3H), 3.13 (d, J=6.4Hz, 2H), 2.55 - 3.30 (m, 2H), 2.30 - 2.50 (m, 1H) 1.35 - 1.95 (m, 4H) | A: 0.18 B: 0.02 |
| 1-94 | 30 | amorphous | 9.62 (s, 1H), 7.63 (d, J=15.4Hz, 1H), 7.30 - 7.58 (m, 5H), 6.94 (d, J=8.5Hz, 2H), 6.86 (d, J=15.4Hz, 1H), 6.79 (d, J=8.4Hz, 2H), 6.20 - 6.50 (m, 1H), 3.95 - 4.85 (m, 3H), 2.57 - 3.30 (m, 4H), 2.35 - 2.51 (m, 1H), 1.50 - 1.95 (m, 4H) | A: 0.15 B: 0.01 |
| 1-95 | 64 | 130.3 to 158.6°C | 9.57 (s, 1H), 8.33 (s, 1H), 7.88 - 8.04 (m, 3H), 7.59 - 7.78 (m, 3H), 6.92 (d, J=8.4Hz, 2H), 6.73 (d, J=8.6Hz, 2H), 5.90 - 6.12 (m, 1H), 4.62 - 4.73 (m, 1H) 3.75 - 3.90 (m, 2H), 3.07 (d, J=6.4Hz, 2H), 2.33 - 2.48 (m, 2H), 1.98 - 2.13 (m, 1H), 1.65 - 1.95 (m, 4H) | A: 0.49 B: 0.09 |

**Example 1-96**

Synthesis of cis-1-(N-benzylcarbamoyl)cyclohexane-4-carboxylic acid-(1S)-(1-formyl-2-phenyl)ethylamide (Compound No. 1-96):

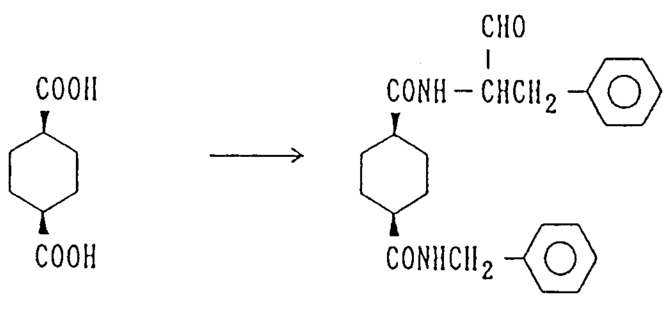

To 200 ml of a chloroform solution containing 3.0 g (17.4 mmol) of commercially available cis-cyclohexane-1,4-dicarboxylic acid and 5.32 g (34.8 mmol) of N-hydroxybenzotriazole hydrate, 100 ml of a chloroform solution of 7.18 g (34.8 mmol) of dicyclohexylcarbodiimide was added dropwise under an ice-cooled condition. After stirring for 1 hour, 50 ml of a chloroform solution containing 2.63 g (17.4 mmol) of

(2S)-2-amino-3-phenylpropanol synthesized in Reference Example 1-48 and 1.76 g (17.4 mmol) of triethylamine was added dropwise to the above prepared reaction mixture. The reaction mixture was heated to room temperature and then stirred overnight.

Insoluble components were removed from the reaction mixture by filtration. The filtrate was washed with 1 N hydrochloric acid and extracted with 100 ml of a 1 N sodium hydroxide solution twice. The resulting water layer was made acid (pH = 1) with the addition of concentrated hydrochloric acid thereto. The water layer was extracted with chloroform twice. The resultant organic extract layer was washed with a saturated aqueous solution of sodium chloride, dried over anhydrous sodium sulfate, and then concentrated under reduced pressure.

To 100 ml of a chloroform solution containing the above obtained residue and 0.857 g (8 mmol) of benzylamine, 0.958 g (5 mmol) of 1-ethyl-3-(3-dimethylaminopropyl)carbodiimide hydrochloride was added, and the reaction mixture was stirred overnight. The reaction mixture was washed successively with 1 N hydrochloric acid, a saturated aqueous solution of sodium hydrogencarbonate, and a saturated aqueous solution of sodium chloride. The reaction mixture was then dried over anhydrous sodium sulfate and concentrated under reduced pressure. The residue thus obtained was chromatographed on a silica gel column for purification, whereby 0.2 g of cis-1-(N-benzylcarbamoyl)cyclohexanecarboxylicacid-(1S)-(1-hydroxymethyl-2-phenyl)ethylamide was obtained in a yield of 2.9% from the cis-cyclohexane-1,4-dicarboxylic acid.

NMR (δ, $CDCl_3$): 7.15 - 7.40 (m, 10H), 5.85 - 6.10 (m, 2H), 4.42 (d, J = 6Hz, 2H), 4.10 - 4.25 (m, 1H), 3.67 (dd, J = 11Hz, 3Hz, 1H), 3.56 (dd, J = 11Hz, 5Hz, 1H), 2.80 - 2.95 (m, 2H), 2.67 (bs, 1H), 2.20 - 2.40 (m, 2H), 1.50 - 2.10 (m, 8H)

0.2 g (0.5 mmol) of the above prepared amide was made acid with pyridine•sulfur trioxide complex in dimethyl sulfoxide in accordance with the same procedure as in Example 1-1, whereby 0.18 g of the captioned Compound No. 1-96 was obtained as an oily material in a yield of 91%.

NMR (δ, $CDCl_3$): 9.58 (s, 1H), 7.10 - 7.40 (m, 10H), 6.10 - 6.30 (m, 1H), 5.80 - 6.00 (m, 1H), 4.69 (q, J = 7Hz, 1H), 4.43 (d, J = 5Hz, 2H), 3.15 (d, J = 7Hz, 2H), 2.20 - 2.40 (m, 2H), 1.50 - 2.20 (m, 8H)

$R_f$ values: 0.33 (Developing Solvent A)
0.04 (Developing Solvent B)

**Example 1-97**

Synthesis of trans-1-(N-benzylcarbamoyl)cyclohexane-4-carboxylic acid-(1S)-(1-formyl-2-phenyl)ethylamide (Compound No. 1-97):

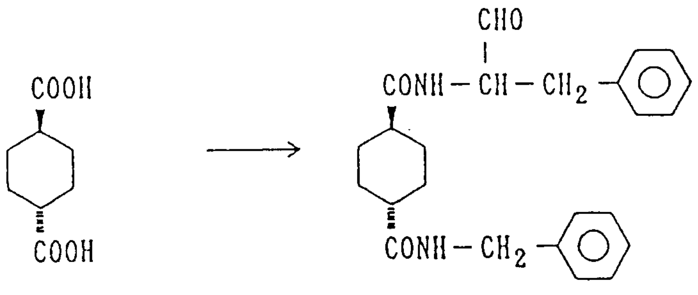

The same reaction procedure as in Example 1-96 was repeated except that the cis-cyclohexane-1,4-didicarboxylic acid used in Example 1-96 was replaced by trans-cyclohexane-1,4-carboxylic acid, whereby trans-1-(N-benzylcarbamoyl)cyclohexane-carboxylic acid-(1S)-(1-hydroxymethyl-2-phenyl)ethylamide was obtained.

NMR (δ, $CDCl_3$): 7.10 - 7.35 (m, 10H), 4.30 - 4.40 (m, 2H), 4.05 - 4.20 (m, 1H), 3.51 (d, J = 5Hz, 1H), 3.10 (d, J = 5Hz, 1H), 2.85 - 3.00 (m, 1H), 2.60 - 2.75 (m, 1H), 2.05 - 2.35 (m, 2H), 1.70 - 2.00 (m, 4H), 1.25 - 1.70 (m, 4H)

The above prepared amide was made acid with pyridine•sulfur trioxide complex in ditrimethyl sulfoxide in accordance with the same procedure as in Example 1-1, whereby the captioned Compound No. 1-97 was obtained as white crystals.

Melting point (° C): 174.9 - 180.7 (dec.)
NMR (δ, $CDCl_3$): 9.61 (s, 1H), 7.05 - 7.41 (m, 10H), 6.13 (d, J=8Hz, 1H), 5.80 - 6.05 (m, 1H), 4.67 (q, J=6Hz, 1H), 4.30 - 4.45 (m, 2H), 3.15 (d, J=6Hz, 2H), 2.05 - 2.20 (m, 2H), 1.70 - 2.00 (m, 4H), 1.40 - 1.60 (m, 4H)
$R_f$ values: 0.14 (Developing Solvent A)
0.02 (Developing Solvent B)

**Reference Example 2-1**

Synthesis of (2S)-2-benzyloxy-4-methylpentanoic acid (Reference Compound No. 2-1):

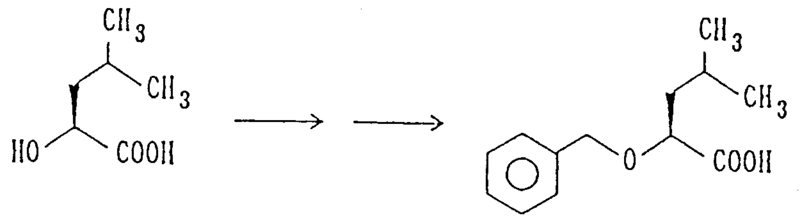

To 40 ml of a N,N-dimethylformamide solution containing 1.6 g (12.1 mmol) of (2S)-2-hydroxy-4-methylpentanoic acid synthesized in accordance with the method described in Tetrahedron, Vol 32, 1101 to 1106, 1976, and 18.64 g (109 mmol) of benzyl bromide, 13.9 g (60 mmol) of silver (I) oxide was added over a period of 40 minutes with stirring. The above prepared reaction mixture was further stirred at room temperature for 4 days and then diluted with ether, and insoluble materials were removed therefrom by filtration through Celite. The filtrate was washed successively with 1 N hydrochloric acid and a saturated aqueous solution of sodium chloride. An organic layer and a water layer in the thus washed filtrate were separated. The resulting water layer was extracted with ethyl acetate again to obtain an another organic layer. The thus extracted layer and the first obtained organic layer were dried over anhydrous sodium sulfate and distilled away under reduced pressure. 1.44 g (36 mmol) of a sodium hydroxide aqueous solution was added to 200 ml of a methanol solution containing the residue thus obtained. The reaction mixture was stirred for 18 hours and concentrated under reduced pressure. The residue was dissolved in water and washed with ether twice. The resulting water layer was made acid (pH=1) with the addition of concentrated hydrochloric acid thereto and extracted with ethyl acetate twice. A mixture of the thus extracted layer and the originally obtained organic layer was washed with a saturated aqueous solution of sodium chloride, dried over anhydrous sodium sulfate. The solvent was distilled away under reduced pressure, whereby 2.15 g of the captioned Reference Compound No. 2-1 was obtained in a yield of 80%.
NMR (δ, $CDCl_3$) 7.26 - 7.40 (m, 5H), 4.74 (d, J=11Hz, 1H), 4.44 (d, J=11Hz, 1H), 3.99 - 4.04 (m, 1H), 1.73 - 1.95 (m, 2H), 1.50 - 1.70 (m, 1H), 0.93 (d, J=6Hz, 3H), 0.84 (d, J=6Hz, 3H)

**Reference Example 2-2**

Synthesis of (2S)-2-(3-phenylpropyloxy)-4-methylpentanoic acid (Reference Compound No. 2-2):

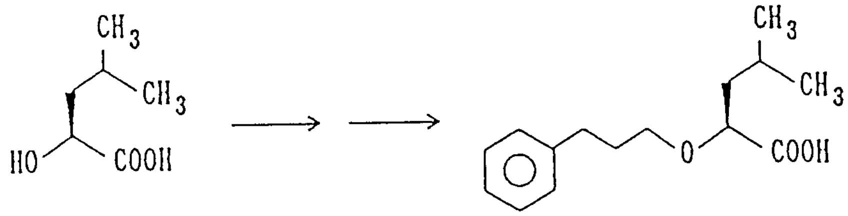

0.756 g (18.9 mmol) of sodium hydride (60% in oil) was added to 60 ml of a N,N-dimethyl-formamide suspension containing sodium chloride prepared by use of 2.5 g (18.9 mmol) of (2S)-2-hydroxy-4-methylpentanoic acid and the above reaction mixture was stirred at 90 ° C for 7 hours. 11.28 g (56.7 mmol) of 3-phenylbromopropane and 0.641 g (1.89 mmol) of tetra-n-butylammonium hydrogensulfate were added to the reaction mixture, followed by stirring at 90 ° C for 18 hours. The temperature of the reaction mixture was decreased to room temperature. The reaction mixture was added to an iced water and extracted with ether twice. The resultant organic extract layer was washed successively with 1 N hydrochloric acid and a saturated aqueous solution of sodium chloride, and then dried over anhydrous sodium sulfate. The solvent was distilled away under reduced pressure. 2.26 g (56.7 mmol) of a sodium hydroxide solution was added to 200 ml of a methanol solution containing the residue thus obtained. The above prepared reaction mixture was stirred for 18 hours and concentrated under reduced pressure. The residue thus obtained was dissolved in water and washed with ether twice. The resulting water layer was made acid (pH = 1) by the addition of concentrated hydrochloric acid and extracted with ethyl acetate twice. The resultant organic extract layer was washed with a saturated aqueous solution of sodium chloride, dried over anhydrous sodium sulfate. The solvent was distilled away under reduced pressure, whereby 1.078 g of the captioned Reference Compound No. 2-2 was obtained in a yield of 22%.

NMR (δ, $CDCl_3$) 7.15 - 7.31 (m, 5H), 3.85 - 3.95 (m, 1H), 3.59 - 3.70 (m, 1H), 3.35 - 3.45 (m, 1H), 2.65 - 2.80 (m, 2H), 1.80 - 2.00 (m, 1H), 1.50 - 1.80 (m, 2H), 0.85 - 1.00 (m, 6H)

**Reference Example 2-3**

Synthesis of (2S)-2-(N-phenylcarbamoyloxy)-4-methylpentanoic acid (Reference Compound No. 2-3):

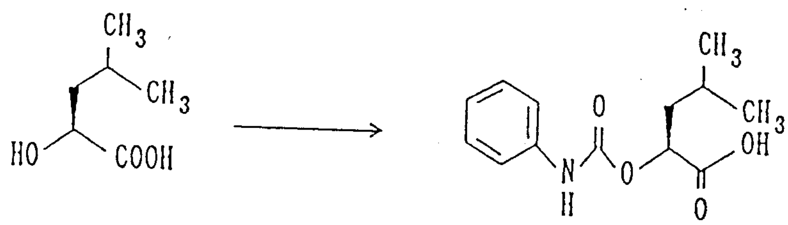

21.7 ml (0.2 mol) of isocyanic acid phenyl ester was added dropwise to a chloroform solution containing 13.3 g (0.1 mol) of (2S)-2-hydroxy-4-methylpentanoic acid and 20.3 ml (0.2 mol) of triethylamine under an ice-cooled condition with stirring. The above prepared reaction mixture was further stirred overnight and extracted with a 10% potassium carbonate solution. The resulting water layer was washed with chloroform. The water layer was made acid by the addition of concentrated hydrochloric acid, extracted with chloroform and dried over anhydrous sodium sulfate. The solvent was distilled away under reduced pressure, whereby 10.5 g of the captioned Reference Compound No. 2-3 was obtained in a yield of 42%.

NMR (δ, $CDCl_3$) 7.28 - 7.40 (m, 4H), 7.08 (t, J = 7Hz, 1H), 6.80 - 6.90 (m, 1H), 5.14 (m, 1H), 4.70 - 5.30 (m, 1H), 1.70 - 1.88 (m, 3H), 0.99 (d, J = 3Hz, 3H), 0.97 (d, J = 3Hz, 3H)

**Reference Examples 2-4 to 2-16**

The same procedure as in Reference Example 2-3 was repeated except that the phenyl isocyanate employed in Reference Example 2-3 was replaced by the isocyanic acid derivative as shown in Table 13, whereby Reference Compound No. 2-4 to 2-16 (carboxylic acid derivatives (II-a)) shown in Table 13 were obtained.

Table 12

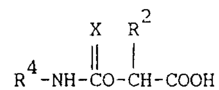 (II-a)

| Ref. Ex. | Compound | $R^4$ | $R^2$ | X | NMR(δ,$CDCl_3$) |
|---|---|---|---|---|---|
| 2-4 | (2R)-2-(N-benzyl-carbamoyloxy)-4-methyl-pentanoic acid | 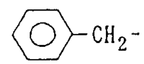 | 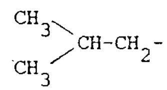 | O | 7.24 - 7.36 (m, 5H), 5.25 - 5.32 (m, 1H), 4.38 (d, J=6Hz, 2H), 4.24 - 4.30 (m, 1H), 1.58 - 1.81 (m, 3H), 0.93 - 0.98 (m, 6H) |
| 2-5 | (2S)-2-[N-(2-chlorophenyl)carbamoyloxy]-4-methylpentanoic acid | 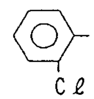 | 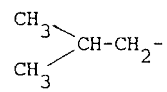 | O | 8.13 - 8.16 (m, 1H), 7.35 (dd, J=8Hz, J=2Hz, 1H), 7.28 (d, J=14Hz, 1H), 7.27(dd, J=16Hz, J=2Hz, 1H), 7.01 (dt, J=9Hz, J=2Hz, 1H), 5.12 - 5.17 (m, 1H), 1.72 - 1.91 (m, 3H), 0.97 (d, J=7Hz, 3H), 0.95 (d, J=4Hz, 3H) |
| 2-6 | (2S)-2-[N-(3-chlorophenyl)carbamoyloxy]-4-methylpentanoic acid | 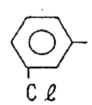 | 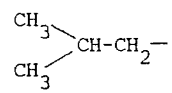 | O | 9.95 - 10.10 (brs, 1H), 7.49 (s, 1H), 7.19 (d, J=5Hz, 2H), 7.02 - 7.05 (m, 2H), 5.11 - 5.16 (m, 1H), 1.69 - 1.88 (m, 3H), 0.95 - 0.98 (m, 6H) |
| 2-7 | (2S)-2-[N-(4-chlorophenyl)carbamoyloxy]-4-methylpentanoic acid | 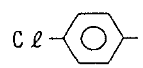 | 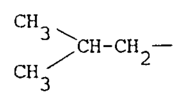 | O | 9.50 - 9.80 (brs, 1H), 7.31 (d, J=9Hz, 2H), 7.24 (d, J=9Hz, 2H), 6.90 - 7.00 (m, 1H), 5.10 - 5.15 (m, 1H), 1.68 - 1.86 (m, 3H), 0.95 - 0.98 (m, 6H) |
| 2-8 | (2S)-2-[N-(1-naphthyl)-carbamoyloxy]-4-methyl-pentane acid | 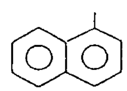 | 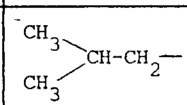 | O | 7.81 - 7.92 (m, 4H), 7.68 (d, J=8Hz, 1H), 7.42 - 7.53 (m, 4H), 5.17 - 5.22 (m, 1H), 1.60 - 1.90 (m, 3H), 0.90 - 1.10 (m, 6H) |
| 2-9 | (2S)-2-(N-propylcarbamoyl-oxy)-4-methylpentanoic acid | $CH_3-(CH_2)_2-$ | 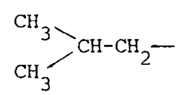 | O | 5.60 - 5.90 (brs, 1H), 5.00 - 5.00 (m, 1H), 4.88 - 4.96 (m, 1H), 3.16 (q, J=65Hz, 2H), 1.54 - 1.82 (m, 8H), 0.94 - 0.98 (m, 6H) |

Table 12

| Ref. Ex. | Compound | $R^4$ | $R^2$ | X | NMR(δ, $CDCl_3$) |
|---|---|---|---|---|---|
| 2-10 | (2R)-2-[N-(t-butyl)-carbamoyloxy)-4-methyl-pentane acid | $(CH_3)C-$ | 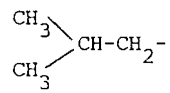 | 0 | 6.40 - 7.00 (brs, 1H), 4.92 - 5.12 (m, 1H), 4.81 - 4.90 (m, 1H), 1.61 - 1.88 (m, 3H), 1.33 (s, 9H), 0.97 (d, J=4Hz, 3Hz), 0.94 (d, J=4Hz, 3H) |
| 2-11 | (2S)-2-[N-(3,4-dichloro-phenyl)carbamoyloxy]-4-methylpentane acid | 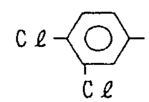 | 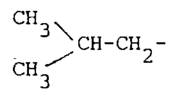 | | 7.62 (brs, 1H), 7.35 (d, J=7Hz, 1H), 7.18 (dd, J=7Hz, J=2Hz, 1H), 6.85 - 6.98 (m, 1H), 5.11 - 5.15 (m, 1H), 1.70 - 1.87 (m, 3H), 0.96 - 1.00 (m, 6H) |
| 2-12 | (2S)-2-(N-phenylthio-carbamoyloxy)-3-methyl-pentane acid | 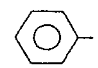 | 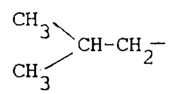 | 0 | 7.37 - 7.40 (m, 2H), 7.31 (t, J=7Hz, 2H), 7.08 (t, J=7Hz, 1H), 6.77 - 6.87 (m, 1H), 5.11 - 5.17 (m, 1H), 1.70 - 1.87 (m, 3H), 0.95 - 1.00 (m, 6H) |
| 2-13 | (2S)-2-(N-phenylcarbamoyl-oxy)-3-methylpentane-acid | 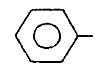 | 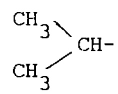 | 0 | 9.00 - 9.50 (brs, 1H), 7.26 - 7.39 (m, 4H), 7.04 - 7.09 (m, 1H), 6.90 - 7.05 (m, 1H), 5.00 (d, J=4Hz, 1H), 2.27 - 2.34 (m, 1H), 1.06 (d, J=7Hz, 3H), 1.03 (d, J=7Hz, 3H) |
| 2-14 | (2S)-2-[N-(1-naphthyl)-carbamoyloxy]-3-methyl-pentane acid | 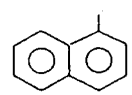 | 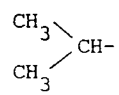 | 0 | 8.00 - 8.50 (brs, 1H), 7.67 - 7.95 (m, 5H) 7.42 - 7.53 (m, 3H), 5.03 (d, J=3Hz, 1H), 2.25 - 2.40 (m, 1H), 1.00 - 1.20 (m, 1H) |
| 2-15 | (2S)-2-(N-propylcarbamoyl-oxy)-3-methylpentane acid | 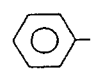 | 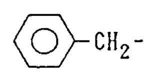 | 0 | 8.35 - 8.70 (m, 1H), 7.17 - 7.25 (m, 10H), 6.97 - 7.02 (m, 1H), 5.23 - 5.27 (m, 1H), 3.23 (dd, J=14Hz, J=4Hz, 1H), 3.10 (dd, J=14Hz, J=9Hz, 1H) |
| 2-16 | (2S)-2-[N-(1-naphthyl)-carbamoyloxy]-3-phenyl propane acid | 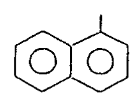 | 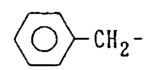 | 0 | 7.70 - 7.88 (m, 2H), 7.41 - 7.52 (m, 2H), 7.45 - 7.25 (m, 9H), 4.51 (m, 1H), 3.20 (dd, J=14Hz, J=4Hz, 1H), 3.10 - 3.35 (m, 1H), 2.99 (dd, J=14Hz, J=7Hz, 1H) |

**Reference Example 2-17**

Synthesis of (2S)-2-amino-4-(methylthio)butanol (Reference Compound No. 2-17):

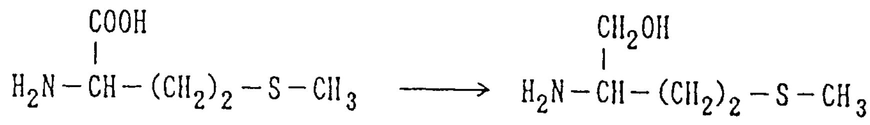

38 ml (300 mmol) of chlorotrimethylsilane was added to 200 ml of an anhydrous tetrahydrofuran suspension containing 3.3 g (150 mmol) of lithium boron hydride under an ice-cooled condition. The above prepared reaction mixture was stirred for 30 minutes and 7.5 g (50 mmol) of L-methionine was gradually added thereto, followed by stirring overnight at room temperature. Methanol was further added to the reaction mixture until the evolution of hydrogen gas ceased. The solvent was distilled away from the reaction mixture under reduced pressure. A 10% sodium hydroxide solution was added to the thus obtained residue, followed by the extraction with chloroform twice. The resultant extract layer was dried over anhydrous sodium sulfate and the solvent was distilled away under reduced pressure, whereby 5.12 g of the captioned Reference Compounds No. 2-17 was obtained in a yield of 75%.

NMR (δ, $CDCl_3$): 3.60 (dd, J = 11Hz, J = 4Hz, 1H), 3.33 (dd, J = 11Hz, J = 7Hz, 1H), 2.96 - 3.04 (m, 1H), 2.57 - 2.64 (m, 2H), 2.22 - 2.43 (m, 3H), 2.11 (s, 3H), 1.68 - 1.80 (m, 1H), 1.50 - 1.62 (m, 1H)

**Reference Example 2-18**

Synthesis of (2R)-amino-3-(2-fluorobenzylthio)propanol (Reference Compound No. 2-18):

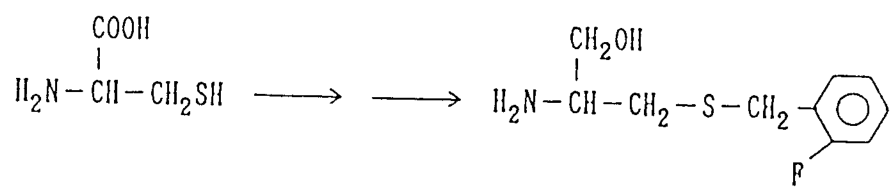

6.9 g (300 mmol) of metallic sodium was dissolved in 500 ml of methanol with stirring. 17.6 g (100 mmol) of a L-cysteine hydrochloride hydrate was added to the above prepared reaction mixture, followed by stirring at room temperature for one hour. 15.0 g (100 mmol) of 2-fluorobenzyl chloride was added dropwise to the reaction mixture, and the mixture was further stirred overnight. The solvent was distilled away from the reaction mixture under reduced pressure. The residue thus obtained was dissolved in water and washed with diethyl ether. The resulting water layer was made acid by the addition of concentrated hydrochloric acid, so that crystals separated out.

The crystals were separated from the reaction mixture by filtration, washed with water, ethanol and diethyl ether, and dried under reduced pressure, whereby 16.5 g of L-S-(2-fluorobenzyl)-cysteine was obtained in a yield of 72%.

The thus obtained L-S-(2-fluorobenzyl)-cysteine was reduced in accordance with the procedure used in Reference Example 2-17 subsequently, whereby the captioned Reference Compound No. 2-18 was obtained.

NMR (δ, $CDCl_3$) 7.20 - 7.36 (m, 2H), 7.01 - 7.13 (m, 2H), 3.75 (s, 2H), 3.62 (dd, J = 11Hz, J = 5Hz, 1H), 3.38 (dd, J = 11Hz, J = 7Hz, 1H), 2.96 - 3.04 (m, 1H), 2.61 (dd, J = 13Hz, J = 5Hz, 1H), 2.42 (dd, J = 13Hz, J = 8Hz, 1H), 2.00 - 2.10 (m, 3H)

**Reference Example 2-19**

Synthesis of (2S)-2-amino-4-phenylbutanol (Reference Compound No. 2-19):

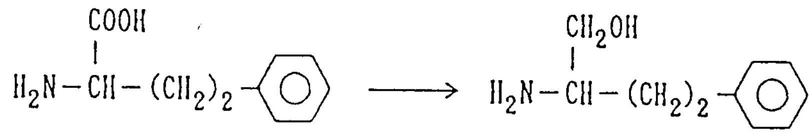

The same procedure as in Reference Example 2-17 was repeated except that the L-methionine was replaced by L-homophenylalanine, whereby the captioned Reference Compound No. 2-19 was obtained.

NMR (δ, $CDCl_3$) 7.17 - 7.31 (m, 5H), 3.60 (dd, J = 11Hz, J = 4Hz, 1H), 3.31 (dd, J = 11Hz, J = 8Hz, 1H), 2.80 - 2.88 (m, 1H), 2.60 - 2.79 (m, 2H), 1.95 (br s, 3H), 1.70 - 1.80 (m, 1H), 1.52 - 1.64 (m, 1H)

**Reference Example 2-20**

Synthesis of (2S)-2-amino-4-(2-fluorobenzylthio)butanol (Reference Compound No. 2-20):

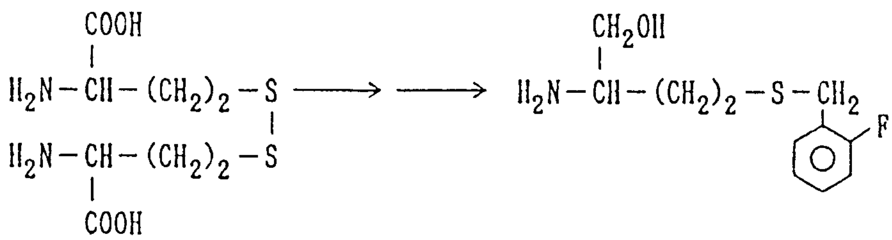

1.0 g of metallic sodium was added to liquid ammonia cooled at -78°, followed by stirring for 30 minutes. After the addition of 2.0 g (7.45 mmol) of homocystine, the above prepared reaction mixture was further stirred for 30 minutes. Ammonium chloride was added to the reaction mixture until the blue color of the reaction mixture faded and 2.17 g (15 mmol) of 2-fluorobenzyl chloride was further added thereto. The liquid ammonia was allowed to evaporate from the reaction mixture at room temperature. The residue thus obtained was dissolved in water, washed with diethyl ether, and made weakly acidic by the addition of concentrated hydrochloric acid, so that crystals were caused to separate out of the reaction mixture under a cooled condition. The crystals were separated by filtration and washed successively with water, ethanol and diethyl ether, and then dried under reduced pressure, whereby 1.80 g of (2S)-2-amino-4-(2-fluorobenzyl-thio)-butanoic acid was obtained in a yield of 81%.

The thus obtained (2S)-2-amino-4-(2-fluorobenzylthio)butanoic acid was reduced in accordance with the procedure used in Reference Example 2-17 subsequently, whereby the captioned Reference Compound No. 2-20 was obtained.

NMR (δ, $CDCl_3$) 7.13 - 7.36 (m, 2H), 7.01 - 7.13 (m, 2H), 3.75 (s, 2H), 3.55 (dd, J = 11Hz, J = 4Hz, 1H), 3.28 (dd, J = 11Hz, J = 8Hz, 1H), 2.89 - 2.97 (m, 1H), 2.48 - 2.62 (m, 2H), 1.95 - 2.10 (m, 3H), 1.65 - 1.77 (m, 1H), 1.47 - 1.59 (m, 1H)

**Reference Example 2-21**

Synthesis of (2S)-2-amino-3-phenylpropanol (Reference Compound No. 2-21):

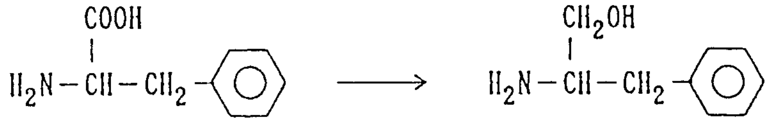

The same procedure as in Reference Example 2-17 was repeated except that the L-methionine was replaced by L-phenylalanine, whereby the captioned Reference Compound No. 2-21 was obtained.

NMR (δ, $CDCl_3$) 7.16 - 7.33 (m, 5H), 3.64 (dd, J = 11Hz, J = 4Hz, 1H), 3.38 (dd, J = 11Hz, J = 7Hz, 1H), 3.07 - 3.16 (m, 1H), 2.79 (dd, J = 14Hz, J = 5Hz, 1H), 2.52 (dd, J = 14Hz, J = 9Hz, 1H), 2.10 (br s, 3H)

**Reference Example 2-22**

Synthesis of (2R)-2-amino-3-[(4-chlorobenzyl)thio]propanol (Reference Compound No. 2-22):

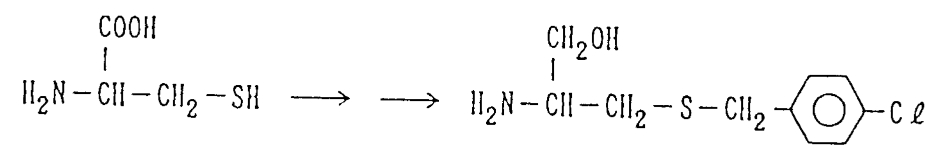

The same procedure as in Reference Example 2-18 was repeated except that the ortho-fluorobenzyl chloride was replaced by para-chlorobenzyl chloride, whereby the captioned Reference Compound No. 2-22 was obtained.

NMR (δ, $CDCl_3$) 7.23 - 7.30 (m, 5H), 3.68 (s, 2H), 3.60 (dd, J = 11Hz, J = 4Hz, 1H), 3.38 (dd, J = 11Hz, J = 7Hz, 1H), 2.92 - 3.00 (m, 1H), 2.54 (dd, J = 13Hz, J = 5Hz, 1H), 2.36 (dd, J = 13Hz, J = 8Hz, 1H), 2.16 - 2.28 (m, 3H)

**Reference Example 2-23**

Synthesis of (2S)-2-amino-4-[(4-chlorobenzyl)thio]butanol (Reference Compound No. 2-23):

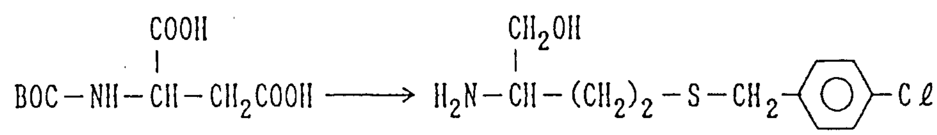

40.1 g (0.4 mol) of potassium carbonate was added to an anhydrous dimethylformamide solution containing 23.4 g (0.1 mol) of t-butoxycarbonyl-L-aspartic acid. The above prepared mixture was stirred at room temperature for 2 hours, and then 31.1 ml (0.5 mol) of methyl iodide was added dropwise thereto. The reaction mixture was further stirred overnight and water was then added thereto, followed by the extraction with ethyl acetate. The resultant organic extract layer was washed with 1 N hydrochloric acid, a saturated aqueous solution of sodium chloride, and dried over anhydrous sodium sulfate. The solvent was concentrated under reduced pressure, whereby 25.8 g of L-t-butoxycarbonylaspartic acid dimethyl ester was obtained in a yield of 99%.

25.7 g (98.4 mmol) of the above prepared diester was added to a tetrahydrofuran suspension containing 4.3 g (196.8 mmol) of lithium boron hydride. To this reaction mixture, 50 ml of methanol was further added dropwise with stirring under an ice-cooled condition, followed by stirring for 2 hours. Water was added to the reaction mixture and the solvent was distilled away therefrom under reduced pressure. The residue thus obtained was made acid by the addition of 1 N hydrochloric acid, extracted with chloroform and dried over anhydrous sodium sulfate. The solvent was distilled away under reduced pressure, whereby 18.5 g of a diol was obtained in a yield of 92%.

83 ml (675 mmol) of 2,2-dimethoxypropane and 1.28 g (6.75 mmol) of paratoluene sulfonic acid were added to a methylene chloride solution containing 27.6 g (153 mmol) of the above prepared diol. The reaction mixture was stirred overnight at room temperature, washed with a saturated aqueous solution of sodium hydrogencarbonate, and dried over anhydrous sodium sulfate. The solvent was distilled away from the reaction mixture under reduced pressure. The thus obtained residue was chromatographed on a silica gel column for purification, whereby 19.9 g of (4S)-N-t-butoxycarbonyl-2,2-dimethyl-4-(2-hydroxyethyl)-1,3-oxazolidine in a yield of 60%.

NMR (δ, $CDCl_3$): 4.18 - 4.27 (m, 1H), 3.99 - 4.04 (m, 1H), 3.50 - 3.71 (m, 3H), 2.70 - 3.00 (br s, 1H), 1.70 - 1.90 (m, 2H), 1.55 (s, 6H), 1.50 (s, 9H)

2.77 g (27.4 mmol) of triethylamine was added to an ethyl acetate solution containing 5.6 g (22.8 mmol) of the above prepared (4S)-N-t-butoxycarbonyl-2,2-dimethyl-4-(2-hydroxyethyl)-1,3-oxazolidine. Under an ice-cooled condition, 2.2 ml (27.4 mmol) of methanesulfonyl chloride was further added dropwise to the above reaction mixture, followed by stirring for 2 hours. The reaction mixture was washed with a saturated aqueous solution of sodium chloride and dried over anhydrous sodium sulfate. The solvent was distilled away from the reaction mixture under reduced pressure, whereby 7.38 g of a methanesulfonate was obtained in a yield of 100%.

1.05 g (26.2 mmol) of sodium hydride (60% in oil) was added to an anhydrous N,N-dimethylformamide solution containing 3.72 ml (28.6 mmol) of 4-chlorobenzylmercaptan, followed by stirring at room temperature for 30 minutes. With the addition of 7.7 g (23.8 mmol) of the above prepared methanesulfonate, the reaction mixture was further stirred overnight. With the addition of water, the reaction mixture was extracted with ethyl acetate. The resultant organic extract layer was washed successively with 1 N hydrochloric acid and a saturated aqueous solution of sodium chloride, and dried over anhydrous sodium sulfate. The solvent was distilled away from the reaction mixture under reduced pressure and the residue thus obtained was chromatographed on a silica gel column for purification, whereby 3.95 g of (4S)-N-t-butoxycarbonyl-2,2-dimethyl-4-[2-(4-chlorobenzylthio)]ethyl-1,3-oxazolidine was obtained in a yield of 43%.

NMR (δ, $CDCl_3$) 7.28 (d, J=10Hz, 2H), 7.24 (d, J=10Hz, 2H), 3.94 - 4.02 (m, 1H), 3.85 - 3.92 (m, 1H), 3.68 (s, 2H), 3.66 - 3.70 (m, 1H), 2.32 - 2.42 (m, 2H), 1.70 - 2.08 (m, 2H), 1.56 (s, 3H), 1.52 (s, 3H), 1.46 (s, 9H)

8 ml of ethyl acetate containing 4 N hydrogen chloride (4 N HCl - AcOEt) was added to a methanol solution containing 1.70 g (4.4 mmol) of the above prepared (4S)-N-t-butoxycarbonyl-2,2-dimethyl-4-[2-(4-chlorobenzylthio]ethyl-1,3-oxazolidine. This reaction mixture was stirred overnight under an ice-cooled condition, and the solvent was distilled away therefrom under reduced pressure. The residue thus obtained was dissolved in water and washed with diethyl ether. The resulting water layer was made basic by the addition of a 10% potassium carbonate solution and extracted with chloroform. The extract layer was dried over anhydrous sodium sulfate. The solvent was distilled away under reduced pressure, whereby 0.81 g of the captioned Reference Compound No. 2-23 was obtained in a yield of 75%.

NMR (δ, $CDCl_3$): 7.23 - 7.30 (m, 4H), 3.68 (s, 2H), 3.54 (dd, J=11Hz, J=4Hz, 1H), 3.27 (dd, J=11Hz, J=7Hz, 1H), 2.88 - 2.98 (m, 1H), 2.39 - 2.58 (m, 2H), 1.77 (br s, 3H), 1.64 - 1.74 (m, 1H), 1.45 - 1.55 (m, 1H)

## Example 2-1

Synthesis of (2S)-2-benzyloxy-4-methylpentanoic acid-(1S)-(1-formyl-3-methylthio)propylamide (Compound No. 2-1):

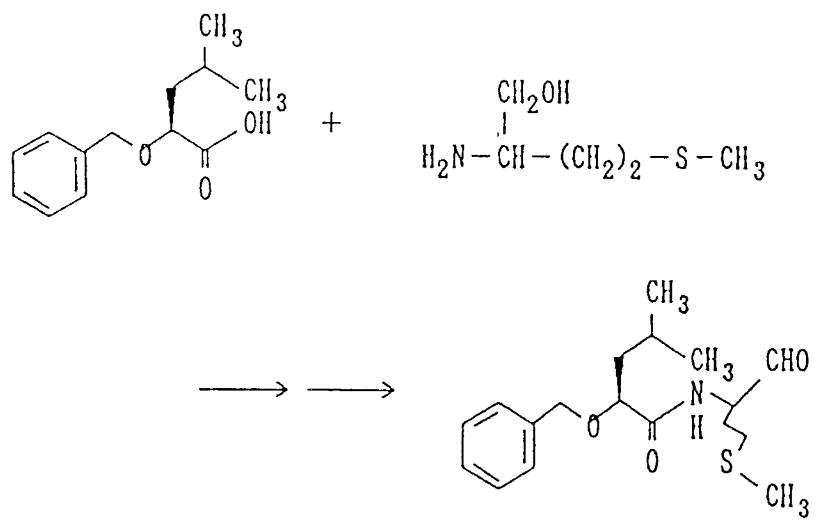

A methylene chloride solution containing 0.752 g (3.38 mmol) of (2S)-2-benzyloxy-4-methylpentanoic acid and 0.267 g (3.38 mmol) of pyridine synthesized in Reference Example 2-1 was cooled in an ice bath containing sodium chloride. 407 mg (3.38 mmol) of pivalic acid chloride was added dropwise to the above prepared solution with stirring. 20 minutes later, a methylene chloride solution containing 0.457 g (3.38 mmol) of (2S)-2-amino-4-(methylthio)butanol and 0.342 g (3.38 mmol) of triethylamine was further added dropwise to the reaction mixture. The temperature of the reaction mixture was raised to room temperature. The reaction mixture was stirred for 18 hours, washed with 1 N hydrochloric acid, a saturated aqueous solution of sodium hydrogencarbonate and a saturated aqueous solution of sodium chloride, and then dried over anhydrous sodium sulfate. The solvent was distilled away from the reaction mixture under reduced pressure. The residue thus obtained and 1.36 g (13.52 mmol) of triethylamine were dissolved in a solvent containing dimethylsulfoxide and methylene chloride at a mixing ratio of 1:1, and cooled in an ice bath containing sodium chloride.

An anhydrous dimethylsulfoxide solution containing 2.15 g (13.52 mmol) of pyridine•sulfur trioxide complex was added dropwise to the cooled reaction mixture. 30 minutes later, the reaction mixture was added to an iced water and extracted with ethyl acetate four times. The resultant organic extract layer was washed successively with a 10% citric acid aqueous solution, water, a saturated aqueous solution of sodium hydrogencarbonate, a saturated aqueous solution of sodium chloride, and then dried over anhydrous sodium sulfate. The solvent was distilled away under reduced pressure. The residue thus obtained was chromatographed on a silica gel column for purification, whereby 0.535 g of the captioned Compound No. 2-1 was obtained in a yield of 50%.

| | |
|---|---|
| Melting Point (°C) | 82.4 to 84.1 |
| NMR (δ, $CDCl_3$) | 9.62 (s, 1H), 7.30 - 7.45 (m, 5H), 7.22 (d, J=7.8Hz, 1H), 4.60 - 4.70 (m, 2H), 4.50 (d, J=12Hz, 1H), 3.91 - 3.95 (m, 1H), 2.45 - 2.60 (m, 2H), 2.20 - 2.35 (m, 1H), 2.07 (s, 3H), 1.80 - 2.00 (m, 1H), 1.50 - 1.70 (m, 3H), 0.93 (d, J=6Hz, 3H), 0.87 (d, J=6Hz, 3H) |
| $R_f$ values | 0.26 (Developing Solvent A: mixture of hexane and ethyl acetate at a mixing ratio of 1:1) |
| | 0.35 (Developing Solvent B: mixture of methylene chloride and acetone at a mixing ratio of 10:1) |

**Examples 2-2 to 2-29**

The same procedure as in Example 2-1 was repeated except that the (2S)-2-benzyloxy-4-methylpentanoic acid and the (2S)-2-amino-4-(methylthio)butanol used in Example 2-1 were respectively replaced by a carboxylic acid derivative (Material 1) and an amine derivative (Material 2) shown in Table 13, whereby Compound No. 2-2 to 2-29 (oxy acid derivatives) as given in Table 14 were obtained.

Furthermore, Table 15 shows the yield, the melting point or state, the NMR analysis data and the $R_f$ values obtained by TLC analysis of each of the prepared oxy acid derivatives. The developing solvent A used in the TLC analysis is a mixture of hexane and ethyl acetate at a mixing ratio of 1:1, and the developing solvent B is a mixture of methylene chloride and acetone at a mixing ratio of 10:1.

Table 13

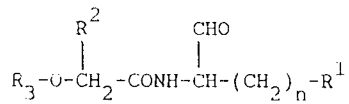

| Ex. | Material a (*) | Material a (*) | $R_3$ | $R_2$ | n | $R_1$ | Compound |
|---|---|---|---|---|---|---|---|
| 2-2 | 2-2 | 2-17 | 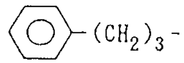 | 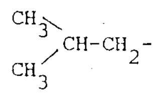 | 2 | $-S-CH_3$ | (2S)-2-(3-phenylpropyloxy-4-methylpentanoic acid-(1S)-(1-formyl-3-methylthio)propylamide |
| 2-3 | 2-3 | 2-17 | 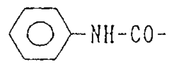 | 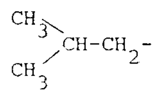 | 2 | $-S-CH_3$ | (2S)-2-(N-phenylcarbamoyloxy)-4-methylpentanoic acid-(1S)-(1-formyl-3-methylthio)propylamide |
| 2-4 | 2-4 | 2-17 | 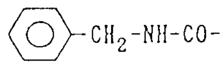 | 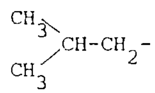 | 2 | $-S-CH_3$ | (2S)-2-(N-benzylcarbamoyloxy)-4-methylpentanoic acid-(1S)-(1-formyl-3-methylthio)propylamide |
| 2-5 | 2-4 | 2-18 | 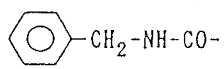 | 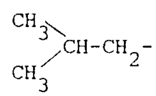 | 1 | 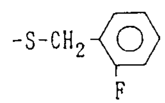 | (2S)-2-(N-benzylcarbamoyloxy-4-methylpentanoic acid-(1R)-[1-formyl-2-(2-fluorobenzylthio)]ethylamide |
| 2-6 | 2-3 | 2-18 | 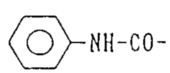 | 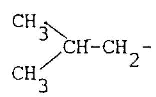 | 1 | 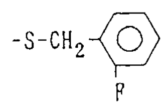 | (2S)-2-(N-phenylcarbamoyloxy)-4-methylpentanoic acid-(1R)-[1-formyl-2-(2-fluorobenzylthio)]ethylamide |

(*) Each material is indicated by the Reference Example Number in which it is synthesized.

Table 13

| Ex. | Material a (*) | Material a (*) | R3 | R2 | n | R1 | Compound |
|---|---|---|---|---|---|---|---|
| 2-7 | 2-5 | 2-18 | 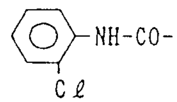 | 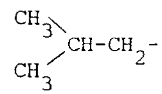 | 1 | 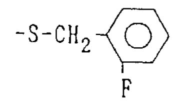 | (2S)-2-[N-(2-chlorophenyl)carbamoyl-oxy]-4-methylpentanoic acid-(1R)-[1-formyl-2-(2-fluorobenzylthio)]ethyl-amide |
| 2-8 | 2-6 | 2-18 | 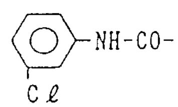 | 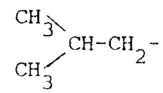 | 1 | 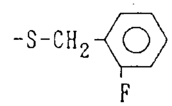 | (2S)-2-[N-(3-chlorophenyl)carbamoyl-oxy]-4-methylpentanoic acid-(1R)-[1-formyl-2-(2-fluorobenzylthio)]ethyl-amide |
| 2-9 | 2-7 | 2-18 | 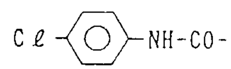 | 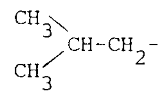 | 1 | 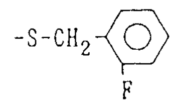 | (2S)-2-[N-(4-chlorophenyl)carbamoyl-oxy]-4-methylpentanoic acid-(1R)-[1-formyl-2-(2-fluorobenzylthio)]ethyl-amide |
| 2-10 | 2-8 | 2-18 | 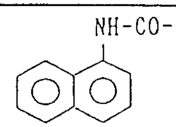 | 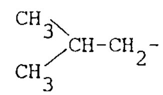 | 1 | 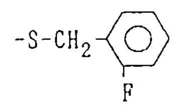 | (2S)-2-[N-(1-naphthyl)carbamoyloxy]-4-methylpentanoic acid-(1R)-[1-formyl-2-(2-fluorobenzylthio)]ethyl-amide |
| 2-11 | 2-9 | 2-18 | $CH_3-(CH_2)_2-NH-CO-$ | 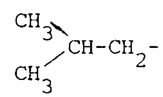 | 1 | 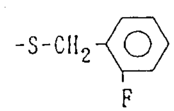 | (2S)-2-[N-propylcarbamoyloxy]-4-methylpentanoic acid-(1R)-[1-formyl-2-(2-fluorobenzylthio)]ethylamide |

(*) Each material is indicated by the Reference Example Number in which it is synthesized.

Table 13

| Ex. | Material a (*) | Material a (*) | $R_3$ | $R_2$ | n | $R_1$ | Compound |
|---|---|---|---|---|---|---|---|
| 2-12 | 2-10 | 2-18 | $(CH_3)_3C-NH-CO-$ | 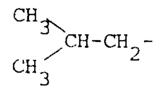 | 1 | 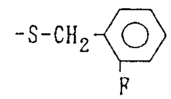 | (2S)-2-(N-t-butylcarbamoyloxy)-4-methylpentanoic acid-(1R)-[1-formyl-2-(2-fluorobenzylthio)]ethylamide |
| 2-13 | 2-8 | 2-19 | 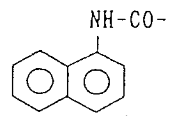 | 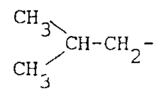 | 2 | 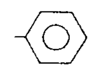 | (2S)-2-[N-(1-naphthyl)carbamoyloxy]-4-methylpentanoic acid-(1S)-(1-formyl-3-phenyl)propylamide |
| 2-14 | 2-8 | 2-17 | 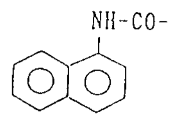 | 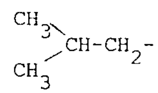 | 2 | $-S-CH_3$ | (2S)-2-[N-(1-naphthyl)carbamoyloxy]-4-methylpentanoic acid-(1S)-(1-formyl-3-methylthio)propylamide |
| 2-15 | 2-11 | 2-17 | 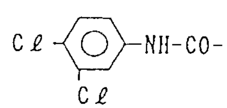 | 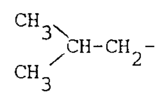 | 2 | $-S-CH_3$ | (2S)-2-[N-(3,4-dichlorophenyl)-carbamoyloxy]-4-methylpentanoic acid-(1S)-(1-formyl-3-methylthio)propyl-amide |
| 2-16 | 2-12 | 2-17 | 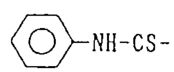 | 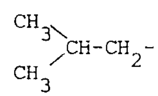 | 2 | $-S-CH_3$ | (2S)-2-(N-phenylthiocarbamoyloxy)-4-methylpentanoic acid-(1S)-(1-formyl-3-methylthio)propylamide |
| 2-17 | 2-3 | 2-20 | 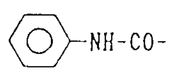 | 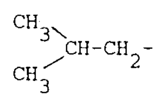 | 2 | 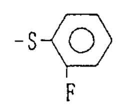 | (2S)-2-(N-phenylcarbamoyloxy)-4-methylpentanoic acid-(1S)-[1-formyl-3-(2-fluorobenzyl)thio)]propylamide |

(*) Each material is indicated by the Reference Example Number in which it is synthesized.

Table 13

| Ex. | Material a (*) | Material a (*) | $R_3$ | $R_2$ | n | $R_1$ | Compound |
|---|---|---|---|---|---|---|---|
| 2-18 | 2-8 | 2-20 | 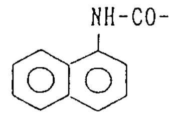 | 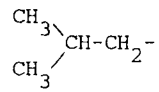 | 2 | 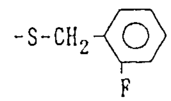 | (2S)-2-[N-(1-naphthyl)carbamoyloxy]-4-methylpentanoic acid-(1S)-[1-formyl-3-(2-fluorobenzylthio)]propylamide |
| 2-19 | 2-3 | 2-21 | 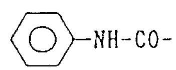 | 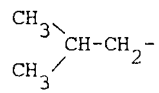 | 1 | 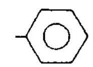 | (2S)-2-(N-phenylcarbamoyloxy)-4-methylpentanoic acid-(1S)-(1-formyl-2-phenyl)ethylamide |
| 2-20 | 2-8 | 2-21 | 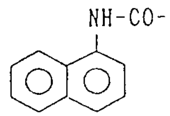 | 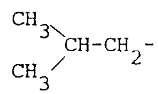 | 1 | 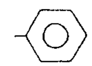 | (2S)-2-[N-(1-naphthyl)carbamoyloxy]-4-methylpentanoic acid-(1S)-(1-formyl-2-phenyl)ethylamide |
| 2-21 | 2-13 | 2-17 | 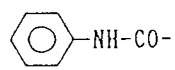 | 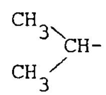 | 2 | $-S-CH_3$ | (2S)-2-(N-phenylcarbamoyloxy)-3-methylbutanoic acid-(1S)-(1-formyl-3-methylthio)propylamide |
| 2-22 | 2-14 | 2-17 | 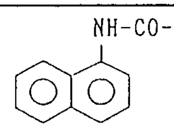 | 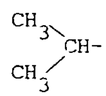 | 2 | $-S-CH_3$ | (2S)-2-[N-(1-naphthyl)carbamoyloxy]-3-methylbutanoic acid-(1S)-(1-formyl-3-methylthio)propylamide |
| 2-23 | 2-15 | 2-17 | 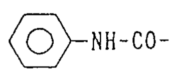 | 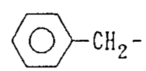 | 2 | $-S-CH_3$ | (2S)-2-(N-phenylcarbamoyloxy)-3-phenylpropanoic acid-(1S)-(1-formyl-3-methylthio)propylamide |

(*) Each material is indicated by the Reference Example Number in which it is synthesized.

Table 13

| Ex. | Material a (*) | Material a (*) | $R_3$ | $R_2$ | n | $R_1$ | Compound |
|---|---|---|---|---|---|---|---|
| 2-24 | 2-16 | 2-17 | 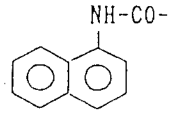 | $CH_3$<br>$CH-CH_2-$<br>$CH_3$ | 2 | $-S-CH_3$ | (2S)-2-[N-(1-naphthyl)phenylcarbamoyloxy]-3-phenylpropanoic acid-(1S)-(1-formyl-3-methylthio)propylamide |
| 2-25 | 2-13 | 2-22 | 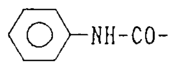 | $CH_3$<br>$CH-$<br>$CH_3$ | 1 | 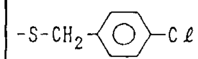 | (2S)-2-(N-phenylcarbamoyloxy)-3-methylpentanoic acid-(1R)-(1-formyl-2-(4-chlorobenzylthio)]ethylamide |
| 2-26 | 2-13 | 2-21 | 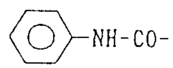 | $CH_3$<br>$CH-$<br>$CH_3$ | 1 | 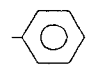 | (2S)-2-(N-phenylcarbamoyloxy)-3-methylbutanoic acid-(1S)-(1-(formyl-2-phenyl)ethylamide |
| 2-27 | 2-3 | 2-23 | 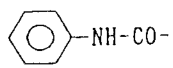 | $CH_3$<br>$CH-CH_2-$<br>$CH_3$ | 2 | 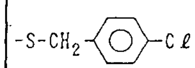 | (2S)-2-(N-phenylcarbamoyloxy)-4-methylpentanoic acid-(1S)-[1-formyl-3-(4-chlorobenzylthio)]propylamide |
| 2-28 | 2-13 | 2-23 | 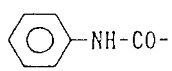 | $CH_3$<br>$CH-$<br>$CH_3$ | 2 | 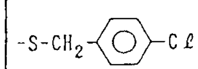 | (2S)-2-(N-phenylcarbamoyloxy)-3-methylbutanoic acid-(1S)-[1-formyl-3-(4-chlorobenzylthio)]propylamide |
| 2-29 | 2-3 | 1-52 | 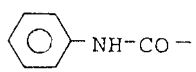 | $CH_3$<br>$CH-CH_2-$<br>$CH_3$ | 1 | 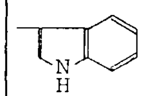 | (2S)-2-(N-phenylcarbamoyloxy)-4-methylpentanoic acid-(1S)-[1-formyl-2-(3-indolyl)]ethylamide |

(*) Each material is indicated by the Reference Example Number in which it is synthesized.

Table 14

| Ex. | Yield (%) | Melting Point (°C) or State | NMR(δ, $CDCl_3$) | $R_f$ Values |
|---|---|---|---|---|
| 2-2 | 49 | amorphous | 9.63 (s, 1H), 7.15 - 7.35 (m, 6H), 4.61 - 4.68 (m, 1H) 3.75 - 3.80 (m, 1H), 3.60 - 3.66 (m, 1H), 3.45 - 3.51 (m, 1H), 2.70 - 2.80 (m, 2H), 2.45 - 2.60 (m, 2H), 2.20 - 2.40 (m, 1H), 2.08 (s, 3H), 1.80 - 2.10 (m, 3H) 1.50 - 1.70 (m, 2H), 0.95 (d, J=6Hz, 3H), 0.94 (d, J=6Hz, 3H) | A:0.27 B:0.43 |
| 2-3 | 50 | oily | 9.62 (s, 1H), 7.30 - 7.45 (m, 4H), 7.10 (t, J=7.5Hz, 1H), 6.85 - 7.05 (m, 2H), 5.23 (t, J=7Hz, 1H), 4.57 (q, J=6Hz, 1H), 2.45 - 2.65 (m, 2H), 2.25 - 2.40 (m, 1H), 1.90 - 2.10 (m, 4H), 1.70 - 1.90 (m, 3H), 0.98 (d, J=6Hz, 6H) | A:0.21 B:0.32 |
| 2-4 | 45 | oily | 9.61 (s, 1H), 7.25 - 7.42 (m, 5H), 6.90 - 6.95 (m, 1H) 5.26 - 5.38 (m, 1H), 5.14 - 5.22 (m, 1H), 4.52 - 4.64 (m, 1H), 4.39 (d, J=4.5Hz, 2H), 2.40 - 2.60 (m, 2H), (m, 2H), 2.25 - 2.40 (m, 1H), 1.90 - 2.10 (m, 4H), 1.65 - 1.85 (m, 3H), 0.95 (d, J=5.4Hz, 6H) | A:0.16 B:0.26 |
| 2-5 | 35 | 95.6 - 97.2 | 9.55 (s, 1H), 7.23 - 7.34 (m, 7H), 7.00 - 7.13 (m, 2H) 6.98 (d, J=6Hz, 1H), 5.19 - 5.25 (m, 2H), 4.62 (q, J=6Hz, 1H), 4.38 - 4.41 (m, 2H), 3.75 (d, J=9Hz, 2H), 2.96 (q, J=6Hz, 2H), 1.58 - 1.78 (m, 3H), 0.94 - 0.97 (m, 6H) | A:0.17 B:0.30 |
| 2-6 | 51 | oily | 9.55 (s, 1H), 7.20 - 7.42 (m, 7H), 6.98 - 7.11 (m, 3H) 6.90 - 6.95 (m, 1H), 5.24 - 5.30 (m, 1H), 4.63 (dt, J=4Hz, 1H), 3.73 - 3.78 (m, 2H), 2.95 - 3.00 (m, 2H), 1.69 - 1.83 (m, 3H), 0.97 - 0.98 (m, 6H) | A:0.27 B:0.41 |
| 2-7 | 30 | oily | 9.56 (s, 1H), 8.12 (d, J=8Hz, 1H), 6.90 - 7.40 (m, 8H) 6.95 (d, J=6Hz, 1H), 5.20 - 5.30 (m, 1H), 4.60 - 4.70 (m, 1H), 3.75 (s, 2H), 2.90 - 3.05 (m, 2H), 1.70 - 1.90 (m, 3H), 0.98 (d, J=6Hz, 6H) | A:0.28 B:0.46 |
| 2-8 | 29 | amorphous | 9.55 (s, 1H), 7.52 (bs, 1H), 6.90 - 7.30 (m, 8H), 6.94 (bs, 1H), 5.25 (t, J=6Hz, 1H), 4.60 - 4.70 (m, 1H), 3.78 (s, 2H), 2.95 - 3.05 (m, 2H), 1.70 - 1.90 (m, 3H) 0.90 - 1.10 (m, 6H) | A:0.25 B:0.40 |
| 2-9 | 51 | amorphous | 9.56 (s, 1H), 6.90 - 7.40 (m, 10H), 5.26 (t, J=6Hz, 1H), 4.60 - 4.70 (m, 1H), 3.78 (s, 2H), 2.90 - 3.05 (m, 2H), 1.70 - 1.90 (m, 3H), 0.90 - 1.05 (m, 6H) | A:0.27 B:0.40 |

Table 14

| Ex. | Yield (%) | Melting Point (°C) or State | NMR(δ, $CDCl_3$) | $R_f$ Values |
|---|---|---|---|---|
| 2-10 | 39 | amorphous | 9.54 (bs, 1H), 6.90 - 8.00 (m, 13H), 5.34 (t, J=6Hz, 1H), 4.55 - 4.70 (m, 1H), 3.71 (d, J=3Hz, 2H), 2.85 - 3.05 (m, 2H), 1.80 - 2.00 (m, 3H), 0.90 - 1.15 (m, 6H) | A:0.21 B:0.36 |
| 2-11 | 52 | oily | 9.57 (s, 1H), 6.90 - 7.40 (m, 5H), 5.19 (t, J=6Hz, 1H) 4.90 - 5.00 (m, 1H), 4.58 - 4.65 (m, 1H), 3.77 (d, J=3Hz, 2H), 3.10 - 3.25 (m, 2H), 2.97 (t, J=6Hz, 2H), 1.60 - 1.85 (m, 3H), 1.45 - 1.60 (m, 2H), 0.85 - 1.05 (m, 9H) | A:0.18 B:0.30 |
| 2-12 | 34 | oily | 9.55 (s, 1H), 7.00 - 7.40 (m, 4H), 6.95 (d, J=6Hz, 2H) 5.05 - 5.20 (m, 1H), 4.85 (bs, 1H), 4.61 (q, J=6Hz, 1H), 3.78 (d, J=6Hz, 2H), 2.90 - 3.05 (m, 2H), 2.60 - 2.80 (m, 3H), 1.33 (s, 9H), 0.94 (d, J=6Hz, 6H) | A:0.26 B:0.36 |
| 2-13 | 56 | amorphous | 9.52 (bs, 1H), 7.10 - 8.11 (m, 14H), 5.25 - 5.35 (m, 1H), 4.45 - 4.55 (m, 1H), 2.45 - 2.80 (m, 2H), 1.45 - 2.10 (m, 5H), 0.80 - 1.10 (m, 6H) | A:0.23 B:0.38 |
| 2-14 | 24 | 119.5 - 124.8 | 9.62 (s, 1H), 7.72 - 7.96 (m, 1H), 7.46 - 7.59 (m, 3H) 6.95 - 7.30 (m, 2H), 5.26 - 5.31 (m, 1H), 4.52 - 4.63 (m, 1H), 2.42 - 2.58 (m, 3H), 2.22 - 2.37 (m, 1H), 2.03 (s, 3H), 1.69 - 1.90 (m, 3H), 0.90 - 1.21 (m, 6H) | A:0.28 B:0.20 |
| 2-15 | 22 | oily | 9.65 (s, 1H), 7.62 - 7.64 (m, 1H), 7.37 (dd, J=9Hz, J=3Hz, 1H), 7.20 - 7.25 (m, 2H), 7.12 - 7.18 (m, 1H), 6.88 - 6.97 (m, 1H), 5.15 - 5.20 (m, 1H), 4.60 - 4.66 (m, 1H), 2.48 - 2.60 (m, 2H), 2.27 - 2.36 (m, 2H), 2.05 (s, 3H), 1.72 - 1.84 (m, 3H), 0.97 - 1.99 (m, 6H) | A:0.30 B:0.24 |
| 2-16 | 30 | oily | 9.63 (s, 1H), 7.30 - 7.42 (m, 4H), 7.10 (t, J=7Hz, 1H) 6.90 - 7.01 (m, 2H), 5.21 - 5.29 (m, 1H), 4.50 - 4.68 (m, 1H), 2.45 - 2.58 (m, 3H), 2.26 - 2.38 (m, 1H), 2.06 (s, 3H), 1.75 - 1.84 (m, 3H), 0.96 - 0.99 (m, 6H) | A:0.32 B:0.25 |
| 2-17 | 48 | oily | 9.56 (s, 1H), 7.12 - 7.41 (m, 6H), 7.00 - 7.12 (m, 3H) 6.83 - 6.92 (m, 2H), 5.20 - 5.28 (m, 1H), 4.52 - 4.64 (m, 1H), 3.71 (s, 2H), 2.47 - 2.55 (m, 2H), 2.20 - 2.37 (m, 1H), 1.92 - 2.04 (m, 1H), 1.71 - 1.81 (m, 3H) 0.95 - 0.98 (m, 6H) | A:0.37 B:0.32 |

Table 14

| Ex. | Yield (%) | Melting Point (°C) or State | NMR(δ, $CDCl_3$) | $R_f$ Values |
|---|---|---|---|---|
| 2-18 | 8 | 99.8 - 104.0 | 9.56 (s, 1H), 7.86 - 7.94 (m, 3H), 7.70 - 7.73 (m, 1H) 7.45 - 7.58 (m, 3H), 7.03 - 7.24 (m, 3H), 6.98 - 7.08 (m, 2H), 5.26 - 5.31 (m, 1H), 4.50 - 4.61 (m, 1H), 3.64 - 3.68 (m, 2H), 2.38 - 2.55 (m, 1H), 2.20 - 2.31 (m, 1H), 1.68 - 1.98 (m, 5H), 0.90 - 1.00 (m, 6H) | A:0.31 B:0.21 |
| 2-19 | 60 | 117.5 - 122.1 | 9.64 (s, 1H), 7.21 - 7.39 (m, 7H), 7.08 - 7.15 (m, 3H) 6.67 - 6.77 (m, 1H), 6.61 - 6.71 (m, 1H), 5.22 (t, J=7Hz, 1H), 4.71 (q, J=7Hz, 1H), 3.21 (d, J=6Hz, 2H), 1.66 - 1.79 (m, 3H), 0.95 (d, J=6Hz, 6H) | A:0.31 B:0.21 |
| 2-20 | 9 | 123.4 - 129.2 | 9.62 (s, 1H), 7.71 - 7.91 (m, 4H), 7.45 - 7.57 (m, 3H) 7.00 - 7.30 (m, 6H), 6.60 - 6.80 (m, 1H), 5.27 (t, J=7Hz, 1H), 4.61 - 4.73 (m, 1H), 3.12 - 3.24 (m, 2H), 1.52 - 1.83 (m, 3H), 0.83 - 1.03 (m, 6H) | A:0.27 B:0.17 |
| 2-21 | 36 | 110.1 - 120.4 | 9.61 (s, 1H), 7.27 - 7.42 (m, 5H), 6.97 - 7.12 (m, 2H) 5.06 (d, J=5Hz, 1H), 4.56 (q, J=5Hz, 1H), 2.51 - 2.57 (m, 2H), 2.27 - 2.36 (m, 2H), 2.05 (s, 3H), 1.99 - 2.04 (m, 1H), 1.00 - 1.06 (m, 6H) | A:0.40 B:0.20 |
| 2-22 | 23 | 137.2 - 138.7 | 9.61 (s, 1H), 7.88 - 7.99 (m, 3H), 7.73 - 7.83 (m, 2H) 7.46 - 7.59 (m, 2H), 6.90 - 7.30 (m, 2H), 5.12 (d, J=5Hz, 1H), 4.50 - 4.70 (m, 1H), 2.26 - 2.55 (m, 4H), 2.03 (s, 3H), 1.90 - 2.00 (m, 1H), 0.90 - 1.10 (m, 6H) | A:0.36 B:0.15 |
| 2-23 | 32 | 112.5 - 116.2 | 9.51 (s, 1H), 7.10 - 7.37 (m, 10H), 6.92 - 7.03 (m, 1H), 6.72 - 6.88 (m, 1H), 5.44 - 5.48 (m, 1H), 4.48 - 4.54 (m, 1H), 2.17 - 2.40 (m, 3H), 1.98 (s, 3H), 1.87 - 1.95 (m, 1H) | A:0.41 B:0.32 |
| 2-24 | 7 | 127.0 - 116.2 | 9.51 (s, 1H), 7.42 - 7.92 (m, 7H), 6.90 - 7.40 (m, 7H) 5.52 (t, J=5Hz, 1H), 4.40 - 4.60 (m, 1H), 3.42 - 3.49 (m, 2H), 2.00 - 2.45 (m, 3H), 1.95 (br s, 3H), 1.81 - 1.85 (m, 1H) | A:0.32 B:0.16 |
| 2-25 | 37 | 118.1 - 125.4 | 9.53 (s, 1H), 7.10 - 7.42 (m, 9H), 6.97 - 7.08 (m, 2H) 5.07 - 5.13 (m, 1H), 4.57 - 4.67 (m, 1H), 3.65 (s, 2H) 2.86 - 2.92 (m, 2H), 2.25 - 2.40 (m, 1H), 0.99 - 1.06 (m, 6H) | A:0.44 B:0.28 |

Table 14

| Ex. | Yield (%) | Melting Point (°C) or State | NMR(δ, $CDCl_3$) | $R_f$ Values |
|---|---|---|---|---|
| 2-26 | 19 | 112.0 - 116.2 | 9.62 (s, 1H), 7.08 - 7.40 (m, 10H), 6.80 (br s, 1H), 6.62 - 6.64 (m, 1H), 5.05 (d, J=5Hz, 1H), 4.71 (q, J=7Hz, 1H), 3.13 - 3.23 (m, 2H), 1.60 - 1.75 (m, 1H), 0.98 (d, J=7Hz, 3H), 0.91 (q, J=7Hz, 3H) | A:0.36 B:0.23 |
| 2-27 | 26 | oily | 9.58 (s, 1H), 7.10 - 7.42 (m, 9H), 6.92 - 7.00 (m, 1H) 6.78 - 6.90 (m, 1H), 5.09 (d, J=4Hz, 1H), 4.63 (q, J=5Hz, 1H), 3.58 (s, 2H), 2.41 - 2.51 (m, 2H), 2.20 - 2.40 (m, 1H), 1.90 - 1.98 (m, 1H), 1.41 - 1.73 (m, 1H) 0.96 - 1.04 (m, 6H) | A:0.41 B:0.25 |
| 2-28 | 24 | amorphous | 9.56 (s, 1H), 7.08 - 7.40 (m, 9H), 6.80 - 6.91 (m, 2H) 5.17 - 5.28 (m, 1H), 4.52 - 4.65 (m, 1H), 3.63 (s, 2H) 2.38 - 2.52 (m, 2H), 2.20 - 2.41 (m, 1H), 1.88 - 1.99 (m, 1H), 1.55 - 1.85 (m, 3H), 0.96 - 0.99 (m, 6H) | A:0.44 B:0.34 |
| 2-29 | 40 | amorphous | 9.54 (s, 1H), 8.15 (s, 1H), 7.90 (s, 1H), 7.63 (d, J=8Hz, 1H), 7.50 (d, J=8Hz, 1H), 6.90 - 7.30 (m, 8H), 6.80 (d, J=7Hz, 1H), 5.20 - 5.30 (m, 1H), 4.65 - 4.75 (m, 1H), 3.40 (dd, J=15Hz, 5Hz, 1H), 1.60 - 1.80 (m, 3H), 0.93 (d, J=6Hz, 6H), 3.25 (dd, J=15Hz, 7Hz, 1H) | A:0.52 B:0.21 |

**Reference Example 3-1**

Synthesis of L-O-(benzyl)serine ethyl ester hydrochloride (Reference Compound No. 3-1):

1 ml of concentrated hydrochloric acid was added to an ethanol solution containing 25 g (8.5 mmol) of L-N-(t-butoxycarbonyl)-O-(benzyl)serine, and the reaction mixture was stirred at room temperature for 2 hours.

The solvent was distilled away from the reaction mixture under reduced pressure. With the addition of 1.3 ml (17 mmol) of thionyl chloride, an ethanol solution of the thus obtained residue was stirred overnight at room temperature. The solvent was distilled away under reduced pressure, whereby 2.01 g of the captioned Reference Compound No. 3-1 was obtained in a yield of 91.6%.

NMR (δ, $CD_3OD$): 7.30 - 7.36 (m, 5H), 4.59 (dd, J = 31.74Hz, J = 12.15Hz, 2H), 4.24 - 4.31 (m, 3H), 3.93 (dd, J = 10.53Hz, J = 4.29Hz, 1H), 3.82 (dd, J = 10.65Hz, J = 3.33Hz, 1H), 1.27 (t, J = 7.17Hz, 3H)

**Reference Example 3-2**

Synthesis of L-S-(2-phenylethyl)cysteine methyl ester hydrochloride (Reference Compound No. 3-2):

4.85 g (29.28 mmol) of a cysteine hydrochloride hydrate was added to 200 ml of a methanol solution of 4.74 g of sodium methoxide under an ice-cooled condition, and the mixture was stirred at room temperature for one hour. A methanol solution containing 5.55 g (30 mmol) of 2-bromoethylbenzene was added dropwise to the above prepared reaction mixture, and the mixture was stirred for one hour under an ice-cooled condition. The solvent was distilled away from the reaction mixture under reduced pressure.

The residue thus obtained was dissolved in water, and washed with diethyl ether. The resultant water layer was made neutral with concentrated hydrochloric acid, so that crystals were caused to separat out.

The crystals separated by filtration were washed successively with water, ethanol and diethyl ether, and then dried, whereby 5.37 g of L-S-phenylethyl cysteine was obtained in a yield of 85.9%.

8.6 ml of thionyl chloride was added dropwise to 200 ml of a methanol solution containing 5.3 g (23.5 mmol) of the above obtained compound under an ice-cooled condition. After the completion of dropping, the reaction mixture was stirred overnight at room temperature. The solvent was distilled away under reduced pressure, so that crystals were caused to separated out. The thus obtained crystals were washed with

diethyl ether and dried, whereby 6.27 g of the captioned Reference Compound No. 3-2 was obtained in a yield of 96.3%.

NMR (δ, $CD_3OD$): 7.20 - 7.31 (m, 5H), 4.25 (dd, J=7.26Hz, J=4.23Hz, 1H), 3.83 (s, 3H), 3.12 (dd, J=14.49Hz, J=4.23Hz, 1H), 3.00 (dd, J=14.54Hz, J=7.27Hz, 1H), 2.82 - 2.94 (m, 4H)

**Reference Example 3-3**

Synthesis of L-S-(3-phenylpropyl)cysteine methyl ester hydrochloride (Reference Compound No. 3-3):

4.85 g (29.28 mmol) of a cysteine hydrochloride hydrate was added to 200 ml of a methanol solution of 4.75 g (87.84 mmol) of sodium methoxide under an ice-cooled condition, and the mixture was stirred at room temperature for one hour. A methanol solution containing 5.97 g (30 mmol) of 3-bromopropylbenzene was added dropwise to the above prepared reaction mixture, and the mixture was stirred for one hour under an ice-cooled condition. The solvent was distilled away from the reaction mixture under reduced pressure.

The residue thus obtained was dissolved in water, and washed with diethyl ether. The resultant water layer became neutral with the addition of concentrated hydrochloric acid, so that crystals were caused to separate out.

The crystals separated by filtration were washed successively with water, ethanol and diethyl ether, and then dried, whereby 4.47 g of (L)-S-(3-phenylpropyl)cysteine was obtained in a yield of 63%.

6.7 ml of thionyl chloride was added dropwise to 200 ml of a methanol solution containing the above obtained compound under an ice-cooled condition. After the completion of dropping, the reaction mixture was stirred overnight at room temperature. The solvent was distilled away under reduced pressure, so that crystals were caused to separate out. The thus obtained crystals were washed with diethyl ether and dried, whereby 5.196 g of the captioned Reference Compound No. 3-3 was obtained.

NMR (δ, $CD_3OD$): 7.16 - 7.30 (m, 5H), 4.27 (dd, J=6.57Hz, J=4.18Hz, 1H), 3.82 (s, 3H), 3.15 (dd, J=14.54Hz, J=4.18Hz, 1H), 3.03 (dd, J=14.49Hz, J=7.54Hz, 1H), 2.73 (t, J=7.33Hz, 2H), 2.58 (t, J=7.54Hz, 2H), 1.86 - 1.97 (m, 2H)

**Reference Example 3-4**

Synthesis of L-O-(3-phenylpropyl)serine ethyl ester hydrochloride (Reference Compound No. 3-4):

1.28 g (32 mmol) of sodium hydride (60% in oil) was added to an anhydrous dimethylformamide solution containing 3.0 g (14.6 mmol) of L-N-(t-butoxycarbonyl)serine with stirring under an ice-cooled condition. After the temperature of the reaction mixture was raised to room temperature, the reaction mixture was stirred for 2 hours. After the addition of 3.18 g (16 mmol) of 3-bromopropylbenzene, the reaction mixture was further stirred overnight.

The reaction mixture was concentrated under reduced pressure. The residue thus obtained was dissolved in water and washed with diethyl ether. The resultant aqueous layer was made acid with the addition of a 1 N hydrochloric acid solution and extracted with ethyl acetate. The organic extract layer was washed with water and dried over anhydrous sodium sulfate. The solvent was distilled away under reduced pressure.

The residue thus obtained was dissolved in ethanol. With the addition of concentrated hydrochloric acid, the above prepared ethanol solution was stirred overnight. Then, 4 ml of thionyl chloride was added to the ethanol solution, the mixture was further stirred overnight. The solvent was distilled away from the reaction mixture under reduced pressure, so that crystals were obtained. The thus obtained crystals were washed with diethyl ether and dried, whereby 1.13 g of the captioned Reference Compound No. 3-4 was obtained.

NMR (δ, $CD_3OD$): 7.15 - 7.28 (m, 5H), 4.32 (q, J=6.52Hz, 2H), 4.25 (t, J=1.68Hz, 1H), 3.92 (dd, J=10.91Hz, J=4.40Hz, 1H), 3.82 (dd, J=10.04Hz, J=2.82Hz, 1H), 3.44 - 3.59 (m, 2H), 2.67 (t, J=7.32, 2H), 1.85 - 1.95 (m, 2H), 1.32 (t, J=7.49, 3H)

**Reference Example 3-5**

Synthesis of L-O-(thiophene-3-ylmethyl)serine ethyl ester hydrochloride (Reference Compound No. 3-5):

172 g (43 mmol) of sodium hydride (60% in oil) was added to an anhydrous dimethylformamide solution containing 40 g (19.5 mmol) of (L)-N-(t-butoxycarbonyl)serine with stirring under an ice-cooled

condition. After the temperature of the reaction mixture was raised to room temperature, the reaction mixture was stirred for 2 hours. After the addition of 4.44 g (25 mmol) of 3-bromomethylthiophene, the reaction mixture was further stirred overnight.

The reaction mixture was concentrated under reduced pressure. The residue thus obtained was dissolved in water and washed with diethyl ether. The resultant water layer was made acid with the addition of a 1 N hydrochloric acid solution and extracted with ethyl acetate. The organic extract layer was washed with water and dried over anhydrous sodium sulfate. The solvent was distilled away under reduced pressure.

The residue thus obtained was dissolved in ethanol. After the addition of 2.2 ml of concentrated hydrochloric acid, the above prepared ethanol solution was stirred overnight. Then, 2.3 ml of thionyl chloride was added to the ethanol solution, the mixture was further stirred overnight. The solvent was distilled away from the reaction mixture under reduced pressure, so that crystals were obtained. The thus obtained crystals were washed with diethyl ether and dried, whereby 1.25 g of the captioned Reference Compound No. 3-5 was obtained.

NMR (δ, $CD_3OD$): 7.36 - 7.42 (m, 2H), 7.08 - 7.10 (m, 1H), 4.60 (q, J = 12.21Hz, 2H), 4.27 (q, J = 7.11Hz, 1H), 4.21 (t, J = 3.18Hz, 1H), 3.90 (dd, J = 4.32Hz, J = 9.87Hz, 1H), 3.81 (dd, J = 3.27Hz, J = 10.47Hz, 1H), 1.27 (t, J = 7.11Hz, 3H)

**Reference Example 3-6**

Synthesis of L-S-(diphenylmethyl)cysteine methyl ester hydrochloride (Reference Compound No. 3-6):

3.0 g (17.1 mmol) of a L-cysteine hydrochloride hydrate and 3.15 g (17.1 mmol) of diphenylmethanol were added to 40 ml of trifluoroacetic acid, and the mixture was stirred at room temperature for one hour.

The trifluoroacetic acid was distilled away from the mixture under reduced pressure. The residue thus obtained was caused to crystallize with the addition of diethyl ether thereto, and washed successively with water, ethanol and diethyl ether, followed by drying under reduced pressure. Thus, 5.2 g of L-S-diphenylmethyl cysteine was obtained in a yield of 100%.

Under an ice-cooled condition, 1.0 ml of thionyl chloride was added dropwise to a methanol solution in which 1.0 g (3.5 mmol) of the above obtained L-S-diphenylmethyl cysteine was dissolved. After the completion of dropping, the temperature of the reaction mixture was raised to room temperature and the reaction mixture was stirred overnight. The solvent was distilled away from the reaction mixture under reduced pressure, so that crystals were obtained. The thus obtained crystals were washed with diethyl ether, whereby 1.11 g of the captioned Reference Compound No. 3-6 was obtained in a yield of 100%.

NMR (δ, $CDCl_3$): 7.41 - 7.44 (m, 4H), 7.15 - 7.29 (m, 6H), 5.42 (s, 1H), 4.22 - 4.32 (m, 1H), 3.61 (s, 3H), 2.98 - 3.03 (m, 2H), 2.78 - 2.89 (m, 2H)

**Reference Example 3-7**

Synthesis of L-S-(cyclohexylmethyl)cysteine ethyl ester hydrochloride (Reference Compound No. 3-7):

The same reaction procedure as used in Reference Example 3-2 was repeated except that the 2-bromoethylbenzene used in Reference Example 3-2 was replaced by cyclohexyl methyl bromide, so that the captioned Reference Compound No. 3-7 was obtained.

NMR (δ, $CDCl_3$): 4.35 - 4.39 (m, 1H), 4.30 (q, J = 7.17Hz, 2H), 3.24 (d, J = 5.22Hz, 2H), 2.50 (dd, J = 6.90Hz, J = 3.18, 2H), 2.20 - 2.65 (m, 2H), 1.38 - 1.88 (m, 5H), 1.33 (t, J = 14.28, J = 7.17, 3H), 0.87 - 1.28 (m, 6H)

**Reference Example 3-8**

Synthesis of L-S-(cyclopentyl)cysteine ethyl ester hydrochloride (Reference Compound No. 3-8):

The same reaction procedure as used in Reference Example 3-2 was repeated except that the 2-bromoethylbenzene used in Reference Example 3-2 was replaced by cyclopentyl bromide, so that the captioned Reference Compound No. 3-8 was obtained.

NMR (δ, $CD_3OD$): 4.44 (t, J = 5.19Hz, 1H), 4.32 (t, J = 6.99Hz, 2H), 4.27 - 4.29 (m, 1H), 3.31 - 3.46 (m, 4H), 3.17 (dd, J = 17.19, J = 4.77, 1H), 3.07 (dd, J = 13.91, J = 6.99, 1H), 2.00 - 2.11 (m, 1H), 1.72 - 1.81 (m, 1H), 1.46 - 1.68 (m, 2H), 1.35 (t, J = 7.20Hz, 3H)

**Reference Example 3-9**

Synthesis of L-S-(thiophene-2-ylmethyl)cysteine methyl ester hydrochloride (Reference Compound No. 3-9):

The same reaction procedure as used in Reference Example 3-2 was repeated except that the 2-bromoethylbenzene used in Reference Example 3-2 was replaced by 2-chloromethylthiophene, so that the captioned Reference Compound No. 3-9 was obtained.

NMR (δ, $CD_3OD$): 7.34 (dd, J = 5.26Hz, J = 1.36Hz, 1H), 7.04 (d, J = 4.56Hz, 1H), 6.95 (dd, J = 5.04Hz, J = 1.62Hz, 1H), 4.22 (dd, J = 7.92Hz, J = 4.5Hz, 1H), 4.06 (d, J = 3.31Hz, 2H), 3.84 (s, 3H), 3.10 (dd, J = 14.81Hz, J = 4.56Hz, 1H), 2.96 (dd, J = 14.92Hz, J = 8.03Hz, 1H)

**Reference Example 3-10**

Synthesis of L-S-(thiophene-3-ylmethyl)cysteine ethyl ester hydrochloride (Reference Compound No. 3-10):

The same reaction procedure as used in Reference Example 3-2 was repeated except that the 2-bromoethylbenzene used in Reference Example 3-2 was replaced by 3-bromomethylthiophene, so that the captioned Reference Compound No. 3-10 was obtained.

NMR (δ, $CD_3OD$): 7.41 (dd, J = 4.95Hz, J = 2.01Hz, 1H), 7.32 (brs, 1H), 7.12 (d, J = 4.89Hz, 1H), 4.29 (q, J = 7.26Hz, 2H), 4.14 (dd, J = 8.04Hz, J = 3.57Hz, 1H), 3.87 (s, 2H), 3.03 (dd, J = 14.76Hz, J = 4.5Hz, 1H), 2.90 (dd, J = 14.82Hz, J = 8.13Hz, 1H), 1.31 (t, J = 7.23Hz, 3H)

**Reference Example 3-11**

Synthesis of L-S-(1-naphthylmethyl)cysteine ethyl ester hydrochloride (Reference Compound No. 3-11):

The same reaction procedure as used in Reference Example 3-2 was repeated except that the 2-bromoethylbenzene used in Reference Example 3-2 was replaced by 1-naphthylmethyl bromide, so that the captioned Reference Compound No. 3-11 was obtained.

NMR (δ, $CD_3OD$): 8.18 (d, J = 8.37Hz, 1H), 7.80 - 7.90 (m, 2H), 7.35 - 7.58 (m, 4H), 4.33 (s, 2H), 4.17 - 4.29 (m, 3H), 3.04 (dd, J = 4.44Hz, J = 9.72, 1H), 2.95 (dd, J = 7.80Hz, J = 14.64Hz, 1H), 1.25 (t, J = 7.02Hz, 3H)

**Reference Example 3-12**

Synthesis of L-S-(2-naphthylmethyl)cysteine ethyl ester hydrochloride (Reference Compound No. 3-12):

The same reaction procedure as used in Reference Example 3-2 was repeated except that the 2-bromoethylbenzene used in Reference Example 3-2 was replaced by 2-naphthylmethyl bromide, so that the captioned Reference Compound No. 3-12 was obtained.

NMR (δ, $CD_3OD$): 7.81 - 7.88 (m, 4H), 7.47 - 7.55 (m, 3H), 4.21 - 4.25 (m, 2H), 4.13 - 4.20 (m, 1H), 4.00 (s, 2H), 3.01 (dd, J = 9.78Hz, J = 4.38Hz, 1H), 2.89 (dd, J = 8.19Hz, J = 6.39Hz, 1H), 1.22 (t, J = 7.05Hz, 3H)

**Reference Example 3-13**

Synthesis of L-S-(2-chlorobenzyl)cysteine ethyl ester hydrochloride (Reference Compound No. 3-13):

The same reaction procedure as used in Reference Example 3-2 was repeated except that the 2-bromoethylbenzene used in Reference Example 3-2 was replaced by 2-chlorobenzyl chloride, so that the captioned Reference Compound No. 3-13 was obtained.

NMR (δ, $CD_3OD$): 7.41 - 7.48 (m, 2H), 7.28 - 7.31 (m, 2H), 4.30 (q, J = 7.33Hz, 2H), 4.25 - 4.29 (m, 1H), 3.12 (dd, J = 14.71Hz, J = 4.45Hz, 1H), 2.97 (dd, J = 14.65Hz, J = 7.86Hz, 1H), 1.31 (t, J = 7.16Hz, 3H)

**Reference Example 3-14**

Synthesis of (2R)-2-amino-3-(2-fluorobenzylthio)propanol (Reference Compound No. 3-14):

30.4 g (173 mmol) of a L-cysteine hydrochloride hydrate was added to a methanol solution containing sodium methoxide which was prepared using 12.0 g (519 mmol) of metallic sodium and 700 ml of methanol. To this mixture, 25.0 g (173 mmol) of 2-fluorobenzyl chloride was added dropwise, and the reaction mixture was stirred overnight at room temperature. The methanol was distilled away from the reaction mixture under reduced pressure.

The residue thus obtained was dissolved in water and washed with ether twice. Then, the pH of the resultant water layer was adjusted to 7.0 with the addition of concentrated hydrochloric acid, so that crystals were caused to separate out.

These crystals were separated by filtration, washed successively with water, ethanol and diethyl ether, and dried under reduced pressure, whereby 29.7 g of (2R)-2-amino-3-(2-fluorobenzylthio)propionic acid was obtained in a yield of 74.8%.

6.7 ml (52.8 mmol) of chlorotrimethylsilane was added to an anhydrous tetrahydrofuran solution of 0.58 g (26.4 mmol) of lithium boron hydride, and the mixture was stirred at room temperature for 30 minutes. To this mixture, 2.02 g (8.8 mmol) of the above prepared (2R)-2-amino-3-(2-fluorobenzylthio)propionic acid was added, and the reaction mixture was further stirred overnight at room temperature. With the addition of methanol, the solvent was distilled away from the reaction mixture under reduced pressure.

The residue thus obtained was dissolved in a 1 N sodium hydroxide solution and extracted with chloroform. After the extract layer was dried over anhydrous sodium sulfate, the solvent was distilled away under reduced pressure, whereby 1.69 g of the captioned Reference Compound No. 3-14 was obtained in a yield of 89.4%.

NMR (δ, $CDCl_3$): 7.19 - 7.36 (m, 2H), 7.00 - 7.12 (m, 2H), 3.75 (s, 2H), 3.65 (dd, J=10.86Hz, J=3.78Hz, 1H), 3.42 (dd, J=10.74Hz, J=6.45Hz, 1H), 3.00 - 3.10 (m, 1H), 2.80 (brs, 3H), 2.62 (dd, J=13.56Hz, J=4.92Hz, 1H), 2.45 (dd, J=13.35Hz, J=8.22Hz, 1H)

**Reference Example 3-15**

Synthesis of (2R)-2-amino-3-(3-chlorobenzylthio)propanol (Reference Compound No. 3-15):

The same reaction procedure as used in Reference Example 3-14 was repeated except that the 2-fluorobenzyl chloride used in Reference Example 3-14 was replaced by 3-chlorobenzyl bromide, so that the captioned Reference Compound No. 3-15 was obtained.

NMR (δ, $CDCl_3$): 7.15 - 7.35 (m, 4H), 3.68 (s, 2H), 3.64 (d, J=3.52Hz, 1H), 3.44 (dd, J=6.45Hz, J=10.85Hz, 1H), 2.98 (brs, 4H), 2.60 (dd, J=5.21Hz, J=13.4Hz, 1H), 2.45 (dd, J=8.08Hz, J=13.45Hz, 1H)

**Reference Example 3-16**

Synthesis of (2R)-2-amino-3-(4-chlorobenzylthio)propanol (Reference Compound No. 3-16):

The same reaction procedure as used in Reference Example 3-14 was repeated except that the 2-fluorobenzyl chloride used in Reference Example 3-14 was replaced by 4-chlorobenzyl chloride, so that the captioned Reference Compound No. 3-16 was obtained.

NMR (δ, $CDCl_3$): 7.26 (q, J=8.52, 4H), 3.68 (s, 2H), 3.60 (dd, J=4.02Hz, J=10.75Hz, 1H), 3.38 (dd, J=6.46Hz, J=10.8Hz, 1H), 2.96 (brs, 1H), 2.54 (dd, J=4.77Hz, J=13.18Hz, 1H), 2.36 (dd, J=8.25Hz, J=13.35Hz, 1H), 2.24 (brs, 3H)

**Reference Example 3-17**

Synthesis of (2R)-2-amino-3-(3-fluorobenzylthio)propanol (Reference Compound No. 3-17):

The same reaction procedure as used in Reference Example 3-14 was repeated except that the 2-fluorobenzyl chloride used in Reference Example 3-14 was replaced by 3-fluorobenzyl chloride, so that the captioned Reference Compound No. 3-17 was obtained.

NMR (δ, $CDCl_3$): 7.21 - 7.28 (m, 1H), 6.89 - 7.10 (m, 3H), 4.76 (s, 3H), 3.76 (dd, J=3.42Hz, J=11.5Hz, 1H), 3.72 (s, 2H), 3.57 (dd, J=7.82Hz, J=12.59Hz, 1H), 3.25 (brs, 1H),

2.62 (d, J=7.05Hz, 2H)

**Reference Example 3-18**

Synthesis of (2R)-2-amino-3-(4-fluorobenzylthio)propanol (Reference Compound No. 3-18):

The same reaction procedure as used in Reference Example 3-14 was repeated except that the 2-fluorobenzyl chloride used in Reference Example 3-14 was replaced by 4-fluorobenzyl chloride, so that the captioned Reference Compound No. 3-18 was obtained.

NMR (δ, $CDCl_3$): 7.25 - 7.30 (m, 2H), 6.97 - 7.03 (m, 2H), 3.69 (s, 2H), 3.60 (dd, J=4.23Hz, J=10.74Hz, 1H), 3.37 (dd, J=6.57Hz, J=10.74Hz, 1H), 2.95 (brs, 1H), 2.55 (dd, J=4.93Hz, J=13.29Hz, 1H), 2.01 (br d, J=10.25Hz, 3H), 2.36 (dd, J=8.31Hz, 13.30Hz, 1H)

**Reference Example 3-19**

Synthesis of (2R)-2-amino-3-(2-methoxybenzylthio)propanol (Reference Compound No. 3-19):

The same reaction procedure as used in Reference Example 3-14 was repeated except that the 2-fluorobenzyl chloride used in Reference Example 3-14 was replaced by 2-methoxybenzyl chloride, so that the captioned Reference Compound No. 3-19 was obtained.

NMR (δ, $CDCl_3$): 7.21 - 7.26 (m, 2H), 6.86 - 6.94 (m, 2H), 3.85 (s, 3H), 3.74 (s, 2H), 3.62 (dd, J=10.74Hz, J=4.13Hz, 1H), 3.37 (dd, J=10.74Hz, J=6.73, 1H), 2.97 - 3.05 (m, 1H), 2.62 (dd, J=13.51Hz, J=4.83Hz, 1H), 2.40 (dd, J=13.57Hz, J=8.31Hz, 1H), 2.08 (brs, 3H)

**Reference Example 3-20**

Synthesis of (2R)-2-amino-3-(3-methoxybenzylthio)propanol (Reference Compound No. 3-20):

The same reaction procedure as used in Reference Example 3-14 was repeated except that the 2-fluorobenzyl chloride used in Reference Example 3-14 was replaced by 3-methoxybenzyl chloride, so that the captioned Reference Compound No. 3-20 was obtained.

NMR (δ, $CDCl_3$): 7.22 (t, J=8.25, 1H), 6.88 (d, J=6.68Hz, 1H), 6.86 (s, 1H), 6.79 (dd, J=2.06Hz, J=7.48Hz, 1H), 3.80 (s, 3H), 3.68 (s, 2H), 3.58 (dd, J=4.18Hz, J=10.8Hz, 1H), 3.36 (dd, J=6.62Hz, J=10.75Hz, 1H), 2.90 - 3.00 (m, 1H), 2.56 (dd, J=4.88Hz, J=13.29Hz, 1H), 2.36 (dd, J=8.19Hz, J=13.35Hz, 1H), 2.11 (brs, 3H)

**Reference Example 3-21**

Synthesis of (2R)-2-amino-3-(4-methoxybenzylthio)propanol (Reference Compound No. 3-21):

The same reaction procedure as used in Reference Example 3-14 was repeated except that the 2-fluorobenzyl chloride used in Reference Example 3-14 was replaced by 4-methoxybenzyl chloride, so that the captioned Reference Compound No. 3-21 was obtained.

NMR (δ, $CDCl_3$): 7.22 (d, J=8.63Hz, 2H), 6.85 (d, J=8.63Hz, 2H), 3.80 (s, 3H), 3.67 (s, 2H), 3.59 (dd, J=4.13Hz, J=10.74Hz, 1H), 3.36 (dd, J=6.62Hz, J=10.75Hz, 1H), 2.90 - 2.98 (m, 1H), 2.55 (dd, J=4.88Hz, J=13.29Hz, 1H), 2.36 (dd, J=8.24Hz, J=13.29Hz, 1H), 1.99 (s, 3H)

**Reference Example 3-22**

Synthesis of (2R)-2-amino-3-(3-nitrobenzylthio)propanol (Reference Compound No. 3-22):

The same reaction procedure as used in Reference Example 3-14 was repeated except that the 2-fluorobenzyl chloride used in Reference Example 3-14 was replaced by 3-nitrobenzyl chloride, so that the captioned Reference Compound No. 3-22 was obtained.

NMR (δ, $CDCl_3$): 8.20 (s, 1H), 8.13 (d, J=8.24Hz, 1H), 7.67 (d, J=7.75Hz, 1H), 7.51 (t, J=7.87Hz,

1H), 3.81 (s, 2H), 3.62 (dd, J=10.75Hz, J=4.24Hz, 1H), 3.42 (dd, J=10.75Hz, J=6.35Hz, 1H), 3.00 - 3.08 (m, 1H), 2.58 (dd, J=13.18Hz, J=4.94Hz, 1H), 2.41 (dd, J=13.23Hz, J=8.13Hz, 1H), 2.05 (brs, 3H)

**Reference Example 3-23**

Synthesis of (2R)-2-amino-3-(4-nitrobenzylthio)propanol (Reference Compound No. 3-23):

To a 1 N sodium hydroxide aqueous solution, 5.27 g (30 mmol) of a L-cysteine hydrochloride hydrate was added. Subsequently, a dioxane solution containing 5.15 g (30 mmol) of 4-nitrobenzyl chloride was added dropwise to the above mixture. The thus obtained reaction mixture was stirred at room temperature for one hour. The reaction mixture was washed with diethyl ether and made weakly acidic with the addition of concentrated hydrochloric acid. On cooling the reaction mixture, crystals were caused to separate out.

The crystals thus obtained were separated by filtration, washed successively with ethanol and diethyl ether, and then dried under reduced pressure, whereby 3.55 g of L-2-amino-3-(4-nitrobenzylthio)propionic acid was obtained in a yield of 45.9%.

9.5 ml (75 mmol) of chlorotrimethylsilane was added to an anhydrous tetrahydrofuran solution of 0.82 g (37.5 mmol) of lithium boron hydride, and the mixture was stirred at room temperature for 30 minutes. To this mixture, 3.0 g (11.70 mmol) of the above prepared L-2-amino-3-(4-nitrobenzylthio)propionic acid was added, and the reaction mixture was further stirred overnight at room temperature. With the addition of methanol, the solvent was distilled away from the reaction mixture under reduced pressure.

The residue thus obtained was dissolved in a 1 N sodium hydroxide solution and extracted with chloroform. After the extract layer was dried over anhydrous sodium sulfate, the solvent was distilled away under reduced pressure, whereby 2.52 g of the captioned Reference Compound No. 3-23 was obtained in a yield of 89%.

NMR (δ, $CDCl_3$): 8.19 (d, J=8.63Hz, 2H), 7.50 (d, J=8.69Hz, 2H), 3.80 (s, 2H), 3.60 (dd, J=4.18Hz, J=10.74Hz, 1H), 3.40 (dd, J=6.35Hz, J=10.74Hz, 1H), 2.92 - 3.02 (m, 1H), 2.57 (dd, J=4.94Hz, J=13.19Hz, 1H), 2.39 (dd, J=8.13Hz, J=13.18Hz, 1H), 2.01 (s, 3H)

**Reference Example 3-24**

Synthesis of (2S)-2-amino-4-phenyloxybutanol hydrochloride (Reference Compound No. 3-24):

320 g (2.94 mol) of ethyl bromide was added dropwise to an anhydrous dimethylformamide solution containing 142 g (0.59 mol) of L-N-(t-butoxycarbonyl)-homoserine potassium salt synthesized in accordance with the method described in Tetrahedron Letters vol. 20, 2243, (1978). The thus obtained reaction mixture was stirred overnight at room temperature. The solvent was distilled away from the reaction mixture under reduced pressure.

The residue thus obtained was dissolved in water, and extracted with ethyl acetate. The extract layer was washed with a saturated aqueous solution of sodium chloride and dried over anhydrous sodium sulfate. The solvent was distilled away under reduced pressure. The thus obtained residue was chromatographed on a silica gel column for purification, whereby 44.5 g of L-N-(t-butoxycarbonyl)homoserine ethyl ester was obtained in a yield of 31%.

3.44 g (30 mmol) of methanesulfonyl chloride was added to an ethyl acetate solution containing 6.18 g (25 mmol) of the above prepared L-N-(t-butoxycarbonyl)-homoserine ethyl ester and 3.04 g (30 mmol) of triethylamine with stirring under an ice-cooled condition. The thus obtained reaction mixture was stirred for one hour. The reaction mixture was washed with water, dried over anhydrous sodium sulfate, and then concentrated under reduced pressure, so that 8.12 g of a methanesulfonate was obtained in a yield of 100%.

0.35 g of sodium hydride (60% in oil) was added to an anhydrous dimethylformamide solution containing 0.88 g of phenol, and the mixture was stirred for one hour. To this mixture, a dimethylformamide solution of 2.55 g (7.83 mmol) of the above prepared methanesulfonate was added dropwise, and the thus obtained reaction mixture was stirred overnight at room temperature. With the addition of a saturated aqueous solution of ammonium chloride, the reaction mixture was extracted with ethyl acetate. The extract layer was washed successively with a saturated aqueous solution of sodium hydrogencarbonate and water, and then dried over anhydrous sodium sulfate. The solvent was distilled away under reduced pressure. The residue thus obtained was chromatographed on a silica gel column for purification, whereby 2.13 g of L-N-(t-butoxycarbonyl)-O-phenylhomoserine ethyl ester was obtained in a yield of 84%.

NMR (δ, $CDCl_3$): 7.20 - 7.30 (m, 2H), 6.83 - 6.97 (m, 3H), 5.34 - 5.42 (m, 1H), 4.45 - 4.52 (m, 1H), 4.18 (q, J=7.22Hz, 2H), 4.04 (t, J=6.08Hz, 2H), 2.20 - 2.37 (m, 2H), 1.44 (s, 9H), 1.25 (t, J=7.06Hz, 3H)

0.28 g of lithium borohydride was added to a tetrahydrofuran solution containing 2.10 g (6.5 mmol) of the above prepared L-N-(t-butoxycarbonyl)-O-phenylhomoserine ethyl ester, followed by dropping of methanol with stirring under an ice-cooled condition. After the reaction mixture was stirred for one hour, water was added to the reaction mixture and the solvent was distilled away therefrom under reduced pressure.

With the addition of a 1 N hydrochloric acid aqueous solution, the thus obtained residue was extracted with ethyl acetate. The resultant extract organic layer was washed successively with a saturated aqueous solution of sodium hydrogencarbonate and water, dried over anhydrous sodium sulfate, and then the solvent was distilled away under reduced pressure.

The residue thus obtained was dissolved in 4 N HCl - AcOEt, and the solution was stirred for one hour. The solvent was distilled away from the reaction solution under reduced pressure, whereby 0.64 g of the captioned Reference Compound No. 3-24 was obtained in a yield of 46%.

**Reference Example 3-25**

Synthesis of (2S)-2-amino-4-(phenylthio)butanol hydrochloride (Reference Compound No. 3-25):

The same reaction procedure for preparation of the L-N-(t-butoxycarbonyl)-O-phenylhomoserine ethyl ester as used in Reference Example 3-24 was repeated except that the ethyl bromide and the phenol used in Reference Example 3-24 were respectively replaced by benzyl bromide and thiophenol, so that L-N-(t-butoxycarbonyl)-S-phenylhomocystein benzyl ester was obtained.

NMR (δ, $CDCl_3$): 7.16 - 7.37 (m, 10H), 5.15 (d, J=3.31Hz, 2H), 5.09 - 5.20 (m, 1H), 4.47 - 4.53 (m, 1H), 2.90 (dt, J=6.3Hz, J=2.11, 2H), 2.10 - 2.20 (m, 1H), 1.89 - 2.00 (m, 1H), 1.43 (s, 9H)

Subsequently, the same reaction procedure for preparation of the Reference Compound No. 3-24 as used in Reference Example 3-24 was repeated, whereby the captioned Reference Compound No. 3-25 was obtained.

**Reference Example 3-26**

Synthesis of (2R)-2-amino-3-(2-chlorobenzyloxy)propanol hydrochloride (Reference Compound No. 3-26):

3.0 g of sodium hydride (60% in oil) was added to an anhydrous dimethylformamide solution containing 7.0 g (34 mmol) of L-N-(t-butoxycarbonyl)serine, and the mixture was stirred at room temperature for three hours. To this mixture, 6.0 g (37 mmol) of 2-chlorobenzyl chloride was added dropwise, and the thus obtained reaction mixture was stirred overnight at room temperature. The solvent was distilled away from the reaction mixture under reduced pressure.

The thus obtained residue was dissolved in a mixed solvent of ethyl acetate and 1 N hydrochloric acid. The resultant ethyl acetate layer was washed with water and dried over anhydrous sodium sulfate. The solvent was distilled away, whereby 4.3 g of L-N-(t-butoxycarbonyl)-O-(2-chlorobenzyl)-serine was obtained in a yield of 38%.

0.93 g of ethyl chlorocarbonate was added dropwise to a tetrahydrofuran solution containing 2.15 g (6.5 mmol) of the above prepared L-N-(t-butoxycarbonyl)-O-(2-chlorobenzyl)-serine and triethylamine by cooling in an ice bath containing salt. After this reaction mixture was stirred for 2 hours, crystals were caused to separate out.

These crystals were removed by filtration, and sodium boron hydride and methanol were successively added dropwise to the filtrate with stirring under an ice-cooled condition. The reaction mixture was stirred for 2 hours. The solvent was distilled away from the reaction mixture under reduced pressure.

With the addition of a 1 N hydrochloric acid aqueous solution, an aqueous solution of the above obtained residue was extracted with ethyl acetate. The resultant extract organic layer was washed successively with a 10% aqueous solution of sodium hydroxide and water and dried over anhydrous sodium sulfate, and then the solvent was distilled away. The residue thus obtained was chromatographed on a silica gel column for purification, so that 0.81 g of an alcohol was obtained.

The thus obtained alcohol was dissolved in 4 N HCl - AcOEt, and the solution was stirred at room temperature for 30 minutes. The solvent was distilled away from the reaction solution, whereby 0.55 g of the

captioned Reference Compound No. 3-26 was obtained in a yield of 19%.

NMR (δ, $CD_3OD$): 7.53 - 7.56 (m, 1H), 7.39 - 7.42 (m, 1H), 7.30 - 7.35 (m, 2H), 4.70 (s, 2H), 3.67 - 3.82 (m, 4H), 3.42 - 3.49 (m, 1H)

**Reference Example 3-27**

Synthesis of (2S)-2-amino-4-(2-fluorophenyloxy)butanol hydrochloride (Reference Compound No. 3-27):

The same reaction procedure for preparation of the L-N-(t-butoxycarbonyl)-O-phenylhomoserine ethyl ester as used in Reference Example 3-24 was repeated except that the phenol used in Reference Example 3-24 was replaced by 2-fluorophenol, so that L-N-(t-butoxycarbonyl)-O-(2-fluorophenyl)homoserine ethyl ester was obtained.

NMR (δ, $CDCl_3$): 6.85 - 7.10 (m, 4H), 5.40 - 5.46 (m, 1H), 4.42 - 4.51 (m, 1H), 4.22 (q, J=7.0Hz, 2H), 4.12 (q, J=5.48Hz, 2H), 2.23 - 2.41 (m, 2H), 1.44 (s, 9H), 1.26 (t, J=7.16Hz, 3H)

Subsequently, the same reaction procedure for preparation of the Reference Compound No. 3-24 as used in Reference Example 3-24 was repeated, whereby the captioned Reference Compound No. 3-27 was obtained.

**Reference Example 3-28**

Synthesis of (2S)-2-amino-4-(3-fluorophenyloxy)butanol hydrochloride (Reference Compound No. 3-28):

The same reaction procedure for preparation of the L-N-(t-butoxycarbonyl)-O-phenylhomoserine ethyl ester as used in Reference Example 3-24 was repeated except that the phenol used in Reference Example 3-24 was replaced by 3-fluorophenol, so that L-N-(t-butoxycarbonyl)-O-(3-fluorophenyl)homoserine ethyl ester was obtained.

NMR (δ, $CDCl_3$): 7.11 - 7.24 (m, 1H), 6.55 - 6.69 (m, 3H), 5.31 - 5.38 (m, 1H), 4.45 - 4.51 (m, 1H), 4.20 (q, J=7.16Hz 2H), 4.03 (t, J=6.02Hz, 2H), 2.20 - 2.38 (m, 2H), 1.44 (s, 9H), 1.26 (t, J=7.11Hz, 3H)

Subsequently, the same reaction procedure for preparation of the Reference Compound No. 3-24 as used in Reference Example 3-24 was repeated, whereby the captioned Reference Compound No. 3-28 was obtained.

**Reference Example 3-29**

Synthesis of (2S)-2-amino-4-(2-chlorophenyloxy)butanol hydrochloride (Reference Compound No. 3-29):

The same reaction procedure for preparation of the L-N-(t-butoxycarbonyl)-O-phenylhomoserine ethyl ester as used in Reference Example 3-24 was repeated except that the ethyl bromide and the phenol used in Reference Example 3-24 were respectively replaced by benzyl bromide and 2-chlorophenol, so that L-N-(t-butoxycarbonyl)-O-(2-chlorophenyl)-homoserine benzyl ester was obtained.

NMR (δ, $CDCl_3$): 7.30 - 7.36 (m, 6H), 7.18 (dt, J=6.67Hz, J=1.6, 1H), 6.90 (dt, J=6.3Hz, J=1.35Hz, 1H), 6.80 (d, J=8.24Hz, 1H), 5.80 - 5.83 (m, 1H), 5.18 (d, J=2.17Hz, 2H), 4.55 - 4.62 (m, 1H), 4.07 - 4.14 (m, 1H), 3.95 - 4.00 (m, 1H), 2.40 - 2.50 (m, 2H), 1.43 (s, 9H)

Subsequently, the same reaction procedure for preparation of the Reference Compound No. 3-24 as used in Reference Example 3-24 was repeated, whereby the captioned Reference Compound No. 3-29 was obtained.

**Reference Example 3-30**

Synthesis of (2S)-2-amino-4-(3-chlorophenyloxy)butanol hydrochloride (Reference Compound No. 3-30):

The same reaction procedure for preparation of the L-N-(t-butoxycarbonyl)-O-phenylhomoserine ethyl ester as used in Reference Example 3-24 was repeated except that the ethyl bromide and the phenol used in Reference Example 3-24 were respectively replaced by benzyl bromide and 3-chlorophenol, so that L-N-(t-butoxycarbonyl)-O-(3-chlorophenyl)-homoserine benzyl ester was obtained.

NMR (δ, $CDCl_3$): 7.34 (s, 5H), 7.16 (t, J=8.14Hz, 1H), 6.92 (dd, J=5.53Hz, J=1.96Hz, 1H), 6.80 (s,

1H), 6.69 (dd, J = 6.35Hz, J = 2.01, 1H), 5.25 - 5.30 (m, 1H), 5.18 (d, J = 2.23Hz, 2H), 4.50 - 4.57 (m, 1H), 3.98 (t, J = 5.96Hz, 2H), 2.25 - 2.37 (m, 2H), 1.43 (s, 9H)

Subsequently, the same reaction procedure for preparation of the Reference Compound No. 3-24 as used in Reference Example 3-24 was repeated, whereby the captioned Reference Compound No. 3-30 was obtained.

**Reference Example 3-31**

Synthesis of (2S)-2-amino-4-benzylthiobutanol (Reference Compound No. 3-31):

1 g of metallic sodium was added to liquid ammonia which was cooled to -78° C, and the mixture was stirred for 30 minutes. With the addition of 2.0 g (7.45 mmol) of homocysteine, the reaction mixture was further stirred for 30 minutes. Ammonium chloride was added to the above reaction mixture until a blue color of the mixture faded. Subsequently, with the addition of 0.69 g (30 mmol) of benzyl bromide, the liquid ammonia was allowed to evaporate at room temperature.

The thus obtained residue was dissolved in water and washed with diethyl ether, and the above prepared solution was made weakly acidic with the addition of concentrated hydrochloric acid, and crystals were allowed to separate out in the cool place.

The thus obtained crystals were washed successively with water, ethanol and ether, and dried under reduced pressure, whereby 2.8 g of (2S)-2-amino-4-benzylthiobutanoic acid was obtained in a yield of 86%.

5.6 ml (44 mmol) of chlorotrimethylsilane was added to an anhydrous tetrahydrofuran solution of 0.49 g (22 mmol) of lithium boron hydride, and the mixture was stirred at room temperature for 30 minutes. To this mixture, 2.5 g (11 mmol) of the above prepared (2S)-2-amino-4-benzylthiobutanoic acid was added, and the thus obtained reaction mixture was stirred overnight at room temperature. Methanol was added to the reaction mixture, and then the solvent was distilled away therefrom under reduced pressure.

The residue thus obtained was dissolved in a 1 N sodium hydroxide aqueous solution, and extracted with chloroform. The extract layer was dried over anhydrous sodium sulfate, and the solvent was distilled away under reduced pressure, whereby 2.0 g of the captioned Reference Compound No. 3-31 was obtained in a yield of 85.5%.

NMR (δ, $CDCl_3$): 7.22 - 7.32 (m, 5H), 3.72 (s, 2H), 3.51 - 3.56 (m, 1H), 3.26 - 3.30 (m, 1H), 2.90 - 3.00 (m, 1H), 2.42 - 2.58 (m, 2H), 2.00 - 2.12 (m, 1H), 1.62 - 1.73 (m, 1H), 1.48 - 1.58 (m, 1H)

**Reference Example 3-32**

Synthesis of (2S)-2-amino-4-(2-fluorobenzylthio)butanol (Reference Compound No. 3-32):

The same reaction procedure as used in Reference Example 3-31 was repeated except that the benzyl bromide used in Reference Example 3-31 was replaced by 2-fluorobenzyl bromide, whereby the captioned Reference Compound No. 3-32 was obtained.

NMR (δ, $CDCl_3$): 7.31 - 7.36 (m, 1H), 7.19 - 7.27 (m, 1H), 7.00 - 7.12 (m, 2H), 3.75 (s, 2H), 3.55 (dd, J = 10.68Hz, J = 3.96Hz, 1H), 3.28 (dd, J = 13.59Hz, J = 7.49Hz, 1H), 2.89 - 2.97 (m, 1H), 2.47 - 2.62 (m, 2H), 1.98 (brs, 3H), 1.65 - 1.76 (m, 1H), 1.49 - 1.59 (m, 1H)

**Reference Example 3-33**

Synthesis of (2S)-2-amino-4-(2-chlorobenzylthio)butanol (Reference Compound No. 3-33):

The same reaction procedure as used in Reference Example 3-31 was repeated except that the benzyl bromide used in Reference Example 3-31 was replaced by 2-chlorobenzyl chloride, whereby the captioned Reference Compound No. 3-33 was obtained.

NMR (δ, $CDCl_3$): 7.33 - 7.39 (m, 2H), 7.19 - 7.26 (m, 2H), 3.84 (s, 2H), 3.57 (dd, J = 10.69Hz, J = 4.01Hz, 1H), 3.29 (dd, J = 10.69Hz, J = 7.49Hz, 1H), 2.93 - 3.01 (m, 1H), 2.50 - 2.66 (m, 2H), 2.04 (brs, 3H), 1.66 - 1.78 (m, 1H), 1.50 - 1.62 (m, 1H)

**Reference Example 3-34**

Synthesis of (2S)-2-amino-4-(2-fluorophenylthio)butanol hydrochloride (Reference Compound No. 3-34):

The same reaction procedure for preparation of the L-N-(t-butoxycarbonyl)-O-phenylhomoserine ethyl ester as used in Reference Example 3-24 was repeated except that the ethyl bromide and the phenol used in Reference Example 3-24 were respectively replaced by benzyl bromide and 2-fluorothiophenol, so that L-N-(t-butoxycarbonyl)-S-(2-fluorophenyl)homocysteine benzyl ester was obtained.

NMR ($\delta$, $CDCl_3$): 7.18 - 7.42 (m, 6H), 7.01 - 7.07 (m, 2H), 5.16 (d, J=2.66Hz, 2H), 5.09 - 5.14 (m, 1H), 4.45 - 4.53 (m, 1H), 2.90 (t, J=7.49Hz, 2H), 2.06 - 2.18 (m, 1H), 1.86 - 1.98 (m, 1H), 1.43 (s, 9H)

Subsequently, the same reaction procedure for preparation of the Reference Compound No. 3-24 as used in Reference Example 3-24 was repeated, whereby the captioned Reference Compound No. 3-34 was obtained.

**Reference Example 3-35**

Synthesis of (2S)-2-amino-4-(2-chlorophenylthio)butanol hydrochloride (Reference Compound No. 3-35):

The same reaction procedure for preparation of the L-N-(t-butoxycarbonyl)-O-phenylhomoserine ethyl ester as used in Reference Example 3-24 was repeated except that the ethyl bromide and the phenol used in Reference Example 3-24 were respectively replaced by benzyl bromide and 2-chlorothiophenol, so that L-N-(t-butoxycarbonyl)-S-(2-chlorophenyl)homocysteine benzyl ester was obtained.

NMR ($\delta$, $CDCl_3$): 7.34 (s, 6H), 7.08 - 7.23 (m, 3H), 5.17 (d, J=3.31Hz, 3H), 4.40 - 4.51 (m, 1H), 2.90 - 2.96 (m, 2H), 2.17 - 2.25 (m, 1H), 1.95 - 2.05 (m, 1H), 1.44 (s, 9H)

Subsequently, the same reaction procedure for preparation of the Reference Compound No. 3-24 as used in Reference Example 3-24 was repeated, whereby the captioned Reference Compound No. 3-35 was obtained.

**Reference Example 3-36**

Synthesis of (2S)-2-amino-4-(4-chlorophenylthio)butanol hydrochloride (Reference Compound No. 3-36):

The same reaction procedure for preparation of the L-N-(t-butoxycarbonyl)-O-phenylhomoserine ethyl ester as used in Reference Example 3-24 was repeated except that the ethyl bromide and the phenol used in Reference Example 3-24 were respectively replaced by benzyl bromide and 4-chlorothiophenol, so that L-N-(t-butoxycarbonyl)-S-(4-chlorophenyl)homocysteine benzyl ester was obtained.

NMR ($\delta$, $CDCl_3$): 7.29 - 7.37 (m, 4H), 7.21 (s, 5H), 5.15 (d, J=7.16Hz, 2H), 5.10 - 5.15 (m, 1H), 4.12 - 4.19 (m, 1H), 2.84 - 2.90 (m, 2H), 2.05 - 2.18 (m, 1H), 1.86 - 1.97 (m, 1H), 1.43 (s, 9H)

Subsequently, the same reaction procedure for preparation of the Reference Compound No. 3-24 as used in Reference Example 3-24 was repeated, whereby the captioned Reference Compound No. 3-36 was obtained.

**Reference Example 3-37**

Synthesis of L-N-[1-(benzyloxycarbonyl)-piperidine-4-carbonyl]leucine (Reference Compound No. 3-37):

2.58 g (25.5 mmol) of triethylamine, 6.7 g (25.5 mmol) of N-(benzyloxycarbonyl)-piperidine-4-carboxylic acid and 4.88 g (25.5 mmol) of 1-ethyl-3-(3-dimethylaminopropyl)carbodiimide hydrochloride were successively added to 100 ml of a chloroform solution containing 5 g (25.5 mmol) of L-leucine ethyl ester hydrochloride with stirring under an ice-cooled condition. After the temperature of the mixture was raised to room temperature, the mixture was stirred overnight. The reaction mixture was washed successively with 1 N hydrochloric acid, a saturated solution of sodium hydrogencarbonate and a saturated aqueous solution of sodium chloride, dried over anhydrous sodium sulfate, and then concentrated under reduced pressure, whereby 10.1 g of L-N-[1-(benzyloxycarbonyl)-piperidine-4-carbonyl]leucine ethyl ester was obtained in a yield of 98%.

With stirring under an ice-cooled condition, 10 ml of an aqueous solution containing 1.02 g (25.5 mmol) of sodium hydroxide was added to a methanol solution in which 10.1 g of the above prepared L-N-[1-(benzyloxycarbonyl)-piperidine-4-carbonyl]leucine ethyl ester was dissolved. This reaction mixture was further stirred for 3 hours.

The reaction mixture was concentrated under reduced pressure. The residue thus obtained was

dissolved in water and washed with ether twice. The resultant water layer was made acid (pH = 1) with the addition of concentrated hydrochloric acid, and then extracted with ethyl acetate twice. The extract layer was washed with a saturated aqueous solution of sodium chloride, dried over anhydrous sodium sulfate and concentrated under reduced pressure, whereby 9.3 g of the captioned Reference Compound No. 3-37 was obtained as an oily material in a yield of 96%.

NMR (δ, $CDCl_3$): 8.80 - 9.00 (m, 1H), 7.32 - 7.38 (m, 5H), 6.05 - 6.15 (m, 1H), 5.14 (s, 2H), 4.58 - 4.68 (m, 1H), 4.16 - 4.26 (m, 2H), 2.82 - 2.95 (m, 2H), 2.37 - 2.48 (m, 1H), 1.82 - 1.92 (m, 2H), 1.56 - 1.75 (m, 5H), 0.95 - 0.97 (m, 6H)

**Reference Example 3-38**

Synthesis of L-N-(N-phenylcarbamoyl)leucine (Reference Compound No. 3-38):

5.16 g (51 mmol) of triethylamine was added to 200 ml of a chloroform solution containing 5 g (25.5 mmol) of L-leucine ethyl ester hydrochloride with stirring under an ice-cooled condition. Subsequently, a chloroform solution containing 2.76 ml (25.5 mmol) of isocyanic acid phenyl ester was added dropwise to the above mixture. After stirring overnight, the reaction mixture was washed successively with 1 N hydrochloric acid, a saturated solution of sodium hydrogencarbonate and a saturated aqueous solution of sodium chloride, dried over anhydrous sodium sulfate, and then concentrated under reduced pressure, whereby 5.85 g of L-N-(N-phenylcarbamoyl)leucine ethyl ester was obtained in a yield of 82%.

With stirring under an ice-cooled condition, 10 ml of an aqueous solution containing 1.848 g (46 mmol) of sodium hydroxide was added to 200 ml of a methanol solution in which 5.85 g of the above prepared L-N-(N-phenylcarbamoyl)leucine ethyl ester was dissolved. This reaction mixture was further stirred for 3 hours.

The reaction mixture was concentrated under reduced pressure. The residue thus obtained was dissolved in water and washed with ether twice. The resultant water layer was made acid (pH = 1) with the addition of concentrated hydrochloric acid, so that crystals were caused to separate out.

These crystals separated by filtration were washed successively with water, cooled ethanol and ether, and then dried, whereby 3.55 g of the captioned Reference Compound No. 3-38 was obtained in a yield of 6.7%.

Melting point (° C): 143.8 - 145.8

NMR (δ, $CD_3OD$): 7.33 (dd, J = 8.5Hz, 0.96Hz, 2H), 7.25 (t, J = 5.1Hz, 2H), 6.96 (td, J = 8.5Hz, 1.2Hz, 1H), 4.37 (dd, J = 9.3Hz, 5.1Hz, 1H), 1.51 - 1.89 (m, 3H), 0.99 (d, J = 3.2Hz, 3H), 0.97 (d, J = 3.0Hz, 3H)

**Reference Example 3-39**

Synthesis of L-N-(4-methylbenzenesulfonyl)leucine (Reference Compound No. 3-39):

The same reaction procedure as used in Reference Example 3-38 was repeated except that the 2.76 ml of isocyanic acid phenyl ester used in Reference Example 3-38 was replaced by 4.86 g of 4-methylbenzenesulfonyl chloride, whereby 6.2 g of the captioned Reference Compound No. 3-39 was obtained.

Melting point (° C): 117.2 - 120

NMR (δ, $CDCl_3$): 7.73 (d, J = 8.3Hz, 2H), 7.28 (d, J = 8.3Hz, 2H), 5.07 (d, J = 9.7Hz, 1H), 3.86 - 3.99 (m, 1H), 2.41 (s, 3H), 1.70 - 1.85 (m, 1H), 1.45 - 1.60 (m, 2H), 0.89 (d, J = 6.6Hz, 3H), 0.82 (d, J = 6.5Hz, 3H)

**Reference Example 3-40**

Synthesis of L-N-methyl-N-(benzyloxycarbonyl)leucine (Reference Compound No. 3-40):

With stirring under an ice-cooled condition, 10 ml of a benzene solution containing 0.982 ml (6.88 mmol) of benzyloxycarbonyl chloride and 10 ml of a 1 N sodium hydroxide aqueous solution were simultaneously added dropwise to 10 ml of a 1 N sodium hydroxide aqueous solution containing 1 g (6.88 mmol) of commercially available L-N-methylleucine. After the temperature of the reaction mixture was raised to room temperature, the reaction mixture was stirred overnight. The reaction mixture was washed with ether twice. The resultant water layer was made acid (pH = 1) with the addition of concentrated hydrochloric acid, and then extracted with ethyl acetate twice. The extract layer was dried over anhydrous sodium sulfate

and concentrated under reduced pressure, whereby 0.46 g of the captioned Reference Compound No. 3-40 was obtained.

NMR (δ, $CDCl_3$): 7.30 - 7.40 (m, 5H), 5.10 - 5.23 (m, 2H), 4.92 (t, J=8.3Hz, 2/3H), 4.77 (dd, J=10.5Hz, 4.5Hz, 1/3H), 2.88 (s, 3H), 1.45 - 1.82 (m, 3H), 0.86 - 1.05 (m, 6H)

**Example 3-1**

Synthesis of L-N-benzyloxycarbonylleucine-(2S)-(1-formyl-2-benzyloxy)ethylamide (Compound No. 3-1):

With stirring under an ice-cooled condition, 784 mg (7.8 mmol) of triethylamine, 23 ml (8.5 mmol) of (L)-N-(benzyloxycarbonyl)leucine (hereinafter referred to as L-Cbz-Len-OH)•toluene solution and 1.18 g (7.8 mmol) of 1-hydroxybenzotriazole were successively added to 200 ml of a chloroform suspension of 2.01 g (7.8 mmol) of the Reference Compound No. 3-1 synthesized in Reference Example 3-1. Subsequently, 50 ml of a chloroform solution of 1.76 g (8.525 mmol) of dicyclohexylcarbodiimide was added dropwise to the above prepared mixture. This reaction mixture was stirred overnight at room temperature, and then, the insoluble components were removed therefrom by filtration. The thus obtained filtrate was washed successively with 1 N hydrochloric acid, a saturated aqueous solution of sodium hydrogencarbonate and a saturated aqueous solution of sodium chloride. The resultant organic layer was dried over anhydrous sodium sulfate and concentrated.

The residue thus obtained was chromatographed on a silica gel column for purification, whereby 2.74 g of L-N-(benzyloxycarbonyl)-leucyl-L-(O-benzyl)-serine ethyl ester was obtained in a yield of 75%.

182 mg (8.4 mmol) of lithium boron hydride was added to 50 ml of an anhydrous tetrahydrofuran solution containing 1.58 g (3.4 mmol) of the above prepared L-N-(benzyloxycarbonyl)-leucyl-L-(O-benzyl)-serine ethyl ester, with stirring under an ice-cooled condition. Then, 3 ml of methanol was added dropwise to the above mixture. After the mixture was further stirred for one hour, 10 ml of water was added dropwise to the mixture. Then, the reaction mixture was concentrated under reduced pressure.

The residue thus obtained was made acid (pH=1) with the addition of 1 N hydrochloric acid and extracted with methylene chloride twice. The extract organic layer was washed successively with a saturated solution of sodium hydrogencarbonate and a saturated aqueous solution of sodium chloride, dried over anhydrous sodium sulfate, and then concentrated. The residue thus obtained was recrystallized from a mixed solvent of benzene and ethyl acetate, so that 0.965 g of a corresponding alcohol was obtained in a yield of 67%.

With stirring at room temperature, 10 ml of an anhydrous dimethyl sulfoxide solution containing 1.33 g (8.4 mmol) of sulfur trioxide•pyridine complex was added dropwise to 10 ml of an anhydrous dimethyl sulfoxide solution containing 900 mg (2.1 mmol) of the above prepared alcohol and 850 mg (8.4 mmol) of triethylamine. After stirred for 30 minutes, the reaction mixture was poured into iced water and extracted with ethyl acetate three times. The organic layer was washed successively with a 10% citric acid aqueous solution, water, a saturated aqueous solution of sodium hydrogencarbonate and a saturated aqueous solution of sodium chloride, dried over anhydrous sodium sulfate, and then concentrated under reduced pressure. The residue thus obtained was chromatographed on a silica gel column for purification, whereby 0.425 g of the captioned Compound No. 3-1 was obtained as an oily material in a yield of 47%.

NMR (δ, $CDCl_3$): 9.56 (s, 1H), 7.25 - 7.36 (m, 10H), 6.75 - 6.95 (m, 1H), 5.20 - 5.22 (m, 1H), 5.11 (s, 2H), 4.55 - 4.59 (m, 1H), 4.43 - 4.50 (m, 2H), 4.25 - 4.35 (m, 1H), 3.99 - 4.03 (m, 1H), 3.66 - 3.70 (m, 1H), 1.49 - 1.73 (m, 3H), 0.92 - 0.95 (m, 6H)

$R_f$ values: 0.19 (Developing Solvent A: mixture of hexane and ethyl acetate at a mixing ratio of 1:1)
0.14 (Developing Solvent B: mixture of methylene chloride and acetone at a mixing ratio of 10:1)

**Example 3-2**

Synthesis of L-N-benzyloxycarbonylleucine-(2S)-(1-formyl-2-benzylthio)ethylamide (Compound No. 3-2):

The same reaction procedure as used in Example 3-1 was repeated except that the 2.01 g of Reference Compound No. 3-1 synthesized in Reference Example 3-1 used in Example 3-1 was replaced by 2.2 g of commercially available L-S-benzylcysteine ethyl ester hydrochloride, whereby 0.14 g of the captioned Compound No. 3-2 was obtained.

Melting point (°C): 116.8 - 122.1

NMR (δ, $CDCl_3$): 9.49 (s; 1H), 7.28 - 7.34 (m, 10H), 6.72 - 6.79 (m, 1H), 5.11 (s, 2H), 5.17 - 5.20 (m, 2H), 4.48 - 4.57 (m, 1H), 4.20 - 4.28 (m, 1H), 3.71 (s, 2H), 2.88 (d, J=5.86Hz, 2H), 1.48 - 1.75 (m, 3H), 0.95 (d, J=5.97Hz, 6H)
$R_f$ values: 0.35 (Developing Solvent A)
0.25 (Developing Solvent B)

**Example 3-3**

Synthesis of L-N-benzyloxycarbonylleucine-(2R)-[1-formyl-2-(2-phenylethylthio)]ethylamide (Compound No. 3-3):

The same reaction procedure as used in Example 3-1 was repeated except that the 2.01 g of Reference Compound No. 3-1 synthesized in Reference Example 3-1 used in Example 3-1 was replaced by the 3.3 g of Reference Compound No. 3-2 synthesized in Reference Example 3-2, whereby 0.57 g of the captioned Compound No. 3-3 was obtained as an oily material.
NMR (δ, $CDCl_3$): 9.57 (s, 1H), 7.18 - 7.36 (m, 10H), 6.90 - 7.00 (m, 1H), 5.15 - 5.20 (m, 1H), 5.10 (s, 2H), 4.48 - 4.55 (m, 1H), 4.24 - 4.27 (m, 1H), 2.77 - 2.96 (m, 6H), 1.49 - 1.71 (m, 3H), 0.92 - 0.95 (m, 6H)
$R_f$ values: 0.30 (Developing Solvent A)
0.23 (Developing Solvent B)

**Example 3-4**

Synthesis of L-N-benzyloxycarbonylleucine-(2R)-[1-formyl-2-(3-phenylpropylthio)]ethylamide (Compound No. 3-4):

The same reaction procedure as used in Example 3-1 was repeated except that the 2.01 g of Reference Compound No. 3-1 synthesized in Reference Example 3-1 used in Example 3-1 was replaced by the 3.47 g of Reference Compound No. 3-3 synthesized in Reference Example 3-3, whereby 0.79 g of the captioned Compound No. 3-4 was obtained as an oily material.
NMR (δ, $CDCl_3$): 9.59 (brs, 1H), 7.28 - 7.36 (m, 8H), 7.15 - 7.21 (m, 2H), 6.90 - 7.00 (m, 1H), 5.11 (s, 2H), 5.10 - 5.20 (m, 1H), 4.50 - 4.58 (m, 1H), 4.22 - 4.30 (m, 1H), 2.95 (brs, 2H), 2.69 (t, J=7.33Hz, 2H), 2.53 (t, J=7.32Hz, 2H), 1.84 - 1.94 (m, 2H), 1.48 - 1.70 (m, 3H), 0.94 (d, J=6.73Hz, 6H)
$R_f$ values: 0.31 (Developing Solvent A)
0.26 (Developing Solvent B)

**Example 3-5**

Synthesis of L-N-benzyloxycarbonylleucine-(2S)-[1-formyl-2-(3-phenylpropyloxy)]ethylamide (Compound No. 3-5):

The same reaction procedure as used in Example 3-1 was repeated except that the 2.01 g of Reference Compound No. 3-1 synthesized in Reference Example 3-1 used in Example 3-1 was replaced by the 1.13 g of Reference Compound No. 3-4 synthesized in Reference Example 3-4, whereby 0.315 g of the captioned Compound No. 3-5 was obtained as an oily material.
NMR (δ, $CDCl_3$): 9.57 (s, 1H), 7.25 - 7.36 (m, 8H), 7.14 - 7.21 (m, 2H), 6.77 - 6.93 (m, 1H), 5.20 - 5.26 (m, 1H), 5.05 - 5.14 (m, 2H), 4.54 - 4.59 (m, 1H), 4.27 - 4.32 (m, 1H), 3.94 - 3.98 (m, 1H), 3.61 - 3,65 (m, 1H), 3.41 (t, J=6.35Hz, 2H), 2.62 (t, J=7.92Hz, 2H), 1.79 - 1.90 (m, 2H), 1.51 - 1.79 (m, 3H), 0.93 - 0.96 (m, 6H)
$R_f$ values: 0.18 (Developing Solvent A)
0.16 (Developing Solvent B)

**Example 3-6**

Synthesis of L-N-benzyloxycarbonylleucine-(2S)-[1-formyl-2-(thiophene-3-ylmethyl)oxy]ethylamide (Compound No. 3-6):

The same reaction procedure as used in Example 3-1 was repeated except that the 2.01 g of Reference Compound No. 3-1 synthesized in Reference Example 3-1 used in Example 3-1 was replaced by the 1.20 g of Reference Compound No. 3-5 synthesized in Reference Example 3-5, whereby 0.33 g of the captioned Compound No. 3-6 was obtained as an oily material.

NMR (δ, $CDCl_3$): 9.55 (s, 1H), 7.26- 7.34 (m, 6H), 7.18 (s, 1H), 6.97 - 7.05 (m, 1H), 6.70 - 6.93 (m, 1H), 5.12 (s, 2H), 5.10 - 5.20 (m, 1H), 4.53 - 4.60 (m, 1H), 4.49 (s, 2H), 4.22 - 4.31 (m, 1H), 3.97 - 4.05 (m, 1H), 3.65 - 3.72 (m, 1H), 1.50 - 1.75 (m, 3H), 0.95 (t, J=4.45Hz, 6H)

$R_f$ values: 0.21 (Developing Solvent A)
0.24 (Developing Solvent B)

**Example 3-7**

Synthesis of L-N-benzyloxycarbonylleucine-(2R)-(1-formyl-2-diphenylmethylthio)ethylamide (Compound No. 3-7):

The same reaction procedure as used in Example 3-1 was repeated except that the 2.01 g of Reference Compound No. 3-1 synthesized in Reference Example 3-1 used in Example 3-1 was replaced by the 1.13 g of Reference Compound No. 3-6 synthesized in Reference Example 3-6, whereby 0.17 g of the captioned Compound No. 3-7 was obtained as an oily material.

NMR (δ, $CDCl_3$): 9.44 (s, 1H), 7.21 - 7.41 (m, 15H), 6.72 - 6.85 (m, 1H), 5.08 - 5.18 (m, 4H), 4.46 - 4.51 (m, 1H), 4.20 - 4.30 (m, 1H), 2.75 - 2.92 (m, 2H), 1.47 - 1.71 (m, 3H), 0.93 - 0.95 (m, 6H)

$R_f$ values: 0.32 (Developing Solvent A)
0.25 iDeveloping Solvent B)

**Example 3-8**

Synthesis of L-N-benzyloxycarbonylleucine-(2R)-(1-formyl-2-cyclohexylmethylthio)ethylamide (Compound No. 3-8):

The same reaction procedure as used in Example 3-1 was repeated except that the 2.01 g of Reference Compound No. 3-1 synthesized in Reference Example 3-1 used in Example 3-1 was replaced by the 2.53 g of Reference Compound No. 3-7 synthesized in Reference Example 3-7, whereby 0.74 g of the captioned Compound No. 3-8 was obtained as an oily material.

NMR (δ, $CDCl_3$): 9.61 (s, 1H), 7.35 (s, 5H), 6,89 - 6.93 (m, 1H), 5.12 (s, 2H), 5.10 - 5.20 (s, 1H), 4.51 - 4.57 (m, 1H), 4.22 - 4.30 (m, 1H), 2.92 - 2.96 (m, 2H), 2.42 (d, J=6,84Hz, 2H), 1.28 - 1.82 (m, 10H), 1.11 - 1.26 (m, 4H), 0.95 (d, J=6.02Hz, 6H)

$R_f$ values: 0.38 (Developing Solvent A)
0.28 (Developing Solvent B)

**Example 3-9**

Synthesis of L-N-benzyloxycarbonylleucine-(2R)-(1-formyl-2-cyclopentylthio)ethylamide (Compound No. 3-9):

The same reaction procedure as used in Example 3-1 was repeated except that the 2.01 g of Reference Compound No. 3-1 synthesized in Reference Example 3-1 used in Example 3-1 was replaced by the 2.0 g of Reference Compound No. 3-8 synthesized in Reference Example 3-8, whereby 0.29 g of the captioned Compound No. 3-9 was obtained as an oily material.

NMR (δ, $CDCl_3$): 9.62 (s, 1H), 7.35 (S, 5H), 6.89 - 6.92 (m, 1H), 5.12 (s, 2H), 5.10 - 5.20 (m, 1H), 4.55 - 4.60 (m, 1H), 4.32 - 4.41 (m, 1H), 2.90 - 3.12 (m, 3H), 1.95 - 2.05 (m, 3H), 1.46 - 1.72 (m, 8H), 0.95 (d, J=5.26Hz, 6H)

$R_f$ values: 0.37 (Developing Solvent A)
0.32 (Developing Solvent B)

**Example 3-10**

Synthesis of L-N-benzyloxycarbonylleucine-(2R)-[1-formyl-2-(thiophene-2-ylmethyl)thio]ethylamide (Compound No. 3-10):

The same reaction procedure as used in Example 3-1 was repeated except that the 2.01 g of Reference Compound No. 3-1 synthesized in Reference Example 3-1 used in Example 3-1 was replaced by the 0.96 g of Reference Compound No. 3-9 synthesized in Reference Example 3-9, whereby 0.22 g of the captioned Compound No. 3-10 was obtained as an oily material.

NMR (δ, $CDCl_3$): 9.50 (s, 1H), 7.34 (s, 5H), 7.21 - 7.23 (m, 1H), 6.89 - 6.96 (m, 2H), 6.79 - 6.83 (m, 1H), 5.12 (s, 2H), 5.11 - 5.17 (m, 1H), 4.48 - 4.53 (m, 1H), 4.21 - 4.30 (m, 1H), 3.92 (s, 2H), 2.94 (d, J=5.76, 2H), 1.49 - 1.72 (m, 3H), 0.95 (d, J=6.08Hz, 6H)

$R_f$ values: 0.28 (Developing Solvent A)
0.18 (Developing Solvent B)

**Example 3-11**

Synthesis of L-N-benzyloxycarbonylleucine-(2R)-[1-formyl-2-(thiophene-3-ylmethyl)thio]ethylamide (Compound No. 3-11):

The same reaction procedure as used in Example 3-1 was repeated except that the 2.01 g of Reference Compound No. 3-1 synthesized in Reference Example 3-1 used in Example 3-1 was replaced by the 2.5 g of Reference Compound No. 3-10 synthesized in Reference Example 3-10, whereby 1.49 g of the captioned Compound No. 3-11 was obtained as an oily material.

NMR (δ, $CDCl_3$): 9.49 (s, 1H), 7.26 - 7.34 (m, 6H), 7.16 (s, 1H), 7.05 (d, J=4.93, 1H), 6.79 - 6.91 (m, 1H), 5.14 - 5.18 (m, 1H), 5.12 (s, 2H), 4.48 - 4.52 (m, 1H), 4.20 - 4.30 (m, 1H), 3.73 (s, 2H), 2.86 (brs, 2H), 1.49 - 1.71 (m, 3H), 0.95 (d, J=5.96Hz, 6H)

$R_f$ values: 0.33 (Developing Solvent A)
0.22 (Developing Solvent B)

**Example 3-12**

Synthesis of L-N-[1-(benzyloxycarbonyl)-piperidine-4-carbonyl]leucine-(2R)-(1-formyl-2-benzylthio)-ethylamide (Compound No. 3-12):

The same reaction procedure as used in Example 3-1 was repeated except that L-cbz-Len-OH used in Example 3-1 was replaced by the Reference Compound No. 3-37 synthesized in Reference Example 3-38, and that the 2.01 g of Reference Compound No. 3-1 synthesized in Reference Example 3-1 used in Example 3-1 was replaced by 0.98 g of commercially available L-S-benzylcysteine ethyl ester hydrochloride, whereby 0.39 g of the captioned Compound No. 3-12 was obtained as an oily material.

NMR (δ, $CDCl_3$): 9.46 (s, 1H), 7.24 - 7.36 (m, 10H), 6.25 - 6.40 (m, 1H), 5.11 (s, 2H), 4.56 - 4.59 (m, 1H), 4.42 - 4.47 (m, 1H), 4.10 - 4.25 (m, 2H), 3.70 (d, J=3.27Hz, 2H), 2.74 - 2.91 (m, 4H), 2.25 - 2.33 (m, 1H), 1.52 - 1.82 (m, 8H), 0.93 (t, J=5.58Hz, 6H)

$R_f$ values: 0.04 (Developing Solvent A)
0.05 (Developing Solvent B)

**Example 3-13**

Synthesis of L-N-(benzyloxycarbonyl)leucine-(2R)-[1-formyl-2-(naphthalene-1-ylmethylthio)]ethylamide (Compound No. 3-13):

With stirring under an ice-cooled condition, 0.81 g (3.98 mmol) of triethylamine was added dropwise to a chloroform solution containing the 1.25 g (3.98 mmol) of Reference Compound No. 3-11 synthesized in Reference Example 3-11 and 1.45 g (3.98 mmol) of L-N-(benzyloxycarbonyl)leucine N-hydroxysuccinimide ester. This reaction mixture was further stirred overnight. The reaction mixture was washed successively with 1 N hydrochloric acid aqueous solution, a saturated aqueous solution of sodium hydrogencarbonate and water, and dried over anhydrous sodium sulfate. The solvent was distilled away from the reaction mixture under reduced pressure. The residue thus obtained was chromatographed on a silica gel column for purification, whereby 0.9 g of L-N-(benzyloxycarbonyl)-leucyl-(L)-S-(naphthalene-1-yl-methyl)cysteine ethyl ester was obtained in a yield of 42%.

To an anhydrous tetrahydrofuran solution of 0.9 g (1.68 mmol) of the above prepared ester, 0.073 g (3.35 mmol) of lithium boron hydride was added, and then methanol was added dropwise under an ice-cooled condition. This reaction mixture was stirred for 3 hours. After stirring, water was added dropwise to the reaction mixture and the solvent was distilled away therefrom under reduced pressure.

An aqueous solution of the thus obtained residue was made acid with the addition of a 1 N hydrochloric acid aqueous solution, and extracted with ethyl acetate. The extract layer was washed with water, and dried over anhydrous sodium sulfate. The solvent was distilled away under reduced pressure, and the residue thus obtained was chromatographed on a silica gel column for purification, so that 0.577 g of an alcohol was obtained in a yield of 69%.

A dimethyl sulfoxide solution of 0.708 g (4.44 mmol) of sulfur trioxide•pyridine complex was added dropwise to an anhydrous dimethyl sulfoxide solution containing 0.55 g (1.11 mmol) of the above prepared alcohol and 0.45 g (4.44 mmol) of triethylamine with stirring at room temperature. This reaction mixture was further stirred at room temperature for one hour. The reaction mixture was poured into iced water, and extracted with ethyl acetate three times. The extract layer was washed successively with a 10% citric acid aqueous solution, a saturated aqueous solution of sodium chloride, a saturated aqueous solution of sodium hydrogencarbonate and a saturated aqueous solution of sodium chloride, and dried over anhydrous sodium sulfate. The solvent was distilled away under reduced pressure, and the residue thus obtained was chromatographed on a silica gel column for purification, whereby 0.26 g of the captioned Compound No. 3-13 was obtained in a yield of 47%.

| | |
|---|---|
| Melting point (°C): | 115.0 - 126.1 (dec.) |
| NMR (δ, $CDCl_3$): | 9.46 (s, 1H), 8.06 (d, J=7.92Hz, 1H), 7.86 (d, J=9.23, 1H), 7.76 - 7.80 (m, 2H), 7.31 - 7.58 (m, 8H), 6.75 - 6.90 (m, 1H), 5.08 (d, J=3.78Hz, 2H), 4.98 - 5.05 (m, 1H), 4.48 - 4.58 (m, 1H), 4.17 - 4.27 (m, 1H), 4.17 (s, 2H), 2.90 - 2.93 (m, 2H), 1.35 - 1.72 (m, 3H), 0.91 - 0.93 (m, 6H) |
| $R_f$ values: | 0.28 (Developing Solvent A)<br>0.22 (Developing Solvent B) |

**Example 3-14**

Synthesis of L-N-benzyloxycarbonylleucine-(2R)-[1-formyl-2-(naphthalene-2-ylmethyl)thio]ethylamide (Compound No. 3-14):

The same reaction procedure as used in Example 3-13 was repeated except that the 1.25 g of Reference Compound No. 3-11 synthesized in Reference Example 3-11 was replaced by the 1.26 g of Reference Compound No. 3-12 synthesized in Reference Example 3-12, whereby 0.1 g of the captioned Compound No. 3-14 was obtained as an oily material.

| | |
|---|---|
| NMR (δ, $CDCl_3$): | 9.48 (d, J=5.10Hz, 1H), 7.71 - 7.83 (m, 3H), 7.32 - 7.49 (m, 9H), 6.80 - 6.82 (m, 1H), 5.11 (d, J=3.36Hz, 2H), 5.07 - 5.18 (m, 1H), 5.50 - 5.57 (m, 1H), 4.22 - 4.30 (m, 1H), 3.87 (s, 2H), 2.87 (d, J=5.01Hz, 2H), 1.45 - 1.80 (m, 3H), 0.93 (d, J=6.36Hz, 6H) |
| $R_f$ values: | 0.29 (Developing Solvent A)<br>0.22 (Developing Solvent B) |

**Example 3-15**

Synthesis of L-N-benzyloxycarbonylleucine-(2R)-[1-formyl-2-(2-chlorobenzyl)thio]ethylamide (Compound No. 3-15):

The same reaction procedure as used in Example 3-13 was repeated except that the 1.25 g of Reference Compound No. 3-11 synthesized in Reference Example 3-11 was replaced by the 1.24 g of Reference Compound No. 3-13 synthesized in Reference Example 3-13, whereby 0.57 g of the captioned Compound No. 3-15 was obtained.

| | |
|---|---|
| Melting point (°C): | 128.6 - 131.9 |
| NMR (δ, $CDCl_3$): | 9.55 (s, 1H), 7.20 - 7.40 (m, 9H), 6.83 - 6.91 (m, 1H), 5.10 - 5.20 (brs, 3H), 4.52 - 4.60 (m, 1H), 4.20 - 4.30 (m, 1H), 3.84 (s, 2H), 2.93 (brs, 2H), 1.49 - 1.73 (m, 4H), 0.95 (d, J=4.29Hz, 6H) |
| $R_f$ values: | 0.31 (Developing Solvent A)<br>0.24 (Developing Solvent B) |

**Example 3-16**

Synthesis of L-N-methyl-N-(benzyloxycarbonyl)leucine-(L)-[1-formyl-2-(4-chlorobenzylthio)]ethylamide (Compound No. 3-16):

A chloroform solution of 0.664 g (3.22 mmol) of dicyclohexylcarbodiimide was added dropwise to 100 ml of a chloroform solution containing the 0.9 g (3.22 mmol) of Reference Compound No. 3-40 synthesized in Reference Example 3-40, 0.326 g (3.22 mmol) of triethylamine, 0.443 g (3.22 mmol) of 1-hydroxybenzotriazole and the 0.748 g (3.22 mmol) of Reference Compound No. 3-16 synthesized in Reference Example 3-16, with stirring in an ice bath containing salt. This reaction mixture was further stirred overnight.

The insoluble components were removed from the reaction mixture by filtration. The filtrate thus obtained was washed successively with 1 N hydrochloric acid, a saturated solution of sodium hydrogencarbonate and a saturated aqueous solution of sodium chloride, dried over anhydrous sodium sulfate and then concentrated under reduced pressure. The residue thus obtained was chromatographed on a silica gel column for purification, whereby 1.2 g of L-N-methyl-N-(benzyloxycarbonyl)leucine-(L)-[1-hydroxymethyl-2-(4-chlorophenyl)]ethylamide was obtained in a yield of 75%.

10 ml of an anhydrous dimethyl sulfoxide solution of 1.14 g (7.22 mmol) of sulfur trioxide•pyridine complex was added dropwise to 10 ml of an anhydrous dimethyl sulfoxide solution containing 0.89 g (1.8 mmol) of the above prepared L-N-methyl-N-(benzyloxycarbonyl)leucine-(2R)-[1-hydroxymethyl-2-(4-chlorophenyl)]ethylamide and 0.13 g (7.22 mmol) of triethylamine with stirring at room temperature. This reaction mixture was further stirred for 30 minutes. The reaction mixture was poured into iced water, and extracted with ethyl acetate three times. The extract organic layer was washed successively with a 10% citric acid aqueous solution, water, a saturated aqueous solution of sodium hydrogencarbonate and a saturated aqueous solution of sodium chloride, dried over anhydrous sodium sulfate, and then concentrated under reduced pressure. The residue thus obtained was chromatographed on a silica gel column for purification, whereby 0.52 g of the captioned Compound No. 3-16 was obtained as an oily material.

NMR ($\delta$, $CDCl_3$): 9.50 (s, 1H), 7.15 - 7.40 (m, 9H), 6.80 - 6.95 (m, 1H), 5.10 - 5.30 (m, 2H), 4.60 - 4.90 (m, 1H), 4.40 - 4.55 (m, 1H), 3.60 - 3.71 (m, 2H), 2.85 (s, 3H), 2.60 - 2.85 (m, 2H), 1.60 - 1.80 (m, 2H), 1.42 - 1.60 (m, 1H), 0.80 - 1.05 (m, 6H)

$R_f$ values: 0.42 (Developing Solvent A)
0.30 (Developing Solvent B)

**Example 3-17**

Synthesis of L-N-(N-phenylcarbamoyl)leucine-(2R)-[1-formyl-2-(4-chlorobenzylthio)]ethylamide (Compound No. 3-17):

The same reaction procedure as used in Example 3-16 was repeated except that the 0.9 g of Reference Compound No. 3-40 synthesized in Reference Example 3-40 was replaced by the 1.0 g of Reference Compound No. 3-38 synthesized in Reference Example 3-38, whereby 0.34 g of the captioned Compound No. 3-17 was obtained as an amorphous material.

NMR ($\delta$, $CDCl_3$): 9.40 (s, 1H), 7.97 (bs, 1H), 7.05 - 7.45 (m, 9H), 6.63 (bs, 1H), 4.50 - 4.60 (m, 1H), 4.30 - 4.40 (m, 1H), 3.60 - 3.75 (m, 2H), 3.10 - 3.30 (m, 2H), 1.45 - 1.85 (m, 3H), 0.85 - 1.10 (m, 6H)

$R_f$ values: 0.20 (Developing Solvent A)
0.10 (Developing Solvent B)

**Example 3-18**

Synthesis of L-N-(4-methylbenzenesulfonyl)leucine-(2R)-[1-formyl-2-(4-chlorobenzylthio)]ethylamide (Compound No. 3-18):

The same reaction procedure as used in Example 3-16 was repeated except that the 0.9 g of Reference Compound No. 3-40 synthesized in Reference Example 3-40 was replaced by the 1.0 g of Reference Compound No. 3-39 synthesized in Reference Example 3-39, whereby 0.54 g of the captioned Compound No. 3-18 was obtained as an amorphous material.

NMR ($\delta$, $CDCl_3$): 9.37 (s, 1H), 7.73 (d, J = 8.2Hz, 2H), 7.20 - 7.35 (m, 6H), 6.61 (d, J = 8.4Hz, 1H), 4.99 (d, J = 5.8Hz, 1H), 4.29 - 4.45 (m, 2H), 3.63 (s, 2H), 2.70 - 2.90 (m, 2H), 2.40 (s, 3H),

| | |
|---|---|
| | 1.40 - 1.70 (m, 3H), 0.90 - 1.10 (m, 6H) |
| $R_f$ values: | 0.33 (Developing Solvent A) |
| | 0.25 (Developing Solvent B) |

**Example 3-19**

Synthesis of L-N-(benzyloxycarbonyl)phenylalanine-(2R)-[1-formyl-2-(4-chlorobenzylthio)]ethylamide (Compound No. 3-19):

The same reaction procedure as used in Example 3-16 was repeated except that the 0.9 g of Reference Compound No. 3-40 synthesized in Reference Example 3-40 was replaced by 1.0 g of commercially available L-N-(benzyloxycarbonyl)phenylalanine, whereby 0.4 g of the captioned Compound No. 3-19 was obtained.

| | |
|---|---|
| Melting point (° C): | 129.4 - 133.4 |
| NMR (δ, $CDCl_3$): | 9.38 (s, 1H), 7.10 - 7.41 (m, 14H), 6.30 - 6.40 (m, 1H), 5.20 - 5.30 (m, 1H), 5.09 (s, 2H), 4.35 - 4.51 (m, 2H), 3.35 - 3.45 (m, 2H), 3.00 - 3.20 (m, 2H), 2.70 - 2.80 (m, 2H) |
| $R_f$ values: | 0.36 (Developing Solvent A) |
| | 0.24 (Developing Solvent B) |

**Example 3-20**

Synthesis of L-N-(benzyloxycarbonyl)-valine-(2R)-[1-formyl-2-(4-chlorobenzylthio)]ethylamide (Compound No. 3-20):

The same reaction procedure as used in Example 3-16 was repeated except that the 0.9 g of Reference Compound No. 3-40 synthesized in Reference Example 3-40 was replaced by 1.0 g of commercially available L-N-(benzyloxycarbonyl)valine, whereby 0.14 g of the captioned Compound No. 3-20 was obtained.

| | |
|---|---|
| Melting point (° C): | 126.2 - 128.7 |
| NMR (δ, $CDCl_3$): | 9.52 (s, 1H), 7.20 - 7.45 (m, 9H), 6.60 - 6.75 (m, 1H), 5.25 - 5.35 (m, 1H), 5.11 (s, 2H), 4.50 - 4.61 (m, 1H), 4.00 - 4.15 (m, 1H), 3.68 (s, 2H), 2.75 - 2.90 (m, 2H), 2.10 - 2.25 (m, 1H), 0.90 - 1.05 (m, 6H) |
| $R_f$ values: | 0.36 (Developing Solvent A) |
| | 0.21 (Developing Solvent B) |

**Example 3-21**

Synthesis of L-N-(benzyloxycarbonyl)leucine-(2R)-[1-formyl-2-(4-chlorobenzyl)thio]ethylamide (Compound No. 3-21):

6.25 g (17.26 mmol) of L-N-(benzyloxycarbonyl)leucine-N-hydroxysuccinimide ester was gradually added to 200 ml of a chloroform solution containing the 4 g (17.26 mmol) of Reference Compound No. 3-16 synthesized in Reference Example 3-16 and 1.74 g (17.26 mmol) of triethylamine with stirring under an ice-cooled condition. After the temperature of the reaction mixture was raised to room temperature, the mixture was stirred overnight.

The reaction mixture was washed successively with 1 N hydrochloric acid, a saturated solution of sodium hydrogencarbonate (three times) and a saturated aqueous solution of sodium chloride, dried over anhydrous sodium sulfate, and then concentrated under reduced pressure, so that 7.8 g of L-N-(benzyloxycarbonyl)leucine-(2R)-[1-hydroxymethyl-2-(4-chlorobenzylthio)]ethylamide was obtained in a yield of 94%.

30 ml of an anhydrous dimethyl sulfoxide solution of 10.34 g (65 mmol) of sulfur trioxide•pyridine complex was added dropwise to 100 ml of an anhydrous dimethyl sulfoxide solution containing 7.8 g (16.28 mmol) of the above prepared L-N-(benzyloxycarbonyl)leucine-(2R)-[1-hydroxymethyl-2-(4-chlorobenzylthio)]-ethylamide and 6.57 g (65 mmol) of triethylamine with stirring at room temperature. This reaction mixture was further stirred for 30 minutes. The reaction mixture was poured into iced water, and extracted with ethyl acetate three times. The extract organic layer was washed successively with a 10% citric acid aqueous solution, water, a saturated solution of sodium hydrogencarbonate and a saturated aqueous solution of

sodium chloride, dried over anhydrous sodium sulfate, and then concentrated under reduced pressure. The residue thus obtained was chromatographed on a silica gel column for purification, whereby 2.4 g of the captioned Compound No. 3-21 was obtained.

Melting point (° C): 116.0 - 130.7 (dec.)
NMR (δ, $CDCl_3$): 9.52 (d, J = 4.88Hz, 1H), 7.22 - 7.33 (m, 10H), 6.83 - 6.91 (m, 1H), 5.11 (d, J = 2.33Hz, 3H), 4.50 - 4.59 (m, 1H), 4.23 - 4.30 (m, 1H), 3.67 (s, 2H), 2.85 (d, J = 4.99Hz, 2H), 1.49 - 1.72 (m, 3H), 0.94 - 0.96 (m, 6H)
$R_f$ values: 0.25 (Developing Solvent A)
0.19 (Developing Solvent B)

**Example 3-22**

Synthesis of L-N-benzyloxycarbonylleucine-(2R)-[1-formyl-2-(3-chlorobenzyl)thio]ethylamide (Compound No. 3-22):

The same reaction procedure as used in Example 3-21 was repeated except that the 4 g of Reference Compound No. 3-16 synthesized in Reference Example 3-16 was replaced by the 3.0 g of Reference Compound No. 3-15 synthesized in Reference Example 3-15, whereby 0.68 g of the captioned Compound No. 3-22 was obtained.

Melting point (° C): 97.6 - 103.3
NMR (δ, $CDCl_3$): 9.53 (s, 1H), 7.16 - 7.34 (m, 9H), 6.84 - 6.90 (m, 1H), 5.11 (s, 2H), 5.10 - 5.17 (m, 1H), 4.50 - 4.60 (m, 1H), 4.20 - 4.30 (m, 1H), 3.68 (s, 2H), 2.80 - 2.93 (m, 2H), 1.48 - 1.76 (m, 3H), 0.94 - 0.97 (m, 6H)
$R_f$ values: 0.25 (Developing Solvent A)
0.18 (Developing Solvent B)

**Example 3-23**

Synthesis of L-N-benzyloxycarbonylleucine-(2R)-[1-formyl-2-(2-fluorobenzyl)thio]ethylamide (Compound No. 3-23):

The same reaction procedure as used in Example 3-21 was repeated except that the 4 g of Reference Compound No. 3-16 synthesized in Reference Example 3-16 was replaced by the 8.7 g of Reference Compound No. 3-14 synthesized in Reference Example 3-14, whereby 2.75 g of the captioned Compound No. 3-23 was obtained.

Melting point (° C): 110.1 - 118.3
NMR (δ, $CDCl_3$): 9.54 (s, 1H), 7.08 - 7.34 (m, 7H), 7.02 - 7.12 (m, 2H), 6.83 (brs, 1H), 5.11 (s, 3H), 4.50 - 4.60 (m, 1H), 4.20 - 4.30 (m, 1H), 3.75 (d, J = 2.93Hz, 2H), 2.93 (d, J = 5.48Hz, 2H), 1.50 - 1.75 (m, 3H), 0.95 (d, J = 6.18Hz, 6H)
$R_f$ values: 0.30 (Developing Solvent A)
0.22 (Developing Solvent B)

**Example 3-24**

Synthesis of L-N-benzyloxycarbonylleucine-(2R)-[1-formyl-2-(3-fluorobenzyl)thio]ethylamide (Compound No. 3-24):

The same reaction procedure as used in Example 3-21 was repeated except that the 4 g of Reference Compound No. 3-16 synthesized in Reference Example 3-16 was replaced by the 6.5 g of Reference Compound No. 3-17 synthesized in Reference Example 3-17, whereby 0.37 g of the captioned Compound No. 3-24 was obtained.

Melting point (° C) : 111.8 - 116.6
NMR (δ, $CDCl_3$): 9.53 (s, 1H), 7.24 - 7.34 (m, 6H), 7.06 (t, J = 8.19Hz, 1H), 6.95 (t, J = 8.96Hz, 1H), 6.81 - 6.87 (m, 1H), 5.11 (s, 3H), 4.50 - 4.59 (m, 1H), 4.21 - 4.30 (m, 1H), 3.70 (s, 7H), 2.87 (d, J = 5.37Hz, 2H), 1.45 - 1.73 (m, 3H), 0.94 - 0.97 (m, 6H)
$R_f$ values: 0.23 (Developing Solvent A)
0.19 (Developing Solvent B)

**Example 3-25**

Synthesis of L-N-benzyloxycarbonylleucine-(2R)-[1-formyl-2-(4-fluorobenzyl)thio]ethylamide (Compound No. 3-25):

The same reaction procedure as used in Example 3-21 was repeated except that the 4 g of Reference Compound No. 3-16 synthesized in Reference Example 3-16 was replaced by the 6.46 g of Reference Compound No. 3-18 synthesized in Reference Example 3-18, whereby 0.8 g of the captioned Compound No. 3-25 was obtained.

| | |
|---|---|
| Melting point (°C): | 71.2 - 76.3 |
| NMR (δ, $CDCl_3$): | 9.52 (d, J=5.37Hz, 1H), 7.25 - 7.34 (m, 7H), 6.95 - 7.07 (m, 2H), 6.82 - 6.90 (m, 1H), 5.11 - 5.20 (m, 3H), 4.50 - 4.60 (m, 1H), 4.20 - 4.30 (m, 1H), 3.69 (s, 2H), 2.80 - 2.90 (m, 2H), 1.49 - 1.73 (m, 3H), 0.95 (d, J=4.12Hz, 6H) |
| $R_f$ values: | 0.25 (Developing Solvent A) |
| | 0.20 (Developing Solvent B) |

**Example 3-26**

Synthesis of L-N-benzyloxycarbonylleucine-(2R)-[1-formyl-2-(2-methoxybenzyl)thio]ethylamide (Compound No. 3-26):

The same reaction procedure as used in Example 3-21 was repeated except that the 4 g of Reference Compound No. 3-16 synthesized in Reference Example 3-16 was replaced by the 5.0 g of Reference Compound No. 3-19 synthesized in Reference Example 3-19, whereby 0.88 g of the captioned Compound No. 3-26 was obtained.

| | |
|---|---|
| Melting point (°C): | 110.8 - 120.2 |
| NMR (δ, $CDCl_3$): | 9.51 (s, 1H), 7.22 - 7.34 (m, 7H), 6.86 - 6.97 (m, 2H), 6.80 - 6.85 (m, 1H), 5.15 - 5.25 (m, 1H), 5.11 (s, 2H), 4.51 - 4.59 (m, 1H), 4.22 - 4.28 (m, 1H), 3.83 (s, 3H), 3.85 (d, J=5.42Hz, 1H), 3.74 (d, J=5.7Hz, 1H), 2.91 (d, J=5.86Hz, 2H), 1.42 - 1.74 (m, 3H), 0.93 - 0.96 (m, 6H) |
| $R_f$ values: | 0.27 (Developing Solvent A) |
| | 0.20 (Developing Solvent B) |

**Example 3-27**

Synthesis of L-N-benzyloxycarbonylleucine-(2R)-[1-formyl-2-(3-methoxybenzyl)thio]ethylamide (Compound No. 3-27):

The same reaction procedure as used in Example 3-21 was repeated except that the 4 g of Reference Compound No. 3-16 synthesized in Reference Example 3-16 was replaced by the 3.41 g of Reference Compound No. 3-20 synthesized in Reference Example 3-20, whereby 0.71 g of the captioned Compound No. 3-27 was obtained.

| | |
|---|---|
| Melting point (°C): | 113.8 - 117.8 |
| NMR (δ, $CDCl_3$): | 9.50 (s, 1H), 7.20 - 7.34 (m, 6H), 6.76 - 6.90 (m, 4H), 4.49 - 4.53 (m, 1H), 4.23 - 4.30 (m, 1H), 3.80 (s, 3H), 3.68 (s, 2H), 2.88 (d, J=5.75Hz, 2H), 1.50 - 1.75 (m, 3H), 0.95 (d, J=5.97Hz, 6H) |
| $R_f$ values: | 0.25 (Developing Solvent A) |
| | 0.19 (Developing Solvent B) |

**Example 3-28**

Synthesis of L-N-benzyloxycarbonylleucine-(2R)-[1-formyl-2-(4-methoxybenzyl)thio]ethylamide (Compound No. 3-28):

The same reaction procedure as used in Example 3-21 was repeated except that the 4 g of Reference Compound No. 3-16 synthesized in Reference Example 3-16 was replaced by the 4.54 g of Reference Compound No. 3-21 synthesized in Reference Example 3-21, whereby 0.13 g of the captioned Compound No. 3-28 was obtained as an oily material.

NMR (δ, $CDCl_3$): 9.45 (brs, 1H), 7.30 - 7.35 (m, 5H), 7.22 (d, J = 8.57Hz, 2H), 6.85 (d, J = 7.54Hz, 2H), 6.76 - 6.89 (m, 1H), 5.11 (s, 2H), 5.08 - 5.18 (m, 1H), 4.45 - 4.52 (m, 1H), 4.20 - 4.30 (m, 1H), 3.79 (s, 3H), 2.78 - 2.89 (m, 2H), 1.46 - 1.85 (m, 3H), 0.94 (d, J = 5.86Hz, 6H)
$R_f$ values: 0.19 (Developing Solvent A)
0.26 (Developing Solvent B)

**Example 3-29**

Synthesis of L-N-benzyloxycarbonylleucine-(2R)-[1-formyl-2-(3-nitrobenzyl)thio]ethylamide (Compound No. 3-29):

The same reaction procedure as used in Example 3-21 was repeated except that the 4 g of Reference Compound No. 3-16 synthesized in Reference Example 3-16 was replaced by the 4.18 g of Reference Compound No. 3-22 synthesized in Reference Example 3-22, whereby 0.37 g of the captioned Compound No. 3-29 was obtained.

Melting point (°C): 72.9 - 104.9 (dec.)
NMR (δ, $CDCl_3$): 9.56 (d, J = 6.95Hz, 1H), 8.21 (s, 1H), 8.13 (dd, J = 2.44Hz, 7.22Hz, 1H), 7.65 (d, J = 7.43Hz), 7.45 - 7.52 (m, 1H), 7.33 (d, J = 4.1Hz, 5H), 6.91 - 7.07 (m, 1H), 5.20 (d, J = 7.70Hz, 1H), 5.11 (d, J = 5.48Hz, 2H), 4.53 - 4.59 (m, 1H), 4.20 - 4.35 (m, 1H), 3.80 (s, 2H), 2.80 - 2.95 (m, 2H), 1.51 - 1.80 (m, 3H), 0.95 (d, J = 5.68Hz, 6H)
$R_f$ values: 0.17 (Developing Solvent A)
0.17 (Developing Solvent B)

**Example 3-30**

Synthesis of L-N-benzyloxycarbonylleucine-(2R)-[1-formyl-2-(4-nitrobenzyl)thio]ethylamide (Compound No. 3-30):

The same reaction procedure as used in Example 3-21 was repeated except that the 4 g of Reference Compound No. 3-16 synthesized in Reference Example 3-16 was replaced by the 2.17 g of Reference Compound No. 3-23 synthesized in Reference Example 3-23, whereby 0.1 g of the captioned Compound No. 3-30 was obtained as an oily material.

NMR (δ, $CDCl_3$): 9.56 (s, 1H), 8.15 - 8.20 (m, 2H), 7.47 - 7.51 (m, 2H), 7.30 - 7.34 (m, 5H), 6.84 - 7.00 (m, 1H), 5.05 - 5.15 (m, 3H), 4.53 - 4.60 (m, 1H), 4.20 - 4.30 (m, 1H), 3.79 (s, 2H), 2.87 (d, J = 5.26Hz, 2H), 1.49 - 1.75 (m, 3H), 0.94 - 0.97 (m, 6H)
$R_f$ values: 0.15 (Developing Solvent A)
0.15 (Developing Solvent B)

**Example 3-31**

Synthesis of L-N-benzyloxycarbonylleucine-(2S)-(1-formyl-3-phenyloxy)propylamide (Compound No. 3-31):

The same reaction procedure as used in Example 3-21 was repeated except that the 4 g of Reference Compound No. 3-16 synthesized in Reference Example 3-16 was replaced by the 0.52 g of Reference Compound No. 3-24 synthesized in Reference Example 3-24, whereby 0.52 g of the captioned Compound No. 3-31 was obtained as an oily material.

NMR (δ, $CDCl_3$): 9.64 (s, 1H), 7.24 - 7.33 (m, 8H), 6.82 - 6.98 (m, 3H), 4.95 - 5.15 (m, 3H), 4.55 - 4.59 (m, 1H), 4.20 - 4.30 (m, 1H), 3.95 - 4.10 (m, 2H), 2.27 - 2.53 (m, 2H), 1.45 - 1.72 (m, 3H), 0.92 (t, J = 6.24Hz, 6H)
$R_f$ values: 0.26 (Developing Solvent A)
0.19 (Developing Solvent B)

**Example 3-32**

Synthesis of L-N-benzyloxycarbonylleucine-(2S)-(1-formyl-3-phenylthio)propylamide (Compound No. 3-32):

The same reaction procedure as used in Example 3-21 was repeated except that the 4 g of Reference Compound No. 3-16 synthesized in Reference Example 3-16 was replaced by the 0.87 g of Reference Compound No. 3-25 synthesized in Reference Example 3-25, whereby 0.42 g of the captioned Compound No. 3-32 was obtained as an oily material.

NMR (δ, $CDCl_3$): 9.52 (d, J=6.24Hz, 1H), 7.12 - 7.38 (m, 10H), 6.71 - 6.86 (m, 1H), 5.14 - 5.16 (d, J=6.95Hz, 1H), 5.10 (s, 2H), 4.51 - 4.59 (m, 1H), 4.20 - 4.25 (m, 1H), 2.91 - 2.96 (m, 2H), 2.25 - 2.35 (m, 1H), 1.85 - 2.00 (m, 1H), 1.48 - 1.72 (m, 3H), 0.92 - 0.96 (m, 6H)

$R_f$ values: 0.31 (Developing Solvent A)
0.24 (Developing Solvent B)

**Example 3-33**

Synthesis of L-N-benzyloxycarbonylleucine-(2S)-[1-formyl-2-(2-chlorobenzyl)oxy]ethylamide (Compound No. 3-33):

The same reaction procedure as used in Example 3-21 was repeated except that the 4 g of Reference Compound No. 3-16 synthesized in Reference Example 3-16 was replaced by the 0.55 g of Reference Compound No. 3-26 synthesized in Reference Example 3-26, whereby 0.50 g of the captioned Compound No. 3-33 was obtained as an oily material.

NMR (δ, $CDCl_3$): 9.61 (s, 1H), 7.23 - 7.36 (m, 9H), 6.80 - 6.93 (m, 1H), 5.09 - 5.17 (m, 3H), 4.55 - 4.63 (m, 3H), 4.25 - 4.35 (m, 1H), 4.08 - 4.16 (m, 1H), 3.75 - 3.79 (m, 1H), 1.49 - 1.71 (m, 3H), 0.92 - 0.96 (m, 6H)

$R_f$ values: 0.31 (Developing Solvent A)
0.24 (Developing Solvent B)

**Example 3-34**

Synthesis of L-N-benzyloxycarbonylleucine-(2S)-[1-formyl-3-(2-fluorophenyl)oxy]propylamide (Compound No. 3-34):

The same reaction procedure as used in Example 3-21 was repeated except that the 4 g of Reference Compound No. 3-16 synthesized in Reference Example 3-16 was replaced by the 0.69 g of Reference Compound No. 3-27 synthesized in Reference Example 3-27, whereby 0.66 g of the captioned Compound No. 3-34 was obtained as an oily material.

NMR (δ, $CDCl_3$): 9.65 (s, 1H), 7.28 - 7.37 (m, 5H), 7.02 - 7.10 (m, 3H), 6.88 - 6.95 (m, 2H), 5.10 - 5.22 (m, 1H), 5.06 (s, 2H), 4.57 - 4.61 (m, 1H), 4.22 - 4.30 (m, 1H), 4.05 - 4.13 (m, 2H), 2.32 - 2.50 (m, 2H), 1.50 - 1.72 (m, 3H), 0.91 - 0.95 (m, 6H)

$R_f$ values: 0.26 (Developing Solvent A)
0.19 (Developing Solvent B)

**Example 3-35**

Synthesis of L-N-benzyloxycarbonylleucine-(2S)-[1-formyl-3-(3-fluorophenyl)oxy]propylamide (Compound No. 3-35):

The same reaction procedure as used in Example 3-21 was repeated except that the 4 g of Reference Compound No. 3-16 synthesized in Reference Example 3-16 was replaced by the 0.27 g of Reference Compound No. 3-28 synthesized in Reference Example 3-28, whereby 0.06 g of the captioned Compound No. 3-35 was obtained as an oily material.

NMR (δ, $CDCl_3$): 9.64 (s, 1H), 7.30 - 7.35 (m, 5H), 7.20 (q, J=8.25Hz, 1H), 6.81 - 6.88 (m, 1H), 6.54 - 6.70 (m, 3H), 5.05 - 5.12 (m, 3H), 4.56 - 4.63 (m, 1H), 4.17 - 4.27 (m, 1H), 3.96 - 4.04 (m, 2H), 2.27 - 2.54 (m, 2H), 1.45 - 1.75 (m, 3H), 0.88 - 0.95 (m, 6H)

$R_f$ values: 0.25 (Developing Solvent A)
0.19 (Developing Solvent B)

**Example 3-36**

Synthesis of L-N-benzyloxycarbonylleucine-(2S)-[1-formyl-3-(2-chlorophenyl)oxy]propylamide (Compound No. 3-36):

The same reaction procedure as used in Example 3-21 was repeated except that the 4 g of Reference Compound No. 3-16 synthesized in Reference Example 3-16 was replaced by the 1.05 g of Reference Compound No. 3-29 synthesized in Reference Example 3-29, whereby 0.41 g of the captioned Compound No. 3-36 was obtained.

Melting point (° C): 107.1 - 111.3

NMR (δ, $CDCl_3$): 9.70 (s, 1H), 7.33 - 7.39 (m, 5H), 7.18 - 7.23 (m, 2H), 7.06 - 7.09 (m, 1H), 6.85 - 6.94 (m, 2H), 5.17 - 5.19 (m, 1H), 5.07 (s, 2H), 4.60 - 4.64 (m, 1H), 4.26 - 4.29 (m, 1H), 4.06 - 4.10 (m, 2H), 2.42 - 2.46 (m, 2H), 1.45 - 1.75 (m, 3H), 0.90 - 0.98 (m, 6H)

$R_f$ values: 0.27 (Developing Solvent A)
0.20 (Developing Solvent B)

**Example 3-37**

Synthesis of L-N-benzyloxycarbonylleucine-(2S)-[1-formyl-3-(3-chlorophenyl)oxy]propylamide (Compound No. 3-37):

The same reaction procedure as used in Example 3-21 was repeated except that the 4 g of Reference Compound No. 3-16 synthesized in Reference Example 3-16 was replaced by the 1.07 g of Reference Compound No. 3-30 synthesized in Reference Example 3-30, whereby 0.42 g of the captioned Compound No. 3-37 was obtained as an oily material.

NMR (δ, $CDCl_3$): 9.63 (s, 1H), 7.28 - 7.35 (m, 6H), 7.17 (t, J = 8.19Hz, 1H), 6.92 - 6.95 (m, 1H), 6.85 (s, 1H), 6.72 (d, J = 8.31Hz, 1H), 5.03 - 5.18 (m, 3H), 4.56 - 4.59 (m, 1H), 4.16 - 4.22 (m, 1H), 3.93 - 4.03 (m, 2H), 2.25 - 2.53 (m, 2H), 1.47 - 1.70 (m, 3H), 0.91 - 0.95 (m, 6H)

$R_f$ values: 0.28 (Developing Solvent A)
0.27 (Developing Solvent B)

**Example 3-38**

Synthesis of L-N-benzyloxycarbonylleucine-(2S)-(1-formyl-3-benzylthio)propylamide (Compound No. 3-38):

The same reaction procedure as used in Example 3-21 was repeated except that the 4 g of Reference Compound No. 3-16 synthesized in Reference Example 3-16 was replaced by the 1.9 g of Reference Compound No. 3-31 synthesized in Reference Example 3-31, whereby 0.8 g of the captioned Compound No. 3-38 was obtained.

Melting point (° C): 72.8 - 81.8

NMR (δ, $CDCl_3$): 9.49 (d, J = 5.76Hz, 1H), 7.24 - 7.33 (m, 10H), 6.67 - 6.69 (m, 1H), 5.14 - 5.18 (m, 1H), 5.10 (s, 2H), 4.55 - 4.64 (m, 1H), 4.15 - 4.21 (m, 1H), 3.66 (s, 2H), 2.39 - 2.43 (m, 2H), 2.11 - 2.20 (m, 1H), 1.82 - 1.90 (m, 1H), 1.47 - 1.69 (m, 3H), 0.92 - 0.95 (m, 6H)

$R_f$ values: 0.52 (Developing Solvent A)
0.25 (Developing Solvent B)

**Example 3-39**

Synthesis of L-N-benzyloxycarbonylleucine-(2S)-[1-formyl-3-(2-fluorobenzyl)thio]propylamide (Compound No. 3-39):

The same reaction procedure as used in Example 3-21 was repeated except that the 4 g of Reference Compound No. 3-16 synthesized in Reference Example 3-16 was replaced by the 1.6 g of Reference Compound No. 3-32 synthesized in Reference Example 3-32, whereby 1.3 g of the captioned Compound No. 3-39 was obtained.

Melting point (° C): 99.6 - 101.0

NMR (δ, $CDCl_3$): 9.54 (s, 1H), 7.19 - 7.37 (m, 7H), 6.67 - 6.69 (m, 1H), 5.11 (s, 2H), 5.10 - 5.15 (m,

1H), 4.50 - 4.54 (m, 1H), 4.18 - 4.23 (m, 1H), 3.70 (s, 2H), 2.45 - 2.48 (m, 2H), 2.18 - 2.30 (m, 2H), 1.86 - 1.97 (m, 2H), 1.48 - 1.72 (m, 3H), 0.95 (d, J=6.24Hz, 6H)

$R_f$ values: 0.51 (Developing Solvent A)
0.24 (Developing Solvent B)

**Example 3-40**

Synthesis of L-N-benzyloxycarbonylleucine-(2S)-[1-formyl-3-(2-chlorobenzyl)thio]propylamide (Compound No. 3-40):

The same reaction procedure as used in Example 3-21 was repeated except that the 4 g of Reference Compound No. 3-16 synthesized in Reference Example 3-16 was replaced by the 1.2 g of Reference Compound No. 3-33 synthesized in Reference Example 3-33, whereby 0.72 g of the captioned Compound No. 3-40 was obtained as an oily material.

NMR (δ, $CDCl_3$): 9.53 (d, J=6.46Hz, 1H), 7.30 - 7.38 (m, 7H), 7.16 - 7.25 (m, 2H), 6.73 - 6.75 (m, 1H), 5.20 (d, J=7.81Hz, 1H), 5.10 (s, 2H), 4.47 - 4.53 (m, 1H), 4.18 - 4.24 (m, 1H), 3.80 (s, 2H), 2.47 - 2.52 (m, 2H), 2.16 - 2.24 (m, 1H), 1.88 - 1.94 (m, 1H), 1.48 - 1.68 (m, 3H), 0.92 - 0.94 (m, 6H)

$R_f$ values: 0.29 (Developing Solvent A)
0.26 (Developing Solvent B)

**Example 3-41**

Synthesis of L-N-benzyloxycarbonylleucine-(2S)-[1-formyl-3-(2-fluorophenyl)thio]propylamide (Compound No. 3-41):

The same reaction procedure as used in Example 3-21 was repeated except that the 4 g of Reference Compound No. 3-16 synthesized in Reference Example 3-16 was replaced by the 1.35 g of Reference Compound No. 3-34 synthesized in Reference Example 3-34, whereby 0.96 g of the captioned Compound No. 3-41 was obtained as an oily material.

NMR (δ, $CDCl_3$): 9.54 (d, J=6.73, 1H), 7.31 - 7.40 (m, 5H), 7.22 - 7.29 (m, 2H), 7.03 - 7.12 (m, 1H), 5.17 (d, J=7.81Hz, 1H), 5.11 (s, 1H), 4.55 - 4.60 (m, 1H), 4.20 - 4.30 (m, 1H), 2.89 - 2.94 (m, 2H), 2.10 - 2.20 (m, 1H), 1.84 - 1.95 (m, 1H), 1.49 - 1.73 (m, 3H), 0.93 - 0.96 (m, 6H)

$R_f$ values: 0.42 (Developing Solvent A)
0.40 (Developing Solvent B)

**Example 3-42**

Synthesis of L-N-benzyloxycarbonylleucine-(2S)-[1-formyl-3-(2-chlorophenyl)thio]propylamide (Compound No. 3-42):

The same reaction procedure as used in Example 3-21 was repeated except that the 4 g of Reference Compound No. 3-16 synthesized in Reference Example 3-16 was replaced by the 1.25 g of Reference Compound No. 3-35 synthesized in Reference Example 3-35, whereby 0.58 g of the captioned Compound No. 3-42 was obtained.

Melting point (°C): 109.5 - 149.0 (dec.)

NMR (δ, $CDCl_3$): 9.54 (s, 1H), 7.12 - 7.40 (m, 9H), 6.62 - 6.85 (m, 1H), 5.09 - 5.15 (m, 3H), 4.54 - 4.60 (m, 1H), 4.21 - 4.25 (m, 1H), 2.94 - 3.00 (m, 2H), 2.27 - 3.40 (m, 1H), 1.90 - 2.02 (m, 1H), 1.49 - 1.71 (m, 3H), 0.93 - 0.95 (m, 6H)

$R_f$ values: 0.27 (Developing Solvent A)
0.27 (Developing Solvent B)

**Example 3-43**

Synthesis of L-N-benzyloxycarbonylleucine-(2S)-[1-formyl-3-(4-chlorophenyl)thio]propylamide (Compound No. 3-43):

The same reaction procedure as used in Example 3-21 was repeated except that the 4 g of Reference Compound No. 3-16 synthesized in Reference Example 3-16 was replaced by the 1.58 g of Reference Compound No. 3-36 synthesized in Reference Example 3-36, whereby 0.71 g of the captioned Compound No. 3-43 was obtained as an oily material.

NMR ($\delta$, $CDCl_3$): 9.52 (d, J=7.21Hz, 1H), 7.32 (s, 5H), 7.25 (s, 4H), 6.72 - 6.90 (m, 1H), 5.14 - 5.17 (m, 1H), 5.10 (d, J=4.88Hz, 2H), 4.53 - 4.60 (m, 1H), 4.15 - 4.25 (m, 1H), 2.88 - 2.93 (m, 2H), 2.20 - 2.35 (m, 1H), 1.82 - 1.98 (m, 1H), 1.49 - 1.68 (m, 3H), 0.93 - 0.98 (m, 6H)

$R_f$ values: 0.30 (Developing Solvent A)
0.30 (Developing Solvent B)

**Test Example 1** Measurement of Calpain Inhibiting Effect

Calpain was separated from a skeletal muscle of a Japanese white rabbit and partially purified according to the method of Tsuji and Imahori as described in J.Biochem. 90, 233-240 (1981), whereby the calpain for use in a test for investigating the calpain inhibiting effect (i.e., the inhibitory effect on calpain activity) of the aldehyde compounds according to the present invention was prepared.

The calpain inhibiting effect was measured in accordance with the method of Yoshimura et al. as described in J. Biol. Chem. 258, 8883-8889 (1983).

Each assay mixture was prepared by mixing 0.05 ml of a 4% casein solution, 0.05 ml of a 50 mM cysteine solution, 0.05 ml of a calpain solution, 0.025 ml of purified water, 0.025 ml of a 10% dimethyl sulfoxide solution containing each compound shown in Table 15 and 0.25 ml of a 200 mM imidazole hydrochloric acid buffer solution (pH=7.5).

The thus prepared assay mixture was preincubated at 30°C for 3 minutes. The reaction was started by the addition of 0.05 ml of a 50 mM calcium chloride solution to the above mixture. After the incubation at 30°C for 30 minutes, the reaction was terminated by the addition of 0.5 ml of 5% trichloroacetic acid.

The amount of a trichloroacetic-acid-soluble protein produced by hydrolysis of casein by calpain was measured by the spectrophotometric analysis in accordance with the method of Ross and Schatz as described in Anal. Biochem. 54, 304-306 (1973), and the absorbance (a) of each sample was measured.

The absorbance (b) of a blank sample which was treated similarly without any of the aldehyde compounds of the present invention, dissolved in a 10% dimethyl sulfoxide solution, was measured.

The inhibition of calpain activity was calculated in accordance with the formula [(b-a)/b] X 100, and the amount of each aldehyde compound necessary to inhibit calpain activity by 50% [$IC_{50}$] was calculated according to the method of Probit. The results are shown in Table 15.

Table 15

| Compound No. in Example | Calpain Inhibiting Effect [$IC_{50}$] (μM) | Compound No. in Example | Calpain Inhibiting Effect [$IC_{50}$] (μM) |
|---|---|---|---|
| 1-1 | 6.6 | 2-3 | 1.60 |
| 1-7 | 3.0 | 2-4 | 2.40 |
| 1-26 | 0.89 | 2-13 | 1.23 |
| 1-27 | 0.75 | 2-14 | 1.90 |
| 1-28 | 0.22 | 2-17 | 3.60 |
| 1-30 | 1.10 | 2-18 | 0.58 |
| 1-32 | 2.00 | 2-19 | 0.59 |
| 1-34 | 0.17 | 2-20 | 4.40 |
| 1-36 | 0.78 | 2-21 | 2.00 |
| 1-38 | 1.90 | 2-22 | 3.30 |
| 1-39 | 2.30 | 3-41 | 0.27 |
| 1-41 | 0.94 | 3-42 | 0.44 |
| 1-42 | 0.35 | 3-43 | 0.52 |
| 1-43 | 0.56 | 3-44 | 0.95 |
| 1-44 | 0.99 | 3-45 | 0.62 |
| 1-51 | 1.40 | 3-46 | 0.61 |
| 1-55 | 0.63 | 3-47 | 2.00 |
| 1-58 | 0.68 | 3-48 | 1.50 |
| 1-61 | 0.88 | 3-49 | 0.85 |
| 1-62 | 1.60 | 3-50 | 0.22 |

Table 15

| Compound No. in Example | Calpain Inhibiting Effect [$IC_{50}$] (μM) | Compound No. in Example | Calpain Inhibiting Effect [$IC_{50}$] (μM) |
|---|---|---|---|
| 3-51 | 0.32 | 3-70 | 0.30 |
| 3-52 | 0.90 | 3-71 | 0.22 |
| 3-53 | 1.30 | 3-72 | 0.20 |
| 3-54 | 1.30 | 3-73 | 0.43 |
| 3-55 | 0.70 | 3-74 | 0.42 |
| 3-61 | 0.41 | 3-75 | 0.17 |
| 3-62 | 0.44 | 3-76 | 0.14 |
| 3-63 | 0.99 | 3-77 | 0.19 |
| 3-64 | 0.29 | 3-78 | 0.14 |
| 3-65 | 1.20 | 3-79 | 0.11 |
| 3-66 | 1.20 | 3-80 | 0.33 |
| 3-67 | 0.61 | 3-81 | 0.58 |
| 3-68 | 1.30 | 3-82 | 1.21 |
| 3-69 | 0.47 | 3-83 | 0.51 |

**Test Example 2** Measurement of Platelet-aggregation Inhibiting Effect

Platelet rich plasma (PRP) was prepared and the platelet aggregation was measured in accordance with the method of Born and Cross as described in J. Phystol. 168, 178-195 (1963).

Blood of a Japanese white rabbit was drawn from a carotid artery of the rabbit. 9 parts by volume of the blood were mixed with 1 part by volume of a 3.8% aqueous solution of sodium citrate. The mixture was immediately centrifuged at 200 x g at 20°C for 15 minutes, and PRP was obtained in the supernate. The number of platelets was calculated by use of an electronic cell counter (MEK-4150 manufactured by Nihon Kohden Kabushiki Kaisha). PRP with 55 X $10^4$ or more platelets per 1 μl was employed in the tests.

10 μl of a test solution containing any of the aldehyde compounds of the present invention was added to 200 μl of PRP. The mixture was preincubated at 37°C for 5 minutes. To this mixture, 10 μl of collagen (22 μg/ml) was added to cause the aggregation. The aggregation was measured by Agricometer (NKK, PAT-4A).

In preparing the above test solution, each compound synthesized in the previously mentioned Examples as shown in Table 16 was dissolved in combined dimethyl sulfoxide and polyoxyethylene 60 hardened castor oil in ethanol. The mixture was diluted with an isotonic saline and the final concentration of the dimethyl sulfoxide and polyoxyethylene 60 hardened castor oil contained in the above mixture was adjusted to less than 1%.

The inhibitory activity of each aldehyde compound on platelet aggregation was expressed by percentages compared with a blank test defined as 100% which was performed without any of the aldehyde compounds.

The amount of each aldehyde compound necessary to inhibit the platelet aggregation by 50% [$IC_{50}$] was calculated according to the method of Probit. The results are shown in Table 16.

Table 16

| Compound No. in Example | Platelet Aggregation Inhibiting Activity [$IC_{50}$] ($\mu$M) | Compound No. in Example | Platelet Aggregation Inhibiting Activity [$IC_{50}$] ($\mu$M) |
|---|---|---|---|
| 1-27 | 2.90 | 3-50 | 25.0 |
| 1-42 | 23.0 | 3-51 | 88.0 |
| 1-44 | 47.0 | 3-52 | 61.0 |
| 1-51 | 10.0 | 3-55 | 44.0 |
| 1-58 | 22.0 | 3-61 | 9.40 |
| 1-61 | 9.60 | 3-62 | 21.0 |
| 1-62 | 17.0 | 3-63 | 23.0 |
| 2-19 | 4.20 | 3-64 | 28.0 |
| 2-27 | 65.0 | 3-67 | 18.0 |
| 3-41 | 56.0 | 3-69 | 13.0 |
| 3-42 | 94.0 | 3-70 | 9.60 |
| 3-43 | 52.0 | 3-72 | 54.0 |
| 3-44 | 60.0 | 3-73 | 9.20 |
| 3-45 | 56.0 | 3-76 | 11.0 |
| 3-46 | 83.0 | 3-83 | 28.0 |

**Test Example 3** Measurement of Inhibitory Effect on Protease other than Calpain

(1) Measurement of Inhibitory Effect on Trypsin Activity

The inhibitory effect on trypsin activity was measured by a partially modified method of Aoyagi as described in J. Antibiotics, 22, 558-568 (1969).

An assay mixture was prepared by mixing 0.5 ml of a 2% casein solution, 0.05 ml of a 50 mM calcium chloride solution, 0.05 ml of a 10% dimethyl sulfoxide solution containing each of Compounds No. 3-61, 3-63, 3-76 and 3-83, 0.1 ml of purified water and 0.25 ml of a 70 mM boric acid buffer solution (pH = 7.4).

The assay mixture was preincubated at 37°C for 3 minutes, and the reaction was started by the addition of 0.05 ml of 0.1 mg/ml trypsin to the above mixture. After incubation at 37°C for 30 minutes, the reaction was terminated by the addition of 1 ml of 1.7 N perchloric acid. After allowing the reaction mixture to stand for 60 minutes, the mixture was centrifuged at 3000 rpm for 10 minutes. The absorbance (a) of the supernate was measured at 280 nm.

The absorbance (b) of a blank sample which was treated similarly, without any aldehyde compound, dissolved in 10% dimethyl sulfoxide, was measured. The inhibition of trypsin activity was calculated in accordance with the formula [(b-a)] X 100.

None of the above compounds showed inhibitory effect at a concentration of $10^{-5}$ M.

(2) Measurement of Inhibitory Effect on $\alpha$-Chymotrypsin

The inhibitory effect on $\alpha$-chymotrypsin was measured by a partially modified method of Erlanger, B. F. as described in Ach. Biochem. Biophys. 115, 206-210 (1966).

An assay mixture was prepared by mixing 0.05 ml of 10 mM glutaryl-L-phenylalanine-p-nitroanilide dissolved in dimethyl sulfoxide and polyoxyethylene 60 hardened castor oil, 0.1 ml of a 50 mM calcium chloride solution, 0.25 ml of purified water, 0.05 ml of a compound to be tested dissolved in combined dimethyl sulfoxide and polyoxyethylene 60 hardened castor oil, and 0.5 ml of 100 mM trishydrochloric acid buffer solution (pH = 7.8).

The mixture was preincubated at 25°C for 3 minutes. To the above mixture, and the reaction was started by the addition of 0.05 ml of 2 mg/ml α-chymotrypsin. The increase of 405 nm absorbance was measured at 25°C with time, and the transition rate (a) of the absorbance per min was calculated.

The rate (b) of the absorbance of a blank sample treated similarly without any compound dissolved in combined dimethyl sulfoxide and 10% polyoxyethylene 60 hardened castor oil was measured. The inhibition of α-chymotrypsin activity was calculated in accordance with the formula [(b-a)/b] X 100, and the amount of each compound necessary to inhibit α-chymotrypsin activity by 50% [$IC_{50}$] was calculated according to the method of Probit.

The inhibitory effect of SUAM-14541, a compound disclosed in Japanese Laid-Open Patent Application 1-121257, on α-chymotrypsin activity was measured for comparison.

The results are shown in Table 17.

Table 17

| Compound tested | Inhibitory Effect [$IC_{50}$] |
|---|---|
| Compound No. 3-6 | $>10^{-4}$ M |
| SUAM-14541 | 2.1 X $10^{-5}$ M |

The aldehyde derivatives according to the present invention are useful for treatment of inveterate diseases such as ischemic diseases, inflammation, progressive muscular dystrophy, cataracts, and hypoimmunity as shown in the above Test Examples since the derivatives have excellent capabilities for inhibiting calpain activity.

The derivatives can be administered perorally, intravenously, hypodermically or intramuscularly. Therefore, the derivatives can be used in various administration forms including pellets, capsules, liquids, and suppositories.

**Claims**

**1.** Aldehyde derivatives represented by formula (I):

$$\begin{array}{l} \qquad\qquad\quad CHO \\ \qquad\qquad\quad | \\ Z\text{-}CONH\text{-}CH\text{-}(CH_2)_n\text{-}R^1 \end{array} \qquad (I)$$

wherein $R^1$ represents an aromatic hydrocarbon group selected from the group consisting of phenyl group, naphthyl group and anthranyl group, a heterocyclic group selected from the group consisting of furyl group, thienyl group, pyrrolyl group, pyridyl group, quinolyl group, isoquinolyl group, and indolyl group, an alkyl group having 1 to 10 carbon atoms, an alkenyl group having 2 to 10 carbon atoms, or a group of $-X-R^3$ in which X represents O, $-S(O)_m-$ (m = 0, 1, or 2), and $R^3$ represents an aromatic hydrocarbon group selected from the group consisting of phenyl group, naphthyl group and anthranyl group, a heterocyclic group selected from the group consisting of furyl group, thienyl group, pyrrolyl group, pyridyl group, quinolyl group, isoquinolyl group, and indolyl group, or an alkyl group having 1 to 10 carbon atoms; Z represents $R^4$-Y- or $R^6$O-CH($R^5$)- in which Y represents a 3- to 7-membered nitrogen-containing saturated heterocyclic group selected from the group consisting of aziridine, azetidine, pyrrolidine, piperidine and perhydroazepine, or a monocyclic saturated hydrocarbon group having 3 to 7 carbon atoms selected from the group consisting of cyclopropane, cyclobutane, cyclopentane and cycloheptane, $R^4$ represents an alkyl group having 1 to 10 carbon atoms, an alkenyl group having 2 to 10 carbon atoms, an alkynyl group having 2 to 10 carbon atoms, an acyl group selected from the group consisting of acetyl group, propionyl group, butyryl group, valeryl group, hexanoyl group, heptanoyl group, iso-valeryl group, cyclohexane carbonyl group, benzoyl group, 1-naphthoyl group, 2-naphthoyl group, toluoyl group, 1-(benzyloxycarbonyl)piperidine-4-carbonyl group, cinnamoyl group, phenylacetyl group, 2-thienylcarbonyl group, trimethyl acetyl group, cyclopentane carbonyl group, 2,6-dichloro benzoyl group, 3,4-dichlorobenzoyl group, 4-phenyl benzoyl group, 2-chlorocinnamoyl group, 3-chlorocinnamoyl group, 4-chlorocinnamoyl group, 2-nitrocinnamoyl group,

indolyl-2-carbonyl group, indolyl-3-carbonyl group, quinolyl-2-carbonyl group, quinolyl-3-carbonyl group, isoquinolyl-3-carbonyl group, diphenylacetyl group, fluorenyl-9-carbonyl group, 3-phenylpropionyl group, 4-phenyl-butyryl group, 3-(3-pyridyl)acryloyl group, 3-(3-thienyl)acryloyl group, 3-phenyl-2-methylacryloyl group, 3-(2-naphthyl)acryloyl group, (2S)-3-phenyl-2-(benzyloxycarbonylamino)propionyl group, and (2R)-3-phenyl-2-(benzyloxycarbonylamino)propionyl group, a sulfonyl group selected from the group consisting of methane sulfonyl group, ethane sulfonyl group, propane sulfonyl group, butane sulfonyl group, pentane sulfonyl group, hexane sulfonyl group, trifluoromethane sulfonyl group, benzene sulfonyl group, naphthalene-2-sulfonyl group, 4-methyl benzene sulfonyl group, iso-quinoline-5-sulfonyl group, and quinoline-8-sulfonyl group, an alkoxycarbonyl group selected from the group consisting of methoxy carbonyl group, ethoxy carbonyl group, propoxy carbonyl group, butoxy carbonyl group, pentyloxy carbonyl group, hexyloxy carbonyl group, heptyloxy carbonyl group, octyloxy carbonyl group, nonyloxy carbonyl group, decyloxy carbonyl group, iso-propoxy carbonyl group, iso-butoxy carbonyl group, s-butoxy carbonyl group, t-butoxy carbonyl group, iso-pentyloxy carbonyl group, neopentyloxy carbonyl group, t-pentyloxy carbonyl group, iso-hexyloxy carbonyl group, cinnamyloxy carbonyl group, and benzyloxy carbonyl group, a carbamoyl group selected from the group consisting of N-methylcarbamoyl group, N-ethylcarbamoyl group, N-phenylcarbamoyl group, N-(2-chlorophenyl)-carbamoyl group, N-(3-chlorophenyl)carbamoyl group, N-(4-chlorophenyl)carbamoyl group, N-(1-naphthyl)carbamoyl group, N-(2-naphthyl)carbamoyl group,and N-benzylcarbamoyl group, or a thiocarbamoyl group selected from the group consisting of N-methylthiocarbamoyl group, N-ethylthiocarbamoyl group, N-phenylthiocarbamoyl group, N-(2-chlorophenyl)thiocarbamoyl group, N-(1-naphthyl)-thiocarbamoyl group, N-(2-naphthyl)thiocarbamoyl group, and N-benzylthiocarbamoyl group, $R^5$ represents hydrogen, an alkyl group having 1 to 10 carbon atoms, or an aromatic hydrocarbon group selected from the group consisting of phenyl group, naphthyl group and anthranyl group, and $R^6$ represents an acyl group selected from the group consisting of acetyl group, propionyl group, butyryl group, valeryl group, hexanoyl group, heptanoyl group, iso-valeryl group, cylcohexane carbonyl group, benzoyl group, 1-naphthoyl group, 2-naphthoyl group, toluoyl group, and 1-(benzyloxycarbonyl)-piperidine-4-carbonyl group, a carbamoyl group selected from the group consisting of N-methylcarbamoyl group, N-ethylcarbamoyl group, N-phenylcarbamoyl group, N-(2-chlorophenyl)carbamoyl group, N-(2-naphthyl)carbamoyl group and N-benzylcarbamoyl group, a thiocarbamoyl group selected from the group consisting of N-methylthiocarbamoyl group, N-ethylthiocarbamoyl group, N-phenylthiocarbamoyl group, N-(2-chlorophenyl)thiocarbamoyl group, N-(1-naphthyl)thiocarbamoyl group and N-benzylthiocarbamoyl group, or an alkyl group having 1 to 10 carbon atoms; and n is an integer of 1 to 5.

**2.** The aldehyde derivatives as claimed in Claim 1, wherein said alkyl group having 1 to 10 carbon atoms represented by $R^1$ has as a substituent an aromatic hydrocarbon group selected from the group consisting of phenyl group, naphthyl group and anthranyl group.

**3.** The aldehyde derivatives as claimed in Claim 1, wherein said alkyl group having 1 to 10 carbon atoms represented by $R^1$ has as a substituent a heterocyclic group selected from the group consisting of furyl group, thienyl group, pyrrolyl group, pyridyl group, quinolyl group, isoquinolyl group, and indolyl group.

**4.** The aldehyde derivatives as claimed in Claim 1, wherein said alkenyl group having 1 to 10 carbon atoms represented by $R^1$ has as a substituent an aromatic hydrocarbon group selected from the group consisting of phenyl group, naphthyl group and anthranyl group.

**5.** The aldehyde derivatives as claimed in Claim 1, wherein said alkenyl group having 1 to 10 carbon atoms represented by $R^1$ has as a substituent a heterocyclic group selected from the group consisting of furyl group, thienyl group, pyrrolyl group, pyridyl group, quinolyl group, isoquinolyl group, and indolyl group.

**6.** The aldehyde derivatives as claimed in Claim 1, wherein said alkyl group having 1 to 10 carbon atoms represented by $R^3$ has as a substituent an aromatic hydrocarbon group selected from the group consisting of phenyl group, naphthyl group and anthranyl group.

**7.** The aldehyde derivatives as claimed in Claim 1, wherein said alkyl group having 1 to 10 carbon atoms represented by $R^3$ has as a substituent a heterocyclic group selected from the group consisting of furyl group, thienyl group, pyrrolyl group, pyridyl group, quinolyl group, isoquinolyl group, and indolyl group.

**8.** The aldehyde derivatives as claimed in Claim 1, wherein said alkyl group having 1 to 10 carbon atoms represented by $R^1$ is selected from the group consisting of methyl group, ethyl group, propyl group, butyl group, pentyl group, hexyl group, heptyl group, octyl group, nonyl group, decyl group, iso-propyl group, s-butyl group, t-butyl group, iso-pentyl group, neopentyl group, t-pentyl group, iso-hexyl group, cyclopropyl group, cyclobutyl group, cyclopentyl group, and cyclohexyl group.

**9.** The aldehyde derivatives as claimed in Claim 8, wherein said alkyl group having 1 to 10 carbon atoms represented by $R^1$ has as a substituent an aromatic hydrocarbon group selected from the group consisting of phenyl group, naphthyl group and anthranyl group.

**10.** The aldehyde derivatives as claimed in Claim 8, wherein said alkyl group having 1 to 10 carbon atoms represented by $R^1$ has as a substituent a heterocyclic group selected from the group consisting of furyl group, thienyl group, pyrrolyl group, pyridyl group, quinolyl group, isoquinolyl group, and indolyl group.

**11.** The aldehyde derivative as claimed in Claim 1, wherein said alkenyl group having 2 to 10 carbon atoms represented by $R^1$ is selected from the group consisting of ethenyl group, 1-propenyl group, 2-propenyl group, iso-propenyl group, 1-butenyl group, 2-butenyl group, 3-butenyl group, 2-methyl-2-propenyl group, 1-pentenyl group, 1-hexenyl group, 1-heptenyl group, 1-cyclohexenyl group, 2-cylcohexenyl group, aromatic hydrocarbon, and heterocyclic group.

**12.** The aldehyde derivatives as claimed in Claim 11, wherein said alkenyl group having 1 to 10 carbon atoms represented by $R^1$ has as a substituent an aromatic hydrocarbon group selected from the group consisting of phenyl group, naphthyl group and anthranyl group.

**13.** The aldehyde derivatives as claimed in Claim 11, wherein said alkenyl group having 1 to 10 carbon atoms represented by $R^1$ has as a substituent a heterocyclic group selected from the group consisting of furyl group, thienyl group, pyrrolyl group, pyridyl group, quinolyl group, isoquinolyl group, and indolyl group.

**14.** The aldehyde derivatives as claimed in Claim 1, wherein said alkyl group having 1 to 10 carbon atoms represented by $R^3$ is selected from the group consisting of methyl group, ethyl group, propyl group, butyl group, pentyl group, hexyl group, heptyl group, octyl group, nonyl group, decyl group, iso-propyl group, s-butyl group, t-butyl group, iso-pentyl group, neopentyl group, t-pentyl group, iso-hexyl group, cyclopropyl group, cyclobutyl group, cyclopentyl group, and cyclohexyl group.

**15.** The aldehyde derivatives as claimed in Claim 14, wherein said alkyl group having 1 to 10 carbon atoms represented by $R^3$ has as a substituent an aromatic hydrocarbon group selected from the group consisting of phenyl group, naphthyl group and anthranyl group.

**16.** The aldehyde derivatives as claimed in Claim 14, wherein said alkyl group having 1 to 10 carbon atoms represented by $R^3$ has as a substituent a heterocyclic group selected from the group consisting of furyl group, thienyl group, pyrrolyl group, pyridyl group, quinolyl group, isoquinolyl group, and indolyl group.

**17.** The aldehyde derivagive as claimed in Claim 1, wherein said alkyl group having 1 to 10 carbon atoms represented by $R^4$ has as a substituent an aromatic hydrocarbon group selected from the group consisting of phenyl group, naphthyl group and anthranyl group.

**18.** The aldehyde derivatives as claimed in Claim 1, wherein said alkyl group having 1 to 10 carbon atoms represented by $R^4$ has as a substituent a heterocyclic group selected from the group consisting of furyl group, thienyl group, pyrrolyl group, pyridyl group, quinolyl group, isoquinolyl group, and indolyl group.

**19.** The aldehyde derivagive as claimed in Claim 1, wherein said alkenyl group having 2 to 10 carbon atoms represented by $R^4$ has as a substituent an aromatic hydrocarbon group selected from the group consisting of phenyl group, naphthyl group and anthranyl group.

**20.** The aldehyde derivatives as claimed in Claim 1, wherein said alkenyl group having 2 to 10 carbon atoms represented by $R^4$ has as a substituent a heterocyclic group selected from the group consisting of furyl group, thienyl group, pyrrolyl group, pyridyl group, quinolyl group, isoquinolyl group, and indolyl

group.

**21.** The aldehyde derivagive as claimed in Claim 1, wherein said alkynyl group having 2 to 10 carbon atoms represented by $R^4$ has as a substituent an aromatic hydrocarbon group selected from the group consisting of phenyl group, naphthyl group and anthranyl group.

**22.** The aldehyde derivatives as claimed in Claim 1, wherein said alkynyl group having 2 to 10 carbon atoms represented by $R^4$ has as a substituent a heterocyclic group selected from the group consisting of furyl group, thienyl group, pyrrolyl group, pyridyl group, quinolyl group, isoquinolyl group, and indolyl group.

**23.** The aldehyde derivatives as claimed in Claim 1, wherein said alkyl group having 1 to 10 carbon atoms represented by $R^4$ is selected from the group consisting of methyl group, ethyl group, propyl group, butyl group, pentyl group, hexyl group, heptyl group, octyl group, nonyl group, decyl group, iso-propyl group, s-butyl group, t-butyl group, iso-pentyl group, neopentyl group, t-pentyl group, iso-hexyl group, cyclopropyl group, cyclobutyl group, cyclopentyl group, and cyclohexyl group.

**24.** The aldehyde derivagive as claimed in Claim 23, wherein said alkyl group having 1 to 10 carbon atoms represented by $R^4$ has as a substituent an aromatic hydrocarbon group selected from the group consisting of phenyl group, naphthyl group and anthranyl group.

**25.** The aldehyde derivatives as claimed in Claim 23, wherein said alkyl group having 1 to 10 carbon atoms represented by $R^4$ has as a substituent a heterocyclic group selected from the group consisting of furyl group, thienyl group, pyrrolyl group, pyridyl group, quinolyl group, isoquinolyl group, and indolyl group.

**26.** The aldehyde derivatives as claimed in Claim 1, wherein said alkenyl group having 2 to 10 carbon atoms represented by $R^4$ is selected from the group consisting of vinyl group, 1-propenyl group, 2-propenyl group, iso-propenyl group, 1-butenyl group, 2-butenyl group, 3-butenyl group, 2-methyl-2-propenyl group, 1-pentenyl group, 1-hexenyl group, 1-heptenyl group, 1-cyclohexenyl group and 2-cyclohexenyl group.

**27.** The aldehyde derivagive as claimed in Claim 26, wherein said alkenyl group having 2 to 10 carbon atoms represented by $R^4$ has as a substituent an aromatic hydrocarbon group selected from the group consisting of phenyl group, naphthyl group and anthranyl group.

**28.** The aldehyde derivatives as claimed in Claim 26, wherein said alkenyl group having 2 to 10 carbon atoms represented by $R^4$ has as a substituent a heterocyclic group selected from the group consisting of furyl group, thienyl group, pyrrolyl group, pyridyl group, quinolyl group, isoquinolyl group, and indolyl group.

**29.** The aldehyde derivatives as claimed in Claim 1, wherein said alkynyl group having 2 to 10 carbon atoms represented by $R^4$ is selected from the group consisting of 1-propynyl group, 2-propynyl group, 1-butynyl group, 2-butynyl group, 1-pentynyl group, 1-hexynyl group, 1-heptynyl group, 1-optynyl group, 1-nonyl group, 1-denyl group and 1-methyl-2-propynyl group.

**30.** The aldehyde derivagive as claimed in Claim 29, wherein said alkynyl group having 2 to 10 carbon atoms represented by $R^4$ has as a substituent an aromatic hydrocarbon group selected from the group consisting of phenyl group, naphthyl group and anthranyl group.

**31.** The aldehyde derivatives as claimed in Claim 29, wherein said alkynyl group having 2 to 10 carbon atoms represented by $R^4$ has as a substituent a heterocyclic group selected from the group consisting of furyl group, thienyl group, pyrrolyl group, pyridyl group, quinolyl group, isoquinolyl group, and indolyl group.

**32.** The aldehyde derivatives as claimed in Claim 1, wherein said aromatic hydrocarbon group represented by $R^1$ has a substituent selected from the group consisting of an alkyl group having 1 to 10 carbon atoms, an alkoxyl group having 1 to 10 carbon atoms, a halogen selected from the group consisting of fluorine, chlorine, bromine and iodine, an amino group, a dimethyl amino group, a diethyl amino group,

a hydroxyl group and a nitro group.

**33.** The aldehyde derivatives as claimed in Claim 1, wherein said heterocyclic group represented by $R^1$ has a substituent selected from the group consisting of an alkyl group having 1 to 10 carbon atoms, an alkoxyl group having 1 to 10 carbon atoms, a halogen selected from the group consisting of fluorine, chlorine, bromine and iodine, an amino group, a dimethyl amino group, a diethyl amino group, a hydroxyl group and a nitro group.

**34.** The aldehyde derivatives as claimed in Claim 2, wherein said aromatic hydrocarbon group which is a substituent of said alkyl group represented by $R^1$ has a substituent selected from the group consisting of an alkyl group having 1 to 10 carbon atoms, an alkoxyl group having 1 to 10 carbon atoms, a halogen selected from the group consisting of fluorine, chlorine, bromine and iodine, an amino group, a dimethyl amino group, a diethyl amino group, a hydroxyl group and a nitro group.

**35.** The aldehyde derivatives as claimed in Claim 3, wherein said heterocyclic group which is a substituent of said alkyl group represented by $R^1$ has a substituent selected from the group consisting of an alkyl group having 1 to 10 carbon atoms, an alkoxyl group having 1 to 10 carbon atoms, a halogen selected from the group consisting of fluorine, chlorine, bromine and iodine, an amino group, a dimethyl amino group, a diethyl amino group, a hydroxyl group and a nitro group.

**36.** The aldehyde derivatives as claimed in Claim 4, wherein said aromatic hydrocarbon group which is a substituent of said alkenyl group represented by $R^1$ has a substituent selected from the group consisting of an alkyl group having 1 to 10 carbon atoms, an alkoxyl group having 1 to 10 carbon atoms, a halogen selected from the group consisting of fluorine, chlorine, bromine and iodine, an amino group, a dimethyl amino group, a diethyl amino group, a hydroxyl group and a nitro group.

**37.** The aldehyde derivatives as claimed in Claim 5, wherein said heterocyclic group which is a substituent of said alkenyl group represented by $R^1$ has a substituent selected from the group consisting of an alkyl group having 1 to 10 carbon atoms, an alkoxyl group having 1 to 10 carbon atoms, a halogen selected from the group consisting of fluorine, chlorine, bromine and iodine, an amino group, a dimethyl amino group, a diethyl amino group, a hydroxyl group and a nitro group.

**38.** Aldehyde derivatives represented by formula (II):

$$R^{10}\text{-}\overset{\displaystyle R^9}{\overset{|}{N}}\text{-}\overset{\displaystyle R^8}{\overset{|}{CH}}\text{-CONH-}\overset{\displaystyle CHO}{\overset{|}{CH}}\text{-}(CH_2)_n\text{-X-}R^7$$

wherein $R^7$ represents an aromatic hydrocarbon group selected from the group consisting of phenyl group, naphthyl group and anthranyl group, a heterocyclic group selected from the group consisting of furyl group, thienyl group, pyrrolyl group, pyridyl group, quinolyl group, isoquinolyl group, and indolyl group, an alkyl group having 1 to 10 carbon atoms with a substituent selected from phenyl group, naphthyl group, anthranyl group, furyl group, thienyl group, pyrrolyl group, pyridyl group, quinolyl group, isoquinolyl group, and indolyl group, or a cyclic alkyl group having 3 to 6 carbon atoms; $R^8$ represents hydrogen, an alkyl group having 1 to 10 carbon atoms, or an aromatic hydrocarbon group selected from the group consisting of phenyl group, naphthyl group and anthranyl group; $R^9$ represents hydrogen, or an alkyl group having 1 to 10 carbon atoms; $R^{10}$ represents an alkoxycarbonyl group selected from the group consisting of methoxy carbonyl group, ethoxy carbonyl group, propoxy carbonyl group, butoxy carbonyl group, pentyloxy carbonyl group, hexyloxy carbonyl group, heptyloxy carbonyl group, octyloxy carbonyl group, nonyloxy carbonyl group, decyloxy carbonyl group, iso-propoxy carbonyl group, iso-butoxy carbonyl group, s-butoxy carbonyl group, t-butoxy carbonyl group, iso-pentyloxy carbonyl group, neopentyloxy carbonyl group, t-pentyloxy carbonyl group, iso-hexyloxy carbonyl group, cinnamyloxy carbonyl group, and benzyloxy carbonyl group, an acyl group selected from the group consisting of acetyl group, propionyl group, butyryl group, valeryl group, hexanoyl group, heptanoyl group, iso-valeryl group, cylcohexane carbonyl group, benzoyl group, 1-naphthoyl group, 2-naphthoyl group, toluyl group, and 1-(benzyloxycarbonyl)piperidine-4-carbonyl group, a carbamoyl group selected from the group consisting of N-methylcarbamoyl group, N-ethylcarbamoyl group, N-phenylcarbamoyl group, N-(2-chlorophenyl)carbamoyl group, N-(2-naphthyl)carbamoyl group and N-benzylcarbamoyl group, or a sulfonyl group selected from the group consisting of methane sulfonyl group, ethane sulfonyl group, propane sulfonyl group, butane sulfonyl group, pentane sulfonyl group, hexane sulfonyl group, trifluoromethane sulfonyl group, benzene sulfonyl group, naphthalene sulfonyl group, 4-methyl benzene sulfonyl group, iso-quinoline-5-sulfonyl group, and quinoline-8-sulfonyl group; X represents oxygen, or a group represented by $-S(O)_m-$ in which m is 0, 1 or 2; and n is an integer of 1 to 5.

**39.** The aldehyde derivatives as claimed in Claim 38, wherein said alkyl group having 1 to 10 carbon atoms represented by $R^7$ is selected from the group consisting of methyl group, ethyl group, propyl group, butyl group, pentyl group, hexyl group, heptyl group, octyl group, nonyl group, decyl group, iso-propyl group, s-butyl group, t-butyl group, iso-pentyl group, neopentyl group, t-pentyl group, iso-hexyl group, cyclopropyl group, cyclobutyl group, cyclopentyl group, and cyclohexyl group.

**40.** The aldehyde derivatives as claimed in Claim 38, wherein said alkyl group having 1 to 10 carbon atoms represented by $R^8$ is selected from the group consisting of methyl group, ethyl group, propyl group, butyl group, pentyl group, hexyl group, heptyl group, octyl group, nonyl group, decyl group, iso-propyl group, s-butyl group, t-butyl group, iso-pentyl group, neopentyl group, t-pentyl group, iso-hexyl group, cyclopropyl group, cyclobutyl group, cyclopentyl group, and cyclohexyl group.

**41.** The aldehyde derivatives as claimed in Claim 38, wherein said alkyl group having 1 to 10 carbon atoms represented by $R^9$ is selected from the group consisting of methyl group, ethyl group, propyl group, butyl group, pentyl group, hexyl group, heptyl group, octyl group, nonyl group, decyl group, iso-propyl group, s-butyl group, t-butyl group, iso-pentyl group, neopentyl group, t-pentyl group, iso-hexyl group, cyclopropyl group, cyclobutyl group, cyclopentyl group, and cyclohexyl group.

**42.** The aldehyde derivatives as claimed in Claim 38, wherein said aromatic hydrocarbon group represented by $R^7$ has a substituent selected from the group consisting of an alkyl group having 1 to 10 carbon atoms, an alkoxyl group having 1 to 10 carbon atoms, a halogen selected from the group consisting of fluorine, chlorine, bromine and iodine, an amino group, a dimethyl amino group, a diethyl amino group, a hydroxyl group and a nitro group.

**43.** The aldehyde derivatives as claimed in Claim 38, wherein said heterocyclic group represented by $R^7$ has a substituent selected from the group consisting of an alkyl group having 1 to 10 carbon atoms, an alkoxyl group having 1 to 10 carbon atoms, a halogen selected from the group consisting of fluorine, chlorine, bromine and iodine, an amino group, a dimethyl amino group, a diethyl amino group, a hydroxyl group and a nitro group.

**44.** The aldehyde derivatives as claimed in Claim 38, wherein said alkyl group represented by $R^8$ has as a substituent an aromatic hydrocarbon group selected from the group consisting of phenyl group, naphthyl group and anthranyl group.

**45.** The aldehyde derivatives as claimed in Claim 38, wherein said alkyl group represented by $R^8$ has as a substituent a heterocyclic group selected from the group consisting of furyl group, thienyl group, pyrrolyl group, pyridyl group, quinolyl group, isoquinolyl group, and indolyl group.

**46.** The aldehyde derivatives as claimed in Claim 38, wherein said alkyl group represented by $R^9$ has as a substituent an aromatic hydrocarbon group selected from the group consisting of phenyl group, naphthyl group and anthranyl group.

**47.** The aldehyde derivatives as claimed in Claim 38, wherein said alkyl group represented by $R^9$ has as a substituent a heterocyclic group selected from the group consisting of furyl group, thienyl group, pyrrolyl group, pyridyl group, quinolyl group, isoquinolyl group, and indolyl group.

**48.** The aldehyde derivatives as claimed in Claim 42, wherein said alkyl group having 1 to 10 carbon atoms is selected from the group consisting of methyl group, ethyl group, propyl group, butyl group, pentyl group, hexyl group, heptyl group, octyl group, nonyl group, decyl group, iso-propyl group, s-butyl

group, t-butyl group, iso-pentyl group, neopentyl group, t-pentyl group, iso-hexyl group, cyclopropyl group, cyclobutyl group, cyclopentyl group, and cyclohexyl group.

**49.** The aldehyde derivatives as claimed in Claim 43, wherein said alkyl group having 1 to 10 carbon atoms is selected from the group consisting of methyl group, ethyl group, propyl group, butyl group, pentyl group, hexyl group, heptyl group, octyl group, nonyl group, decyl group, iso-propyl group, s-butyl group, t-butyl group, iso-pentyl group, neopentyl group, t-pentyl group, iso-hexyl group, cyclopropyl group, cyclobutyl group, cyclopentyl group, and cyclohexyl group.

**50.** The aldehyde derivatives as claimed in Claim 42, wherein said alkoxyl group having 1 to 10 carbon atoms is selected from the group consisting of methoxy group, ethoxy group, propoxy group, butoxy group, pentyloxy group, hexyloxy group, butyloxy group, octyloxy group, nonyloxy group, decyloxy group, iso-propoxy group, iso-butoxy group, s-butoxy group, t-butoxy group, iso-pentyloxy group, neopentyloxy group, t-pentyloxy group and iso-hexyloxy group, and benzyloxy group.

**51.** The aldehyde derivatives as claimed in Claim 43, wherein said alkoxyl group having 1 to 10 carbon atoms is selected from the group consisting of methoxy group, ethoxy group, propoxy group, butoxy group, pentyloxy group, hexyloxy group, butyloxy group, octyloxy group, nonyloxy group, decyloxy group, iso-propoxy group, iso-butoxy group, s-butoxy group, t-butoxy group, iso-pentyloxy group, neopentyloxy group, t-pentyloxy group and iso-hexyloxy group, and benzyloxy group.